(19) Europäisches Patentamt
European Patent Office
Office européen des brevets

(11) **EP 1 997 804 A1**

(12) **EUROPEAN PATENT APPLICATION**
published in accordance with Art. 158(3) EPC

(43) Date of publication:
**03.12.2008 Bulletin 2008/49**

(21) Application number: **07715112.4**

(22) Date of filing: **01.03.2007**

(51) Int Cl.:
***C07D 209/86*** (2006.01) ***A61K 31/451*** (2006.01)
***A61K 31/495*** (2006.01) ***A61K 31/496*** (2006.01)
***A61P 1/02*** (2006.01) ***A61P 19/02*** (2006.01)
***A61P 19/10*** (2006.01) ***A61P 27/02*** (2006.01)
***A61P 29/00*** (2006.01) ***A61P 31/12*** (2006.01)
***A61P 31/18*** (2006.01) ***A61P 35/00*** (2006.01)
***A61P 43/00*** (2006.01) ***C07D 211/96*** (2006.01)
***C07D 243/08*** (2006.01) ***C07D 295/22*** (2006.01)

(86) International application number:
**PCT/JP2007/053899**

(87) International publication number:
**WO 2007/102392 (13.09.2007 Gazette 2007/37)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.03.2006 JP 2006057486**
**02.11.2006 JP 2006298795**

(71) Applicant: **SHIONOGI & CO., LTD.**
**Osaka-shi,**
**Osaka 541-0045 (JP)**

(72) Inventors:
- **HAYASHI, Mikayo**
  **Naruto-shi, Tokushima 772-0003 (JP)**
- **WATANABE, Fumihiko**
  **Kitakatsuragi-gun, Nara 639-0203 (JP)**

(74) Representative: **HOFFMANN EITLE**
**Patent- und Rechtsanwälte**
**Arabellastrasse 4**
**81925 München (DE)**

(54) **MMP-13-SELECTIVE INHIBITOR**

(57) Since it is thought that if the activity of MMP-13 can be inhibited, this will largely contribute to improvement or prevention of progession of pathological states, particularly, osteoarthritis (OA), resulting from or associated with the MMP-13 activity, the development of MMP-13 inhibitors is anticipated.

There are provided a compound represented by the general formula (I):

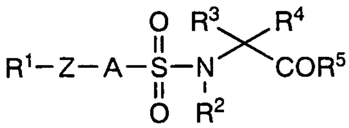

(I)

wherein $R^1$ is optionally substituted aryl etc. Z is C1-C5 alkylene which may be substituted and may be interrupted with a substituent selected from Substituent group a etc.; A is the formula:

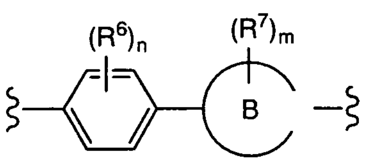

or

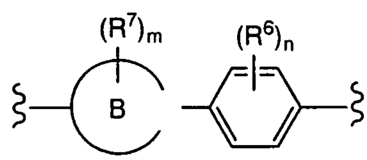

($R^6$ and $R^7$ are each independently halogen, lower alkyl etc.; m and n are each independently 0, 1, or 2); $R^2$ is a hydrogen

EP 1 997 804 A1

atom, optionally substituted lower alkyl etc.; $R^3$ is a hydrogen atom, optionally substituted lower alkyl etc.; $R^4$ is a hydrogen atom; or $R^3$ and $R^4$ may be taken together with an adjacent carbon atom to from a ring; $R^5$ is hydroxy, lower alkyloxy etc.), or an optically active isomer, a pharmaceutically acceptable salt or a solvate thereof, and a pharmaceutical composition containing it as an active ingredient, which has the MMP-13 inhibiting activity.

## Description

[Technical field]

The present invention relates to sulfonamide derivatives which selectively inhibit MMP-13.

[Background technique]

An extracellular matrix is composed of collagen, fibronectin, laminine, proteoglycan and the like, and has a role of tissue supporting function, proliferation, differentiation and adhesion of cells, and the like. Degradation of an extracellular matrix is associated with metalloprotease which is a protease containing a metal ion in an active center, particularly, matrix metalloprotease (MMP). As for the MMP family, many members were reported from MMP-1 (I type collagenase) to MMP-28, and they act on growth, tissue metabolism and the like under the original physiological conditions. However, it was reported that the progress were correlated with the increase in expression (activity) of the above enzymes in various pathological states which were accompanied by the destruction of tissues and fibrosis (osteoarthritis, articular rheumatism, corneal ulcer, periodontic disease, metastasis or infiltration of tumor, virus infectious disease (HIV infectious disease)).
The sulfonamide derivatives having MMP inhibiting activities were described in Patent Literature 1, Patent Literature 2, Patent Literature 3 and Non-Patent Literature 1.
The sulfonamide derivatives having MMP-12 inhibiting activity, and having a naphthyl group as a substituent were described in Patent Literature 4.
The thiazoles or oxazolesulfonamide derivatives having a naphtyl group as a substituent iwere described in Patent Literature 5.
The thiazoles or oxazolesulfonamide derivatives having a naphtyl group as a substituent were described in Patent Literature 6.
The sulfonamide derivatives having aglycanase inhibiting activity, and having a naphtyl group as a substituent were described in Patent Literature 7.
The sulfonamide derivatives having aggrecanase inhibiting activity and MMP-13 inhibiting activity were described in Patent Literature 8.
The sulfonamide derivatives having aggrecanase inhibiting activity and MMP inhibiting activities were described in Patent Literature 9.
The sulfonamide derivatives having TNF-$\alpha$ inhibiting activity and the MMP inhibiting activities were described in Patent Literature 10.

[Patent Literature 1] International Publication WO 97/27174
[Patent Litarature 2] International Publication WO 99/04780
[Patent Litarature 3] International Publication WO 00/15213
[Patent Litarature 4] International Publication WO 01/83431
[Patent Litarature 5 International Publication WO 02/28844
[Patent Litarature 6] International Publication WO 01/83461
[Patent Litarature 7] International Publication WO 03/080042
[Patent Litarature 8] Japanese Patent Application Laid-Open (JP-A) No.2000-319250
[Patent Litarature 9] International Publication WO 05/061459
[Patent Litarature 10] International Publication WO 98/32748
[Non-Patent Literature 1] Yoshinori Tamura et al., J. Med. Chem., 1998, vol.41, No.4, p.640-649

[Disclosure of the invention]

[Problems to be solved by the invention]

Since it is thought that if the activity of MMP-13 can be inhibited, this will contribute to improvement or prevention of progession of pathological states, particularly, osteoarthritis (OA), resulting from or associated with the MMP-13 activity, the development of MMP-13 inhibitors is anticipated.

[Means to solve the problems]

In view of the aforementioned respects, the present inventors intensively continued to study and they completed the following invention by finding that some novel sulfonamide derivatives exhibit the MMP-13 selective inhibiting activity.

The present invention relates to: 1) a compound represented by the general formula (I):

[Chemical formula 1]

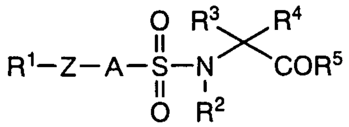

(I)

wherein $R^1$ is optionally substituted aryl, optionally substituted heteroaryl, an optionally substituted non-aromatic carbocyclic group, or an optionally substituted non-aromatic heterocyclic group;
Z is -O-, -S-, -SO-, $-SO_2-$, $-N(R^{10})-$, $-N(R^{10})-C(=O)-$, $-C(=O)-N(R^{10})-$, $-N(R^{10})-SO_2-$, $-SO_2-N(R^{10})-$, -C(=O)-, -O-C(=O)-, -C(=O)-O-, $-N(R^{10})-C(=O)-N(R^{10})-$, - $N(R^{10})-C(=S)-N(R^{10})-$, $-O-N=C(R^8)-$, $-C(R^8)=N-O-$, $-O-C(=O)-N(R^{10})-$, $-N(R^{10})-C(=O)-O-$, -C(=O)C(=O)-, an optionally substituted non-aromatic carbocyclic group, an optionally substituted non-aromatic heterocyclic group, C1-C5 alkylene which is optionally substituted and may be intervened a substituent selected from Substituent group a, or C1-C5 alkenylene which is optionally substituted and may be intervened with a substituent selected from Substituent group a, or C1-C5 alkynylene which is optionally substituted and may be intervened with a substituent selected from Substituent group a, wherein the Substituent group a consists of -O-, -S-, - SO-, $-SO_2-$, $-N(R^{10})-$, $-N(R^{10})-C(=O)-$, $-C(=O)-N(R^{10})-$, $-N(R^{10})-SO_2-$, $-SO_2-N(R^{10})-$, -C(=O)-, -O-C(=O)-, -C(=O)-O-, $-N(R^{10})-C(=O)-N(R^{10})-$, $-N(R^{10})-C(=S)-N(R^{10})-$, $-O-N=C(R^8)-$, $-C(R^8)=N-O-$, $-O-C(=O)-N(R^{10})-$, $-N(R^{10})-C(=O)-O-$, and - C(=O)-C(=O)-,
$R^8$ and $R^{10}$ are each independently a hydrogen atom or lower alkyl;
A is a group represented by the formula;

[Chemical formula 2]

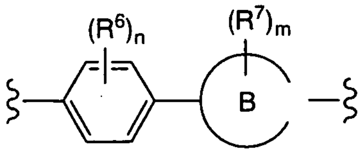

or

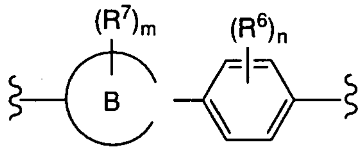

wherein $R^6$ and $R^7$ are each independently halogen, lower alkyl, cycloalkyl, lower alkenyl, lower alkynyl, lower alkyloxy, lower alkenyloxy, lower alkylthio, halo lower alkyl, halo lower alkyloxy, halo lower alkylthio, hydroxy, hydroxy lower alkyl, mercapto, carboxy, lower alkyloxycarbonyl, lower alkylsulfonyl, acyl, acyloxy, nitro, cyano, optionally substituted amino, or optionally substituted aminocarbonyl;
B ring is a nitrogen-containing heterocycle optionally further containing a nitrogen atom, an oxygen atom, and/or a sulfur atom in the ring; m and n are each independently an integer of 0 to 3;
$R^2$ is a hydrogen atom, optionally substituted lower alkyl, optionally substituted aralkyl, optionally substituted heteroarylalkyl, optionally substituted aryl, or optionally substituted heteroaryl;
$R^3$ is a hydrogen atom, optionally substituted lower alkyl, optionally substituted aralkyl, optionally substituted heteroarylalkyl, optionally substituted aryl, or optionally substituted heteroaryl;
$R^4$ is a hydrogen atom, or
$R^3$ and $R^4$ may be taken together with an adjacent carbon atom to form a 5-to 6-membered non-aromatic carbocyclic ring or a 5- to 6-membered non-aromatic heterocyclic ring;
$R^5$ is hydroxy, lower alkyloxy, or -NHOH;
provided that when B ring is piperazine, and Z is $-CH_2-O-$, $R^1$ is not pyridyl an optically active isomer , a pharmaceutically acceptable salt or a solvate thereof.

More particularly, the present invention relates to the following 2) to 27).

2) The compound according to 1), which is represented by the general formula (II):

[Chemical formula 3]

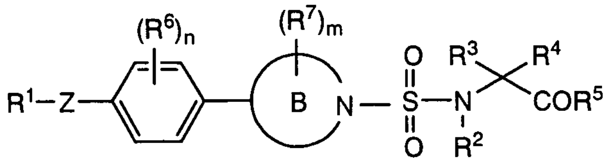

(II)

wherein $R^1$, $R^2$, $R^3$, $R^4$, $R^5$, $R^6$, $R^7$, Z, m, n and B ring are as defined in 1), an optically active isomer, a pharmaceutically acceptable salt or a solvate thereof.

3) The compound according to 1), which is represented by the general formula (III):

[Chemical formula 4]

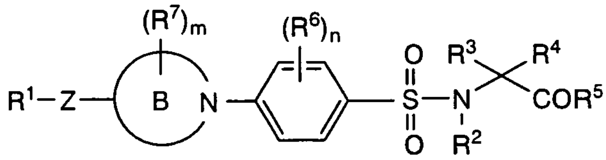

(III)

wherein $R^1$, $R^2$, $R^3$, $R^4$, $R^5$, $R^6$, $R^7$, Z, m, n and B ring are as defined in 1), an optically active isomer, a pharmaceutically acceptable salt or a solvate thereof.

4) The compound according to any one of 1) to 3), wherein $R^1$ is optionally substituted naphthyl or optionally substituted carbazolyl, an optically active isomer, a pharmaceutically acceptable salt or a solvate thereof.
5) The compound according to any one of 1) to 3), wherein $R^1$ is optionally substituted naphthyl, or an optically active isomer,a pharmaceutically acceptable salt or a solvate thereof.
6) The compound according to 5), wherein $R^1$ is a group represented by the formula:

[Chemical formula 5]

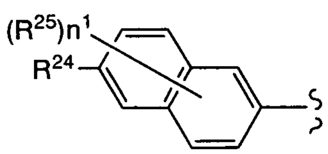

wherein $R^{24}$ is a hydrogen atom; lower alkyl optionally substituted with carboxy or lower alkyloxyimino; lower alkenyl optionally substituted with carboxy; hydroxy; lower alkyloxy optionally susbstituted with cycloalkyl, hydroxy, lower alkyloxy, carboxy, or aminocarbonyl optionally substituted with 1 or 2 lower alkyl(s); carboxy; acyl; amino optionally substitute with 1 or 2 acyl(s) or lower alkylsulfonyl(s); aminocarbonyl optionally substituted with 1 or 2 lower alkyl (s); or a non-aromatic heterocycle;
$R^{25}$ is halogen, cyano, or acyl;
$n^1$ is an integer of 0 to 2, and $R^{25}$ may be substituted on all replaceable constituent carbon atoms, an optically active isomer, a pharmaceutically acceptable salt, or a solvate thereof. Among the abovementioned compounds, the

compounds in which $n^1$ is 0 are preferable.

7) The compound according to 5), wherein $R^1$ is a group represented by the formula:

[Chemical formula 6]

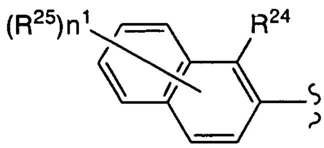

wherein $R^{24}$, $R^{25}$, and $n^1$ are as defined in 6,);
an optically active isomer, a pharmaceutically acceptable salt or a solvate thereof. Among the abovementioned compounds, the compounds in which $n^1$ is 0 are preferable.

8) The compound according to 5), wherein $R^1$ is a group represented by the formula:

[Chemical formula 7]

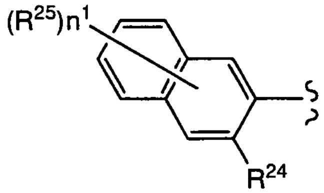

wherein $R^{24}$, $R^{25}$, and $n^1$ are as defined in 6);,
an optically active isomer, a pharmaceutically acceptable salt or a solvate thereof. Among the abovementioned compounds, the compounds in which $n^1$ is 0 are preferable.

9) The compound according to any one of 6) to 8), wherein $R^{24}$ is a hydrogen atom, lower alkyloxy, or amino, or an optically active isomer, a pharmaceutically acceptable salt or a solvate thereof.

10) The compound according to any one of 1) to 3), wherein $R^1$ is optionally substituted carbazolyl, or an optically active isomer, a pharmaceutically acceptable salt or a solvate thereof. Among the abovemented compounds, the compounds in which $R^1$ is unsubstituted carbazolyl are preferable.

11) The compound according to any one of 1) to 10), wherein Z is $-C(R^8)(R^9)-$, -O-, -S-, -SO-, $-SO_2-$, $-N(R^{10})-$, $-C(R^8)(R^9)-C(R^8)(R^9)-$, $-C(R^8)=C(R^9)-$, -C≡C-, $-O-C(R^8)(R^9)-$, $-C(R^8)(R^9)-O-$, $-S-C(R^8)(R^9)-$, $-C(R^8)(R^9)-S-$, $-SO-C(R^8)(R^9)-$, $- C(R^8)(R^9)-SO-$, $-SO_2-C(R^8)(R^9)-$, $-C(R^8)(R^9)-SO_2-$, $-N(R^{10})-C(R^8)(R^9)-$, $- C(R^8)(R^9)-N(R^{10})-$, $-N(R^{10})-C(=O)-$, $-C(=O)-N(R^{10})-$, $-N(R^{10})-SO_2-$, $\cdot SO_2-N(R^{10})-$, $-C(R^8)(R^9)-C(=O)-$, $-C(=O)-C(R^8)(R^9)-$, -C(=O)C(=O)-, $-C(R8)(R9)-C(R^8)(R^9)-C(R^8)(R^9)-$, $-C(R^8)(R^9)-C(R^8)=C(R^9)-$, $-C(R^8)=C(R^9)-C(R^8)(R^9)-$, $-C(R^8)(R^9)-C\equiv C-$, $-C\equiv C-C(R^8)(R^9)-$, $-O-C(R^8)(R^9)-C(R^8)(R^9)-$, $-C(R^8)(R^9)-O-C(R^8)(R^9)-$, $-C(R^8)(R^9)-C(R^8)(R^9)-O-$, $-S-C(R^8)(R^9)-C(R^8)(R^9)-$, $-C(R^8)(R^9)-S-C(R^8)(R^9)-$, $-C(R^8)(R^9)-C(R^8)(R^9)-S-$, $-SO-C(R^8)(R^9)-C(R^8)(R^9)-$, $-C(R^8)(R^9)-SO-C(R^8)(R^9)-$, $-C(R^8)(R^9)-C(R^8)(R^9)-SO-$, $-SO_2-C(R^8)(R^9)-C(R^8)(R^9)-$, $-C(R^8)(R^9)-SO_2-C(R^8)(R^9)-$, $- C(R^8)(R^9)-C(R^8)(R^9)-SO_2-$, $-N(R^{10})-C(R^8)(R^9)-C(R^8)(R^9)-$, $-C(R^8)(R^9)-N(R^{10})-C(R8)(R9)-$, $-C(R^8)(R^9)-C(R^8)(R^9)-N(R^{10})-$, $-C(=O)-C(R^8)(R^9)-C(R^8)(R^9)-$, $- C(R^8)(R^9)-C(=O)-C(R^8)(R^9)-$, $-C(R^8)(R^9)-C(R^8)(R^9)-C(=O)-$, $-C(R^8)(R^9)-N(R^{10})-C(=O)-$, $-N(R^{10})-C(=O)-C(R^8)(R^9)-$, $-C(R^8)(R^9)-C(=O)-N(R^{10})-$, $-C(=O)-N(R^{10})-C(R^8)(R^9)-$, $-C(R^8)(R^9)-N(R^{10})-SO_2-$, $-N(R^{10})-SO_2-C(R^8)(R^9)-$, $-C(R^8)(R^9)-SO_2-N(R^{10})-$, $-SO_2-N(R^{10})-C(R^8)(R^9)-$, $N(R^{10})-C(=O)-N(R^{10})-$, $-N(R^{10})-C(=S)-N(R^{10})-$, $-O-N=C(R^8)-$, $-C(R^8)=N-O-$, $-O-C(=O)-C(R^8)(R^9)-$, $-C(R^8)(R^9)-C(=O)-O-$, $-O-C(=O)-N(R^{10})-$, or $-N(R^{10})-C(=O)-O-$, wherein $R^8$, $R^9$ and $R^{10}$ are each independently a hydrogen atom or lower alkyl, an optically active isomer, a pharmaceutically acceptable salt or a sovate thereof.

12) The compound according to any one of 1) to 10), wherein Z is $-C(R^8)(R^9)-C(R^8)(R^9)-$, $-C(R^8)=C(R^9)-$, -C≡C-, $-O-C(R^8)(R^9)-$, $-C(R^8)(R^9)-O-$, $-S-C(R^8)(R^9)-$, $-C(R^8)(R^9)-S-$, $-SO-C(R^8)(R^9)-$, $-C(R^8)(R^9)-SO-$, $-SO_2-C(R^8)(R^9)-$, $-C(R^8)(R^9)-SO_2-$, $-N(R^{10})-C(R^8)(R^9)-$, $-C(R^8)(R^9)-N(R^{10})-$, $-N(R^{10})-C(=O)-$, $-C(=O)-N(R^{10})-$, $-N(R^{10})-SO_2-$, $-SO_2-N(R^{10})-$, $-C(R^8)(R^9)-C(=O)-$, $-C(=O)-C(R^8)(R^9)-$, or - C(=O)C(=O)-, wherein $R^8$, $R^9$ and $R^{10}$ are each independently a hydrogen atom or lower alkyl, an optically active isomer, a pharmaceutically acceptable salt or a solvate thereof.
13) The compound according to any one of 1) to 10), wherein Z is $-CH_2-CH_2-$, -CH=CH-, -C≡C-, $-O-CH_2-$, or $-CH_2-O-$, an optically active isomer, a pharmaceutically acceptable salt or a solvate thereof.
14) The compound according to any one of 1) to 10), wherein Z is -C≡C-, an optically active isomer, a pharmaceutically acceptable salt or a solvate thereof.

15) The compound according to any one of 1) to 14), wherein a group represented by the formula:

[Chemical formula 8]

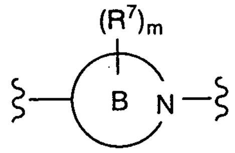

is a group represented by the formula:

[Chemical formula 9]

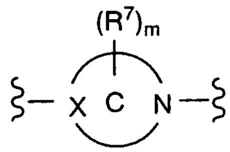

wherein X is a carbon atom or a nitrogen atom, a C ring is a 5- to 8-membered nitrogten-containing heterocycle, and $R^7$ and m are as defined in 1), an optically active isomer, a pharmaceutically acceptable salt or a solvate thereof.
16) The compound according to any one of 1) to 14), wherein a group represented by the formula:

[Chemical formula 10]

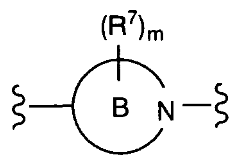

is a group represented by the formula:

[Chemical formula 11]

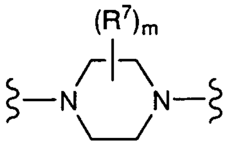,
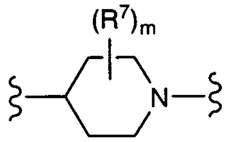,
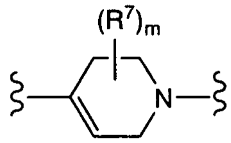,

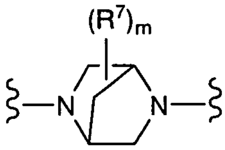
or
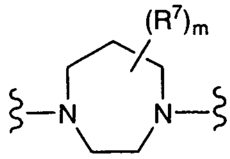

wherein $R^7$ and m are as defined in 1), an optically active isomer, a pharmaceutically acceptable salt or a solvate thereof.

17) The compound according to any one of 1) to 16), wherein $R^2$ is a hydrogen atom, or an optically active isomer, a pharmaceutically acceptable salt or a solvate thereof.

18) The compound according to any one of 1) to 17), wherein $R^3$ is a hydrogen atom, methyl, ethyl, n-propyl, isopropyl, n-butyl, s-butyl, isobutyl, t-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl;
$R^4$ is a hydrogen atom; or
$R^3$ and $R^4$ may be taken together with an adjacent carbon to form a ring represented by the formula:

[Chemical formula 12]

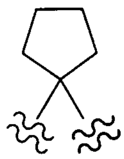,
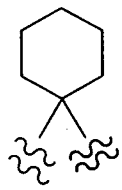
or
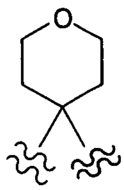

an optically active isomer, a pharmaceutically acceptable salt or a solvate thereof.

19) The compound according to any one of 1) to 17), wherein $R^3$ is a hydrogen atom, methyl, ethyl, n-propyl, isopropyl, n-butyl, s-butyl, isobutyl, t-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl, and
$R^4$ is a hydrogen atom, an optically active isomer, a pharmaceutically acceptable salt or a solvate thereof.

20) The compound according to any one of 1) to 19), wherein $R^5$ is hydroxy, an optically active isomer, a pharmaceutically acceptable salt or a solvate thereof.

21) A pharmaceutical composition containing the compound as defined in any one of 1) to 20) as an active ingredient. -

22) A pharmaceutical composition for inhibiting MMP-13 which contains the compound as defined in any one of 1) to 20) as an active ingredient.

23) An agent for treating or preventing osteoarthritis containing the compound as defined in any one of 1) to 20) as an active ingredient.

24) A method of treating a disease resulting from, or associated with MMP-13 of a mammal, comprising administering

an amount for exhibiting the therapeutic effect of the compound as defined in any one of 1) to 20) to a mammal including a human.

25) A method of treating osteoarthritis of a mammal, comprising administering an amount for exhibiting the therapeutic effect of the compound as defined in any one of 1) to 20) to a mammal including a human.

26) Use of the compound as defined in any one of 1) to 20), for preparing a medicament for treating a disease resulting from associated with MMP-13.

27) Use of the compound as defined in any one of 1) to 20), for preparing a medicament for treating osteoarthritis.

Herein, a term "lower alkyl" which is used alone or in combination with other terms includes linear or branched monovalent hydrocarbon groups comprised of 1 to 8 carbon atoms. For example, they are methyl, ethyl, n-propyl, isopropyl, n-butyl, isobutyl, s-butyl, t-butyl, n-pentyl, isopentyl, neopentyl, n-hexyl, isohexyl, n-heptyl, n-octyl and the like. Preferable examples are C1-C6 alkyls. Further preferable examples are C1-C3 alkyls.

Herein, the "cycloalkyl" includes cycloalkyls comprised of 3 to 8 carbon atoms. For example they are cyclopropyl, cylobutyl, cyclopentyl, cyclohexyl, cycloheptyl and cyclooctyl. Preferable examples are C3-C6 cycloalkyls.

Herein, the "lower alkenyl" which is used alone or in combination with other terms includes linear or branched monovalent hydrocarbon groups comprised of 2 to 8 carbon atoms, having one or more double bonds. For example, they are vinyl, allyl, propenyl, crotonyl, isopentenyl, various butenyl isomers and the like. Preferable examples are C2-C6 alkenyls. Further preferable examples are C2-C4 alkenyls.

Herein, the "cycloalkenyl" includes cycloalkenyls comprised of 3 to 8 carbon atoms, having one or more double bonds in the ring. For example, they are cyclopropenyl, cyclobutenyl, cyclopentenyl, cyclohexenyl, cycloheptenyl, and cyclooctenyl. Preferable examples are C3-C6 cycloalkenyls.

Herein, the "lower alkynyl" includes linear or branched monovalent hydrocarbon groups comprised of 2 to 8 carbon atoms, having one or more triple bonds, which may have a double bond. For example, they are ethynyl, 2-propynyl, 3-butynyl, 4-pentynyl, 5-hexynyl, 6-heptynyl, 7-octynyl and the like. Preferable examples are C2-C6 alkynyls. Further preferable examples are C2-C4 alkynyls.

Herein, the "cycloalkynyl" includes cycloalkynyls comprised of 4 to 8 carbon atoms, having one or more triple bonds in the ring, which may have a double bond. For example, they are cyclobutynyl, cyclopentynyl, cyclohexynyl, cycloheptynyl, and cyclooctynyl. Preferable examples are C3-C6 cycloalkynyls.

Herein, the "alkylene" includes divalent hydrocarbon groups corresponding to the above "lower alkyl".

Herein, the "alkenylene" includes divalent hydrocarbon groups corresponding to the above "lower alkenyl".

Herein, the "alkynylene" includes divalent hydrocarbon groups corresponding to the above "lower alkynyl".

Herein, the "C1-C5 alkylene which be intervened with a substituent selected from Substituent group a" includes (substituent selected from Substituent group a)-(C1-C5 alkylene), (C1-C5 alkylene)-(substituent selected from Substituent group a), (alkylene)-(substituent selected from Substituent group a)-(alkylene) (provided that the sum of lengths of both alkylene chains is an integer of 1 to 5).

Herein, the "aryl" which is used alone or in combination with other terms includes monocyclic aromatic hydrocarbon groups or fused aromatic hydrocarbon groups in which 2 or 3 aromatic rings are fused. For example, they are phenyl, 1-naphthyl, 2-naphthyl, anthryl and the like.

As the "aryl" in $R^1$, naphthyl is preferable, and 2-naphthyl is particularly preferable.

As the "aryl" in $R^2$, phenyl is preferable.

As the "aryl" in $R^3$, phenyl is preferable.

Herein, the "naphthyl" includes 1-naphthyl and 2-naphthyl.

Herein, the "aralkyl" which is used alone or in combination with other terms is the abovementioned "lower alkyl" which is substituted with one or more of the abovementioned "aryl"s, which may be incorporated at all possible positions. For example, they are benzyl, phenylethyl (e.g. 2-phenylethyl etc.), phenylpropyl (e.g. 3-phenylpropyl etc.), naphthylmethyl (e.g. 1-naphthylmethyl, 2-naphthylmethyl etc.), anthrylmethyl (e.g. 9-anthrylmethyl etc.), biphenylmethyl (e.g. 4-biphenylmethyl etc.) and the like. Preferable examples are benzyl, and phenylethyl.

As the "aralkyl" in $R^2$, phenyl C1-C3 alkyls are preferable, and benzyl is particularly preferable.

As the "aralkyl" in $R^3$, phenyl C1-C6 alkyls and naphthyl C1-C6 alkyls are preferable. Further preferable are phenyl C1-C3 alkyls and naphthyl C1-C3 alkyls, for example, benzyl, phenylethyl, 1-naphthylmethyl, and 2-naphthylmethyl.

Herein, the "heteroaryl" which is used alone or in combination with other terms includes 5- to 6-membered aromatic rings containing, in the ring, one or more atoms arbitrarily selected from oxygen atoms, sulfur atoms and nitrogen atoms, and these may be fused with cycloalkyl, aryl, non-aromatic heterocycle, or other heteroaryl, and may be fused at all possible positions. For example, they are pyrrolyl (e.g. 1-pyrrolyl, 2-pyrrolyl, 3-pyrrolyl), furyl (e.g. 2-furyl, 3-furyl), thienyl (e.g. 2-thienyl, 3-thienyl), imidazolyl (e.g. 2-imidazolyl, 4-imidazolyl), pyrazolyl (e.g. 1-pyrazolyl, 3-pyrazolyl), isothiazolyl (e.g. 3-isothiazolyl), isoxazolyl (e.g. 3-isoxazolyl), oxazolyl (e.g. 2-oxazolyl), thiazolyl (e.g. 2-thiazolyl), pyridyl (e.g. 2-pyridyl, 3-pyridyl, 4-pyridyl), pyrazinyl (e.g. 2-pyrazinyl), pyrimidinyl (e.g. 2-pyrimidinyl, 4-pyrimidinyl), pyridazinyl (e.g. 3-pyridazinyl), tetrazolyl (e.g. 1H-tetrazolyl), oxadiazolyl (e.g. 1,3,4-oxadiazolyl), thiadiazolyl (e.g. 1,3,4-thiadiazolyl), indolizinyl (e.g. 2-indolizinyl, 6-indolizinyl), isoindolyl - (e.g. 2-isoindolyl), indolyl (e.g. 1-indolyl, 2-indolyl, 3-indolyl, 5-indolyl, 6-indolyl), indazolyl (e.g. 3-indazolyl), purinyl (e.g. 8-purinyl), quinolizinyl (e.g. 2-quinolizinyl), isoquinolyl (e.g. 3-isoquinolyl), quinolyl (e.g. 2-quinolyl, 5-quinolyl), phthalazinyl (e.g. 1-phthalazinyl), naphthyridinyl (e.g. 2-naphthyridinyl), quinoxanyl (e.g. 2-quinoxanyl), quinazolinyl (e.g. 2-quinazolinyl), cinnolinyl (e.g. 3-cinnolinyl), pteridinyl (e.g. 2-pteridinyl), carbazolyl (e.g. 2-carbazolyl, 3-carbazolyl), phenanthridinyl (e.g. 2-phenanthridinyl, 3-phenanthridinyl), acridinyl (e.g. 1-acridinyl, 2-acridinyl), dibenzofuranyl (e.g. 1-dibenzofuranyl, 2-dibenzofuranyl, 3-dibenzofuranyl), benzimidazolyl (e.g. 2-benzimidazolyl), benzisoxazolyl (e.g. 3-benzisoxazolyl), benzoxazolyl (e.g. 2-benzoxazolyl), benzoxadiazolyl (e.g. 4-benzoxadiazolyl), benzisothiazolyl (e.g. 3-benzisothiazolyl), benzthiazolyl (e.g. 2-benzthiazolyl, 5-benzthiazolyl), benzofuryl (e.g. 2-benzofuryl, 3-benzofuryl, 5-benzofuryl), benzothienyl (e.g. 2-benzothienyl), thienopyrimidinyl, and the like.
As the "heteroaryl" in $R^1$, carbazolyl is preferable.
As the "heteroaryl" in $R^2$, pyridyl, thienyl, furyl, imidazolyl and the like are preferable.
As the "heteroaryl" in $R^3$, indolyl, imidazolyl, furyl, thienyl, and the like are preferable.

Herein, the "heteroarylalkyl" is the abovementioned "lower alkyl" substituted with one or more of the abovementioned "heteroaryl"s at the arbitrary positions, and these can be incorporated at all possible positions. For example, they are oxazolylmethyl (e.g. 2-oxazolylmethyl, 4-oxaszolylmethyl), thiazolylmethyl (e.g. 2-thiazolylmethtyl, 4-thiazolylmethyl), oxazolylethyl (e.g. 5-oxazolyl-2-ethyl), thiazolylyethyl (e.g. 5-thiazolyl-2-ethyl), benzoxazolylmethyl (e.g. benzoxazol-2-ylmethyl), benzothiazolylmethyl (e.g. benzothiazol-2-ylmethyl), indolylmethyl (e.g. indol-3-ylmethyl), imidazolylmethyl (e.g. 4-imidazolylmethyl), indazolylmethyl (e.g. 1-indazolylmethyl), benzotriazolylmethyl (e.g. 1-benzotriazolylmethyl), benzoquinolylmethyl (e.g. 2-benzoquinolylmethyl), benzimidazolylmethyl (e.g. benzimidazol-2-methyl), pyridylmethyl (e.g. 4-pyridylmethyl), furylmethyl (e.g. furan-2-ylmethyl), thienylmethyl (e.g. thiophen-2-ylmethyl) and the like.
Examples of the "heteroarylalkyl" in $R^2$ are indolylmethyl (e.g. indol-3-ylmethyl), imidazolylmethyl (e.g. imidazol-5-ylmethyl), thienylmethyl (e.g. thiophen-2-ylmethyl), thiazolylmethyl (e.g. 2-thiazolylmethyl, 4-thiazolylmethyl), pyridylmethyl and the like.
Examples of the "heteroarylalkyl" in $R^3$ are indolylmethyl (e.g. indol-3-ylmethyl), imidazolylmethyl (e.g. imidazol-5-ylmethyl), benzoxazolylmethyl (e.g. benzoxazol-2-ylmethyl), benzothiazolylmethyl (e.g. benzothiazol-2-ylmethyl), benzimidazolylmethyl (e.g. benzimidazol-2-ylmethyl), thienylmethyl (e.g. thiophen-2-ylmethyl), thiazolylmethyl (e.g. 2-thiazolylmethyl, 4-thiazolylmethyl) and the like.

Herein, a term "non-aromatic hydrocarbon group" which is used alone or in combination with other terms includes cycloalkyls, cycloalkenyls or cycloalkynyls, or cyclic groups in which they are fused with the abovementioned "aryl". For example, they are cyclobutyl, cyclopentyl, cyclohexyl, indanyl, tetrahydronaphthyl and the like.
As the "non-aromatic carbocyclic group" in Z, cyclobutyl is preferable.
Examples of the "non-aromatic carbocycle" in which $R^3$ and $R^4$ are taken together with an adjacent carbon to form a 5- to 6- membered non-aromatic carbocycle, are cycloalkane, cycloalkene and cycloalkyne.
Cyclopentane, cyclohexane, and the like are preferable.
Herein, a term "non-aromatic heterocyclic group" which is used alone or in combination with other terms includes a non-aromatic 5- to 7-membered ring containing, in the ring, one or more atoms, arbitrarily selected from oxygen atoms, sulfer atoms and nitrogen atoms, and the ring groups in which two or more of the above rings are fused, and the ring groups in which the abovementioned "ring" is fused with the abovementioned "aryl". For example, they are pyrrolidinyl (e.g. 1-pyrrolidinyl, 2-pyrrolidinyl), pyrrolinyl (e.g. 3-pyrrolinyl), imidazolidinyl (e.g. 2-imidazolidinyl), imidazolinyl (e.g. imidazolinyl), pyrazolidinyl (e.g. 1-pyrazolidinyl, 2-pyrazolidinyl), pyrazolinyl (e.g. pyrazolinyl), piperidyl (e.g. piperidino, 2-piperidyl), piperazinyl (e.g. 1-piperazinyl), indolinyl (e.g. 1-indolinyl), isoindolinyl (e.g. isoindolinyl), morpholinyl (e.g. morpholino, 3-morpholinyl), tetrahydroquinolyl, tetrahydrofuryl, tetrahydropyranyl, 1,3-benzodioxolanyl, 1,4-benzodioxanyl, 1-benzoxolanyl, oxetanyl, azetidinyl, diazetidinyl, chromanyl, and the like.
As the "non-aromatic heterocyclic group" in Z, oxetanyl, azetidinyl, and diazetidinyl are preferable.
As the "non-aromatic heterocycle" in which $R^3$ and $R^4$ are taken together with an adjacent carbon to form a 5- to 6-membered non-aromatic heterocycle, tetrahydropyran and the like are preferable.

The "nitrogen-containing heterocycle optionally further containing a nitrogen atom, an oxygen atom, and/or a sulfur atom in a ring" in the B ring means a nitrogen-containing heterocycle optionally further containing a nitrogen atom, an oxygen atom, and/or a sulfur atom in the ring, in addition to a nitrogen atom on the B ring. The "nitrogen-containing heterocycle" includes monocyclic 5- to 8- membered nitrogen-containing heterocycles, and bridged nitrogen-containing heterocycles. Preferable are nitrogen-containing heterocycles represented by the formula:

[Chemical formula 13]

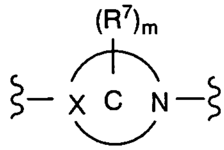

(wherein, X is a carbon atom or a nitrogen atom, a C ring is a 5- to 8-membered nitrogen-containg heterocycle or bridged ring, $R^7$ is halogen, lower alkyl, cycloalkyl, lower alkenyl, lower alkynyl, lower alkyloxy, lower alkenyloxy, lower alkylthio, halo lower alkyl, halo lower alkyloxy, halo lower alkylthio, hydroxy, hydroxy lower alkyl, mercapto, carboxy, lower alkyloxycarbonyl, lower alkylsulfonyl, acyl, acyloxy, nitro, cyano, optionally substituted amino, or optionally substituted aminocarbonyl; m is an integer of 0 to 3). Further preferable are nitrogen-containing heterocycles represented by the formula:

[Chemical formula 14]

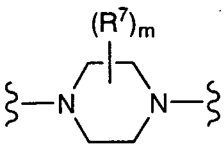 , 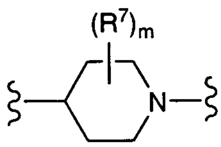 , 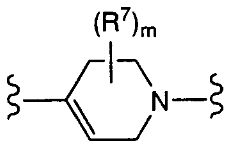 ,

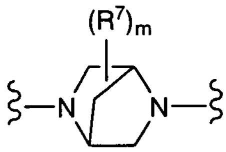 or 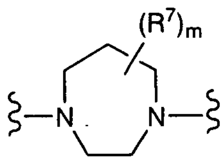

(wherein $R^7$ and m are defined above). Particularly preferable are nitrogen-containing heterocycles represented the formula:

[Chemical formula 15]

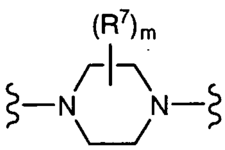

(wherein $R^7$ and m are defined above).

Herein, "halogen" means fluorine, chlorine, bromine and iodine.

Preferable are fluorine, chlorine, and bromine.

Herein, examples of the "lower alkyloxy" are methyloxy, ethyloxy, n-propyloxy, isopropyloxy, n-butyloxy, isobutyloxy, s-butyloxy, t-butyloxy, n-pentyloxy, n-hexyloxy, and the like. Preferable is C1-C6 alkyloxy.

Further preferable is C1-C3 alkyloxy.

Herein, examples of the "lower alkenyloxy" are vinyloxy, allyloxy, propenyloxy, 3-butenyloxy, 2-butenyloxy (crotonyloxy,

isocrotonyloxy), 1-butenyloxy, isopentenyloxy and the like. C2-C3 alkenyloxy is preferable.
Herein, examples of the "lower alkylthio" are methylthio, ethylthio, propylthio, isopropylthio, butylthio, s-butylthio, isobutylthio, t-butylthio and the like. C1-C3 alkylthio is preferable.

Herein, a term "halo lower alkyl" which is used alone or in combination with other terms includes the abovementioned "lower alkyl", 1 to 8 atoms, preferably 1 to 5 atoms of which are substituted with the abovementioned "halogen". For example, they are trifluoromethyl, trichloromethyl, difluoroethyl, trifluoroethyl, dichloroethyl, trichloroethyl and the like. Preferable examples are C1-C3 lower alkyls, 1 to 5 atoms of which are substituted with the "halogen". Particularly preferable examples are trifluoromethyl.
Herein, as the "halo lower alkyloxy", C1-C3 lower alkyloxys, 1 to 5 atoms of which are substituted with the "halogen", is preferable.
Particularly preferable examples are trifluoromethyloxy.
Herein, as the "halo lower alkylthio", C1-C3 lower alkylthios, 1 to 5 atoms of which are substituted with the "halogen", is preferable.
Particularly preferable examples are trifluoromethylthio.
Herein, a term "hydroxy lower alkyl" includes the abovementioned "lower alkyl" substituted with a hydroxy group. For example, they are hydroxymethyl, 1-hydroxyethyl, 2-hydroxyethyl, 1-hydroxypropyl, 2-hydroxypropyl, 3-hydroxypropyl and the like. Preferable examples are hydroxymethyl, 1-hydroxyethyl, and 2-hydroxyethyl.
Herein, examples of the "lower alkyloxycarbonyl" are methyloxycarbonyl, ethyloxycarbonyl, n-propyloxycarbonyl, isopropyloxycarbonyl and the like.
Herein, examples of the "lower alkylsulfonyl" are methylsulfonyl, ethylsulfonyl and the like. Preferable example is methylsulfonyl.
Herein, examples of the "aralkyloxy" are phenyl C1-C3 alkyloxy, naphthyl C1-C3 oxy and the like. Preferable example is benzyl.
Herein, a term "lower alkyloxy lower alkyl" includes the abovemenrioned "lower alkyl" substituted with the abovementioned "lower alkyloxy". For example, they are methyloxymethyl, ethyloxymethyl, propyloxymethyl, 1-methyloxyethyl, 2-methyloxyethyl, 1-methyloxypropyl, 2-methyloxypropyl, 3-methyloxypropyl, and the like. Preferable example is methyloxymethyl.
Herein, examples of the "aryloxy lower alkyl" are phenyloxymethyl, 1-phenyloxyethyl, 2-phenyloxyethyl, 1-naphthyloxymethyl, 2-naphtyloxymethyl and the like.
Herein, a term "acyl" which is used alone or in combination with other terms includes alkylcarbonyl in which an alkyl moiety is the abovementioned "lower alkyl", arylcarbonyl in which an aryl moiety is the abovementioned "aryl", and heteroarylcarbonyl in which a heteroaryl moiety is the abovementioned "heteroaryl". For example, they are acetyl, propionyl, benzoyl, pyridylcarbonyl and the like. The "lower alkyl" and the "aryl" may be substituted with a substituent described later.
Herein, examples of the "acyloxy" are acetyloxy, propionyloxy, benzoyloxy, and the like.

Herein, the "optionally substituted amino" which is used alone or in combination with other terms includes amino and amino, 1 or 2 atoms of which are substituted with the abovementioned "lower alkyl", the abovementioned "alalkyl", the abovementioned "heteroarylalkyl", the abovementioned "lower alkylsulfonyl", or the abovementioned "acyl". Preferable examples are unsubstituted amino, and amino substituted with 1 or 2 substituents selected from the group consisting of C1-C6 alkyl, phenyl C1-C3 alkyl, pyridyl C1-C3 alkyl, C1-C6 alkylsulfonyl, C1-C6 alkylcarbonyl and arylcarbonyl. For example, they are amino, methylamino, dimethylamino, ethylmethylamino, diethylamino, benzylamino, methylsulfonylamino, acetylamino, benzoylamino and the like. Preferable examples are amino, methylamino, dimethylamino, ethylmethylamino, diethylamino, methylsulfonylamino and acetylamino.
Herein, as the "optionally substituted aminocarbonyl", unsubstitued aminocarbonyl, or aminocarbonyl, 1 or 2 atoms of which are substituted with C1-C6 alkyl, is preferable. For example, they are aminocarbonyl, methylaminocarbonyl, dimethylaminocarbonyl, ethylmethylaminocarbonyl, diethylaminocarbonyl and the like. Preferable examples are aminocarbonyl and dimethylaminocarbonyl.

Herein, examples of a substituent in the "optionally substituted lower alkyl" are lower alkylthio, cycloalkyl, carboxy, lower alkyloxy, hydroxy, mercapto, halogen, nitro, cyano, lower alkyloxycarbonyl, halo lower alkyl, halo lower alkyloxy, optionally substituted amino, optionally substituted aminocarbonyl, acyl, acyloxy, a non-aromatic heterocyclic group optionally substituted with a substituent selected from Substituent group b, aryloxy, aralkyloxy, lower alkylsulfonyl, guanidino, azo group, ureido optionally substituted with a substituent selected from Substituent group b, carbamoyl and the like. These may substitute at one or more of all possible positions.
Substituent group b: lower alkyl, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo lower alkyl, halo lower alkyloxy, halo lower alkylthio, optionally substituted amino, optionally substituted aminocarbonyl, acyl, acyloxy, aryl, heteroaryl, non-aromatic heterocycle, aralkyl, lower alkylsulfonyl, guanidino, azo group, and ureido.
As a substituent of the "optionally substituted lower alkyl" in $R^2$, hydroxy, lower alkyloxy, and a non-aromatic heterocyclic

group optionally substituted with a substituent selected from Substituent group b are preferable.
As a substituent of the "optionally substituted lower alkyl" in $R^3$, lower alkyloxy, lower alkylthio, cycloalkyl, carboxy, halogen, hydroxy, carbamoyl, and mercapto are preferable.

Herein, examples of the substituents in the "optionally substituted aryl", the "optionally substituted heteroaryl", the "optionally substituted aralkyl", and the "optionally substituted heteroarylalkyl" in $R^2$ and $R^3$ are lower alkyl optionally substituted with a substituent selected from Substituent group b, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo lower alkyl, halo lower alkyloxy, halo lower alkylthio, optionally substituted amino, optionally substituted aminocarbonyl, acyl, acyloxy, aryl optionally substituted with a substituent setected from Substituent group b, hetroaryl optionally substituted with a substituent setected from Substituent group b, non-aromatic heterocycle optionally substituted with a substituent setected from Substituent group b, aralkyl optionally substituted with a substituent setected from Substituent group b, lower alkylsulfonyl, guanidino, azo group, and ureido optionally substituted with a substituent setected from Substituent group b. These can substitute at one or more of all possible positions.
Substituent b: lower alkyl, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo lower alkyl, halo lower alkyloxy, halo lower alkylthio, optionally substituted amino, optionally substituted aminocarbonyl, acyl, acyloxy, aryl, heteroaryl, non-aromatic heterocycle, aralkyl, lower alkylsulfonyl, guanidino, azo group, and ureido
As a substituent of the "optionally substituted aryl" in $R^2$, hydroxy, lower alkyloxy, halogen, and halo lower alkyl are preferable.
As a substitutent of the "optionally substituted aryl" in $R^3$, hydroxy is preferable.
As a substituent of the "optionally substituted heteroaryl" in $R^2$, hydroxy, lower alkyloxy, halogen, and halo lower alkyl and preferable.
As a substitutent of the "optionally substituted heteroaryl" in $R^3$, hydroxy and halogen are preferable.
As a substituent of the "optionally substituted aralkyl" in $R^2$, hydroxy, lower alkyloxy, halogen, halo lower alkyl, and nitro are preferable.
As a substituent of the "optionally substituted aralkyl" in $R^3$, hydroxy, halogen, nitro, lower alkyloxy, halo lower alkyl, and carboxy lower alkyloxy are preferable.
As a substituent of "optinally substituted heteroarylalkyl" in $R^2$, hydroxy, lower alkyloxy, halogen, and halo lower alkyl are preferable.
As a substituent of the "optionally substituted heteroarylalkyl" in $R^3$, halogen, hydroxy, and carboxy are preferable.

Herein, examples of the substituents in the "optionally substituted aryl", the "optionally substituted heteroaryl", the "optionally substituted non-aromatic carbocyclic group", the "optionaly substituted non-aromatic heterocyclic group", the "optionally substituted naphtyl", the "optionally substituted carbazolyl" in $R^1$ are halogen, lower alkyl optionally substituted with a substituent selected from Susbstituent group e, lower alkyloxy optionally substituted with a substituent selected from Substituent group f, cycloalkyl, lower alkenyl optionally substituen with carboxy, lower alkynyl, lower alkenyloxy, lower alkylthio, halo lower alkyl, halo lower alkyloxy, halo lower alkylthio, hydroxy, hydroxy lower alkyl, mercapto, carboxy, lower alkyloxycarbonyl, lower alkylsulfonyl, acyl, acyloxy, nitro, cyano, oxo, hydroxyimino, lower alkyloxyimino, optionally substituted amino, optionally substituted aminocarbonyl, aryl optionally substituted with a substituent selected from Substituent group b, heteroaryl optionally substituted with a substituent selected from Substituent group b, aryloxy optionally substituted with a substituent selected from Substituent group b, aralkyloxy optionally substituted with a substituent selected from Substituent group b, lower alkyloxy lower alkyl, aryloxy lower alkyl optionally substituted with a substituent selected from Substituent group b, ureido optionally substituted with a substituent selected from Substituent group b, and non-aromatic heterocycric group optionally substituted with a substituent selected from Substituent group b. These can substitute at one or more of all possible positions.
Substituent group b: lower alkyl, cycloalkyl, lower alkenyl, lower alkynyl, hydroxy, lower alkyloxy, mercapto, lower alkylthio, lower alkenyloxy, halogen, nitro, cyano, carboxy, lower alkyloxycarbonyl, halo lower alkyl, halo lower alkyloxy, halo lower alkylthio, optionally substituted amino, optionally substituted aminocarbonyl, acyl, acyloxy, aryl, heteroaryl, non-aromatic heterocycle, aralkyl, lower alkylsulfonyl, guanidino, azo group, and ureido Substituent group e: carboxy, cyano, lower alkyloxyimino, and optionally substituted aminocarbonyl
Substituent group f: hydroxy, lower alkyloxy, cycloalkyl, aryl, carboxy, and optionally substituted aminocarbonyl Herein, as the "optionally substituted naphtyl" in $R^1$, 2-naphtyl which has no substituents either at the position 5 or 8, is preferable. Herein, as the "optionally substituted carbazolyl" in $R^1$, unsubstituted carbozolyl is preferable.

As the present compound, particularly, compounds represented by the following A) to AC) are preferable.

A) A compound represented by the general formula (IV):

[Chemical formula 16]

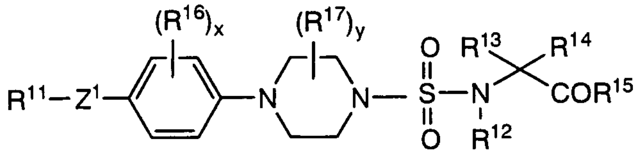

(IV)

[wherein $R^{11}$ is optionally substituted naphthyl or optionally substituted carbazolyl;
$Z^1$ is -C($R^8$)($R^9$)-C($R^8$)($R^9$)-, -C($R^8$)=C($R^9$)-, -C≡C-, -O-C($R^8$)($R^9$)-, -C($R^8$)($R^9$)-O-, -S-C($R^8$)($R^9$)- -C($R^8$)($R^9$)-S-, -SO-C($R^8$)($R^9$)-, -C($R^8$)($R^9$)-SO-, -$SO_2$-C($R^8$)($R^9$)-, - C($R^8$)($R^9$)-$SO_2$-, -N($R^{10}$)-C($R^8$)($R^9$)-, -C($R^8$)($R^9$)-N($R^{10}$)-, -N($R^{10}$)-C(=O)-, -C(=O)-N($R^{10}$)-, - -N($R^{10}$)-$SO_2$-, -$SO_2$-N($R^{10}$)-, -C($R^8$)($R^9$)-C(=O)-, -C(=O)-C($R^8$)($R^9$)-, or - C(=O)C(=O)- (wherein, $R^8$, $R^9$, and $R^{10}$ are each independently a hydrogen atom, or lower alkyl);
$R^{12}$ is a hydrogen atom or lower alkyl;
$R^{13}$ is a hydrogen atom, methyl, ethyl, n-propyl, isopropyl, n-butyl, s-butyl, isobutyl, t-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl,
$R^{14}$ is a hydrogen atom; or
$R^{13}$ and $R^{14}$ may be taken together with an adjacent carbon to form the ring represented by the formula:

[Chemical formula 17]

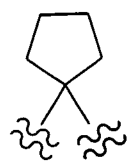 , 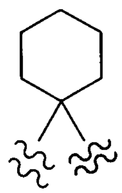 or 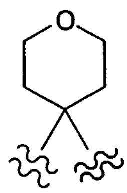

$R^{15}$ is hydroxy or lower alkyloxy;
$R^{16}$ and $R^{17}$ are each independently halogen, lower alkyl, lower, alkyloxy, or hydroxy;
x and y are each independently an integer of 0 to 3],
an optically active isomer, a pharmaceutically acceptable salt, or a solvate thereof.
B) A compound represented by the general formula (V):

[Chemical formula 18]

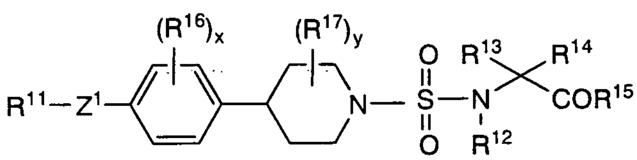

(V)

wherein, $R^{11}$, $Z^1$, $R^{12}$, $R^{13}$, $R^{14}$, $R^{15}$, $R^{16}$, $R^{17}$, x, and y are as defined in A),

an optically active isomer, a pharmaceutically acceptable salt or a solvate thereof.

C) A compound represented by the general formula (VI):

[Chemical formula 19]

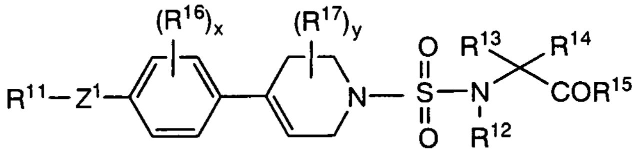

(VI)

wherein $R^{11}$, $Z^1$, $R^{12}$, $R^{13}$, $R^{14}$, $R^{15}$, $R^{16}$, $R^{17}$, x, and y are as defined in A), an optically active isomer, a pharmaceutically acceptable salt, or a solvate thereof.

D) A compound represented by the general formula (VII):

[Chemical formula 20]

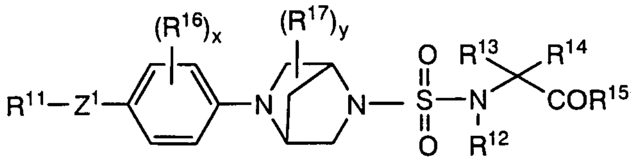

(VII)

wherein $R^{11}$, $Z^1$, $R^{12}$, $R^{13}$, $R^{14}$, $R^{15}$, $R^{16}$, $R^{17}$, x, and y are as defined in A), an optically active body isomer, a pharmaceutically acceptable salt, or a solvate thereof.

E) A compound represented by the general formula (VIII):

[Chemical formula 21]

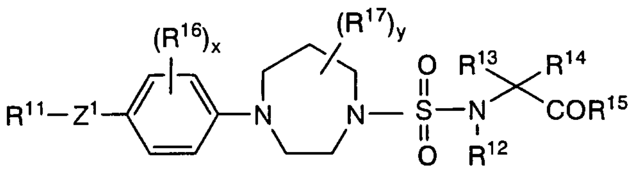

(VIII)

wherein $R^{11}$, $Z^1$, $R^{12}$, $R^{13}$, $R^{14}$, $R^{15}$, $R^{16}$, $R^{17}$, x, and y are as defined in A), an optically active body isomer, a pharmaceutically acceptable salt, or a solvate thereof.

F) A compound represented by the general formula (IX):

[Chemical formula 22]

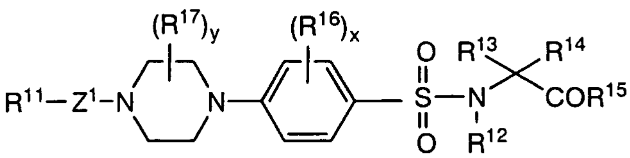

(IX)

wherein $R^{11}$, $Z^1$, $R^{12}$, $R^{13}$, $R^{14}$, $R^{15}$, $R^{16}$, $R^{17}$, x, and y are as defined in A) an optically active isomer, a pharmaceutically acceptable salt, or a solvate thereof.

G) A comound represented by the general formula (X):

[Chemical formula 23]

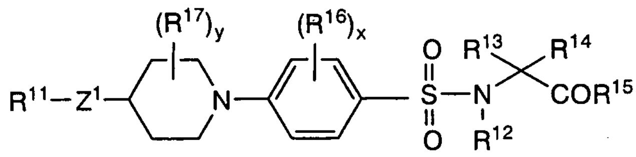

(X)

wherein $R^{11}$, $Z^1$, R12, $R^{13}$, $R^{14}$, $R^{15}$, $R^{16}$, $R^{17}$, x, and y are as defined in A)] an optically active isomer, a pharmaceutically acceptable salt, or a solvate thereof.

H) A compound represented by the general formula (XI)

[Chemical formula 24]

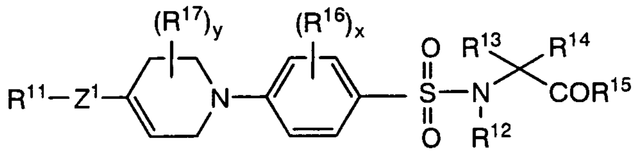

(XI)

wherein $R^{11}$, $Z^1$, $R^{12}$, $R^{13}$, $R^{14}$, $R^{15}$, $R^{16}$, $R^{17}$, x and y are as defined in A), an optically active isomer, a pharmaceutically acceptable salt, or a solvate thereof.

I) A compound represented by the general formula (XII):

[Chemical formula 25]

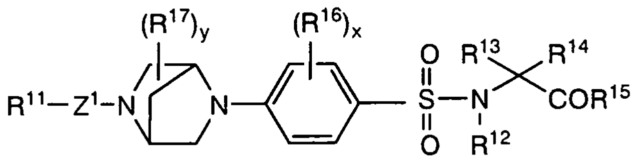

(XII)

wherein $R^{11}$, $Z^1$, $R^{12}$, $R^{13}$, $R^{14}$, $R^{15}$, $R^{16}$, $R^{17}$, x and y are as defined in A) an optically active isomer, a pharmaceutically acceptable salt, or a solvate thereof.

J) A compound represented by the general formula (XIII)

[Chemical formula 26]

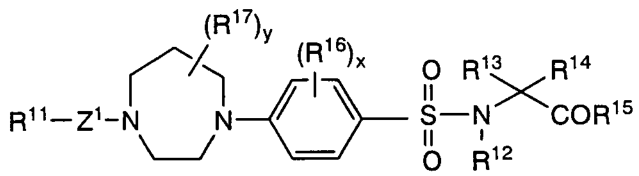

(XIII)

wherein $R^{11}$, $Z^1$, $R^{12}$, $R^{13}$, $R^{14}$, $R^{15}$, $R^{16}$, $R^{17}$, x and y are as defined in A), an optically active isomer, a pharmaceutically acceptable salt, or a solvate thereof.

K) The compound described in any of A) to J), wherein $R^{11}$ is optionally substituted naphthyl, an optically active isomer, a pharmaceutically acceptable salt or a solvate thereof.
L) The compound described in any of A) to K), wherein $R^{11}$ is a group represented by the formula:

[Chemical formula 27]

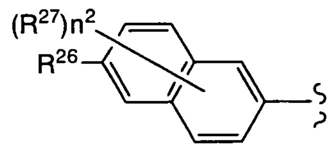

wherein, $R^{26}$ is a hydrogen atom, optionally substituted lower alkyl, optionally substituted lower alkenyl, hydroxy, optionally substituted lower alkyloxy, carboxy, acyl, optionally substituted amino, optionally substituted aminocarbonyl or non-aromatic heterocycle;
$R^{27}$ is halogen, cyano, or acyl;
$n^2$ is an integer of 0 to 2, and $R^{27}$ can substitute at all incorporable ring-constituting atoms, an optically active isomer, a pharmaceutically acceptable salt, or a solvate thereof. Among the above compounds, the compounds in which $n^2$ is 0 are preferable.
M) The compound described in any of A) to L), wherein $R^{11}$ is a group represented by the formula:

[Chemical formula 28]

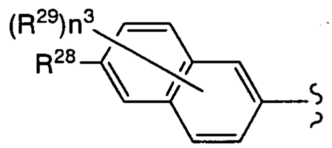

wherein $R^{28}$ is a hydrogen atom; lower alkyl optionally substituted with carboxy or lower alkyloxylmino; lower alkenyl optionally substituted with carboxy; hydroxy; lower alkyloxy optionally substituted with cycloalkyl, hydroxy, lower alkyloxy, carboxy, or aminocarbonyl optionally substituted with 1 or 2 lower alkyl(s); carboxy; acyl; amino optionally substituted with 1 or 2 acyl(s) or lower alkylsulfonyl; aminocarbonyl optionally substituted with 1 or 2 lower alkyl(s); or non-aromatic heterocycle;
$R^{29}$ is halogen, cyano, or acyl;
$n^3$ is an integer of 0 to 2, and $R^{29}$ can substitute at all incorporable ring-constituting atoms, an optically active isomer, a pharmaceutically acceptable salt, or a solvate thereof. Among the above compounds, the compounds in which $n^3$ is 0 are preferable.

N) The compound described in any of A) to K), wherein $R^{11}$ is a group represented by the formula:

[Chemical formula 29]

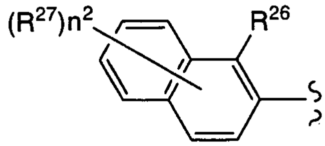

wherein $R^{26}$, $R^{27}$, and $n^2$ are as defined in L), an optically active isomer, a pharmaceutically acceptable salt, or a solvate thereof. Among the above compounds, the compound in which $n^2$ is 0 are preferable.

O) The compound described in any of A) to K), and N), wherein $R^{11}$ is a group represented by the formula:

[Chemical formula 30]

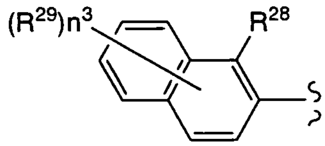

wherein $R^{28}$, $R^{29}$, and $n^3$ are as defined in M), an optically active isomer, a pharmaceutically acceptable salt, or a solvate thereof. Among the above compounds, the compounds in which $n^3$ is 0 are preferable.

P) The compound described in any of A) to K), wherein $R^{11}$ is a group represented by the formula:

[Chemical formula 31]

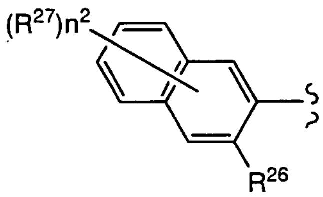

wherein $R^{26}$, $R^{27}$, and $n^2$ are as defined in L), an optically active isomer, a pharmaceutically acceptable salt, or a solvate thereof. Among the above compounds, the compounds in which $n^2$ is 0 are preferable.

Q) The compound described in any of A) to K), and P), wherein $R^{11}$ is a group represented by the formula:

[Chemical formula 32]

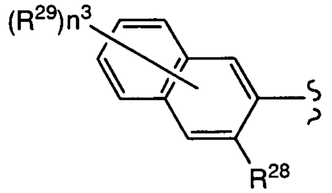

wherein $R^{28}$, $R^{29}$ and $n^3$ are as defined in M), an optically active isomer, a pharmaceutically acceptable salt, or a solvate thereof. Among the above compounds, the compounds in which $n^3$ is 0 are preferable.

R) The compound described in any of A) to K), wherein $R^{11}$ is optionally substituted carbazolyl, an optically active isomer, a pharmaceutically acceptable salt, or a solvate thereof. Among the compounds, the compounds in which $R^{11}$ is unsubstituted carbazolyl are preferable.

S) The compound described in any of A) to R), wherein $Z^1$ is $-CH_2-CH_2-$, - CH=CH-, -C≡C-, $-O-CH_2-$, $-CH_2-O-$, $-S-CH_2-$, $-CH_2-S-$, $-SO-CH_2-$, $-CH_2-SO-$, - $SO_2-CH_2-$, $-CH_2-SO_2-$, $-N(R^{10})-CH_2-$, $-CH_2-N(R^{10})-$, -NH-C(=O)-, -C (=O)-NH-, - $CH_2-C(=O)-$, $-C(=O)-CH_2-$, or -C(=O)C(=O)-, an optically active isomer, a pharmaceutically acceptable salt, or a solvate thereof.

T) The compound described in any of A) to S), wherein $Z^1$ is $-CH_2-CH_{2-}$, - CH=CH-, -C≡C-, $-O-CH_2-$, or $-CH_2-O-$, an optically active isomer, a pharmaceutically acceptable salt, or a solvate thereof.

U) The compound described in any of A) to T), wherein $Z^1$ is $-CH_2-CH_2-$, - CH=CH- or -C≡C-, an optically active isomer, a pharmaceutically acceptable salt, or a solvate thereof.

V) The compound described in any of A) to U), wherein $Z^1$ is -C≡C-, an optically active isomer, a pharmaceutically acceptable salt, or a solvate thereof.

W) The compound described in any of A) to V), wherein $R^{12}$ is a hydrogen atom, an optically active isomer, a pharmaceutically acceptable salt, or a solvate thereof.

X) The compound described in any of A) to W), wherein $R^{13}$ is a hydrogen atom, methyl, ethyl, n-propyl, isopropyl, n-butyl, s-butyl, isobutyl, t-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl, and $R^{14}$ is a hydrogen atom, an optically active isomer, a pharmaceutically acceptable salt, or a solvate thereof.

Y) The compound described in any of A) to X), wherein $R^{13}$ is methyl, ethyl, n-propyl, isopropyl, n-butyl, s-butyl, isobutyl, t-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl, and $R^{14}$ is a hydrogen atom, an optically active isomer, a pharmaceutically acceptable salt, or a solvate thereof.

Z) The compound described in any of A) to W), wherein $R^{13}$, and $R^{14}$ are taken together with an adjacent carbon to form a ring represented by the formula:

[Chemical formula 33]

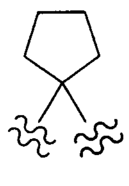 , 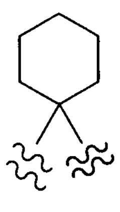 or 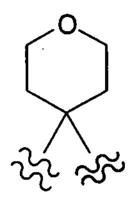

an optically active isomer, a pharmaceutically acceptable salt, or a solvate thereof.

AA) The compound described in any of A) to Z), wherein $R^{15}$ is hydroxy, an optically active isomer, a pharmaceutically acceptable salt, or a solvate thereof.

AB) The compound described in any of A), F), and K) to AA), wherein the group represented by the formula:

[Chemical formula 34]

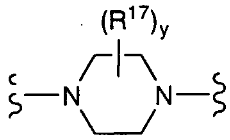

is the group represented by the formula:

[Chemical formula 35]

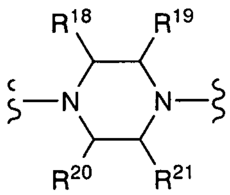

wherein in $R^{18}$, $R^{19}$, $R^{20}$, and $R^{21}$, i) all are hydrogen atoms, or ii) only $R^{18}$ is lower alkyl, and all of the rest are hydrogen atoms, iii) only $R^{19}$ is lower alkyl, and all of the rest are hydrogen atoms, or iv) $R^{18}$ and $R^{20}$ are each independently lower alkyls, and all of the rest are hydrogen atoms, or v) $R^{18}$ and $R^{21}$ are each independently lower alkyls, and all of the rest are hydrogen atoms, or vi) $R^{19}$ and $R^{21}$ are each independently lower alkyls, and all of the rest are hydrogen atoms, an optically active isomer, a pharmaceutically acceptable salt, or a solvate thereof.

AC) The compound described in any of B), G), and K) to AA), wherein the group represented by the formula:

[Chemical formula 36]

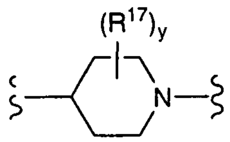

is the group represented by the formula:

[Chemical formula 37]

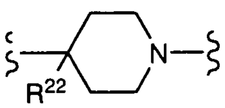

wherein $R^{22}$ is a hydrogen atom, halogen, hydroxy, or lower alkyloxy, an optically active isomer, a pharmaceutically acceptable salt, or a solvate thereof.

[Effect of the invention]

It was found that certain novel sulfonamide derivatives exhibit the MMP-13 selective inhibiting activity. These derivatives can be utilized as an agent for treating or preventing a disease resulting from, or associated with MMP-13, particularly, osteoarthritis (OA). Also, the invented compounds have excellent profile as a medicament, such as relatively low protein binding rate, excellent solubility, good oral absorbability, and/or low toxicity.

[Best mode for carrying out the invention]

The present compound can be synthesized according to the method described in WO97/27174, WO00/46189 and the like, and the known method. As the methods for synthesizing the invented compounds, Method A and Method B are exemplified below.

(Method A)

[Chemical formula 38]

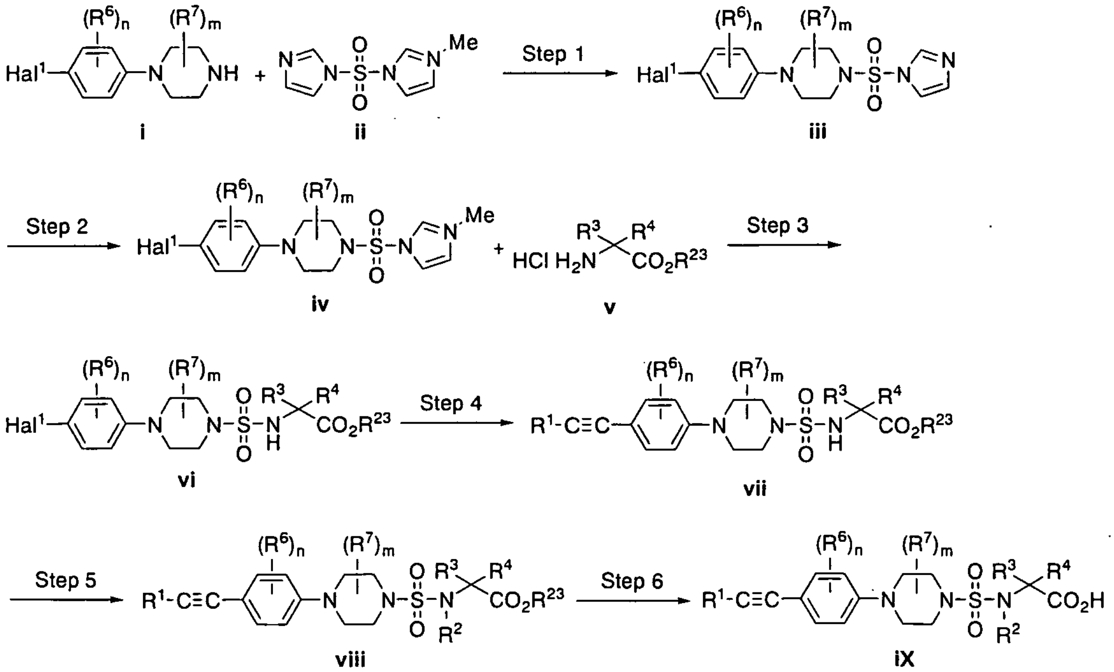


(wherein $R^1$, $R^2$, $R^3$, $R^4$, $R^6$, $R^7$, m and n are as defined above; $R^{23}$ is a protecting group of a carboxyl group; and $Hal^1$ is halogen)

(Step 1)

Some compounds of the starting material represented by the formula (i) are commertially available. The other compounds can be synthesized according to the method described in Tetrahedron Lett. 39 (1998) 617-620.
Some compounds of the starting material represented by the formula (ii) are commertially available.
A compound represented by the formula (iii) can be obtained by reacting a compound represented by the formula (i) or a salt thereof, and a compound represented by the formula (ii) at ice-bath temperature to 80 degree, preferably 0 degree to 30 degree, for 1 to 38 hours, preferably 3 to 24 hours in a solvent such as acetonitrile, tetrahydrofuran, N,N-dimethylformamide and the like, in the presence of a base such as triethylamine, diisopropylethylamine, potassium carbonate and the like.

(Step 2)

A compound represented by the formula (iv) can be obtained by reacting the compound represented by the formula (iii) with methyl trifluoromethanesulfonate at ice-bath temperature to room temperature for 1 to 24 hours, preferably 1 to 5 hours.

(Step 3)

Some of the starting amino acids and their acid addition salts (e.g. hydrochloride, *p*-toluenesulfonate, trifluoroacetate) represented by the formula (v) are commertially available. The other amino acids and their acid addition salts can be synthesized according to the method for amino acid synthesis, described in Experimental Chemistry Course, vol.22, 4 edition (edited by The Chemical Society of Japan), or the method described in J. Med. Chem. 38, 1689-1700 (1995) Gary M. Ksander et. al. and the like.
In addition, some of sulfonylating reagents (e.g. sulfonic acid halide) are commertially available and the other reagent can be synthesized according to the method described in New Experimental Chemistry Course vol.14, p1787, (1978), Synthesis 852-854 (1986) Tatsuo Hamada et. al. and the like.
The protecting groups for carboxyl groups include, for example, an esterified carboxyl group (e.g. methyl ester, tert-butyl ester, benzyl ester). Removal of these protecting groups can be performed by hydrolysis in the presence of an acid (e.g. hydrochloric acid, trifluoroacetic acid) or a base (e.g. sodium hydroxide etc), or hydrogenolysis (e.g. in the presence of 10% palladium-carbon catalyst), depending on the protecting groups.
Solvents for the sulfonylation include dimethylformamide, tetrahydrofuran, dioxane, dimethyl sulfoxide, acetonitrile, water and a mixed solvent thereof, and a mixed solvent of a water-insoluble solvent (e.g. benzene, dichloromethane) and the aforementioned solvent.
The base used for the sulfonylation is an organic base such as triethylamine, N-methylmorpholine and the like, or an inorganic base such as sodium hydroxide, potassium hydroxide, potassium carbonate and the like. The reaction temperature is usually ice-bath temperature ~ room temperature. When $R^3$ and $R^4$ are groups having a substituent which is harmful for sulfonylation (e.g. hydroxy, mercapto, amino, guanidino), the group can be protected in advance by the method described in Protective Groups in Organic Synthesis, Theodora W Green (John Wiley & Sons), and the protecting group may be removed at a desirable stage.

(Step 4)

By reacting with an optionally substituted naphthyl derivative having an ethynyl group such as ethynylnaphthalene and the like in the presence of a palladium catalyst (e.g. $Pd(Ph_3P)_2Cl_2$ etc.), a divalent copper reagent (e.g. CuI etc.), and an organic base (e.g. triethylamine, diisopropylethylamine etc.) in a solvent such as dimethylformamide, toluene, xylene, benzene, tetrahydrofuran and the like (Sonogashira reaction), the compound (vi) can be converted into the desired compound.
The reaction temperature is room temperature to 100°C, preferably room temperature to 80°C, and the reaction time is 3 to 30 hours, preferably 10 to 20 hours. When the optionally substituted naphthyl derivative has a substituent which is harmful to the present reaction, the group can be protected in advance by the method described in Protective Groups in Organic Synthesis, Theodora W Green (John Wiley & Sons), and the protecting group may be removed at a preferable stage.

(Step 5)

The desired N-$R^2$ analogs can be obtained by adding alkyl halide (e.g. methyl iodide, ethyl iodide etc.) or aralkyl halide (e.g. benzyl chloride, benzyl bromide etc.) to the compound (vii) at ice-bath temperature to 80°C, preferably at ice-bath temperature to room temperature, in a solvent such as dimethylformamide, tetrahydrofuran, dioxane and the like, and by stirring the mixture for 3 to 30 hours, preferably 10 to 20 hours.

(Step 6)

The compound represented by the general formula (I) can be synthesized by deprotecting the protective group of the compound (viii) according to a conventional method.

(B method)

[Chemical formula 39]

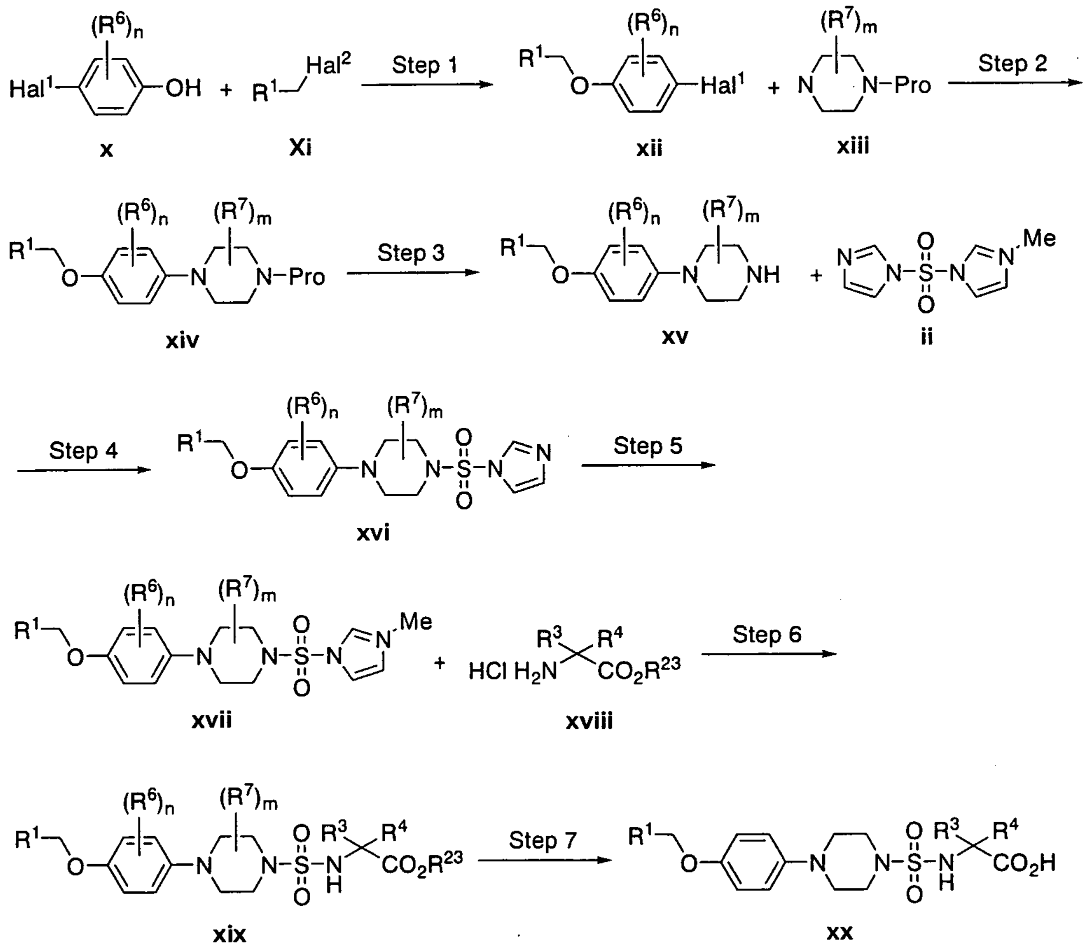


x Xi xii xiii xiv xv ii xvi xvii xviii xix xx

(wherein $R^1$, $R^3$, $R^4$, $R^6$, $R^7$, $R^{23}$, m and n are as defined above; $R^{10}$ is a protecting group for a carboxyl group; $Hal^1$ and $Hal^2$ are each independently halogen; Pro is a protecting group such as Boc etc.)

(Step 1)

Some compounds of the starting material represented by the formula (x) are commercially available. Some compounds of the starting material represented by the formula (xi) are commercially available. The other compounds can be synthesized according to the method described in Tetrahedron Lett. 37 (1996) 6965-6968.
A compound represented by the formula (xii) can be obtained by reacting the compound represented by the formula (x) or a salt thereof and a compound represented by the formula (xi) at ice-bath temparature to 110 degree, preferably 0 degree to 80 degree for 1 to 36 hours, preferably 1 to 24 hours in the presence of a base such as potassium carbonate, cesium carbonate, sodium hydroxide and the like, in a solvent such as dimethylformamide, 1,4-dioxane, dichloromethane and the like.

(Step 2)

Some compounds of the starting material represented by the formula (xiii) are commercially available. The other compounds can be synthesized according to the method described in Angew Chem, Int Ed 37 (1998) 325-329.
A compound represented by the formula (xiv) can be obtained by reacting a compound represented by the formula (xii) and a compound represented by the formula (xiii) at room temperature to 150 degree, preferably room temperature to 120 degree, for 1 to 24 hours, preferably 1 to 8 hours, in the presence of a base such as sodium t-butoxide, cesium carbonate and the like, a palladium catalyst such as palladium acetate and the like, and a ligand such as tri-t-butylphosphine, triphenylphosphine and the like in a mixed solvent of toluene, and N-methylpiperidone, N,N-dimethylformamide,

1,4-dioxane and the like.

(Step 3)

Step 3 is a step of deprotecting a protecting group. The step can be performed by a method which is usually conducted.

(Step 4)

Step 4 can be conducted by the same method as the Method A Step 1.

(Step 5)

Step 5 can be conducted by the same method as the Method A Step 2.

(Step 6)

Step 6 can be conducted by the same method as the Method A Step 3.

(Step 7)

Step 7 can be conducted by the same method as the Method A Step 6.
The compound represented by the general formula (iii) can be also synthesized according to the aforementioned method, the method described in WO 97/27174, WO 00/46189 and the like, and the known method.
The therapeutic effect of the present compound regarding osteoarthritis and adjuvant arthritis can be confirmed by the following method.

A) Osteoarthritis

Using a 12 week-old female Hartley guinea pig (Charles River Laboratories Japan, Inc.), a right knee joint meniscus and an inside collateral ligament are excised according to the method of Meacock et al. (J. Exp. Path. 71: 279-293, 1990), by which a guinea pig knee osteoarthritis model is prepared. From the following date of operation, a 0.5% methylcellulose solution as a solvent, or a 30mg/kg test compound solution is orally administered once a day every day for 10 days.
On the following day of the final administration date, a right os femoris distal portion and a cnemis proximal part are taken. After a joint cartilage surface is stained with India ink (manufactured by Kuretake Co, Ltd.), a cartilage surface is photographed with a digital camera (manufactured by Nikon Corporation). Assessment of compounds is performed by scoring a degree of an OA lesion on a joint cartilage surface using a photographed image by a blind method.
In addition, similarly, the effect can be also confirmed by oral administration every day for 6 weeks using a 12 week-old female NZW rabbit (KITAYAMA LABES Co., Ltd.) or a 12 week-old female SD rat (CLEA Japan, Inc.).

B) Adjuvant arthritis

Using a 7-week old female Lewis rat (manufactured by Charles River Laboratories Japan, Inc.), and according to the method of Fletcher et al. (J. Pharmacol. Exp. Ther. 284(2): 714-721), dead mycobacterium butyricum is administered to a right posterior limb footpad to prepare a rat adjuvant arthritis model. From the following day of administration, a 0.5% methylcellulose solution which is a solvent, or a 30mg/kg test compound solution is orally administered once a day. After 21 days from administration, a degree of posterior limb bloating is measured using a volume recording meter (manufactured by Shionogi & Co., Ltd.), a spleen and a thymus are taken, and their weight are measured. In addition, both posterior limbs are subjected to X-ray photographing (manufactured by OHMIC Co., Ltd.). Determination of X-ray image is performed by a blind method using a score from 0 (normal) to 3 (bone/cartilage complete destruction), depending on a degree of joint destruction.
Herein, the "solvate" includes, for example, a solvate with an organic solvent, a hydrate and the like. When a hydrate is formed, an arbitrary number of water molecules may be coordinated.
The "present compound" also includes a pharmaceutically acceptable salt, and a solvate thereof. For example they are salts with alkali metals (lithium, sodium, potassium etc.), alkaline earth metals (magnesium, calcium etc.), ammonium, organic bases and amino acids, and salts with inorganic acids (hydrochloric acid, hydrobromic acid, phosphoric acid, sulfuric acid etc.), and organic acids (acetic acid, citric acid, maleic acid, fumaric acid, benzenesulfonic acid, p-toluenesulfonic acid etc.). These salts can be formed by the method which is conventionally performed.
And, the present compound is not limited to a specified isomer, and includes all possible isomers (e.g. optically active body) or racemates.
The present compound does not exhibit the inhibiting activity on MMP-2 and MMP-9, but exhibits the selective and excellent MMP-13 inhibiting activity as described in Test Example described later.
The present compound can be used as a remedy for osteoarthritis, arthritis rheumatism, adjuvant arthritis, periodontitis, cirrhosis, osteoporosis, bone resorption, gingivitis, large intestine-rectum cancer, squamous epithelial cancer, epithelial cell cancer, prostate cancer, ovary cancer, tongue cancer, endometrium cancer, or stomach cancer.
When the present compound is administered to a human for the purpose of treating the aforementioned diseases, it can be orally administered as powders, granules, tablets, capsules, pills, solutions or the like, or can be parenterally administered as injectables, suppositories, transdermally absorbing agents, inhalants or the like. And, pharmaceutical additives suitable for a dosage form, such as excipients, binders, wetting agents, disintegrating agents, lubricants and the like, can be, if necessary, mixed into an effective amount of the present compound to obtain pharmaceutical preparations. In the case of injectables, they are sterilization-treated together with suitable carriers to obtain preparations.
A dose is different depending on condition of a patient, an administration route, an age and a weight of a patient and, when orally administered to an adult, the dose is usually 0.1 to 100 mg/kg/day, preferably 1 to 20 mg/kg/day.
The present invention will be explained in more detail below by way of Examples and Test Examples, but the present invention is not limited to them.

## Examples

In Examples, following abbreviations are used.

Me: methyl
Tf: trifluoromethanesulfonyl
Boc: t-butoxycarbonyl

[Examples 1]

Synthesis of (S)-2-{4-[4-(6-methoxy-naphthalen-2-ylethynyl)-phenyl]-piperazine-1-sufonylammo}-pentanoic acid (I-1)

[Chemical formula 40]

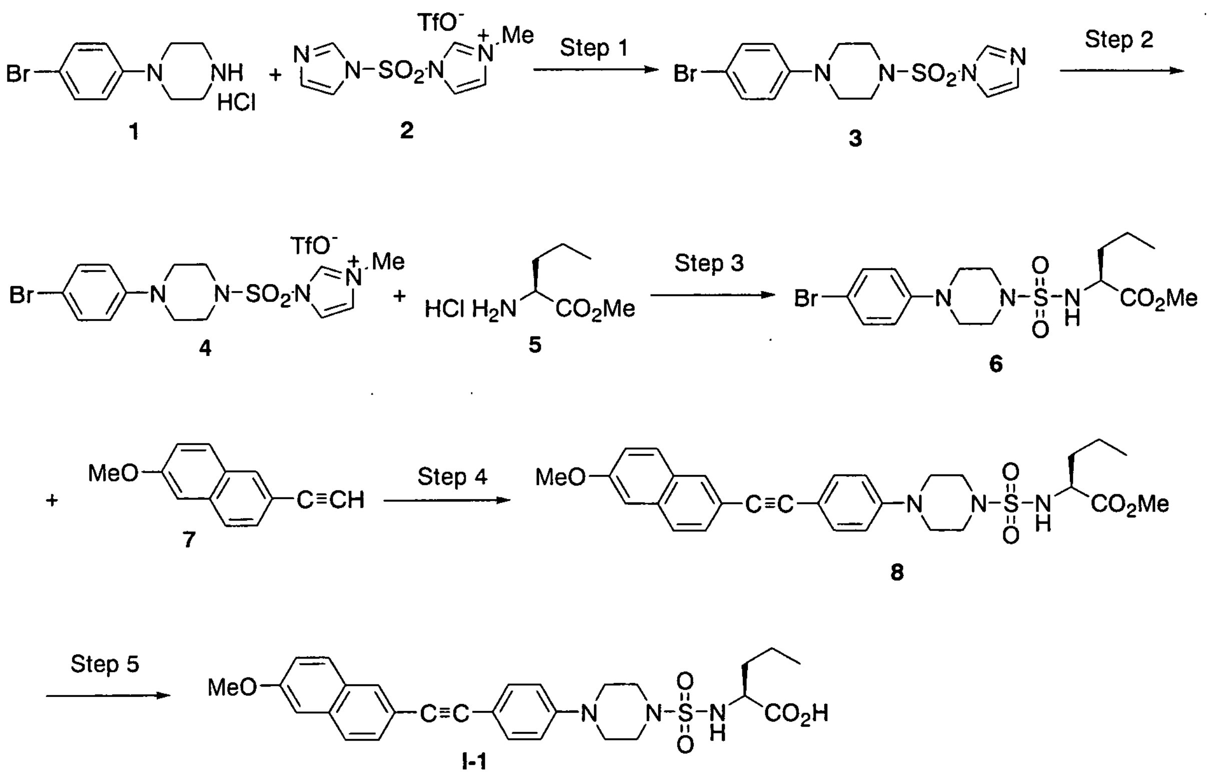


1 2 3 4 5 6 7 8 I-1

Step 1

To a solution of 1-(4-bromophenyl)piperazine hydrochloride (1) (5 g, 18.0 mmol) and triethylamine (2.8 mL, 20.1 mmol) in acetonitrile (80 mL) was added 3-(imidazol-1-sulfonyl)-1-methyl-3H-imidazol-1-ium triflate (2: J. Org. Chem., 68, 115(2003) ) (9.8 g, 27.0 mmol), and the mixture was stirred at room temperature for 24 hours. The reaction mixture was poured into ice-water, this was extracted with ethyl acetate, and the organic layer was washed with water. After dried over anhydrous sodium sulfate, the organic layer was concentrated under reduced pressure. The residue was subjected to silica gel chromatography, and the fractions eluted with hexane/ethyl acetate=1/1 were collected to give a pale brown compound (3) (2.91 g, yield 45.3%). The compound (3) was used in a next step without purification. mp 153-154°C

$^1$H NMR ($CDCl_3$) δ: 3.20-3.26 (m, 4H), 3.36-3.40 (m, 4H), 6.76 (d, J = 9.0 Hz, 2H), 7.22 (m, 1H), 7.29 (m, 1H), 7.37 (d, J = 9.0 Hz, 2H), 8.07 (m, 1H).

Step 2

To a solution of the compound (3) (4.82 g, 13.0 mmol) in dichloromethane (80 mL) was added methyl trifluoromethanesulfonate (1.6 mL, 14.1 mmol) under ice cooling, and this was stirred for 2.5 hours. The precipitated solid was collected by filtration, washed with dichloromethane and, then, n-hexane, and air-dried to give a compound (4) (6.3 g, yield 90.6%). The compound (4) was used in a next step without purification. mp 154-156°C

$^1$H-NMR (DMSO-$d_6$) δ: 3.28-3.35 (m, 4H), 3.46-3.53 (m, 4H), 3.88 (s, 3H), 6.93 (d, J = 9.0 Hz, 2H), 7.38 (d, J = 9.0 Hz, 2H), 7.98 (m, 1H), 8.24 (m, 1H), 9.85 (s, 1H).

Step 3

To a solution of the compound (4) (0.8 g, 1.49 mmol) in acetonitrile (8 mL) were added L-norvaline methyl ester hydrochloride (5) (0.38 g, 2.27 mmol) and, then, triethylamine (0.31 mL, 2.22 mmol) at room temperature, and the mixture was stirred for 40 hours. The reaction mixture was poured into ice water, and extracted with ethyl acetate. The organic layer was washed with water, dried over anhydrous sodium sulfate, and concentrated under reduced pressure. The

residue was subjected to silica gel chromatography, and the fractions eluted with ethyl acetate/hexane=1/3 were collected to give a pale yellow compound (6) (0.35 g, yield 53.8 %). The compound (6) was used in a next step without purification.
$^1$H NMR ($CDCl_3$) δ: 0.95 (t, J = 7.2 Hz, 3H), 1.35-1.50 (m, 2H), 1.60-1.85 (m, 2H), 3.13-3.22 (m, 4H), 3.32-3.40 (m, 4H), 3.77 (s, 3H), 3.98 (m, 1H), 4.91 (d, J = 9.6 Hz, 1H), 6.82 (d, J = 9.0 Hz, 2H), 7.37 (d, J = 9.0 Hz, 2H).

Step 4

To a solution of the compound (6) (0.34 g, 0.78 mmol) in dimethylformamide (5 mL) was added 2-ethynyl-6-methoxynaphthalene (7) (0.19 g, 1.04 mmol), and this was degassed under the nitrogen gas atmosphere. Then, bis (triphenylphosphine)palladium (II) chloride (0.06 g, 0.08 mmol), copper (I) iodide (0.03 g, 0.16 mmol), and triethylamine (0.63 mL, 4.50 mmol) were added, this was degassed again under the nitrogen gas atmosphere, and stirred at 90°C for 14 hours. The reaction mixture was poured into ice water-2 mol/L hydrochloric acid, and extracted with ethyl acetate. The organic layer was washed successively with an aqueous saturated sodium bicarbonate solution, and an aqueous saturated sodium chloride solution, dried over anhydrous sodium sulfate, and concentrated under reduced pressure. The residue was subjected to silica gel chromatography, and the fractions eluted with ethyl/n-hexane/chloroform=1/2/1 were collected, and crystallized with ethyl acetate/n-hexane=1/3 to give a pale brown compound (8) (0.09 g, yield 21.0%).
mp 174-176°C
$^1$H NMR ($CDCl_3$) δ: 0.96 (t, J = 7.5 Hz, 3H), 1.35-1.52 (m, 2H), 1.60-1.85 (m, 2H), 3.23-3.40 (m, 8H), 3.77 (s, 3H), 3.93 (s, 3H), 3.99 (m, 1H), 4.92 (d, J = 9.6 Hz, 1H), 6.90 (d, J = 9.0 Hz, 2H), 7.11 (d, J = 2.7 Hz, 1H), 7.16 (dd, J = 2.4, 9.0 Hz, 1H), 7.48 (d, J = 8.7 Hz, 2H), 7.53 (dd, J = 1.8, 8.7 Hz, 1H), 7.69 (d, J = 8.4 Hz, 1H), 7.70 (d, J = 9.0 Hz, 1H), 7.95 (s, 1H).

Step 5

To a solution of the compound (8) (0.08 g, 0.15 mmol) in dimethyl sulfoxide (0.9 mL) was added a 1 mol/L aqueous sodium hydroxide solution (0.9 mL) at room temperature, and the mixture was stirred at 60°C for 14 hours. The reaction mixture was poured into ice water-5% aqueous citric acid solution, and extracted with ethyl acetate. The organic layer was washed with water, dried over anhydrous sodium sulfate, and concentrated under reduced pressure. The residue was recrystallized from acetone/n-hexane to give (S)-2-{4-[4-(6-methoxy-naphthalen-2-ylethynyl)-phenyl]-piperazine-1-sulfonylamino}-pentanoic acid (I-1) (0.07 g, yield 90.0%). mp 187-190°C (dec.)
$^1$H-NMR (DMSO-$d_6$) δ: 0.88 (t, J = 7.5 Hz, 3H), 1.30-1.50 (m, 2H), 1.50-1.65 (m, 2H), 3.05-3.45 (m, 8H), 3.69 (m, 1H), 3.89 (s, 3H), 7.00 (d, J = 9.0 Hz, 2H), 7.21 (dd, J = 2.7, 9.0 Hz, 1H), 7.35 (d, J = 2.7 Hz, 1H), 7.43 (d, J = 8.7 Hz, 2H), 7.52 (dd, J = 1.5, 8.4 Hz, 1H), 7.82 (d, J = 8.4 Hz, 1H), 7.85 (d, J = 9.0 Hz, 1H), 7.91 (d, J = 9.0 Hz, 1H), 8.04 (s, 1H), 12.78 (br s, 1H).

Synthesis of (R)-2-{4-[4-(naphthalen-2-ylmethoxy)-phenyl]-piperazine-1-sulfonylamino}-propionic acid (I-2)

[Chemical formula 41]

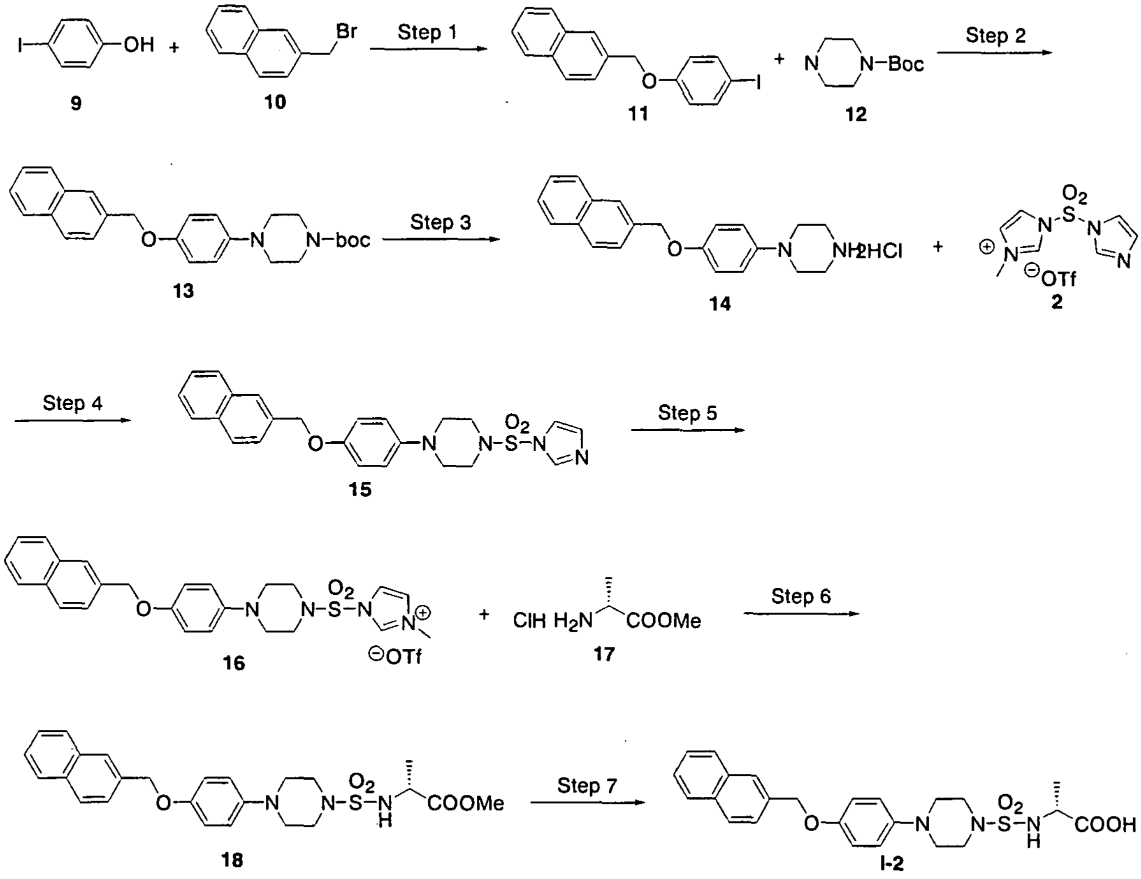


9 10 11 12 13 14 2 15 16 17 18 I-2

Step 1

A solution of *p*-iodophenol (9) (10.0 g, 45.6 mmol) and potassium carbonate (9.42 g, 68.2 mmol) in dimethylformamide (100 mL) was degassed well, and the atmosphere was replaced with a nitrogen gas. Then, 2-bromomethylnaphthalene (10) (11.1 mg, 50.0 mmol) was added, and the mixture was stirred at 70°C for 1 hour. The reaction mixture was cooled to room temperature, and poured into ice-2mol/L hydrochloric acid, and this was stirred for 5 minutes. The precipitated crystal was collected by filtration, and washed with water to give a compound (3) (7.6 g, yield 46%). The mother liquor was extracted with ethyl acetate, and the organic layer was washed successively with water, and aqueous saturated sodium chloride solution, dried over anhydrous sodium sulfate, and concentrated under reduced pressure. The residue was crystallized with tetrahydrofuran/water to obtain a compound (11) (8.6 g, yield 53%). (total amount 16.2 g, total yield 90%) mp 136-137°C

$^1$H NMR ($CDCl_3$) δ: 5.20 (s, 2H), 6.80 (dd, *J* = 2.1 Hz, 9.0 Hz, 2H), 7.48-7.59 (m, 5H), 7.82-7.88 (m, 4H).

Step 2

A solution of a solution of the compound (11) (2.5 g, 9.7 mmol), 1-t-butoxycarbonylpiperazine (12) (10.9 g, 58.3 mmol), sodium t-butoxide (13.1 g, 13.6 mmol) in toluene and N-methylpiperidone (7 mL) was degassed well, and the atmosphere was replaced with a nitrogen gas. Then, palladium acetate (0.11 g, 0.49 mmol) and tri-*t*-butylphosphine (0.47 mL, 1.94 mmol) were added, the atmosphere was replaced again with a nitrogen gas well, and the mixture was stirred at 120 °C for 5 hours. The reaction mixture was filtered through Celite and water was added to the filtrate, which was extracted with ethyl acetate. The organic layer was washed with aqueous saturated sodium chloride, dried over anhydrous sodium sulfate, and concentrated under reduced pressure. The residue was subjected to silica gel chromatography, and the fractions eluted with ethyl acetate/n-hexane=2/3 were collected, and concentrated under reduced

pressure to give a compound (13). (2.6 g, yield 64%) mp 147-149°C
$^1$H NMR ($CDCl_3$) δ: 1.48 (s, 9H), 3.01 (bs, 4H), 3.57 (bs, 4H), 5.19 (s, 2H), 6.91-6.98 (m, 4H), 7.26-7.55 (m, 3H), 7.82-7.88 (m, 4H).

Step 3

To the compound (13) was added a 4mol/L hydrochloric acid solution in ethyl acetate (50 mL), and the mixture was stirred at room temperature for 30 minutes. The reaction mixture was concentrated under reduced pressure, and the residue was dissolved in methanol, and concentrated again under reduced pressure. The residue was washed with ethyl acetate, and collected by filtration to obtain a compound (14). (2.4 g, 99%) mp 200-201°C
$^1$H NMR (DMSO-$d_6$) δ: 3.24 (bs, 8H), 5.22 (s, 2H), 4.98 (s, 4H), 7.51-7.57 (m, 3H), 7.90-7.96 (m, 4H), 9.13 (bs, 2H).

Step 4

Compound (15) was prepared from compound (14) and compound (2) according to Step 1 of Example 1.
$^1$H NMR ($CDCl_3$) δ: 3.15 (t, J = 4.8 Hz, 4H), 3.38 (t, *J* = 4.8 Hz, 4H), 5.19 (s, 2H), 6.88-6.97 (m, 4H), 7.18 (bs, 1H), 7.27 (bs, 1H), 7.47-7.53 (m, 3H), 7.82-7.87 (m, 5H), 7.95 (s, 1H).
Compound (16) was prepared from compound (15) according to Step 2 of Example 1.
$^1$H NMR (DMSO-$d_6$) δ: 3.16 (bs, 4H), 3.51 (bs, 4H), 3.89 (s, 3H), 5.21 (s, 2H), 6.90-6.93 (m, 4H), 7.50-7.54 (m, 3H), 7.90-7.99 (m, 5H), 8.23 (t, J = 1.8 Hz, 1H), 9.85 (s, 1H).

Step 6

Compound (18) was prepared from compound (16) and compound (17) according to Step 3 of Example 1.
$^1$H NMR (DMSO-$d_6$) δ: 1.30 (d, J = 6.8 Hz, 2H), 3.04 (bs, 4H), 3.14 (bs, 4H), 3.65 (s, 3H), 3.83-3.95 (m, 1H), 5.21 (s, 2H), 6.90-6.97 (m, 4H), 7.51-7.57 (m, 3H), 7.91-7.99 (m, 4H), 8.03 (d, J = 8.8 Hz, 1H).

Step 7

(*R*)-2-{4-[4-(naphthalen-2-ylmethoxy)-phenyl]-piperazine-1-sulfonylamino}-propionic acid (I-2) was prepared from compound (18) according to Step 5 of Example 1.
mp 167-171°C
$^1$H NMR (DMSO-$d_6$) δ: 1.30 (d, J=7.2Hz, 3H), 3.01 (bs, 4H), 3.14 (bs, 4H), 3.81 (t, J=7.2Hz, 1H), 5.21 (s, 2H), 6.91-6.98 (m, 4H), 7.51-7.57 (m, 3H), 7.86-7.95 (m, 5H), 12.69 (bs, 1H).
According to the same manner as that described above, the following compounds were synthesized.

Synthesis of (*S*)-2-{4-[4-(naphthalen-2-ylmethoxy)-phenyl]-piperazine-1-sulfonylamino}-propionic acid (I-3)

$^1$H NMR (DMSO-$d_6$) δ: 1.29 (d, J=4.8Hz, 3H), 3.05 (bs, 4H), 3.14 (bs, 4H), 5.21 (s, 2H), 6.92-6.95 (m, 4H), 7.51-7.60 (m, 3H), 7.85-7.95 (m, 5H)

Synthesis of (*R*)-2-{4-[4-(6-methoxy-naphthalen-2-ylethynyl)-phenyl]-piperazine-1-sulfonylamino}-3-methyl-butanoic acid (I-4)

$^1$H NMR (DMSO-$d_6$) δ: 0.80-0.97 (m, 6H), 1.98 (m, 1H), 3.00-3.60 (m, 9H), 3.89 (s, 3H), 7.00 (d, J=8.7 Hz, 2H), 7.21 (m, 1H), 7.35 (m, 1H), 7.43 (d, J = 8.7 Hz, 2H), 7.52 (m, 1H), 7.68-7.90 (m, 3H), 8.04 (s, 1H)

Synthesis of (*S*)-2-{4-[4-(6-methoxy-naphthalen-2-ylethynyl)-phenyl]-piperazine-1-sulfonylamino}-3-methyl-butanoic acid (I-5)

$^1$H NMR (DMSO-$d_6$) δ: 0.91 (d, J=6.6 Hz, 3H), 0.93 (d, J=6.6 Hz, 3H), 1.98 (m, 1H), 3.05-3.35 (m, 8H), 3.50 (m, 1H), 3.89 (s, 3H), 7.00 (d, J=9.0 Hz, 2H), 7.21 (dd, J=2.4, 9.0 Hz, 1H), 7.35 (m, 1H), 7.43 (d, J = 9.3 Hz, 2H), 7.52 (m, 1H), 7.78-7.90 (m, 3H), 8.03 (s, 1H), 12.74 (br s, 1H)

Synthesis of (*S*)-2-{4-[4-(6-methoxy-naphthalen-2-ylethynyl)-phenyl]-piperazine-1-sulfonylamino}-hexanoic acid (I-6)

$^1$H NMR (DMSO-$d_6$) δ: 0.86 (t, J=7.2 Hz, 3H), 1.20-1.44 (m, 4H), 1.50-1.72 (m, 2H), 3.05-3.35 (m, 8H), 3.66 (m, 1H), 3.88 (s, 3H), 6.99 (d, J=9.0 Hz, 2H), 7.20 (dd, J=2.4, 9.0 Hz, 1H), 7.34 (d, J=2.7 Hz, 1H), 7.42 (d, J=8.7 Hz,

2H), 7.51 (dd, J=1.8, 8.4 Hz, 1H), 7.81 (d, J=8.7 Hz, 1H), 7.84 (d, J=9.3 Hz, 1H), 7.90 (d, J=8.1 Hz, 1H), 8.02 (s, 1H), 12.76 (br s, 1H)

Synthesis of (*R*)-2-{4-[4-(naphthalen-2-yloxymethyl)-phenyl]-piperazine-1-sulfonylamino}-propionic acid (I-7)

$^1$H-NMR (DMSO-$d_6$) δ: 1.30 (d, J=8.8Hz, 3H), 3.17-3.20 (m, 8H), 3.80 (bs, - 1H), 5.10 (s, 2H), 6.99 (d, J=7.7Hz, 2H), 7.20 (d, J=9.6Hz, 1H), 7.34-7.47 (m, 5H), 7.78-7.83 (4H, m)

Synthesis of (*R*)-2-{4-[4-(6-methoxy-naphthalen-2-ylethynyl)-phenyl]-piperazine-1-sulfonylamino}-propionic acid (I-8)

$^1$H-NMR (DMSO-$d_6$) δ: 1.30 (d, J=6.9 Hz, 3H), 3.05-3.40 (m, 8H), 3.80 (m, 1H), 3.88 (s, 3H), 6.99 (d, J=8.7 Hz, 2H), 7.20 (dd, J=2.4, 9.0 Hz, 1H), 7.34 (d, J=2.1 Hz, 1H), 7.42 (d, J=8.7 Hz, 2H), 7.51 (dd, J=1.5, 8.1 Hz, 1H), 7.81 (d, J=8.4 Hz, 1H), 7.83 (d, J=9.0 Hz, 1H), 7.91 (d, J=8.1 Hz, 1H), 8.02 (s, 1H), 12.72 (br s, 1H)

Synthesis of (*S*)-2-{4-[4-(6-methoxy-naphthalen-2-ylethynyl)-phenyl]-piperazine-1-sulfonylamino}-4-methyl-pentanoic acid (I-9)

$^1$H-NMR (DMSO-$d_6$) δ: 0.89 (d, J=6.3 Hz, 3H), 0.90 (d, J=6.6 Hz, 3H), 1.35-1.60 (m, 2H), 1.74 (m, 1H), 3.05-3.40 (m, 8H), 3.70 (m, 1H), 3.89 (s, 3H), 7.00 (d, J=9.0 Hz, 2H), 7.20 (dd, J=2.4, 9.0 Hz, 1H), 7.35 (d, J=3.0 Hz, 1H), 7.43 (d, J=9.0 Hz, 2H), 7.52 (m, 1H), 7.82 (d, J=8.7 Hz, 1H), 7.84 (d, J=9.0 Hz, 1H), 7.95 (d, J=8.1 Hz, 1H), 8.03 (s, 1H), 12.76 (br s, 1H)

Synthesis of (*R*)-2-{4-[4-(6-methoxy-naphthalen-2-ylethynyl)-phenyl]-3,6-dihydro-2H-pyridine-1-sulfonylamino}-propionic acid (I-10)

1H-NMR (DMSO-$d_6$) δ: 1.30 (d, J=7.2 Hz, 3H), 2.50-2.60 (m, 2H), 3.20-3.30 (m, 2H), 3.70-3.90 (m, 3H), 3.90 (s, 3H), 6.32 (m, 1H), 7.22 (dd, J=2.4, 8.7 Hz, 1H), 7.37 (d, J=2.4 Hz, 1H), 7.50-7.60 (m, 5H), 7.80-7.95 (m, 3H), 8.10 (s, 1H), 12.71 (br s, 1H)

Synthesis of (*R*)-2-{4-[4-(6-methoxy-naphthalen-2-ylethynyl)-phenyl]-piperidine-1-sulfonylamino}-propionic acid (I-11)

$^1$H-NMR (DMSO-$d_6$) δ: 1.31 (d, J=7.2 Hz, 3H), 1.55-1.75 (m, 2H), 1.75-1.92 (m, 2H), 2.60-2.80 (m, 3H), 3.58-3.70 (m, 2H), 3.79 (m, 1H), 3.90 (s, 3H), 7.22 (dd, J=2.7, 9.3 Hz, 1H), 7.32 (d, J=8.4 Hz, 1H), 7.37 (d, J=2.4 Hz, 2H), 7.52 (d, J=8.4 Hz, 1H), 7.56 (m, 1H), 7.77 (d, J=7.8 Hz, 1H), 7.85 (d, J=8.4 Hz, 1H), 7.87 (d, J=9.3 Hz, 1H), 8.09 (s, 1H), 12.75 (br s, 1H)

Synthesis of (*R*)-2-{4-hydroxy-4-[4-(6-methoxy-naphthalen-2-ylethynyl)-phenyl]-piperidine-1-sulfonylamino}-propionic acid (I-12)

$^1$H-NMR (DMSO-$d_6$) δ: 1.30 (d, J=7.2 Hz, 3H), 1.60-1.75 (m, 2H), 1.85-2.02 (m, 2H), 2.95-3.15 (m, 2H), 3.30-3.50 (m, 2H), 3.79 (m, 1H), 3.89 (s, 3H), 5.18 (s, 1H), 7.21 (dd, J=2.7, 8.7 Hz, 1H), 7.36 (d, J=2.4 Hz, 1H), 7.48-7.58 (m, 5H), 7.74 (d, J=7.2 Hz, 1H), 7.84 (d, J=8.4 Hz, 1H), 7.86 (d, J=8.7 Hz, 1H), 8.08 (s, 1H), 12.69 (br s, 1H)

Synthesis of (*S*)-2-{4-[4-(((E)-naphthalen-2-yl-vinyl)-phenyl]-piperazine-1-sulfonylamino}-pentanoic acid (I-13)

$^1$H-NMR (DMSO-$d_6$) δ: 0.89 (t, J=6.8 Hz, 3H), 1.32-1.44 (m, 2H), 1.54-1.63 (m, 2H), 3.16-3.33 (m, 8H), 3.66-3.74 (m, 1H), 7.00 (d, J=7.2 Hz, 2H), 7.23 (d, J=16.8 Hz, 1H), 7.32 (d, J=16.8 Hz, 1H), 7.43-7.54 (m, 4H), 7.82-7.88 (m, 5H), 7.94(s, 1H), 12.70 (br s, 1H)

Synthesis of (*S*)-2-{4-[4-(2-naphthalen-2-yl-ethyl)-phenyl]-piperazine-1-sulfonylamino}-pentanoic acid (I-14)

$^1$H-NMR (DMSO-$d_6$) δ: 0.88 (t, J=6.8 Hz, 3H), 1.32-1.44 (m, 2H), 1.54-1.63 (m, 2H), 2.87-2.91 (m, 2H), 2.96-3.02 (m, 2H), 3.11 (s, 4H), 3.32 (s, 4H), 3.63-3.71 (m, 1H), 6.86 (d, J=7.2 Hz, 2H), 7.11 (d, J=7.2 Hz, 2H), 7.41-7.48 (m, 3H), 7.70 (s, 1H), 7.81-7.86 (m, 4H), 12.70 (br s, 1H)

Synthesis of (*S*)-2-{4-[4-(6-methoxy-naphthalen-2-ylmethoxy)-phenyl]-piperazine-1-sulfonylamino}-propionic acid (I-15)

$^1$H-NMR (DMSO-$d_6$) δ: 1.20 (d, J = 6.3 Hz, 3H), 3.03 (bs, 4H), 3.16 (bs, 4H), 3.88 (s, 3H), 5.14 (s, 2H), 6.89-6.93

(m, 4H), 7.17 (d, J = 8.8 Hz, 1H), 7.32 (s, 1H), 7.51 (d, J = 8.3 Hz, 1H), 7.85 (d, J = 8.8 Hz, 2H), 7.92 (s, 1H).

Synthesis of (*R*)-2-{4-[4-(6-methoxy-naphthalen-2-ylmethoxy)-phenyl]-piperazine-1-sulfonylamino}-propionic acid (I-16)

$^1$H-NMR (DMSO-$d_6$) δ: 1.20 (d, J = 6.3 Hz, 3H), 3.03 (bs, 4H), 3.16 (bs, 4H), 3.88 (s, 3H), 5.14 (s, 2H), 6.93 (d, J = 7.3 Hz, 4H), 7.17 (d, J = 8.8 Hz, 1H), 7.32 (s, 1H), 7.51 (d, J = 8.3 Hz, 1H), 7.83 (d, J = 8.8 Hz, 2H), 7.87 (s, 1H).

Synthesis of (*S*)-2-{4-[4-(6-methoxy-naphthalen-2-ylethynyl)-phenyl]-piperidine-1-sulfonylamino}-pentanoic acid (I-17)

$^1$H-NMR (DMSO-$d_6$) δ: 0.90 (d, J=7.2 Hz, 3H), 1.30-1.74 (m, 6H), 1.74-1.92 (m, 2H), 2.56-2.80 (m, 3H), 3.58-3.73 (m, 3H), 3.89 (s, 3H), 7.22 (dd, J=2.4, 8.7 Hz, 1H), 7.32 (d, J=8.4 Hz, 1H), 7.37 (d, J=2.1 Hz, 2H), 7.49-7.58 (m, 3H), 7.77 (d, J=9.0 Hz, 1H), 7.85 (d, J=8.4 Hz, 1H), 7.87 (d, J=8.7 Hz, 1H), 8.09 (s, 1H), 12.73 (br s, 1H).

Synthesis of (*R*)-2-{4-fluoro-4-[4-(6-methoxy-naphthalen-2-ylethynyl)-phenyl]-piperidine-1-sulfonylamino}-propionic acid (I-18)

$^1$H-NMR (DMSO-$d_6$) δ: 1.31 (d, J=6.9 Hz, 3H), 1.90-2.30 (m, 4H), 2.85-3.05 (m, 2H), 3.50-3.63 (m, 2H), 3.83 (m, 1H), 3.89 (s, 3H), 7.22 (dd, J=2.4, 9.0 Hz, 1H), 7.36 (d, J=2.4 Hz, 1H), 7.49 (d, J=8.4 Hz, 2H), 7.56 (m, 1H), 7.61 (d, J=8.4 Hz, 1H), 7.80-7.95 (m, 3H), 8.10 (s, 1H), 12.83 (br s, 1H).

Synthesis of (*R*)-2-{4-[4-(6-methoxy-naphthalen-2-ylmethoxy)-phenyl]-piperidine-1-sulfonylamino}-propionic acid (I-19)

$^1$H-NMR (DMSO-$d_6$) δ: 1.30 (d, J = 7.2 Hz, 2H), 1.57 (t, J = 8.8Hz, 2H), 1.78 (t, J = 11.2 Hz, 2H), 2.62-2.73 (m, 2H), 3.60 (bs, 2H), 3.79 (t, J = 7.2 Hz1H), 3.88 (s, 3H), 5.19 (s, 2H), 6.98 (d, J = 8.6 Hz, 2H), 7.16 (t, J = 7.1 Hz, 3H), 7.32 (s, 1H), 7.52 (d, J = 9.1 Hz, 1H), 7.71 (d, J = 9.1 Hz, 1H), 7.83 (d, J = 8.8 Hz, 2H), 7.89 (s, 1H).

Synthesis of (*S*)-2-(4-{4-[2-(6-methoxy-naphthalen-2-yl)-ethyl]-phenyl}-piperidine-1-sulfonylamino}-pentanoic acid (I-20)

$^1$H NMR (DMSO-$d_6$) δ: 0.90 (d, J = 6.6 Hz, 3H), 1.38-1.40 (m, 2H), 1.58-1.61 (m, 4H), 1.75-1.79 (m, 2H), 2.65-2.72 (m, 2H), 2.95 (d, J = 12.1 Hz, 4H), 3.61-3.63 (m, 3H), 3.83 (s, 3H), 7.14-7.17 (m, 5H), 7.27 (s, 1H), 7.38 (d, J = 6.6 Hz, 1H), 7.63 (s, 1H), 7.71 (t, J = 9.7 Hz, 3H).

Synthesis of (*S*)-2-(4-{4-[(*E*)-2-(6-methoxy-naphthalen-2-yl)-vinyl]-phenyl}-piperazine-1-sulfonylamino}-propionic acid (I-21)

$^1$H NMR (DMSO-$d_6$) δ: 1.27 (d, J = 6.8 Hz, 3H), 3.38-3.28 (m, 8H), 3.65 (bs, 1H), 3.86 (s, 3H), 6.98 (d, J = 8.4 Hz, 2H), 7.14-7.16 (m, 1H), 7.20 (d, J=9.6 Hz, 2H), 7.14-7.16 (m, 1H), 7.20 (d, J=9.6 Hz, 2H), 7.29(s, 1H), 7.50 (d, J = 8.4 Hz, 2H), 7.73-7.80(m, 3H), 7.87 (s, 1H).

Synthesis of (*S*)-2-(4-{4-[2-(6-methoxy-naphthalen-2-yl)-ethyl]-phenyl}-piperazine-1-sulfonylamino}-propionic acid (I-22)

$^1$H NMR (DMSO-$d_6$) δ: 1.29 (d, J = 7.6 Hz, 3H), 2.86-2.91 (m, 2H), 2.93-2.95 (m, 2H), 3.12 (bs, 8H), 3.80-3.82 (bs, 1H), 3.85 (s, 3H), 6.86 (d, J = 8.4 Hz, 2H), 7.09 (bs, 3H), 7.26 (s, 1H), 7.35 (d, J=7.2 Hz, 1H), 7.61 (s, 1H), 7.72 (d, J = 9.2Hz, 2H), 7.83-7.84(m, 1H).

Synthesis of (*R*)-2-{4-methoxy-4-[4-(6-methoxy-naphthalen-2-ylethynyl)-phenyl]-piperidine-1-sulfonylamino}-propionic acid (I-23)

$^1$H NMR (DMSO-$d_6$) δ: 1.29 (d, J=6.9 Hz, 3H), 1.80-2.15 (m, 4H), 2.91 (s, 3H), 2.90-3.05 (m, 2H), 3.78 (m, 1H), 3.90 (s, 3H), 7.23 (dd, J=2.7, 9.3 Hz, 1H), 7.38 (d, J=2.4 Hz, 1H), 7.46 (d, J=8.4 Hz, 2H), 7.56 (dd, J=1.8, 8.7 Hz, 1H), 7.60 (d, J=8.4 Hz, 2H), 7.79 (m, 1H), 7.86 (d, J=8.4 Hz, 1H), 7.88 (d, J=8.7 Hz, 1H), 8.11 (s, 1H)

Synthesis of (*S*)-2-(4-{4-[(*E*)-2-(6-methoxy-naphthalen-2-yl)-vinyl]-phenyl}-piperidine-1-sulfonylamino}-pentanoic acid (I-24)

$^1$H NMR (DMSO-$d_6$) δ: 0.88 (t, J=7.2 Hz, 3H), 1.38 (t, J = 7.2 Hz, 2H), 1.58-1.62 (m, 4H), 2.64-2.76 (m, 3H),

3.61-3.64 (m, 2H), 3.69 (s, 3H), 3.77-3.81 (m, 1H), 7.27 (d, J = 8.0 Hz, 2H), 7.38 (s, 2H), 7.47-7.52 (m, 2H), 7.59 (d, J=6.8 Hz, 2H), 7.85-7.92 (m, 4H), 8.00(bs, 1H).

Synthesis of (*S*)-2-{4-[4-((*E*)-2-naphthalen-2-yl-vinyl)-phenyl]-piperidine-1-sulfonylamino)-pentanoic acid (I-25)

$^1$H NMR (DMSO-$d_6$) δ: 0.90 (d, J=7.6 Hz, 3H), 1.36-1.40 (m, 2H), 1.58-1.68 (m, 4H), 1.81-1.84 (m, 2H), 2.64-2.74 (m, 3H), 3.64-3.66 (m, 2H), 7.27 (bs, 2H), 7.38 (d, J=4.4 Hz, 2H), 7.46-7.50 (m, 2H), 7.56-7.60 (m, 2H), 7.69-7.72 (m, 1H), 7.86-7.89 (m,4H), 7.99-8.01 (m, 1H).

Synthesis of (*R*)-2-{4-[4-((E)-2-naphthalen-2-yl-vinyl)-phenyl]-piperidine-1-sulfonylamino)-propionic acid (I-26)

$^1$H NMR (DMSO-$d_6$) δ: 1.31 (d, J=6.8 Hz, 3H), 1.59-1.66 (m, 2H), 1.84-1.87 (m, 2H), 2.57-2.77 (m, 4H), 3.65 (bs, 2H), 3.79 (bs, 1H), 7.28 (d, J=7.2Hz, 2H), 7.38 (s, 2H), 7.49-7.51 (m, 2H), 7.68-7.72 (m, 1H), 7.85-7.90 (m, 4H), 7.99 (bs, 1H).

Synthesis of (*R*)-2-{4-[4-(6-ethoxy-naphthalen-2-ylethynyl)-phenyl]-piperazine-1-sulfonylamino)-propionic acid (I-27)

$^1$H NMR (DMSO-$d_6$) δ: 1.30 (d, J=7.2 Hz, 3H), 1.40 (t, J=6.9 Hz, 3H), 3.05-3.40 (m, 8H), 3.81 (m, 1H), 4.16 (q, J=6.9Hz, 2H), 7.00 (d, J=9.0 Hz, 2H), 7.19 (dd, J=2.7, 9.3 Hz, 1H), 7.33 (d, J=1.8 Hz, 1H), 7.43 (d, J=8.7 Hz, 2H), 7.51 (dd, J=1.5, 8.4 Hz, 1H), 7.76-7.88 (m, 2H), 7.93 (d, J=9.0 Hz, 1H), 8.03 (s, 1H), 12.72 (br s, 1H).

Synthesis of (*S*)-2-{4-[4-(6-ethoxy-naphthalen-2-ylethynyl)-phenyl]-piperazine-1-sulfonylamino)-pentanoic acid (I-28)

$^1$H NMR (DMSO-$d_6$) δ: 0.88 (t, J=7.2 Hz, 3H), 1.40 (t, J=7.2 Hz, 3H), 1.28-1.48 (m, 2H), 1.50-1.68 (m, 2H), 3.05-3.40 (m, 8H), 3.68 (m, 1H), 4.15 (q, J=6.9Hz, 2H), 6.99 (d, J=9.0 Hz, 2H), 7.18 (dd, J=2.4, 9.0 Hz, 1H), 7.32 (d, J=2.4 Hz, 1H), 7.42 (d, J=8.4 Hz, 2H), 7.50 (dd, J=1.5, 8.7 Hz, 1H), 7.76-7.86 (m, 2H), 7.91 (d, J=9.0 Hz, 1H), 8.02 (s, 1H), 12.71 (br s, 1H).

Synthesis of (*R*)-2-{4-[4-(naphthalen-2-ylmethoxy)-phenyl]-piperidine-1-sulfonylamino)-propionic acid (I-29)

$^1$H NMR (DMSO-$d_6$) δ: 1.29-1.31 (m, 3H), 1.57-1.59 (m, 2H), 1.75-1.78 (m, 2H), 2.65-2.77 (m, 2H), 3.60 (bs, 2H), 3.78 (bs, 1H), 6.99 (d, J=6.8 Hz, 2H), 7.15-7.17 (m, 2H), 7.51-7.58 (m, 3H), 7.69-7.74 (m, 4H).

Synthesis of (*S*)-2-{4-[4-(naphthalen-2-ylmethoxy)-phenyl]-piperidine-1-sulfonylamino)-pentanoic acid (I-30)

$^1$H NMR (DMSO-$d_6$) δ: 0.89 (t, J=7.2 Hz, 3H), 1.37-1.40 (m, 2H), 1.53-1.58 (m, 3H), 1.74-1.81 (m, 2H), 2.60-2.72 (m, 2H), 3.59-3.69 (m, 2H), 5.25 (s, 2H), 6.99 (d, J=7.2 Hz, 2H), 7.15 (d, J=7.2 Hz, 2H), 7.51-7.58 (m, 3H), 7.68 (d, J=5.6 Hz, 1H), 7.92-7.97 (m, 4H).

Synthesis of (*S*)-2-{4-[4-(6-methoxymethoxy-naphthalen-2-ylethynyl)-phenyl]-piperazine-1-sulfonylamino)-propionic acid (I-31)

$^1$H NMR (DMSO-$d_6$) δ: 1.30 (d, J=7.2 Hz, 3H), 3.05-3.40 (m, 8H), 3.42 (s, 3H), 3.80 (m, 1H), 5.33 (s, 2H), 7.00 (d, J=8.7 Hz, 2H), 7.28 (dd, J=2.1, 8.4 Hz, 1H), 7.36-7.56 (m, 4H), 7.81 (d, J=8.7 Hz, 1H), 7.87 (d, J=9.0 Hz, 1H), 7.92 (m, 1H), 8.04 (s, 1H), 12.70 (br s, 1H).

Synthesis of (*R*)-2-{4-[4-(6-cyclopropylmethoxy-naphthalen-2-ylethynyl)-phenyl]-piperazine-1-sulfonylamino)-propionic acid (I-32)

$^1$H NMR (DMSO-$d_6$) δ: 0.32-0.42 (m, 2H), 0.56-0.66 (m, 2H), 1.30 (d, J=7.2 Hz, 3H), 3.05-3.40 (m, 8H), 3.81 (m, 1H), 3.95 (d, J=6.9 Hz, 2H), 7.00 (d, J=9.0 Hz, 2H), 7.22 (dd, J=2.1, 8.7 Hz, 1H), 7.36 (d, J=2.4 Hz, 1H), 7.43 (d, J=9.0 Hz, 2H), 7.50 (dd, J=1.2, 8.7 Hz, 1H), 7.79 (d, J=8.4 Hz, 1H), 7.84 (d, J=9.3 Hz, 1H), 7.93 (d, J=8.7 Hz, 1H), 8.02 (m, 1H), 12.72 (br s, 1H).

Synthesis of (*R*)-2-{4-[4-(6-hydroxy-naphthalen-2-ylethynyl)-phenyl]-piperazine-1-sulfonylamino)-propionic acid (I-33)

$^1$H NMR (DMSO-$d_6$) δ: 1.30 (d, J=6.9 Hz, 3H), 3.05-3.40 (m, 8H), 3.81 (m, 1H), 7.00 (d, J=8.7 Hz, 2H), 7.08-7.16 (m, 2H), 7.38-7.48 (m, 3H), 7.69 (d, J=9.0 Hz, 1H), 7.78 (d, J=9.0 Hz, 1H), 7.93 (d, J=8.1 Hz, 1H), 7.97 (s, 1H), 9.94 (s,

1H), 12.75 (br s, 1H).

Synthesis of (*R*)-2-{4-[4-(naphthalen-2-ylmethylsulfanyl)-phenyl]-piperazine-1-sulfonylamino)-propionic acid (I-34)

$^1$H NMR (DMSO-$d_6$) δ: 1.28 (d, J=6.8 Hz, 3H), 3.32 (bs, 8H), 3.79 (bs, 1H), 4.24 (s, 2H), 6.86 (d, J=8.4 Hz, 2H), 7.22 (d, J=8.4 Hz, 2H), 7.46-7.47 (m, 3H), 7.70 (s, 1H), 7.79-7.85 (m, 4H).

Synthesis of (*R*)-2-{4-[4-(naphthalen-2-ylmethanesulfinyl)-phenyl]-piperazine-1-sulfonylamino)-propionic acid (I-35)

$^1$H NMR (DMSO-$d_6$) δ: 1.30 (d, J=6.8 Hz, 3H), 3.14-3.17 (m, 4H), 3.79-3.83 (m, 1H), 4.20 (d, J=8.4 Hz, 1H), 4.33 (d, J=8.4 Hz, 1H), 7.06 (d, J=8.8 Hz, 2H), 7.24 (d, J=8.0 Hz, 1H), 7.39 (d, J=8.8 Hz, 2H), 7.47-7.52 (m, 2H), 7.65 (s, 1H), 7.81 (d, J=7.2 Hz, 2H), 7.89 (bs, 2H).

Synthesis of (*R*)-2-{4-[4-(6-hydroxy-naphthalen-2-ylethynyl)-phenyl]-piperazine-1-sulfonylamino)-propionic acid (I-36)

$^1$H NMR (DMSO-$d_6$) δ: 1.31 (d, J=6.8 Hz, 3H), 1.60-1.65 (m, 2H), 1.81-1.84 (m, 2H), 2.62-2.76 (m, 3H), 3.64 (bs, 2H), 3.80 (bs, 1H), 7.14 (d, J=8.0 Hz, 2H), 7.31 (d, J=8.0 Hz, 2H), 7.46-7.52 (m, 3H), 7.71 (d, J=8.8 Hz, 2H), 7.79 (d, J=8.8 Hz, 1H), 8.02 (bs, 1H).

Synthesis of (*R*)-2-{4-[4-(naphthalen-2-ylmethanesulfonyl)-phenyl]-4-oxy-piperazine-1-sulfonylamino)-propionic acid (I-37)

$^1$H NMR (DMSO-$d_6$) δ: 1.28 (d, J=7.2 Hz, 3H), 3.24 (d, J=9.2 Hz, 2H), 3.53 (t, J=13.6 Hz, 2H), 3.70-3.82 (m, 3H), 4.00-4.06 (m, 2H), 4.94 (s, 2H), 7.31 (d, J=8.4 Hz, 2H), 7.53 (d, J=4.0 Hz, 2H), 7.69 (s, 1H), 7.82-7.92 (m, 6H), 8.38 (d, J=8.4 Hz, 2H).

Synthesis of (*R*)-2-{4-[4-(naphthalen-2-ylmethanesulfonyl)-phenyl]-piperazine-1-sulfonylamino)-propionic acid (I-38)

$^1$H NMR (DMSO-$d_6$) δ: 1.30 (d, J=7.2 Hz, 3H), 3.09-3.17 (m, 4H), 3.37-3.39 (m, 4H), 3.77-3.83 (m, 1H), 4.70 (s, 2H), 7.02 (d, J=8.8 Hz, 2H), 7.27 (d, J=8.8 Hz, 1H), 7.47-7.53 (m, 4H), 7.69 (bs, 1H), 7.82 (d, J=8.0 Hz, 2H), 7.88-7.93 (m, 4H).

Synthesis of (*R*)-2-(4-{4-[6-(2-hydroxy-ethoxy)-naphthalen-2-ylethynyl]-phenyl}-piperazine-1-sulfonylamino)-propionic acid (I-39)

$^1$H NMR (DMSO-$d_6$) δ: 1.30 (d, J=7.2 Hz, 3H), 3.10-3.40 (m, 8H), 3.74-3.86 (m, 3H), 4.12 (t, J=5.4 Hz, 2H), 4.94 (m, 1H), 7.10 (d, J=9.0 Hz, 2H), 7.22 (dd, J=2.4, 9.3 Hz, 1H), 7.35 (d, J=2.4 Hz, 1H), 7.43 (d, J=8.7 Hz, 2H), 7.51 (d, J=8.7 Hz, 1H), 7.79-7.88 (m, 2H), 7.92 (d, J=7.2 Hz, 1H), 8.03 (s, 1H), 12.77 (br s, 1H).

Synthesis of (*R*)-2-{(*R*)-3-methyl-4-[4-(naphthalen-2-ylmethoxy)-phenyl]-piperazine-1-sulfonylamino)-propionic acid (I-40)

$^1$H NMR (DMSO-$d_6$) δ: 0.85 (d, J=5.6 Hz, 3H), 1.32 (d, J=6.8 Hz, 3H), 2.96-2.98 (m, 2H), 3.08 (bs, 3H), 3.66 (bs, 1H), 3.82 (bs, 1H), 5.20 (s, 2H), 6.94 (dd, J=8.0 Hz, 9.2 Hz, 4H), 7.52-7.58 (m, 3H), 7.79 (bs, 1H), 7.92-7.94 (m, 4H).

Synthesis of (*R*)-2-{(*2R*,*6S*)-2,6-dimethyl-4-[4-(naphthalen-2-ylmethoxy)-phenyl]-piperazine-1-sulfonylamino)-propionic acid (I-41)

$^1$H NMR (DMSO-$d_6$) δ: 1.27 (d, J=7.2 Hz, 3H), 1.40 (t, J=6.4 Hz, 6H), 2.97-2.71 (m, 2H), 3.62-3.66 (m, 1H), 3.91 (bs, 2H), 5.22 (s, 2H), 6.93 (dd, J=8.8 Hz, 9.2 Hz, 4H), 7.51-7.58 (m, 4H), 7.91-7.96 (m, 4H).

Synthesis of (*R*)-2-(4-{4-[4-(naphthalene-2-carbonyl)-amino]-phenyl}-piperazine-1-sulfonylamino)-propionic acid (I-42)

$^1$H NMR (DMSO-$d_6$) δ: 1.31 (d, J=6.8 Hz, 3H), 3.18 (s, 8H), 3.80-3.85 (m, 1H), 6.99 (d, J=8.0 Hz, 2H), 7.61-7.71 (m, 4H), 7.91 (d, J=8.4 Hz, 1H), 7.99-8.09 (m, 4H), 8.56 (s, 1H), 10.27 (s, 1H), 12.73 (br s, 1H).

Synthesis of (*R*)-2-{4-[4-(methyl-naphthalen-2-ylmehyl-amino)-phenyl]-piperazine-1-sulfonylamino}-propionic acid (I-43)

$^1$H NMR (DMSO-$d_6$) δ: 1.29 (d, J=6.8 Hz, 3H), 2.96 (s, 7H), 3.12 (s, 4H), 3.77-3.83 (m, 1H), 4.70 (s, 2H), 6.72 (d, J=7.6 Hz, 2H), 6.83 (d, J=7.6 Hz, 2H), 7.38 (d, J=8.0 Hz, 1H), 7.46-7.48 (m, 2H), 7.70 (s, 1H), 7.82-7.87 (m, 4H), 12.69 (br s, 1H).

Synthesis of (*R*)-2-(4-{4-[4-(naphthalen-2-ylmehyl)-amino]-phenyl}-piperazine-1-sulfonylamino)-propionic acid (I-44)

$^1$H NMR (DMSO-$d_6$) δ: 1.28 (d, J=6.8 Hz, 3H), 2.93 (s, 4H), 3.10 (s, 4H), 3.76-3.80 (m, 1H), 4.38 (s, 2H), 6.56 (d, J=7.6 Hz, 2H), 6.73 (d, J=7.6 Hz, 2H), 7.46-7.53 (m, 3H), 7.81-7.87 (m, 5H), 12.61 (br s, 1H).

Synthesis of (*R*)-2-(4-{4-[4-(6-methoxy-naphthalen-2-ylmehyl)-amino]-phenyl}-piperazine-1-sulfonylamino)-propionic acid (I-45)

$^1$H NMR (DMSO-$d_6$) δ: 1.28 (d, J=6.8 Hz, 3H), 2.92 (s, 4H), 3.10 (s, 4H), 3.76-3.80 (m, 1H), 3.85 (s, 3H), 4.32 (s, 2H), 6.54 (d, J=7.6 Hz, 2H), 6.72 (d, J=7.6 Hz, 2H), 7.12 (d, J=8.6 Hz, 1H), 7.28 (s, 1H), 7.46 (d, J=8.6 Hz, 1H), 7.76 (s, 3H), 7.82 (d, J=7.6 Hz, 1H), 12.12 (br s, 1H).

Synthesis of (*R*)-2-(4-{4-[4-(6-carboxymethoxy-naphthalen-2-ylethynyl)-phenyl]-piperazine-1-sulfonylamino)-propionic acid (I-46)

$^1$H NMR (DMSO-$d_6$) δ: 1.30 (d, J=6.6 Hz, 3H), 3.05-3.40 (m, 8H), 3.81 (m, 1H), 4.82 (s, 2H), 7.00 (d, J=8.4 Hz, 2H), 7.20-7.32 (m, 2H), 7.43 (d, J=8.1 Hz, 2H), 7.51 (d, J=8.7 Hz, 1H), 7.80 (d, J=8.4 Hz, 1H), 7.87 (d, J=9.3 Hz, 1H), 7.93 (m, 1H), 8.04 (s, 1H), 12.40-13.40 (m, 2H).

Synthesis of (*R*)-2-(4-{4-[4-(6-methoxy-naphthalen-2-ylethynyl)-phenyl]-2-methyl-piperazine-1-sulfonylamino)-propionic acid (I-47)

$^1$H NMR (DMSO-$d_6$) δ: 1.22-1.32 (m, 6H), 2.65-3.30 (m, 4H), 3.55-3.80 (m, 3H), 3.89 (s, 3H), 3.95 (m, 1H), 6.97 (d, J=9.0 Hz, 2H), 7.21 (dd, J=2.7, 9.0 Hz, 1H), 7.35 (d, J=2.1 Hz, 1H), 7.42 (d, J=8.7 Hz, 2H), 7.52 (dd, J=1.5, 8.7 Hz, 1H), 7.82 (d, J=8.4 Hz, 1H), 7.84 (d, J=9.3 Hz, 1H), 7.92 (m, 1H), 8.03 (s, 1H), 12.72 (br s, 1H).

Synthesis of (*R*)-2-(4-{4-[4-(6-methoxy-naphthalen-2-ylethynyl)-phenyl]-[1,4]diazepane-1-sulfonylamino)-propionic acid (I-48)

$^1$H NMR (DMSO-$d_6$) δ: 1.25 (d, J=7.1 Hz, 3H), 3.22-3.61 (m, 11H), 3.93 (s, 3H), 6.81-6.84 (m, 2H), 7.23-7.26 (m, 1H), 7.39-7.42 (m, 3H), 7.52-7.64 (m, 2H), 7.81-7.91 (m, 2H), 8.03 (br s, 1H).

Synthesis of (*S*)-2-(4-{4-[(*E*)-2-(6-methoxymethoxy-naphthalen-2-yl)-vinyl]-phenyl}-piperazine-1-sulfonylamino)-pentanoic acid (I-49)

$^1$H NMR (DMSO-$d_6$) δ: 0.89 (t, J=6.8 Hz, 3H), 1.33-1.43 (m, 2H), 1.55-1.62 (m, 2H), 3.14-3.26 (m, 8H), 3.44-3.71 (m, 4H), 5.32 (s, 2H), 7.00 (d, J=8.4 Hz, 2H), 7.17-7,28 (m, 3H), 7.42 (s, 1H), 7.51 (d, J=8.4 Hz, 2H), 7.78 (s, 2H), 7.82-7.92 (m, 3H).

$^1$H NMR (DMSO-$d_6$) δ: 0.89 (t, J=6.8 Hz, 3H), 1.33-1.43 (m, 2H), 1.55-1.62 (m, 2H), 3.14-3.26 (m, 8H), 3.67-3.71 (m, 1H), 6.98 (d, J=8.0 Hz, 2H), 7.05-7.23 (m, 4H), 7.49 (d, J=8.4 Hz, 2H), 7.64-7.73 (m, 3H) 7.81 (s, 1H), 7.90 (d, J=8.8 Hz, 1H), 9.75 (s, 1H), 12.73 (s, br s, 1H).

Synthesis of (*S*)-2-(4-{4-[(*E*)-2-(6-methoxy-naphthalen-2-yl)-vinyl]-phenyl}-[1,4]diazepane-1-sulfonylamino)-pentanoic acid (I-51)

$^1$H NMR (DMSO-$d_6$) δ: 0.84-0.87 (m, 3H), 1.25-1.35 (m, 3H), 1.48-1.58 (m, 2H), 1.84-1.94 (m, 2H), 3.10-3.63 (m, 11H), 3.89 (s, 3H), 6.77-6.80 (m, 2H), 7.18-7.22 (m, 1H), 7.35-7.38 (m, 3H), 7.49-7.51 (m, 1H), 7.60-7.63 (m, 1H), 7.80-7.85 (m, 2H), 8.00 (br s, 1H).

Synthesis of (*R*)-2-{4-[4-(6-ethoxy-naphthalen-2-ylmethoxy)-phenyl]-piperazine-1-sulfonylamino}-propionic acid (I-52)

$^1$H NMR (DMSO-$d_6$) δ: 1.29 (d, J=8.0 Hz, 3H), 1.39 (t, J=8.0 Hz, 3H), 3.04-3.17 (m, 8H), 3.77-3.81 (m, 1H), 4.14 (dd, J=8.0 Hz, 12.0 Hz, 2H), 5.14 (s, 2H), 6.89-6.95 (m, 4H), 7.15 (d, J=8.4 Hz, 1H), 7.30 (s, 1H), 7.49 (d, J=8.0 Hz, 1H), 7.79-7.86 (m, 4H).

Synthesis of (*R*)-2-{(*S*)-4-[4-(6-methoxy-naphthalen-2-ylethynyl)-phenyl]-3-methylpiperazine-1-sulfonylamino}-propionic acid (I-53)

$^1$H NMR (DMSO-$d_6$) δ: 1.02 (d, J=6.3 Hz, 3H), 1.28-1.35 (m, 3H), 2.60-3.10 (m, 4H), 3.45-3.60 (m, 2H), 3.85 (m, 1H), 3.89 (s, 3H), 4.25 (m, 1H), 6.94 (d, J=8.7 Hz, 2H), 7.21 (dd, J=2.7, 9.0 Hz, 1H), 7.35 (d, J=2.1 Hz, 1H), 7.42 (d, J=8.7 Hz, 2H), 7.52 (d, J=9.0 Hz, 1H), 7.78-7.88 (m, 2H), 8.00 (m, 1H), 8.03 (s, 1H), 12.70 (br s, 1H).

Synthesis of (*R*)-2-(4-{4-[6-((*E*)-3-hydroxy-buta-1,3-dienyl)-naphthalen-2-ylethynyl)-phenyl]-piperazine-1-sulfonylamino}-propionic acid (I-55)

$^1$H NMR (DMSO-$d_6$) δ: 1.30 (d, J=7.2 Hz, 3H), 3.13-3.33 (m, 8H), 3.81 (br s, 1H), 6.69 (d, J=15.9 Hz, 1H), 7.02 (d, J=8.7 Hz, 2H), 7.46 (d, J=8.7 Hz, 2H), 7.59-7.62 (m, 1H), 7.74 (d, J=15.9 Hz, 1H), 7.90-7.97 (m, 4H), 8.13 (s, 1H), 8.20 (s, 1H), 12.6(br s, 1H).

Synthesis of (*R*)-2-{4-[4-(6-acetylamino-naphthalen-2-ylethynyl)-phenyl]-piperazine-1-sulfonylamino}-propionic acid (I-56)

$^1$H NMR (DMSO-$d_6$) δ: 1.31 (d, J=7.2 Hz, 3H), 2.12 (s, 3H), 3.14-3.22 (m, 4H), 3.79-3.84 (m, 1H), 7.00 (d, J=8.8 Hz, 2H), 7.44 (d, J=8.4 Hz, 2H), 7.49-7.65 (m, 3H), 7.80-7.92 (m, 2H), 8.02 (s, 1H), 8.30 (s, 1H).

Synthesis of (*R*)-2-{4-[4-(6-amino-naphthalen-2-ylethynyl)-phenyl]-piperazine-1-sulfonylamino}-propionic acid (I-57)

$^1$H NMR (DMSO-$d_6$) δ: 1.30 (d, J=7.2 Hz, 3H), 2.54 (s, 2H), 3.15-3.17 (m, 4H), 3.80-3.84 (m, 1H), 6.95-7.00 (m, 3H), 7.40 (d, J=8.4 Hz, 2H), 7.49 (d, J=8.4 Hz, 1H), 7.60 (d, J=8.8 Hz, 1H), 7.81 (bs, 1H), 7.90 (d, J=8.4 Hz, 1H).

Synthesis of (*R*)-2-[4-(4-{2-[6-(2-carboxy-ethyl)-naphthalen-2-yl]ethyl}-phenyl)-piperazine-1-sulfonylamino]-propionic acid (I-58)

$^1$H NMR (DMSO-$d_6$) δ: 1.29 (d, J=7.2 Hz, 3H), 2.59-2.65 (m, 2H), 2.84-2.91 (m, 2H), 2.94-2.99 (m, 4H), 3.07-3.17 (m, 8H), 3.79 (br s, 1H),6.86 (d, J=8.4 Hz, 2H), 7.10 (d, J=8.4 Hz, 2H),7.34-7.40 (m, 2H), 7.63-7.67 (m, 2H), 7.71-7.76 (m, 2H), 7.86 (br s, 1H).

Synthesis of (*R*)-2-{4-[4-(6-dimethylcarbamoylmethoxy-naphthalen-2-ylethynyl)-phenyl]-piperazine-1-sulfonylamino}]-propionic acid (I-59)

$^1$H NMR (DMSO-$d_6$) δ: 1.30 (d, J=6.8 Hz, 3H), 2.87 (s, 3H), 3.05 (s, 3H), 3.13-3.22 (m, 4H), 3.25-3.31 (m, 4H), 3.78-3.82 (m, 1H), 4.94 (s, 2H), 7.00 (d, J=8.4 Hz, 2H), 7.23-7.29 (m, 2H), 7.43 (d, J=8.4 Hz, 2H), 7.51 (d, J=8.4 Hz, 1H), 7.78 (d, J=8.8 Hz, 1H), 7.84-7.91(m, 2H), 8.03 (s, 1H).

Synthesis of 6-{4-[4-((*R*)-1-carboxy-ethylsulfamoyl)-piperazin-1-yl]-phenylethynyl}-naphthalene-2-carboxylic acid (I-60)

$^1$H NMR (DMSO-$d_6$) δ: 1.30 (d, J=7.5 Hz, 3H), 3.05-3.40 (m, 8H), 3.81 (m, 1H), 7.02 (d, J=8.7 Hz, 2H), 7.47 (d, J=8.7 Hz, 2H), 7.65 (d, J=8.4 Hz, 1H), 7.92 (m, 1H), 8.01 (s, 2H), 8.14 (d, J=9.0 Hz, 1H), 8.19 (s, 1H), 8.61 (s, 1H), 13.00 (m, 1H).

Synthesis of (*R*)-2-[4-(4-naphthalen-2-ylethynyl-phenyl)-piperazine-1-sulfonylamino]-propionic acid (I-61)

$^1$H NMR (DMSO-$d_6$) δ: 1.30 (d, J=7.2 Hz, 3H), 3.08-3.35 (m, 8H), 3.81 (m, 1H), 7.02 (d, J=9.0 Hz, 2H), 7.46 (d, J=8.7 Hz, 2H), 7.52-7.60 (m, 3H), 7.88-7.98 (m, 4H), 8.12 (s, 1H), 12.69 (br s, 1H).

Synthesis of (R)-2-{4-[4-(6-methanesulfonylamino-naphthalen-2-ylethynyl)-phenyl]-piperazine-1-sulfonylamino]-propionic acid (I-62)

$^1$H NMR (DMSO-$d_6$) δ: 1.31 (d, J=7.2 Hz, 3H), 3.09 (s, 3H), 3.16-3.20 (m, 4H), 3.30 (bs, 4H), 7.01 (d, J=8.8 Hz, 2H), 7.41-7.45 (m, 3H), 7.54 (d, J=8.4 Hz, 1H), 7.69 (s, 1H), 7.84-7.92 (m, 3H), 8.05 (s, 1H).

Synthesis of (*S*)-2-(4-{4-[(*E*)-2-(6-carboxymethoxy-naphthalen-2-yl)-vinyl]-phenyl}-piperazine-1-sulfonylamino]-pentanoic acid (I-63)

$^1$H NMR (DMSO-$d_6$) δ: 0.84-0.91 (m, 3H), 1.34-1.44 (m, 2H), 1.57-1.64 (m, 2H), 3.16-3.24 (m, 8H), 3.70-3.73 (m, 1H), 4.80 (s, 2H), 7.15-7.25 (m, 4H), 7.50 (d, J=8.4 Hz, 2H), 7.50 (d, J=8.4 Hz, 2H), 7.77-7.88 (m, 5H).

Synthesis of (*R*)-2-(4-{4-[6-(2-carboxy-ethyl)-naphthalen-2-ylethynyl]-phenyl}-piperazine-1-sulfonylamino]-propionic acid (I-64)

$^1$H NMR (DMSO-$d_6$) δ: 1.29 (d, J=7.0 Hz, 3H), 2.64 (t, J=7.5 Hz, 2H), 2.99 (t, J=7.5 Hz, 2H), 3.10-3.42 (m, 8H), 3.79 (br s, 1H), 6.99 (d, J=8.7 Hz, 2H), 7.41-7.47 (m, 3H), 7.51 (d, J=7.6 Hz, 1H), 7.72 (s, 1H), 7.83 (d, J=8.7 Hz, 2H), 7.88 (br s, 1H), 8.05 (s, 1H).

Synthesis of (*R*-)-2-(4-{4-[6-(methoxyimino-methyl)-naphthalen-2-ylethynyl]-phenyl}-piperazine-1-sulfonylamino]-propionic acid (I-66)

$^1$H NMR (DMSO-$d_6$) δ: 1.30 (d, J=7.2 Hz, 3H), 3.10-3.40 (m, 8H), 3.80 (m, 1H), 3.95 (s, 3H), 7.02 (d, J=9.0 Hz, 2H), 7.46 (d, J=8.7 Hz, 2H), 7.61 (d, J=8.7 Hz, 1H), 7.80-8.20 (m, 4H), 8.10 (d, J=12.6 Hz, 1H), 8.38 (s, 1H).

Synthesis of (*R*)-2-{4-[4-(6-morpholin-4-yl-naphthalen-2-ylethynyl)-phenyl]-piperazine-1-sulfonylamino}-propionic acid (I-67)

$^1$H NMR (DMSO-$d_6$) δ: 1.29 (d, J=7.2 Hz, 3H), 3.15-3.20 (m, 4H), 3.75-3.79 (m, 4H), 6.99 (d, J=8.4 Hz, 2H), 7.19 (s, 1H), 7.41-7.48 (m, 4H), 7.71 (d, J=8.0 Hz, 2H), 7.94 (s, 1H).

Synthesis of (*R*)-2-{4-[4-(6-dimethylcarbamoyl-naphthalen-2-ylethynyl)-phenyl]-piperazine-1-sulfonylamino}-propionic acid (I-68)

$^1$H NMR (DMSO-$d_6$) δ: 1.30 (d, J=7.2 Hz, 3H), 2.98 (s, 3H), 3.04 (s, 3H), 3.12-3.40 (m, 8H), 3.80 (br s, 1H), 7.02 (d, J=8.9 Hz, 2H), 7.46 (d, J=8.9 Hz, 2H), 7.53-7.58 (m, 1H), 7.60-7.66 (m, 1H), 7.90 (br s, 1H), 7.96-8.02 (m, 3H), 8.16 (s, 1H).

Synthesis of (*R*)-2-{4-[4-(4-cyano-6-methoxymethoxy-naphthalen-2-ylethynyl)-phenyl]-piperazine-1-sulfonylamino}-propionic acid (I-83)

$^1$H NMR (DMSO-$d_6$) δ: 1.31 (d, J=6.8 Hz, 3H), 3.15-3.21 (m, 4H), 3.46 (s, 3H), 3.82 (bs, 1H), 5.42 (s, 2H), 7.02 (d, J=8.8 Hz, 2H), 7.45-7.48 (m, 3H), 7.45-7.48 (m, 3H), 7.56 (s, 1H), 7.91 (bs, 1H), 8.08 (d, J=8.8, 1H), 8.20 (s, 1H), 8.41 (s, 1H).

Synthesis of (*R*)-2-{4-[4-(4-cyano-6-hydroxy-naphthalen-2-ylethynyl)-phenyl]-piperazine-1-sulfonylamino}-propionic acid (I-84)

$^1$H NMR (DMSO-$d_6$) δ: 1.30 (d, J=6.8 Hz, 3H), 3.14-3.17 (m, 4H), 3.82 (bs, 1H), 7.01 (d, J=7.6 Hz, 2H), 7.28 (d, J=8.0 Hz, 1H), 7.36 (s, 1H), 7.44 (d, J=7.6 Hz, 2H), 7.99 (d, J=8.8, 1H), 8.12 (s; 1H), 8.34 (s, 1H).

Synthesis of 3-{4-[4-((*R*)-1-carboxy-ethylsulfamoyl)-piperazin-1-yl]-phenylethynyl}-naphthalene-2-carboxylic acid (I-97)

$^1$H NMR (DMSO-$d_6$) δ: 1.32 (d, J=6.8 Hz, 3H), 3.18 (bs, 8H), 3.81-3.85 (m, 1H), 7.03 (d, J=8.0 Hz, 2H), 7.43 (d, J=8.4 Hz, 2H), 7.61-7.69 (m, 2H), 7.91 (d, J=8.0 Hz, 1H), 7.99 (d, J=8.0 Hz, 1H), 8.08 (d, J=8.4 Hz, 1H), 8.22 (s, 1H), 8.52 (s, 1H).

Synthesis of (*R*)-2-{4-[4-(6-acetyl-naphthalen-2-ylethynyl)-phenyl]-piperazine-1-sulfonylamino}-propionic acid (I-116)

$^1H$ NMR (DMSO-$d_6$) δ: 1.30 (d, J=7.2 Hz, 3H), 2.71 (s, 3H), 3.05-3.40 (m, 8H), 3.81 (m, 1H), 7.03 (d, J=9.0 Hz, 2H), 7.47 (d, J=9.0 Hz, 2H), 7.67 (d, J=1.5, 8.4 Hz, 1H), 7.92 (m, 1H), 7.96-8.06 (m, 2H), 8.15 (d, J=8.4 Hz, 1H), 8.19(s, 1H), 8.68 (s, 1H), 12.77 (br s, 1H).

Synthesis of (*R*)-2-{4-[4-(9H-carbazol-3-ylethynyl)-phenyl]-piperazine-1-sulfonylamino}-proplonic acid (I-129)

$^1H$ NMR (DMSO-$d_6$) δ: 1.30 (d, J=7.2 Hz, 3H), 3.15-3.21 (m, 4H), 3.82 (bs, 1H), 6.99 (d, J=8.8 Hz, 2H), 7.19 (d, J=7.2 Hz, 1H), 7.42 (d, J=8.8 Hz, 3H), 7.49-7.51 (m, 1H), 8.18 (d, J=8.4 Hz, 1H), 8.32 (s, 1H).

Synthesis of (*S*)-2-[4-(4-naphthalen-2-ylethynyl-phenyl)-piperazine-1-sulfonylamino]-pentanoic acid (I-132)

$^1H$ NMR (DMSO-$d_6$) δ: 0.88 (t, J=6.9 Hz, 3H), 1.30-1.70 (m, 4H), 3.05-3.40 (m, 8H), 3.69 (m, 1H), 7.02 (d, J=9.0 Hz, 2H), 7.46 (d, J=8.7 Hz, 2H), 7.52-7.60 (m, 3H), 7.86-8.00 (m, 4H), 8.12 (s, 1H), 12.77 (br s, 1H).

Synthesis of (*R*)-2-{4-[4-(4-cyano-6-methoxy-naphthalen-2-ylethynyl)-phenyl]-piperazine-1-sulfonylamino}-propionic acid (I-133)

$^1H$ NMR (DMSO-$d_6$) δ: 1.31 (d, J=7.2 Hz, 3H), 3.15-3.20 (m, 4H), 3.81(t, J=6.0 Hz, 1H), 3.98 (s, 3H), 7.02 (d, J=8.4 Hz, 2H), 7.34 (s, 1H), 7.40 (d, J=9.2 Hz, 1H), 7.45 (d, J=8.4 Hz, 1H), 7.92 (d, J=9.2 Hz, 1H), 8.05 (d, J=9.2 Hz, 1H), 8.20 (s, 1H), 8.41(s, 1H).

According to the same manner as that described above, in the compounds represented by the general formula (I'):

[Chemical formula 42]

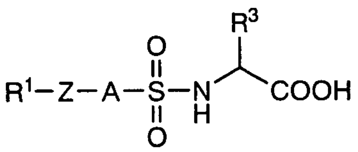

(I')

wherein $R^1$ is a group represented by:

[Chemical formula 43]

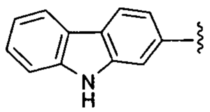
,
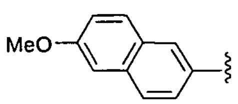
,
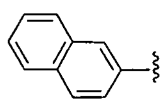
,

(a1) (a2) (a3)

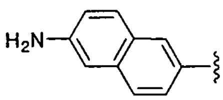
,
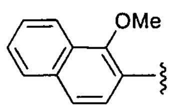
, or
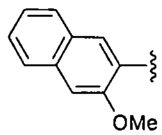

(a4) (a5) (a6)

;

Z is: (b1)-$CH_2O$-, (b2) -$OCH_2$-, (b3) -$NHCH_2$-, (b4) -$CH_2NH$-, (b5) -$CH_2S$-, (b6)- $CH_2SO$-, (b7) -$CH_2SO_2$-, (b8) -$SCH_2$-,

(b9) $-SOCH_2-$, (b10) $-SO_2CH_2-$, (b11) -C(=O)C(=O)-, (b12) $-CH_2CH_2-$, (b 13) -CH=CH-, (b14) -C≡C-, or (b15) -NH-C(=O)-;
A is a group represented by:

[Chemical formula 44]

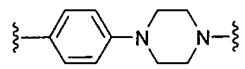
,
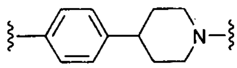
,
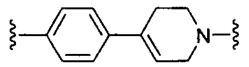
,
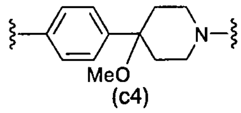
,

(c1) (c2) (c3) (c4)

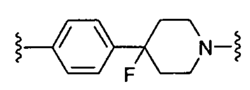
,
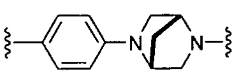
,
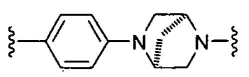
,
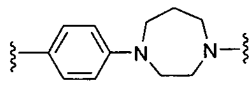
,

(c5) (c6) (c7) (c8)

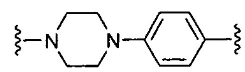
,
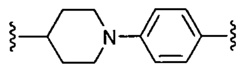
,
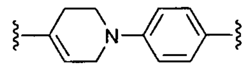
,

(c9) (c10) (c11)

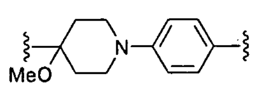
, or
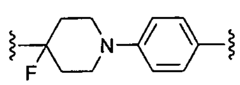

(c12) (c13)

;

$R^3$ is (d1) benzyl, (d2) indol-3-ylmethyl, (d3) isobutyl, (d4) methyl, (d5) 2-methylthioethyl, (d6) isopropyl, (d7) sec-butyl, (d8) t-butyl, (d9) n-propyl, (d10) n-butyl, (d11) hydroxymethyl, or (d12) 4-hydroxybenzyl, ($R^1$, Z, A, $R^3$) = (a1, b1, c1, d1), (a1, b1, c1, d2), (a1, b1, c1, d3), (a1, b1, c1, d4), (a1, b1, c1, d5), (a1, b1, c1, d6), (a1, b1, c1, d7), (a1, b1, c1, d8), (a1, b1, c1, d9), (a1, b1, c1, d10), (a1, b1, c1, d11), (a1, b1, c1, d12), (a1, b1, c2, d1), (a1, b1, c2, d2), (a1, b1, c2, d3), (a1, b1, c2, d4), (a1, b1, c2, d5), (a1, b1, c2, d6), (a1, b1, c2, d7), (a1, b1, c2, d8), (a1, b1, c2, d9), (a1, b1, c2, d10), (a1, b1, c2, d11) (a1, b1, c2, d12), (a1, b1, c3, d1), (a1, b1, c3, d2), (a1, b1, c3, d3), (a1, b1, c3, d4), (a1, b1, c3, d5), (a1, b1, c3, d6), (a1, b1, c3, d7), (a1, b1, c3, d8), (a1, b1, c3, d9), (a1, b1, c3, d10), (a1, b1, c3, d11), (a1, b1, c3, d12), (a1, b1, c4, d1), (a1, b1, c4, d2), (a1, b1, c4, d3), (a1, b1, c4, d4), (a1, b1, c4, d5), (a1, b1, c4, d6), (a1, b1, c4, d7), (a1, b1, c4, d8), (a1, b1, c4, d9), (a1, b1, c4, d10), (a1, b1, c4, d11), (a1, b1, c4, d12), (a1, b1, c5, d1), (a1, b1, c5, d2), (a1, b1, c5, d3), (a1, b1, c5, d4), (a1, b1, c5, d5), (a1, b1, c5, d6), (a1, b1, c5, d7), (a1, b1, c5, d8), (a1, b1, c5, d9), (a1, b1, c5, d10), (a1, b1, c5, d11), (a1, b1, c5, d12), (a1, b1, c6, d1), (a1, b1, c6, d2), (a1, b1, c6, d3), (a1, b1, c6, d4), (a1, b1, c6, d5), (a1, b1, c6, d6), (a1, b1, c6, d7), (a1, b1, c6, d8), (a1, b1, c6, d9), (a1, b1, c6, d10), (a1, b1, c6, d11), (a1, b1, c6, d12), (a1, b1, c7, d1), (a1, b1, c7, d2), (a1, b1, c7, d3), (a1, b1, c7, d4), (a1, b1, c7, d5), (a1, b1, c7, d6), (a1, b1, c7, d7), (a1, b1, c7, d8), (a1, b1, c7, d9), (a1, b1, c7, d10), (a1, b1, c7, d11), (a1, b1, c7, d12), (a1, b1, c8, d1), (a1, b1, c8, d2), (a1, b1, c8, d3), (a1, b1, c8, d4), (a1, b1, c8, d5), (a1, b1, c8, d6), (a1, b1, c8, d7), (a1, b1, c8, d8), (a1, b1, c8, d9), (a1, b1, c8, d10), (a1, b1, c8, d11), (a1, b1, c8, d12), (a1, b1, c9, d1), (a1, b1, c9, d2), (a1, b1, c9, d3), (a1, b1, c9, d4), (a1, b1, c9, d5), (a1, b1, c9, d6), (a1, b1, c9, d7), (a1, b1, c9, d8), (a1, b1, c9, d9), (a1, b1, c9, d10), (a1, b1, c9, d11), (a1, b1, c9, d12), (a1, b1, c10, d1), (a1, b1, c10, d2), (a1, b1, c10, d3), (a1, b1, c10, d4), (a1, b1, c10, d5), (a1, b1, c10, d6), (a1, b1, c10, d7), (a1, b1, c10, d8), (a1, b1, c10, d9), (a1, b1, c10, d10), (a1, b1, c10, d11), (a1, b1, c10, d12), (a1, b1, c11, d1), (a1, b1, c11, d2), (a1, b1, c11, d3), (a1, b1, c11, d4), (a1, b1, c11, d5), (a1, b1, c11, d6), (a1, b1, c11, d7), (a1, b1, c11, d8), (a1, b1, c11, d9), (a1, b1, c11, d10), (a1, b1, c11, d11), (a1, b1, c11, d12), (a1, b1, c12, d1), (a1, b1, c12, d2), (a1, b1, c12, d3), (a1, b1, c12, d4), (a1, b1, c12, d5), (a1, b1, c12, d6), (a1, b1, c12, d7), (a1, b1, c12, d8), (a1, b1, c12, d9), (a1, b1, c12, d10), (a1, b1, c12, d11), (a1, b1, c12, d12), (a1, b1, c13, d1), (a1, b1, c13, d2), (a1, b1, c13, d3), (a1, b1, c13, d4), (a1, b1, c13, d5), (a1, b1, c13, d6), (a1, b1, c13, d7), (a1, b1, c13, d8), (a1, b1, c13, d9), (a1, b1, c13, d10), (a1, b1, c13, d11), (a1, b1, c13, d12), (a1, b2, c1, d1), (a1, b2, c1, d2), (a1, b2, c1, d3), (a1, b2, c1, d4), (a1, b2, c1, d5), (a1, b2, c1, d6), (a1, b2, c1, d7), (a1, b2, c1, d8), (a1, b2, c1, d9), (a1, b2, c1, d10), (a1, b2, c1, d11), (a1, b2, c1, d12), (a1, b2, c2, d1), (a1, b2, c2, d2), (a1, b2, c2, d3), (a1, b2, c2, d4), (a1, b2, c2, d5), (a1, b2, c2, d6), (a1, b2, c2, d7), (a1, b2, c2, d8), (a1, b2, c2, d9), (a1, b2, c2, d10), (a1, b2, c2, d11), (a1, b2, c2, d12), (a1, b2, c3, d1), (a1, b2, c3, d2), (a1, b2, c3, d3), (a1, b2, c3, d4), (a1, b2, c3, d5), (a1, b2, c3, d6), (a1, b2, c3, d7), (a1, b2, c3,

d8), (a1, b2, c3, d9), (a1, b2, c3, d10), (a1, b2, c3, d11), (a1, b2, c3, d12), (a1, b2, c4, d1), (a1, b2, c4, d2), (a1, b2, c4, d3), (a1, b2, c4, d4), (a1, b2, c4, d5), (a1, b2, c4, d6), (a1, b2, c4, d7), (a1, b2, c4, d8), (a1, b2, c4, d9), (a1, b2, c4, d10), (a1, b2, c4, d11), (a1, b2, c4, d12), (a1, b2, c5, d1), (a1, b2, c5, d2), (a1, b2, c5, d3), (a1, b2, c5, d4), (a1, b2, c5, d5), (a1, b2, c5, d6), (a1, b2, c5, d7), (a1, b2, c5, d8), (a1, b2, c5, d9), (a1, b2, c5, d10), (a1, b2, c5, d11), (a1, b2, c5, d12), (a1, b2, c6, d1), (a1, b2, c6, d2), (a1, b2, c6, d3), (a1, b2, c6, d4), (a1, b2, c6, d5), (a1, b2, c6, d6), (a1, b2, c6, d7), (a1, b2, c6, d8), (a1, b2, c6, d9), (a1, b2, c6, d10), (a1, b2, c6, d11), (a1, b2, c6, d12), (a1, b2, c7, d1), (a1, b2, c7, d2), (a1, b2, c7, d3), (a1, b2, c7, d4), (a1, b2, c7, d5), (a1, b2, c7, d6), (a1, b2, c7, d7), (a1, b2, c7, d8), (a1, b2, c7, d9), (a1, b2, c7, d10), (a1, b2, c7, d11), (a1, b2, c7, d12), (a1, b2, c8, d1), (a1, b2, c8, d2), (a1, b2, c8, d3), (a1, b2, c8, d4), (a1, b2, c8, d5), (a1, b2, c8, d6), (a1, b2, c8, d7), (a1, b2, c8, d8), (a1, b2, c8, d9), (a1, b2, c8, d10), (a1, b2, c8, d11), (a1, b2, c8, d12), (a1, b2, c9, d1), (a1, b2, c9, d2), (a1, b2, c9, d3), (a1, b2, c9, d4), (a1, b2, c9, d5), (a1, b2, c9, d6), (a1, b2, c9, d7), (a1, b2, c9, d8), (a1, b2, c9, d9), (a1, b2, c9, d10), (a1, b2, c9, d11), (a1, b2, c9, d12), (a1, b2, c10, d1), (a1, b2, c10, d2), (a1, b2, c10, d3), (a1, b2, c10, d4), (a1, b2, c10, d5), (a1, b2, c10, d6), (a1, b2, c10, d7), (a1, b2, c10, d8), (a1, b2, c10, d9), (a1, b2, c10, d10), (a1, b2, c10, d11), (a1, b2, c10, d12), (a1, b2, c11, d1), (a1, b2, c11, d2), (a1, b2, c11, d3), (a1, b2, c11, d4), (a1, b2, c11, d5), (a1, b2, c11, d6), (a1, b2, c11, d7), (a1, b2, c11, d8), (a1, b2, c11, d9), (a1, b2, c11, d10), (a1, b2, c11, d11), (a1, b2, c11, d12), (a1, b2, c12, d1), (a1, b2, c12, d2), (a1, b2, c12, d3), (a1, b2, c12, d4), (a1, b2, c12, d5), (a1, b2, c12, d6), (a1, b2, c12, d7), (a1, b2, c12, d8), (a1, b2, c12, d9), (a1, b2, c12, d10), (a1, b2, c12, d11), (a1, b2, c12, d12), (a1, b2, c13, d1), (a1, b2, c13, d2), (a1, b2, c13, d3), (a1, b2, c13, d4), (a1, b2, c13, d5), (a1, b2, c13, d6), (a1, b2, c13, d7), (a1, b2, c13, d8), (a1, b2, c13, d9), (a1, b2, c13, d10), (a1, b2, c13, d11), (a1, b2, c13, d12), (a1, b3, c1, d1), (a1, b3, c1, d2), (a1, b3, c1, d3), (a1, b3, c1, d4), (a1, b3, c1, d5), (a1, b3, c1, d6), (a1, b3, c1, d7), (a1, b3, c1, d8), (a1, b3, c1, d9), (a1, b3, c1, d10), (a1, b3, c1, d11), (a1, b3, c1, d12), (a1, b3, c2, d1), (a1, b3, c2, d2), (a1, b3, c2, d3), (a1, b3, c2, d4), (a1, b3, c2, d5), (a1, b3, c2, d6), (a1, b3, c2, d7), (a1, b3, c2, d8), (a1, b3, c2, d9), (a1, b3, c2, d10), (a1, b3, c2, d11), (a1, b3, c2, d12), (a1, b3, c3, d1), (a1, b3, c3, d2), (a1, b3, c3, d3), (a1, b3, c3, d4), (a1, b3, c3, d5), (a1, b3, c3, d6), (a1, b3, c3, d7), (a1, b3, c3, d8), (a1, b3, c3, d9), (a1, b3, c3, d10), (a1, b3, c3, d11), (a1, b3, c3, d12), (a1, b3, c4, d1), (a1, b3, c4, d2), (a1, b3, c4, d3), (a1, b3, c4, d4), (a1, b3, c4, d5), (a1, b3, c4, d6), (a1, b3, c4, d7), (a1, b3, c4, d8), (a1, b3, c4, d9), (a1, b3, c4, d10), (a1, b3, c4, d11), (a1, b3, c4, d12), (a1, b3, c5, d1), (a1, b3, c5, d2), (a1, b3, c5, d3), (a1, b3, c5, d4), (a1, b3, c5, d5), (a1, b3, c5, d6), (a1, b3, c5, d7), (a1, b3, c5, d8), (a1, b3, c5, d9), (a1, b3, c5, d10), (a1, b3, c5, d11), (a1, b3, c5, d12), (a1, b3, c6, d1), (a1, b3, c6, d2), (a1, b3, c6, d3), (a1, b3, c6, d4), (a1, b3, c6, d5), (a1, b3, c6, d6), (a1, b3, c6, d7), (a1, b3, c6, d8), (a1, b3, c6, d9), (a1, b3, c6, d10), (a1, b3, c6, d11), (a1, b3, c6, d12), (a1, b3, c7, d1), (a1, b3, c7, d2), (a1, b3, c7, d3), (a1, b3, c7, d4), (a1, b3, c7, d5), (a1, b3, c7, d6), (a1, b3, c7, d7), (a1, b3, c7, d8), (a1, b3, c7, d9), (a1, b3, c7, d10), (a1, b3, c7, d11), (a1, b3, c7, d12), (a1, b3, c8, d1), (a1, b3, c8, d2), (a1, b3, c8, d3), (a1, b3, c8, d4), (a1, b3, c8, d5), (a1, b3, c8, d6), (a1, b3, c8, d7), (a1, b3, c8, d8), (a1, b3, c8, d9), (a1, b3, c8, d10), (a1, b3, c8, d11), (a1, b3, c8, d12), (a1, b3, c9, d1), (a1, b3, c9, d2), (a1, b3, c9, d3), (a1, b3, c9, d4), (a1, b3, c9, d5), (a1, b3, c9, d6), (a1, b3, c9, d7), (a1, b3, c9, d8), (a1, b3, c9, d9), (a1, b3, c9, d10), (a1, b3, c9, d11), (a1, b3, c9, d12), (a1, b3, c10, d1), (a1, b3, c10, d2), (a1, b3, c10, d3), (a1, b3, c10, d4), (a1, b3, c10, d5), (a1, b3, c10, d6), (a1, b3, c10, d7), (a1, b3, c10, d8), (a1, b3, c10, d9), (a1, b3, c10, d10), (a1, b3, c10, d11), (a1, b3, c10, d12), (a1, b3, c11, d1), (a1, b3, c11, d2), (a1, b3, c11, d3), (a1, b3, c11, d4), (a1, b3, c11, d5), (a1, b3, c11, d6), (a1, b3, c11, d7), (a1, b3, c11, d8), (a1, b3, c11, d9), (a1, b3, c11, d10), (a1, b3, c11, d11), (a1, b3, c11, d12), (a1, b3, c12, d1), (a1, b3, c12, d2), (a1, b3, c12, d3), (a1, b3, c12, d4), (a1, b3, c12, d5), (a1, b3, c12, d6), (a1, b3, c12, d7), (a1, b3, c12, d8), (a1, b3, c12, d9), (a1, b3, c12, d10), (a1, b3, c12, d11), (a1, b3, c12, d12), (a1, b3, c13, d1), (a1, b3, c13, d2), (a1, b3, c13, d3), (a1, b3, c13, d4), (a1, b3, c13, d5), (a1, b3, c13, d6), (a1, b3, c13, d7), (a1, b3, c13, d8), (a1, b3, c13, d9), (a1, b3, c13, d10), (a1, b3, c13, d11), (a1, b3, c13, d12), (a1, b4, c1, d1), (a1, b4, c1, d2), (a1, b4, c1, d3), (a1, b4, c1, d4), (a1, b4, c1, d5), (a1, b4, c1, d6), (a1, b4, c1, d7), (a1, b4, c1, d8), (a1, b4, c1, d9), (a1, b4, c1, d10), (a1, b4, c1, d11), (a1, b4, c1, d12), (a1, b4, c2, d1), (a1, b4, c2, d2), (a1, b4, c2, d3), (a1, b4, c2, d4), (a1, b4, c2, d5), (a1, -b4, c2, d6), (a1, b4, c2, d7), (a1, b4, c2, d8), (a1, b4, c2, d9), (a1, b4, c2, d10), (a1, b4, c2, d11), (a1, b4, c2, d12), (a1, b4, c3, d1), (a1, b4, c3, d2), (a1, b4, c3, d3), (a1, b4, c3, d4), (a1, b4, c3, d5), (a1, b4, c3, d6), (a1, b4, c3, d7), (a1, b4, c3, d8), (a1, b4, c3, d9), (a1, b4, c3, d10), (a1, b4, c3, d11), (a1, b4, c3, d12), (a1, b4, c4, d1), (a1, b4, c4, d2), (a1, b4, c4, d3), (a1, b4, c4, d4), (a1, b4, c4, d5), (a1, b4, c4, d6), (a1, b4, c4, d7), (a1, b4, c4, d8), (a1, b4, c4, d9), (a1, b4, c4, d10), (a1, b4, c4, d11), (a1, b4, c4, d12), (a1, b4, c5, d1), (a1, b4, c5, d2), (a1, b4, c5, d3), (a1, b4, c5, d4), (a1, b4, c5, d5), (a1, b4, c5, d6), (a1, b4, c5, d7), (a1, b4, c5, d8), (a1, b4, c5, d9), (a1, b4, c5, d10), (a1, b4, c5, d11), (a1, b4, c5, d12), (a1, b4, c6, d1), (a1, b4, c6, d2), (a1, b4, c6, d3), (a1, b4, c6, d4), (a1, b4, c6, d5), (a1, b4, c6, d6), (a1, b4, c6, d7), (a1, b4, c6, d8), (a1, b4, c6, d9), (a1, b4, c6, d10), (a1, b4, c6, d11), (a1, b4, c6, d12), (a1, b4, c7, d1), (a1, b4, c7, d2), (a1, b4, c7, d3), (a1, b4, c7, d4), (a1, b4, c7, d5), (a1, b4, c7, d6), (a1, b4, c7, d7), (a1, b4, c7, d8), (a1, b4, c7, d9), (a1, b4, c7, d10), (a1, b4, c7, d11), (a1, b4, c7, d12), (a1, b4, c8, d1), (a1, b4, c8, d2), (a1, b4, c8, d3), (a1, b4, c8, d4), (a1, b4, c8, d5), (a1, b4, c8, d6), (a1, b4, c8, d7), (a1, b4, c8, d8), (a1, b4, c8, d9), (a1, b4, c8, d10), (a1, b4, c8, d11), (a1, b4, c8, d12), (a1, b4, c9, d1), (a1, b4, c9, d2), (a1, b4, c9, d3), (a1, b4, c9, d4), (a1, b4, c9, d5), (a1, b4, c9, d6), (a1, b4, c9, d7), (a1, b4, c9, d8), (a1, b4, c9, d9), (a1, b4, c9, d10), (a1, b4, c9, d11), (a1, b4, c9, d12), (a1, b4, c10, d1), (a1, b4, c10, d2), (a1, b4, c10, d3), (a1, b4, c10, d4), (a1, b4, c10, d5), (a1, b4, c10, d6), (a1, b4, c10, d7), (a1, b4, c10, d8), (a1, b4, c10, d9), (a1, b4, c10, d10), (a1, b4, c10, d11), (a1, b4, c10, d12), (a1, b4, c11, d1), (a1,

b4, c11, d2), (a1, b4, c11, d3), (a1, b4, c11, d4), (a1, b4, c11, d5), (a1, b4, c11, d6), (a1, b4, c11, d7), (a1, b4, c11, d8), (a1, b4, c11, d9), (a1, b4, c11, d10), (a1, b4, c11, d11), (a1, b4, c11, d12), (a1, b4, c12, d1), (a1, b4, c12, d2), (a1, b4, c12, d3), (a1, b4, c12, d4), (a1, b4, c12, d5), (a1, b4, c12, d6), (a1, b4, c12, d7), (a1, b4, c12, d8), (a1, b4, c12, d9), (a1, b4, c12, d10), (a1, b4, c12, d11), (a1, b4, c12, d12), (a1, b4, c13, d1), (a1, b4, c13, d2), (a1, b4, c13, d3), (a1, b4, c13, d4), (a1, b4, c13, d5), (a1, b4, c13, d6), (a1, b4, c13, d7), (a1, b4, c13, d8), (a1, b4, c13, d9), (a1, b4, c13, d10), (a1, b4, c13, d11), (a1, b4, c13, d12), (a1, b5, c1, d1), (a1, b5, c1, d2), (a1, b5, c1, d3), (a1, b5, c1, d4), (a1, b5, c1, d5), (a1, b5, c1, d6), (a1, b5, c1, d7), (a1, b5, c1, d8), (a1, b5, c1, d9), (a1, b5, c1, d10), (a1, b5, c1, d11), (a1, b5, c1, d12), (a1, b5, c2, d1), (a1, b5, c2, d2), (a1, b5, c2, d3), (a1, b5, c2, d4), (a1, b5, c2, d5), (a1, b5, c2, d6), (a1, b5, c2, d7), (a1, b5, c2, d8), (a1, b5, c2, d9), (a1, b5, c2, d10), (a1, b5, c2, d11), (a1, b5, c2, d12), (a1, b5, c3, d1), (a1, b5, c3, d2), (a1, b5, c3, d3), (a1, b5, c3, d4), (a1, b5, c3, d5), (a1, b5, c3, d6), (a1, b5, c3, d7), (a1, b5, c3, d8), (a1, b5, c3, d9), (a1, b5, c3, d10), (a1, b5, c3, d11), (a1, b5, c3, d12), (a1, b5, c4, d1), (a1, b5, c4, d2), (a1, b5, c4, d3), (a1, b5, c4, d4), (a1, b5, c4, d5), (a1, b5, c4, d6), (a1, b5, c4, d7), (a1, b5, c4, d8), (a1, b5, c4, d9), (a1, b5, c4, d10), (a1, b5, c4, d11), (a1, b5, c4, d12), (a1, b5, c5, d1), (a1, b5, c5, d2), (a1, b5, c5, d3), (a1, b5, c5, d4), (a1, b5, c5, d5), (a1, b5, c5, d6), (a1, b5, c5, d7), (a1, b5, c5, d8), (a1, b5, c5, d9), (a1, b5, c5, d10), (a1, b5, c5, d11), (a1, b5, c5, d12), (a1, b5, c6, d1), (a1, b5, c6, d2), (a1, b5, c6, d3), (a1, b5, c6, d4), (a1, b5, c6, d5), (a1, b5, c6, d6), (a1, b5, c6, d7), (a1, b5, c6, d8), (a1, b5, c6, d9), (a1, b5, c6, d10), (a1, b5, c6, d11), (a1, b5, c6, d12), (a1, b5, c7, d1), (a1, b5, c7, d2), (a1, b5, c7, d3), (a1, b5, c7, d4), (a1, b5, c7, d5), (a1, b5, c7, d6), (a1, b5, c7, d7), (a1, b5, c7, d8), (a1, b5, c7, d9), (a1, b5, c7, d10), (a1, b5, c7, d11), (a1, b5, c7, d12), (a1, b5, c8, d1), (a1, b5, c8, d2), (a1, b5, c8, d3), (a1, b5, c8, d4), (a1, b5, c8, d5), (a1, b5, c8, d6), (a1, b5, c8, d7), (a1, b5, c8, d8), (a1, b5, c8, d9), (a1, b5, c8, d10), (a1, b5, c8, d11), (a1, b5, c8, d12), (a1, b5, c9, d1), (a1, b5, c9, d2), (a1, b5, c9, d3), (a1, b5, c9, d4), (a1, b5, c9, d5), (a1, b5, c9, d6), (a1, b5, c9, d7), (a1, b5, c9, d8), (a1, b5, c9, d9), (a1, b5, c9, d10), (a1, b5, c9, d11), (a1, b5, c9, d12), (a1, b5, c10, d1), (a1, b5, c10, d2), (a1, b5, c10, d3), (a1, b5, c10, d4), (a1, b5, c10, d5), (a1, b5, c10, d6), (a1, b5, c10, d7), (a1, b5, c10, d8), (a1, b5, c10, d9), (a1, b5, c10, d10), (a1, b5, c10, d11), (a1, b5, c10, d12), (a1, b5, c11, d1), (a1, b5, c11, d2), (a1, b5, c11, d3), (a1, b5, c11, d4), (a1, b5, c11, d5), (a1, b5, c11, d6), (a1, b5, c11, d7), (a1, b5, c11, d8), (a1, b5, c11, d9), (a1, b5, c11, d10), (a1, b5, c11, d11), (a1, b5, c11, d12), (a1, b5, c12, d1), (a1, b5, c12, d2), (a1, b5, c12, d3), (a1, b5, c12, d4), (a1, b5, c12, d5), (a1, b5, c12, d6), (a1, b5, c12, d7), (a1, b5, c12, d8), (a1, b5, c12, d9), (a1, b5, c12, d10), (a1, b5, c12, d11), (a1, b5, c12, d12), (a1, b5, c13, d1), (a1, b5, c13, d2), (a1, b5, c13, d3), (a1, b5, c13, d4), (a1, b5, c13, d5), (a1, b5, c13, d6), (a1, b5, c13, d7), (a1, b5, c13, d8), (a1, b5, c13, d9), (a1, b5, c13, d10), (a1, b5, c13, d11), (a1, b5, c13, d12), (a1, b6, c1, d1), (a1, b6, c1, d2), (a1, b6, c1, d3), (a1, b6, c1, d4), (a1, b6, c1, d5), (a1, b6, c1, d6), (a1, b6, c1, d7), (a1, b6, c1, d8), (a1, b6, c1, d9), (a1, b6, c1, d10), (a1, b6, c1, d11), (a1, b6, c1, d12), (a1, b6, c2, d1), (a1, b6, c2, d2), (a1, b6, c2, d3), (a1, b6, c2, d4), (a1, b6, c2, d5), (a1, b6, c2, d6), (a1, b6, c2, d7), (a1, b6, c2, d8), (a1, b6, c2, d9), (a1, b6, c2, d10), (a1, b6, c2, d11), (a1, b6, c2, d12), (a1, b6, c3, d1), (a1, b6, c3, d2), (a1, b6, c3, d3), (a1, b6, c3, d4), (a1, b6, c3, d5), (a1, b6, c3, d6), (a1, b6, c3, d7), (a1, b6, c3, d8), (a1, b6, c3, d9), (a1, b6, c3, d10), (a1, b6, c3, d11), (a1, b6, c3, d12), (a1, b6, c4, d1), (a1, b6, c4, d2), (a1, b6, c4, d3), (a1, b6, c4, d4), (a1, b6, c4, d5), (a1, b6, c4, d6), (a1, b6, c4, d7), (a1, b6, c4, d8), (a1, b6, c4, d9), (a1, b6, c4, d10), (a1, b6, c4, d11), (a1, b6, c4, d12), (a1, b6, c5, d1), (a1, b6, c5, d2), (a1, b6, c5, d3), (a1, b6, c5, d4), (a1, b6, c5, d5), (a1, b6, c5, d6), (a1, b6, c5, d7), (a1, b6, c5, d8), (a1, b6, c5, d9), (a1, b6, c5, d10), (a1, b6, c5, d11), (a1, b6, c5, d12), (a1, b6, c6, d1), (a1, b6, c6, d2), (a1, b6, c6, d3), (a1, b6, c6, d4), (a1, b6, c6, d5), (a1, b6, c6, d6), (a1, b6, c6, d7), (a1, b6, c6, d8), (a1, b6, c6, d9), (a1, b6, c6, d10), (a1, b6, c6, d11), (a1, b6, c6, d12), (a1, b6, c7, d1), (a1, b6, c7, d2), (a1, b6, c7, d3), (a1, b6, c7, d4), (a1, b6, c7, d5), (a1, b6, c7, d6), (a1, b6, c7, d7), (a1, b6, c7, d8), (a1, b6, c7, d9), (a1, b6, c7, d10), (a1, b6, c7, d11), (a1, b6, c7, d12), (a1, b6, c8, d1), (a1, b6, c8, d2), (a1, b6, c8, d3), (a1, b6, c8, d4), (a1, b6, c8, d5), (a1, b6, c8, d6), (a1, b6, c8, d7), (a1, b6, c8, d8), (a1, b6, c8, d9), (a1, b6, c8, d10), (a1, b6, c8, d11), (a1, b6, c8, d12), (a1, b6, c9, d1), (a1, b6, c9, d2), (a1, b6, c9, d3), (a1, b6, c9, d4), (a1, b6, c9, d5), (a1, b6, c9, d6), (a1, b6, c9, d7), (a1, b6, c9, d8), (a1, b6, c9, d9), (a1, b6, c9, d10), (a1, b6, c9, d11), (a1, b6, c9, d12), (a1, b6, c10, d1), (a1, b6, c10, d2), (a1, b6, c10, d3), (a1, b6, c10, d4), (a1, b6, c10, d5), (a1, b6, c10, d6), (a1, b6, c10, d7), (a1, b6, c10, d8), (a1, b6, c10, d9), (a1, b6, c10, d10), (a1, b6, c10, d11), (a1, b6, c10, d12), (a1, b6, c11, d1), (a1, b6, c11, d2), (a1, b6, c11, d3), (a1, b6, c11, d4), (a1, b6, c11, d5), (a1, b6, c11, d6), (a1, b6, c11, d7), (a1, b6, c11, d8), (a1, b6, c11, d9), (a1, b6, c11, d10), (a1, b6, c11, d11), (a1, b6, c11, d12), (a1, b6, c12, d1), (a1, b6, c12, d2), (a1, b6, c12, d3), (a1, b6, c12, d4), (a1, b6, c12, d5), (a1, b6, c12, d6), (a1, b6, c12, d7), (a1, b6, c12, d8), (a1, b6, c12, d9), (a1, b6, c12, d10), (a1, b6, c12, d11), (a1, b6, c12, d12), (a1, b6, c13, d1), (a1, b6, c13, d2), (a1, b6, c13, d3), (a1, b6, c13, d4), (a1, b6, c13, d5), (a1, b6, c13, d6), (a1, b6, c13, d7), (a1, b6, c13, d8), (a1, b6, c13, d9), (a1, b6, c13, d10), (a1, b6, c13, d11), (a1, b6, c13, d12), (a1, b7, c1, d1), (a1, b7, c1, d2), (a1, b7, c1, d3), (a1, b7, c1, d4), (a1, b7, c1, d5), (a1, b7, c1, d6), (a1, b7, c1, d7), (a1, b7, c1, d8), (a1, b7, c1, d9), (a1, b7, c1, d10), (a1, b7, c1, d11), (a1, b7, c1, d12), (a1, b7, c2, d1), (a1, b7, c2, d2), (a1, b7, c2, d3), (a1, b7, c2, d4), (a1, b7, c2, d5), (a1, b7, c2, d6), (a1, b7, c2, d7), (a1, b7, c2, d8), (a1, b7, c2, d9), (a1, b7, c2, d10), (a1, b7, c2, d11), (a1, b7, c2, d12), (a1, b7, c3, d1), (a1, b7, c3, d2), (a1, b7, c3, d3), (a1, b7, c3, d4), (a1, b7, c3, d5), (a1, b7, c3, d6), (a1, b7, c3, d7), (a1, b7, c3, d8), (a1, b7, c3, d9), (a1, b7, c3, d10), (a1, b7, c3, d11), (a1, b7, c3, d12), (a1, b7, c4, d1), (a1, b7, c4, d2), (a1, b7, c4, d3), (a1, b7, c4, d4), (a1, b7, c4, d5), (a1, b7, c4, d6), (a1, b7, c4, d7), (a1, b7, c4, d8), (a1, b7, c4, d9), (a1, b7, c4, d10), (a1, b7, c4, d11), (a1, b7, c4, d12), (a1, b7, c5, d1), (a1, b7, c5, d2), (a1, b7, c5, d3), (a1, b7, c5, d4), (a1, b7, c5, d5),

(a1, b7, c5, d6), (a1, b7, c5, d7), (a1, b7, c5, d8), (a1, b7, c5, d9), (a1, b7, c5, d10), (a1, b7, c5, d11), (a1, b7, c5, d12), (a1, b7, c6, d1), (a1, b7, c6, d2), (a1, b7, c6, d3), (a1, b7, c6, d4), (a1, b7, c6, d5), (a1, b7, c6, d6), (a1, b7, c6, d7), (a1, b7, c6, d8), (a1, b7, c6, d9), (a1, b7, c6, d10), (a1, b7, c6, d11), (a1, b7, c6, d12), (a1, b7, c7, d1), (a1, b7, c7, d2), (a1, b7, c7, d3), (a1, b7, c7, d4), (a1, b7, c7, d5), (a1, b7, c7, d6), (a1, b7, c7, d7), (a1, b7, c7, d8), (a1, b7, c7, d9), (a1, b7, c7, d10), (a1, b7, c7, d11), (a1, b7, c7, d12), (a1, b7, c8, d1), (a1, b7, c8, d2), (a1, b7, c8, d3), (a1, b7, c8, d4), (a1, b7, c8, d5), (a1, b7, c8, d6), (a1, b7, c8, d7), (a1, b7, c8, d8), (a1, b7, c8, d9), (a1, b7, c8, d10), (a1, b7, c8, d11), (a1, b7, c8, d12), (a1, b7, c9, d1), (a1, b7, c9, d2), (a1, b7, c9, d3), (a1, b7, c9, d4), (a1, b7, c9, d5), (a1, b7, c9, d6), (a1, b7, c9, d7), (a1, b7, c9, d8), (a1, b7, c9, d9), (a1, b7, c9, d10), (a1, b7, c9, d11), (a1, b7, c9, d12), (a1, b7, c10, d1), (a1, b7, c10, d2), (a1, b7, c10, d3), (a1, b7, c10, d4), (a1, b7, c10, d5), (a1, b7, c10, d6), (a1, b7, c10, d7), (a1, b7, c10, d8), (a1, b7, c10, d9), (a1, b7, c10, d10), (a1, b7, c10, d11), (a1, b7, c10, d12), (a1, b7, c11, d1), (a1, b7, c11, d2), (a1, b7, c11, d3), (a1, b7, c11, d4), (a1, b7, c11, d5), (a1, b7, c11, d6), (a1, b7, c11, d7), (a1, b7, c11, d8), (a1, b7, c11, d9), (a1, b7, c11, d10), (a1, b7, c11, d11), (a1, b7, c11, d12), (a1, b7, c12, d1), (a1, b7, c12, d2), (a1, b7, c12, d3), (a1, b7, c12, d4), (a1, b7, c12, d5), (a1, b7, c12, d6), (a1, b7, c12, d7), (a1, b7, c12, d8), (a1, b7, c12, d9), (a1, b7, c12, d10), (a1, b7, c12, d11), (a1, b7, c12, d12), (a1, b7, c13, d1), (a1, b7, c13, d2), (a1, b7, c13, d3), (a1, b7, c13, d4), (a1, b7, c13, d5), (a1, b7, c13, d6), (a1, b7, c13, d7), (a1, b7, c13, d8), (a1, b7, c13, d9), (a1, b7, c13, d10), (a1, b7, c13, d11), (a1, b7, c13, d12), (a1, b8, c1, d1), (a1, b8, c1, d2), (a1, b8, c1, d3), (a1, b8, c1, d4), (a1, b8, c1, d5), (a1, b8, c1, d6), (a1, b8, c1, d7), (a1, b8, c1, d8), (a1, b8, c1, d9), (a1, b8, c1, d10), (a1, b8, c1, d11), (a1, b8, c1, d12), (a1, b8, c2, d1), (a1, b8, c2, d2), (a1, b8, c2, d3), (a1, b8, c2, d4), (a1, b8, c2, d5), (a1, b8, c2, d6), (a1, b8, c2, d7), (a1, b8, c2, d8), (a1, b8, c2, d9), (a1, b8, c2, d10), (a1, b8, c2, d11), (a1, b8, c2, d12), (a1, b8, c3, d1), (a1, b8, c3, d2), (a1, b8, c3, d3), (a1, b8, c3, d4), (a1, b8, c3, d5), (a1, b8, c3, d6), (a1, b8, c3, d7), (a1, b8, c3, d8), (a1, b8, c3, d9), (a1, b8, c3, d10), (a1, b8, c3, d11), (a1, b8, c3, d12), (a1, b8, c4, d1), (a1, b8, c4, d2), (a1, b8, c4, d3), (a1, b8, c4, d4), (a1, b8, c4, d5), (a1, b8, c4, d6), (a1, b8, c4, d7), (a1, b8, c4, d8), (a1, b8, c4, d9), (a1, b8, c4, d10), (a1, b8, c4, d11), (a1, b8, c4, d12), (a1, b8, c5, d1), (a1, b8, c5, d2), (a1, b8, c5, d3), (a1, b8, c5, d4), (a1, b8, c5, d5), (a1, b8, c5, d6), (a1, b8, c5, d7), (a1, b8, c5, d8), (a1, b8, c5, d9), (a1, b8, c5, d10), (a1, b8, c5, d11), (a1, b8, c5, d12), (a1, b8, c6, d1), (a1, b8, c6, d2), (a1, b8, c6, d3), (a1, b8, c6, d4), (a1, b8, c6, d5), (a1, b8, c6, d6), (a1, b8, c6, d7), (a1, b8, c6, d8), (a1, b8, c6, d9), (a1, b8, c6, d10), (a1, b8, c6, d11), (a1, b8, c6, d12), (a1, b8, c7, d1), (a1, b8, c7, d2), (a1, b8, c7, d3), (a1, b8, c7, d4), (a1, b8, c7, d5), (a1, b8, c7, d6), (a1, b8, c7, d7), (a1, b8, c7, d8), (a1, b8, c7, d9), (a1, b8, c7, d10), (a1, b8, c7, d11), (a1, b8, c7, d12), (a1, b8, c8, d1), (a1, b8, c8, d2), (a1, b8, c8, d3), (a1, b8, c8, d4), (a1, b8, c8, d5), (a1, b8, c8, d6), (a1, b8, c8, d7), (a1, b8, c8, d8), . (a1, b8, c8, d9), (a1, b8, c8, d10), (a1, b8, c8, d11), (a1, b8, c8, d12), (a1, b8, c9, d1), (a1, b8, c9, d2), (a1, b8, c9, d3), (a1, b8, c9, d4), (a1, b8, c9, d5), (a1, b8, c9, d6), (a1, b8, c9, d7), (a1, b8, c9, d8), (a1, b8, c9, d9), (a1, b8, c9, d10), (a1, b8, c9, d11), (a1, b8, c9, d12), (a1, b8, c10, d1), (a1, b8, c10, d2), (a1, b8, c10, d3), (a1, b8, c10, d4), (a1, b8, c10, d5), (a1, b8, c10, d6), (a1, b8, c10, d7), (a1, b8, c10, d8), (a1, b8, c10, d9), (a1, b8, c10, d10), (a1, b8, c10, d11), (a1, b8, c10, d12), (a1, b8, c11, d1), (a1, b8, c11, d2), (a1, b8, c11, d3), (a1, b8, c11, d4), (a1, b8, c11, d5), (a1, b8, c11, d6), (a1, b8, c11, d7), (a1, b8, c11, d8), (a1, b8, c11, d9), (a1, b8, c11, d10), (a1, b8, c11, d11), (a1, b8, c11, d12), (a1, b8, c12, d1), (a1, b8, c12, d2), (a1, b8, c12, d3), (a1, b8, c12, d4), (a1, b8, c12, d5), (a1, b8, c12, d6), (a1, b8, c12, d7), (a1, b8, c12, d8), (a1, b8, c12, d9), (a1, b8, c12, d10), (a1, b8, c12, d11), (a1, b8, c12, d12), (a1, b8, c13, d1), (a1, b8, c13, d2), (a1, b8, c13, d3), (a1, b8, c13, d4), (a1, b8, c13, d5), (a1, b8, c13, d6), (a1, b8, c13, d7), (a1, b8, c13, d8), (a1, b8, c13, d9), (a1, b8, c13, d10), (a1, b8, c13, d11), (a1, b8, c13, d12), (a1, b9, c1, d1), (a1, b9, c1, d2), (a1, b9, c1, d3), (a1, b9, c1, d4), (a1, b9, c1, d5), (a1, b9, c1, d6), (a1, b9, c1, d7), (a1, b9, c1, d8), (a1, b9, c1, d9), (a1, b9, c1, d10), (a1, b9, c1, d11), (a1, b9, c1, d12), (a1, b9, c2, d1), (a1, b9, c2, d2), (a1, b9, c2, d3), (a1, b9, c2, d4), (a1, b9, c2, d5), (a1, b9, c2, d6), (a1, b9, c2, d7), (a1, b9, c2, d8), (a1, b9, c2, d9), (a1, b9, c2, d10), (a1, b9, c2, d11), (a1, b9, c2, d12), (a1, b9, c3, d1), (a1, b9, c3, d2), (a1, b9, c3, d3), (a1, b9, c3, d4), (a1, b9, c3, d5), (a1, b9, c3, d6), (a1, b9, c3, d7), (a1, b9, c3, d8), (a1, b9, c3, d9), (a1, b9, c3, d10), (a1, b9, c3, d11), (a1, b9, c3, d12), (a1, b9, c4, d1), (a1, b9, c4, d2), (a1, b9, c4, d3), (a1, b9, c4, d4), (a1, b9, c4, d5), (a1, b9, c4, d6), (a1, b9, c4, d7), (a1, b9, c4, d8), (a1, b9, c4, d9), (a1, b9, c4, d10), (a1, b9, c4, d11), (a1, b9, c4, d12), (a1, b9, c5, d1), (a1, b9, c5, d2), (a1, b9, c5, d3), (a1, b9, c5, d4), (a1, b9, c5, d5), (a1, b9, c5, d6), (a1, b9, c5, d7), (a1, b9, c5, d8), (a1, b9, c5, d9), (a1, b9, c5, d10), (a1, b9, c5, d11), (a1, b9, c5, d12), (a1, b9, c6, d1), (a1, b9, c6, d2), (a1, b9, c6, d3), (a1, b9, c6, d4), (a1, b9, c6, d5), (a1, b9, c6, d6), (a1, b9, c6, d7), (a1, b9, c6, d8), (a1, b9, c6, d9), (a1, b9, c6, d10), (a1, b9, c6, d11), (a1, b9, c6, d12), (a1, b9, c7, d1), (a1, b9, c7, d2), (a1, b9, c7, d3), (a1, b9, c7, d4), (a1, b9, c7, d5), (a1, b9, c7, d6), (a1, b9, c7, d7), (a1, b9, c7, d8), (a1, b9, c7, d9), (a1, b9, c7, d10), (a1, b9, c7, d11), (a1, b9, c7, d12), (a1, b9, c8, d1), (a1, b9, c8, d2), (a1, b9, c8, d3), (a1, b9, c8, d4), (a1, b9, c8, d5), (a1, b9, c8, d6), (a1, b9, c8, d7), (a1, b9, c8, d8), (a1, b9, c8, d9), (a1, b9, c8, d10), (a1, b9, c8, d11), (a1, b9, c8, d12), (a1, b9, c9, d1), (a1, b9, c9, d2), (a1, b9, c9, d3), (a1, b9, c9, d4), (a1, b9, c9, d5), (a1, b9, c9, d6), (a1, b9, c9, d7), (a1, b9, c9, d8), (a1, b9, c9, d9), (a1, b9, c9, d10), (a1, b9, c9, d11), (a1, b9, c9, d12), (a1, b9, c10, d1), (a1, b9, c10, d2), (a1, b9, c10, d3), (a1, b9, c10, d4), (a1, b9, c10, d5), (a1, b9, c10, d6), (a1, b9, c10, d7), (a1, b9, c10, d8), (a1, b9, c10, d9), (a1, b9, c10, d10), (a1, b9, c10, d11), (a1, b9, c10, d12), (a1, b9, c11, d1), (a1, b9, c11, d2), (a1, b9, c11, d3), (a1, b9, c11, d4), (a1, b9, c11, d5), (a1, b9, c11, d6), (a1, b9, c11, d7), (a1, b9, c11, d8), (a1, b9, c11, d9), (a1, b9, c11, d10), (a1, b9, c11, d11), (a1, b9, c11, d12), (a1, b9, c12, d1), (a1, b9, c12, d2), (a1, b9, c12, d3), (a1, b9, c12, d4), (a1, b9, c12, d5), (a1, b9, c12, d6), (a1, b9, c12, d7), (a1, b9, c12, d8), (a1, b9, c12, d9), (a1,

b9, c12, d10), (a1, b9, c12, d11), (a1, b9, c12, d12), (a1, b9, c13, d1), (a1, b9, c13, d2), (a1, b9, c13, d3), (a1, b9, c13, d4), (a1, b9, c13, d5), (a1, b9, c13, d6), (a1, b9, c13, d7), (a1, b9, c13, d8), (a1, b9, c13, d9), (a1, b9, c13, d10), (a1, b9, c13, d11), (a1, b9, c13, d12), (a1, b10, c1, d1), (a1, b10, c1, d2), (a1, b10, c1, d3), (a1, b10, c1, d4), (a1, b10, c1, d5), (a1, b10, c1, d6), (a1, b10, c1, d7), (a1, b10, c1, d8), (a1, b10, c1, d9), (a1, b10, c1, d10), (a1, b10, c1, d11), (a1, b10, c1, d12), (a1, b10, c2, d1), (a1, b10, c2, d2), (a1, b10, c2, d3), (a1, b10, c2, d4), (a1, b10, c2, d5), (a1, b10, c2, d6), (a1, b10, c2, d7), (a1, b10, c2, d8), (a1, b10, c2, d9), (a1, b10, c2, d10), (a1, b10, c2, d11), (a1, b10, c2, d12), (a1, b10, c3, d1), (a1, b10, c3, d2), (a1, b10, c3, d3), (a1, b10, c3, d4), (a1, b10, c3, d5), (a1, b10, c3, d6), (a1, b10, c3, d7), (a1, b10, c3, d8), (a1, b10, c3, d9), (a1, b10, c3, d10), (a1, b10, c3, d11), (a1, b10, c3, d12), (a1, b10, c4, d1), (a1, b10, c4, d2), (a1, b10, c4, d3), (a1, b10, c4, d4), (a1, b10, c4, d5), (a1, b10, c4, d6), (a1, b10, c4, d7), (a1, b10, c4, d8), (a1, b10, c4, d9), (a1, b10, c4, d10), (a1, b10, c4, d11), (a1, b10, c4, d12), (a1, b10, c5, d1), (a1, b10, c5, d2), (a1, b10, c5, d3), (a1, b10, c5, d4), (a1, b10, c5, d5), (a1, b10, c5, d6), (a1, b10, c5, d7), (a1, b10, c5, d8), (a1, b10, c5, d9), (a1, b10, c5, d10), (a1, b10, c5, d11), (a1, b10, c5, d12), (a1, b10, c6, d1), (a1, b10, c6, d2), (a1, b10, c6, d3), (a1, b10, c6, d4), (a1, b10, c6, d5), (a1, b10, c6, d6), (a1, b10, c6, d7), (a1, b10, c6, d8), (a1, b10, c6, d9), (a1, b10, c6, d10), (a1, b10, c6, d11), (a1, b10, c6, d12), (a1, b10, c7, d1), (a1, b10, c7, d2), (a1, b10, c7, d3), (a1, b10, c7, d4), (a1, b10, c7, d5), (a1, b10, c7, d6), (a1, b10, c7, d7), (a11, b10, c7, d8), (a1, b10, c7, d9), (a1, b10, c7, d10), (a1, b10, c7, d11), (a1, b10, c7, d12), (a1, b10, c8, d1), (a1, b10, c8, d2), (a1, b10, c8, d3), (a1, b10, c8, d4), (a1, b10, c8, d5), (a1, b10, c8, d6), (a1, b10, c8, d7), (a1, b10, c8, d8), (a1, b10, c8, d9), (a1, b10, c8, d10), (a1, b10, c8, d11), (a1, b10, c8, d12), (a1, b10, c9, d1), (a1, b10, c9, d2), (a1, b10, c9, d3), (a1, b10, c9, d4), (a1, b10, c9, d5), (a1, b10, c9, d6), (a1, b10, c9, d7), (a1, b10, c9, d8), (a1, b10, c9, d9), (a1, b10, c9, d10), (a1, b10, c9, d11), (a1, b10, c9, d12), (a1, b10, c10, d1), (a1, b10, c10, d2), (a1, b10, c10, d3), (a1, b10, c10, d4), (a1, b10, c10, d5), (a1, b10, c10, d6), (a1, b10, c10, d7), (a1, b10, c10, d8), (a1, b10, c10, d9), (a1, b10, c10, d10), (a1, b10, c10, d11), (a1, b10, c10, d12), (a1, b10, c11, d1), (a1, b10, c11, d2), (a1, b10, c11, d3), (a1, b10, c11, d4), (a1, b10, c11, d5), (a1, b10, c11, d6), (a1, b10, c11, d7), (a1, b10, c11, d8), (a1, b10, c11, d9), (a1, b10, c11, d10), (a1, b10, c11, d11), (a1, b10, c11, d12), (a1, b10, c12, d1), (a1, b10, c12, d2), (a1, b10, c12, d3), (a1, b10, c12, d4), (a1, b10, c12, d5), (a1, b10, c12, d6), (a1, b10, c12, d7), (a1, b10, c12, d8), (a1, b10, c12, d9), (a1, b10, c12, d10), (a1, b10, c12, d11), (a1, b10, c12, d12), (a1, b10, c13, d1), (a1, b10, c13, d2), (a1, b10, c13, d3), (a1, b10, c13, d4), (a1, b10, c13, d5), (a1, b10, c13, d6), (a1, b10, c13, d7), (a1, b10, c13, d8), (a1, b10, c13, d9), (a1, b10, c13, d10), (a1, b10, c13, d11), (a1, b10, c13, d12), (a1, b11, c1, d1), (a1, b11, c1, d2), (a1, b11, c1, d3), (a1, b11, c1, d4), (a1, b11, c1, d5), (a1, b11, c1, d6), (a1, b11, c1, d7), (a1, b11, c1, d8), (a1, b11, c1, d9), (a1, b11, c1, d10), (a1, b11, c1, d11), (a1, b11, c1, d12), (a1, b11, c2, d1), (a1, b11, c2, d2), (a1, b11, c2, d3), (a1, b11, c2, d4), (a1, b11, c2, d5), (a1, b11, c2, d6), (a1, b11, c2, d7), (a1, b11, c2, d8), (a1, b11, c2, d9), (a1, b11, c2, d10), (a1, b11, c2, d11), (a1, b11, c2, d12), (a1, b11, c3, d1), (a1, b11, c3, d2), (a1, b11, c3, d3), (a1, b11, c3, d4), (a1, b11, c3, d5), (a1, b11, c3, d6), (a1, b11, c3, d7), (a1, b11, c3, d8), (a1, b11, c3, d9), (a1, b11, c3, d10), (a1, b11, c3, d11), (a1, b11, c3, d12), (a1, b11, c4, d1), (a1, b11, c4, d2), (a1, b11, c4, d3), (a1, b11, c4, d4), (a1, b11, c4, d5), (a1, b11, c4, d6), (a1, b11, c4, d7), (a1, b11, c4, d8), (a1, b11, c4, d9), (a1, b11, c4, d10), (a1, b11, c4, d11), (a1, b11, c4, d12), (a1, b11, c5, d1), (a1, b11, c5, d2), (a1, b11, c5, d3), (a1, b11, c5, d4), (a1, b11, c5, d5), (a1, b11, c5, d6), (a1, b11, c5, d7), (a1, b11, c5, d8), (a1, b11, c5, d9), (a1, b11, c5, d10), (a1, b11, c5, d11), (a1, b11, c5, d12), (a1, b11, c6, d1), (a1, b11, c6, d2), (a1, b11, c6, d3), (a1, b11, c6, d4), (a1, b11, c6, d5), (a1, b11, c6, d6), (a1, b11, c6, d7), (a1, b11, c6, d8), (a1, b11, c6, d9), (a1, b11, c6, d10), (a1, b11, c6, d11), (a1, b11, c6, d12), (a1, b11, c7, d1), (a1, b11, c7, d2), (a1, b11, c7, d3), (a1, b11, c7, d4), (a1, b11, c7, d5), (a1, b11, c7, d6), (a1, b11, c7, d7), (a1, b11, c7, d8), (a1, b11, c7, d9), (a1, b11, c7, d10), (a1, b11, c7, d11), (a1, b11, c7, d12), (a1, b11, c8, d1), (a1, b11, c8, d2), (a1, b11, c8, d3), (a1, b11, c8, d4), (a1, b11, c8, d5), (a1, b11, c8, d6), (a1, b11, c8, d7), (a1, b11, c8, d8), (a1, b11, c8, d9), (a1, b11, c8, d10), (a1, b11, c8, d11), (a1, b11, c8, d12), (a1, b11, c9, d1), (a1, b11, c9, d2), (a1, b11, c9, d3), (a1, b11, c9, d4), (a1, b11, c9, d5), (a1, b11, c9, d6), (a1, b11, c9, d7), (a1, b11, c9, d8), (a1, b11, c9, d9), (a1, b11, c9, d10), (a1, b11, c9, d11), (a1, b11, c9, d12), (a1, b11, c10, d1), (a1, b11, c10, d2), (a1, b11, c10, d3), (a1, b11, c10, d4), (a1, b11, c10, d5), (a1, b11, c10, d6), (a1, b11, c10, d7), (a1, b11, c10, d8), (a1, b11, c10, d9), (a1, b11, c10, d10), (a1, b11, c10, d11), (a1, b11, c10, d12), (a1, b11, c11, d1), (a1, b11, c11, d2), (a1, b11, c11, d3), (a1, b11, c11, d4), (a1, b11, c11, d5), (a1, b11, c11, d6), (a1, b11, c11, d7), (a1, b11, c11, d8), (a1, b11, c11, d9), (a1, b11, c11, d10), (a1, b11, c11, d11), (a1, b11, c11, d12), (a1, b11, c12, d1), (a1, b11, c12, d2), (a1, b11, c12, d3), (a1, b11, c12, d4), (a1, b11, c12, d5), (a1, b11, c12, d6), (a1, b11, c12, d7), (a1, b11, c12, d8), (a1, b11, c12, d9), (a1, b11, c12, d10), (a1, b11, c12, d11), (a1, b11, c12, d12), (a1, b11, c13, d1), (a1, b11, c13, d2), (a1, b11, c13, d3), (a1, b11, c13, d4), (a1, b11, c13, d5), (a1, b11, c13, d6), (a1, b11, c13, d7), (a1, b11, c13, d8), (a1, b11, c13, d9), (a1, b11, c13, d10), (a1, b11, c13, d11), (a1, b11, c13, d12), (a1, b12, c1, d1), (a1, b12, c1, d2), (a1, b12, c1, d3), (a1, b12, c1, d4), (a1, b12, c1, d5), (a1, b12, c1, d6), (a1, b12, c1, d7), (a1, b12, c1, d8), (a1, b12, c1, d9), (a1, b12, c1, d10), (a1, b12, c1, d11), (a1, b12, c1, d12), (a1, b12, c2, d1), (a1, b12, c2, d2), (a1, b12, c2, d3), (a1, b12, c2, d4), (a1, b12, c2, d5), (a1, b12, c2, d6), (a1, b12, c2, d7), (a1, b12, c2, d8), (a1, b12, c2, d9), (a1, b12, c2, d10), (a1, b12, c2, d11), (a1, b12, c2, d12), (a1, b12, c3, d1), (a1, b12, c3, d2), (a1, b12, c3, d3), (a1, b12, c3, d4), (a1, b12, c3, d5), (a1, b12, c3, d6), (a1, b12, c3, d7), (a1, b12, c3, d8), (a1, b12, c3, d9), (a1, b12, c3, d10), (a1, b12, c3, d11), (a1, b12, c3, d12), (a1, b12, c4, d1), (a1, b12, c4, d2), (a1, b12, c4, d3), (a1, b12, c4, d4), (a1, b12, c4, d5), (a1, b12, c4, d6), (a1, b12, c4, d7), (a1, b12, c4, d8), (a1, b12, c4, d9), (a1, b12, c4, d10), (a1, b12, c4, d11), (a1,

b12, c4, d12), (a1, b12, c5, d1), (a1, b12, c5, d2), (a1, b12, c5, d3), (a1, b12, c5, d4), (a1, b12, c5, d5), (a1, b12, c5, d6), (a1, b12, c5, d7), (a1, b12, c5, d8), (a1, b12, c5, d9), (a1, b12, c5, d10), (a1, b12, c5, d11), (a1, b12, c5, d12), (a1, b12, c6, d1), (a1, b12, c6, d2), (a1, b12, c6, d3), (a1, b12, c6, d4), (a1, b12, c6, d5), (a1, b12, c6, d6), (a1, b12, c6, d7), (a1, b12, c6, d8), (a1, b12, c6, d9), (a1, b12, c6, d10), (a1, b12, c6, d11), (a1, b12, c6, d12), (a1, b12, c7, d1), (a1, b12, c7, d2), (a1, b12, c7, d3), (a1, b12, c7, d4), (a1, b12, c7, d5), (a1, b12, c7, d6), (a1, b12, c7, d7), (a1, b12, c7, d8), (a1, b12, c7, d9), (a1, b12, c7, d10), (a1, b12, c7, d11), (a1, b12, c7, d12), (a1, b12, c8, d1), (a1, b12, c8, d2), (a1, b12, c8, d3), (a1, b12, c8, d4), (a1, b12, c8, d5), (a1, b12, c8, d6), (a1, b12, c8, d7), (a1, b12, c8, d8), (a1, b12, c8, d9), (a1, b12, c8, d10), (a1, b12, c8, d11), (a1, b12, c8, d12), (a1, b12, c9, d1), (a1, b12, c9, d2), (a1, b12, c9, d3), (a1, b12, c9, d4), (a1, b12, c9, d5), (a1, b12, c9, d6), (a1, b12, c9, d7), (a1, b12, c9, d8), (a1, b12, c9, d9), (a1, b12, c9, d10), (a1, b12, c9, d11), (a1, b12, c9, d12), (a1, b12, c10, d1), (a1, b12, c10, d2), (a1, b12, c10, d3), (a1, b12, c10, d4), (a1, b12, c10, d5), (a1, b12, c10, d6), (a1, b12, c10, d7), (a1, b12, c10, d8), (a1, b12, c10, d9), (a1, b12, c10, d10), (a1, b12, c10, d11), (a1, b12, c10, d12), (a1, b12, c11, d1), (a1, b12, c11, d2), (a1, b12, c11, d3), (a1, b12, c11, d4), (a1, b12, c11, d5), (a1, b12, c11, d6), (a1, b12, c11, d7), (a1, b12, c11, d8), (a1, b12, c11, d9), (a1, b12, c11, d10), (a1, b12, c11, d11), (a1, b12, c11, d12), (a1, b12, c12, d1), (a1, b12, c12, d2), (a1, b12, c12, d3), (a1, b12, c12, d4), (a1, b12, c12, d5), (a1, b12, c12, d6), (a1, b12, c12, d7), (a1, b12, c12, d8), (a1, b12, c12, d9), (a1, b12, c12, d10), (a1, b12, c12, d11), (a1, b12, c12, d12), (a1, b12, c13, d1), (a1, b12, c13, d2), (a1, b12, c13, d3), (a1, b12, c13, d4), (a1, b12, c13, d5), (a1, b12, c13, d6), (a1, b12, c13, d7), (a1, b12, c13, d8), (a1, b12, c13, d9), (a1, b12, c13, d10), (a1, b12, c13, d11), (a1, b12, c13, d12), (a1, b13, c1, d1), (a1, b13, c1, d2), (a1, b13, c1, d3), (a1, b13, c1, d4), (a1, b13, c1, d5), (a1, b13, c1, d6), (a1, b13, c1, d7), (a1, b13, c1, d8), (a1, b13, c1, d9), (a1, b13, c1, d10), (a1, b13, c1, d11), (a1, b13, c1, d12), (a1, b13, c2, d1), (a1, b13, c2, d2), (a1, b13, c2, d3), (a1, b13, c2, d4), (a1, b13, c2, d5), (a1, b13, c2, d6), (a1, b13, c2, d7), (a1, b13, c2, d8), (a1, b13, c2, d9), (a1, b13, c2, d10), (a1, b13, c2, d11), (a1, b13, c2, d12), (a1, b13, c3, d1), (al, b13, c3, d2), (a1, b13, c3, d3), (a1, b13, c3, d4), (a1, b13, c3, d5), (a1, b13, c3, d6), (a1, b13, c3, d7), (a1, b13, c3, d8), (a1, b13, c3, d9), (a1, b13, c3, d10), (a1, b13, c3, d11), (a1, b13, c3, d12), (a1, b13, c4, d1), (a1, b13, c4, d2), (a1, b13, c4, d3), (a1, b13, c4, d4), (a1, b13, c4, d5), (a1, b13, c4, d6), (a1, b13, c4, d7), (a1, b13, c4, d8), (a1, b13, c4, d9), (a1, b13, c4, d10), (a1, b13, c4, d11), (a1, b13, c4, d12), (a1, b13, c5, d1), (a1, b13, c5, d2), (a1, b13, c5, d3), (a1, b13, c5, d4), (a1, b13, c5, d5), (a1, b13, c5, d6), (a1, b13, c5, d7), (a1, b13, c5, d8), (a1, b13, c5, d9), (a1, b13, c5, d10), (a1, b13, c5, d11), (a1, b13, c5, d12), (a1, b13, c6, d1), (a1, b13, c6, d2), (a1, b13, c6, d3), (a1, b13, c6, d4), (a1, b13, c6, d5), (a1, b13, c6, d6), (a1, b13, c6, d7), (a1, b13, c6, d8), (a1, b13, c6, d9), (a1, b13, c6, d10), (a1, b13, c6, d11), (a1, b13, c6, d12), (a1, b13, c7, d1), (a1, b13, c7, d2), (a1, b13, c7, d3), (a1, b13, c7, d4), (a1, b13, c7, d5), (a1, b13, c7, d6), (a1, b13, c7, d7), (a1, b13, c7, d8), (a1, b13, c7, d9), (a1, b13, c7, d10), (a1, b13, c7, d11), (a1, b13, c7, d12), (a1, b13, c8, d1), (a1, b13, c8, d2), (a1, b13, c8, d3), (a1, b13, c8, d4), (a1, b13, c8, d5), (a1, b13, c8, d6), (a1, b13, c8, d7), (a1, b13, c8, d8), (a1, b13, c8, d9), (a1, b13, c8, d10), (a1, b13, c8, d11), (a1, b13, c8, d12), (a1, b13, c9, d1), (a1, b13, c9, d2), (a1, b13, c9, d3), (a1, b13, c9, d4), (a1, b13, c9, d5), (a1, b13, c9, d6), (a1, b13, c9, d7), (a1, b13, c9, d8), (a1, b13, c9, d9), (a1, b13, c9, d10), (a1, b13, c9, d11), (a1, b13, c9, d12), (a1, b13, c10, d1), (a1, b13, c10, d2), (a1, b13, c10, d3), (a1, b13, c10, d4), (a1, b13, c10, d5), (a1, b13, c10, d6), (a1, b13, c10, d7), (a1, b13, c10, d8), (a1, b13, c10, d9), (a1, b13, c10, d10), (a1, b13, c10, d11), (a1, b13, c10, d12), (a1, b13, c11, d1), (a1, b13, c11, d2), (a1, b13, c11, d3), (a1, b13, c11, d4), (a1, b13, c11, d5), (a1, b13, c11, d6), (a1, b13, c11, d7), (a1, b13, c11, d8), (a1, b13, c11, d9), (a1, b13, c11, d10), (a1, b13, c11, d11), (a1, b13, c11, d12), (a1, b13, c12, d1), (a1, b13, c12, d2), (a1, b13, c12, d3), (a1, b13, c12, d4), (a1, b13, c12, d5), (a1, b13, c12, d6), (a1, b13, c12, d7), (a1, b13, c12, d8), (a1, b13, c12, d9), (a1, b13, c12, d10), (a1, b13, c12, d11), (a1, b13, c12, d12), (a1, b13, c13, d1), (a1, b13, c13, d2), (a1, b13, c13, d3), (a1, b13, c13, d4), (a1, b13, c13, d5), (a1, b13, c13, d6), (a1, b13, c13, d7), (a1, b13, c13, d8), (a1, b13, c13, d9), (a1, b13, c13, d10), (a1, b13, c13, d11), (a1, b13, c13, d12), (a1, b14, c1, d1), (a1, b14, c1, d2), (a1, b14, c1, d3), (a1, b14, c1, d4), (a1, b14, c1, d5), (a1, b14, c1, d6), (a1, b14, c1, d7), (a1, b14, c1, d8), (a1, b14, c1, d9), (a1, b14, c1, d10), (a1, b14, c1, d11), (a1, b14, c1, d12), (a1, b14, c2, d1), (a1, b14, c2, d2), (a1, b14, c2, d3), (a1, b14, c2, d4), (a1, b14, c2, d5), (a1, b14, c2, d6), (a1, b14, c2, d7), (a1, b14, c2, d8), (a1, b14, c2, d9), (a1, b14, c2, d10), (a1, b14, c2, d11), (a1, b14, c2, d12), (a1, b14, c3, d1), (a1, b14, c3, d2), (a1, b14, c3, d3), (a1, b14, c3, d4), (a1, b14, c3, d5), (a1, b14, c3, d6), (a1, b14, c3, d7), (a1, b14, c3, d8), (a1, b14, c3, d9), (a1, b14, c3, d10), (a1, b14, c3, d11), (a1, b14, c3, d12), (a1, b14, c4, d1), (a1, b14, c4, d2), (a1, b14, c4, d3), (a1, b14, c4, d4), (a1, b14, c4, d5), (a1, b14, c4, d6), (a1, b14, c4, d7), (a1, b14, c4, d8), (a1, b14, c4, d9), (a1, b14, c4, d10), (a1, b14, c4, d11), (a1, b14, c4, d12), (a1, b14, c5, d1), (a1, b14, c5, d2), (a1, b14, c5, d3), (a1, b14, c5, d4), (a1, b14, c5, d5), (a1, b14, c5, d6), (a1, b14, c5, d7), (a1, b14, c5, d8), (a1, b14, c5, d9), (a1, b14, c5, d10), (a1, b14, c5, d11), (a1, b14, c5, d12), (a1, b14, c6, d1), (a1, b14, c6, d2), (a1, b14, c6, d3), (a1, b14, c6, d4), (a1, b14, c6, d5), (a1, b14, c6, d6), (a1, b14, c6, d7), (a1, b14, c6, d8), (a1, b14, c6, d9), (a1, b14, c6, d10), (a1, b14, c6, d11), (a1, b14, c6, d12), (a1, b14, c7, d1), (a1, b14, c7, d2), (a1, b14, c7, d3), (a1, b14, c7, d4), (a1, b14, c7, d5), (a1, b14, c7, d6), (a1, b14, c7, d7), (a1, b14, c7, d8), (a1, b14, c7, d9), (a1, b14, c7, d10), (a1, b14, c7, d11), (a1, b14, c7, d12), (a1, b14, c8, d1), (a1, b14, c8, d2), (a1, b14, c8, d3), (a1, b14, c8, d4), (a1, b14, c8, d5), (a1, b14, c8, d6), (a1, b14, c8, d7), (a1, b14, c8, d8), (a1, b14, c8, d9), (a1, b14, c8, d10), (a1, b14, c8, d11), (a1, b14, c8, d12), (a1, b14, c9, d1), (a1, b14, c9, d2), (a1, b14, c9, d3), (a1, b14, c9, d4), (a1, b14, c9, d5), (a1, b14, c9, d6), (a1, b14, c9, d7), (a1, b14, c9, d8), (a1, b14, c9, d9), (a1, b14, c9, d10), (a1, b14, c9, d11), (a1, b14, c9, d12), (a1, b14, c10, d1), (a1, b14, c10, d2), (a1,

b14, c10, d3), (a1, b14, c10, d4), (a1, b14, c10, d5), (a1, b14, c10, d6), (a1, b14, c10, d7), (a1, b14, c10, d8), (a1, b14, c10, d9), (a1, b14, c10, d10), (a1, b14, c10, d11), (a1, b14, c10, d12), (a1, b14, c11, d1), (a1, b14, c11, d2), (a1, b14, c11, d3), (a1, b14, c11, d4), (a1, b14, c11, d5), (a1, b14, c11, d6), (a1, b14, c11, d7), (a1, b14, c11, d8), (a1, b14, c11, d9), (a1, b14, c11, d10), (a1, b14, c11, d11), (a1, b14, c11, d12), (a1, b14, c12, d1), (a1, b14, c12, d2), (a1, b14, c12, d3), (a1, b14, c12, d4), (a1, b14, c12, d5), (a1, b14, c12, d6), (a1, b14, c12, d7), (a1, b14, c12, d8), (a1, b14, c12, d9), (a1, b14, c12, d10), (a1, b14, c12, d11), (a1, b14, c12, d12), (a1, b14, c13, d1), (a1, b14, c13, d2), (a1, b14, c13, d3), (a1, b14, c13, d4), (a1, b14, c13, d5), (a1, b14, c13, d6), (a1, b14, c13, d7), (a1, b14, c13, d8), (a1, b14, c13, d9), (a1, b14, c13, d10), (a1, b14, c13, d11), (a1, b14, c13, d12), (a1, b15, c1, d1), (a1, b15, c1, d2), (a1, b15, c1, d3), (a1, b15 c1, d4), (a1, b15, c1, d5), (a1, b15, c1, d6), (a1, b15, c1, d7), (a1, b15, c1, d8), (a1, b15, c1, d9), (a1, b15, c1, d10), (a1, b15, c1, d11), (a1, b15, c1, d12), (a1, b15, c2, d1), (a1, b15, c2, d2), (a1, b15, c2, d3), (a1, b15, c2, d4), (a1, b15, c2, d5), (a1, b15, c2, d6), (a1, b15, c2, d7), (a1, b15, c2, d8), (a1, b15, c2, d9), (a1, b15, c2, d10), (a1, b15, c2, d11), (a1, b15, c2, d12), (a1, b15, c3, d1), (a1, b15, c3, d2), (a1, b15, c3, d3), (a1, b15, c3, d4), (a1, b15, c3, d5), (a1, b15, c3, d6), (a1, b15, c3, d7), (a1, b15, c3, d8), (a1, b15, c3, d9), (a1, b15, c3, d10), (a1, b15, c3, d11), (a1, b15, c3, d12), (a1, b15, c4, d1), (a1, b15, c4, d2), (a1, b15, c4, d3), (a1, b15, c4, d4), (a1, b15, c4, d5), (a1, b15, c4, d6), (a1, b15, c4, d7), (a1, b15, c4, d8), (a1, b15, c4, d9), (a1, b15, c4, d10), (a1, b15, c4, d11), (a1, b15, c4, d12), (a1, b15, c5, d1), (a1, b15, c5, d2), (a1, b15, c5, d3), (a1, b15, c5, d4), (a1, b15, c5, d5), (a1, b15, c5, d6), (a1, b15, c5, d7), (a1, b15, c5, d8), (a1, b15, c5, d9), (a1, b15, c5, d10), (a1, b15, c5, d11), (a1, b15, c5, d12), (a1, b15, c6, d1), (a1, b15, c6, d2), (a1, b15, c6, d3), (a1, b15, c6, d4), (a1, b15, c6, d5), (a1, b15, c6, d6), (a1, b15, c6, d7), (a1, b15, c6, d8), (a1, b15, c6, d9), (a1, b15, c6, d10), (a1, b15, c6, d11), (a1, b15, c6, d12), (a1, b15, c7, d1), (a1, b15, c7, d2), (a1, b15, c7, d3), (a1, b15, c7, d4), (a1, b15, c7, d5), (a1, b15, c7, d6), (a1, b15, c7, d7), (a1, b15, c7, d8), (a1, b15, c7, d9), (a1, b15, c7, d10), (a1, b15, c7, d11), (a1, b15, c7, d12), (a1, b15, c8, d1), (a1, b15, c8, d2), (a1, b15, c8, d3), (a1, b15, c8, d4), (a1, b15, c8, d5), (a1, b15, c8, d6), (a1, b15, c8, d7), (a1, b15, c8, d8), (a1, b15, c8, d9), (a1, b15, c8, d10), (a1, b15, c8, d11), (a1, b15, c8, d12), (a1, b15, c9, d1), (a1, b15, c9, d2), (a1, b15, c9, d3), (a1, b15, c9, d4), (a1, b15, c9, d5), (a1, b15, c9, d6), (a1, b15, c9, d7), (a1, b15, c9, d8), (a1, b15, c9, d9), (a1, b15, c9, d10), (a1, b15, c9, d11), (a1, b15, c9, d12), (a1, b15, c10, d1), (a1, b15, c10, d2), (a1, b15, c10, d3), (a1, b15, c10, d4), (a1, b15, c10, d5), (a1, b15, c10, d6), (a1, b15, c10, d7), (a1, b15, c10, d8), (a1, b15, c10, d9), (a1, b15, c10, d10), (a1, b15, c10, d11), (a1, b15, c10, d12), (a1, b15, c11, d1), (a1, b15, c11, d2), (a1, b15, c11, d3), (a1, b15, c11, d4), (a1, b15, c11, d5), (a1, b15, c11, d6), (a1, b15, c11, d7), (a1, b15, c11, d8), (a1, b15, c11, d9), (a1, b15, c11, d10), (a1, b15, c11, d11), (a1, b15, c11, d12), (a1, b15, c12, d1), (a1, b15, c12, d2), (a1, b15, c12, d3), (a1, b15, c12, d4), (a1, b15, c12, d5), (a1, b15, c12, d6), (a1, b15, c12, d7), (a1, b15, c12, d8), (a1, b15, c12, d9), (a1, b15, c12, d10), (a1, b15, c12, d11), (a1, b15, c12, d12), (a1, b15, c13, d1), (a1, b15, c13, d2), (a1, b15, c13, d3), (a1, b15, c13, d4), (a1, b15, c13, d5), (a1, b15, c13, d6), (a1, b15, c13, d7), (a1, b15, c13, d8), (a1, b15, c13, d9), (a1, b15, c13, d10), (a1, b15, c13, d11), (a1, b15, c13, d12), (a2, b1, c1, d1), (a2, b1, c1, d2), (a2, b1, c1, d3), (a2, b1, c1, d4), (a2, b1, c1, d5), (a2, b1, c1, d6), (a2, b1, c1, d7), (a2, b1, c1, d8), (a2, b1, c1, d9), (a2, b1, c1, d10), (a2, b1, c1, d11), (a2, b1, c1, d12), (a2, b1, c2, d1), (a2, b1, c2, d2), (a2, b1, c2, d3), (a2, b1, c2, d4), (a2, b1, c2, d5), (a2, b1, c2, d6), (a2, b1, c2, d7), (a2, b1, c2, d8), (a2, b1, c2, d9), (a2, b1, c2, d10), (a2, b1, c2, d11), (a2, b1, c2, d12), (a2, b1, c3, d1), (a2, b1, c3, d2), (a2, b1, c3, d3), (a2, b1, c3, d4), (a2, b1, c3, d5), (a2, b1, c3, d6), (a2, b1, c3, d7), (a2, b1, c3, d8), (a2, b1, c3, d9), (a2, b1, c3, d10), (a2, b1, c3, d11), (a2, b1, c3, d12), (a2, b1, c4, d1), (a2, b1, c4, d2), (a2, b1, c4, d3), (a2, b1, c4, d4), (a2, b1, c4, d5), (a2, b1, c4, d6), (a2, b1, c4, d7), (a2, b1, c4, d8), (a2, b1, c4, d9), (a2, b1, c4, d10), (a2, b1, c4, d11), (a2, b1, c4, d12), (a2, b1, c5, d1), (a2, b1, c5, d2), (a2, b1, c5, d3), (a2, b1, c5, d4), (a2, b1, c5, d5), (a2, b1, c5, d6), (a2, b1, c5, d7), (a2, b1, c5, d8), (a2, b1, c5, d9), (a2, b1, c5, d10), (a2, b1, c5, d11), (a2, b1, c5, d12), (a2, b1, c6, d1), (a2, b1, c6, d2), (a2, b1, c6, d3), (a2, b1, c6, d4), (a2, b1, c6, d5), (a2, b1, c6, d6), (a2, b1, c6, d7), (a2, b1, c6, d8), (a2, b1, c6, d9), (a2, b1, c6, d10), (a2, b1, c6, d11), (a2, b1, c6, d12), (a2, b1, c7, d1), (a2, b1, c7, d2), (a2, b1, c7, d3), (a2, b1, c7, d4), (a2, b1, c7, d5), (a2, b1, c7, d6), (a2, b1, c7, d7), (a2, b1, c7, d8), (a2, b1, c7, d9), (a2, b1, c7, d10), (a2, b1, c7, d11), (a2, b1, c7, d12), (a2, b1, c8, d1), (a2, b1, c8, d2), (a2, b1, c8, d3), (a2, b1, c8, d4), (a2, b1, c8, d5), (a2, b1, c8, d6), (a2, b1, c8, d7), (a2, b1, c8, d8), (a2, b1, c8, d9), (a2, b1, c8, d10), (a2, b1, c8, d11), (a2, b1, c8, d12), (a2, b1, c9, d1), (a2, b1, c9, d2), (a2, b1, c9, d3), (a2, b1, c9, d4), (a2, b1, c9, d5), (a2, b1, c9, d6), (a2, b1, c9, d7), (a2, b1, c9, d8), (a2, b1, c9, d9), (a2, b1, c9, d10), (a2, b1, c9, d11), (a2, b1, c9, d12), (a2, b1, c10, d1), (a2, b1, c10, d2), (a2, b1, c10, d3), (a2, b1, c10, d4), (a2, b1, c10, d5), (a2, b1, c10, d6), (a2, b1, c10, d7), (a2, b1, c10, d8), (a2, b1, c10, d9), (a2, b1, c10, d10), (a2, b1, c10, d11), (a2, b1, c10, d12), (a2, b1, c11, d1), (a2, b, c11, d2), (a2, b1, c11, d3), (a2, b1, c11, d4), (a2, b1, c11, d5), (a2, b1, c11, d6), (a2, b1, c11, d7), (a2, b1, c11, d8), (a2, b1, c11, d9), (a2, b1, c11, d10), (a2, b1, c11, d11), (a2, b1, c11, d12), (a2, b1, c12, d1), (a2, b1, c12, d2), (a2, b1, c12, d3), (a2, b1, c12, d4), (a2, b1, c12, d5), (a2, b1, c12, d6), (a2, b1, c12, d7), (a2, b1, c12, d8), (a2, b1, c12, d9), (a2, b1, c12, d10), (a2, b1, c12, d11), (a2, b1, c12, d12), (a2, b1, c13, d1), (a2, b1, c13, d2), (a2, b1, c13, d3), (a2, b1, c13, d4), (a2, b1, c13, d5), (a2, b1, c13, d6), (a2, b1, c13, d7), (a2, b1, c13, d8), (a2, b1, c13, d9), (a2, b1, c13, d10), (a2, b1, c13, d11), (a2, b1, c13, d12), (a2, b2, c1, d1), (a2, b2, c1, d2), (a2, b2, c1, d3), (a2, b2, c1, d4), (a2, b2, c1, d5), (a2, b2, c1, d6), (a2, b2, c1, d7), (a2, b2, c1, d8), (a2, b2, c1, d9), (a2, b2, c1, d10), (a2, b2, c1, d11), (a2, b2, c1, d12), (a2, b2, c2, d1), (a2, b2, c2, d2), (a2, b2, c2, d3), (a2, b2, c2, d4), (a2, b2, c2, d5), (a2, b2, c2, d6), (a2, b2, c2, d7), (a2, b2, c2, d8), (a2, b2, c2, d9), (a2, b2, c2, d10), (a2, b2, c2, d11), (a2, b2, c2, d12), (a2, b2, c3, d1), (a2, b2, c3, d2), (a2, b2,

c3, d3), (a2, b2, c3, d4), (a2, b2, c3, d5), (a2, b2, c3, d6), (a2, b2, c3, d7), (a2, b2, c3, d8), (a2, b2, c3, d9), (a2, b2, c3, d10), (a2, b2, c3, d11), (a2, b2, c3, d12), (a2, b2, c4, d1), (a2, b2, c4, d2), (a2, b2, c4, d3), (a2, b2, c4, d4), (a2, b2, c4, d5), (a2, b2, c4, d6), (a2, b2, c4, d7), (a2, b2, c4, d8), (a2, b2, c4, d9), (a2, b2, c4, d10), (a2, b2, c4, d11), (a2, b2, c4, d12), (a2, b2, c5, d1), (a2, b2, c5, d2), (a2, b2, c5, d3), (a2, b2, c5, d4), (a2, b2, c5, d5), (a2, b2, c5, d6), (a2, b2, c5, d7), (a2, b2, c5, d8), (a2, b2, c5, d9), (a2, b2, c5, d10), (a2, b2, c5, d11), (a2, b2, c5, d12), (a2, b2, c6, d1), (a2, b2, c6, d2), (a2, b2, c6, d3), (a2, b2, c6, d4), (a2, b2, c6, d5), (a2, b2, c6, d6), (a2, b2, c6, d7), (a2, b2, c6, d8), (a2, b2, c6, d9), (a2, b2, c6, d10), (a2, b2, c6, d11), (a2, b2, c6, d12), (a2, b2, c7, d1), (a2, b2, c7, d2), (a2, b2, c7, d3), (a2, b2, c7, d4), (a2, b2, c7, d5), (a2, b2, c7, d6), (a2, b2, c7, d7), (a2, b2, c7, d8), (a2, b2, c7, d9), (a2, b2, c7, d10), (a2, b2, c7, d11), (a2, b2, c7, d12), (a2, b2, c8, d1), (a2, b2, c8, d2), (a2, b2, c8, d3), (a2, b2, c8, d4), (a2, b2, c8, d5), (a2, b2, c8, d6), (a2, b2, c8, d7), (a2, b2, c8, d8), (a2, b2, c8, d9), (a2, b2, c8, d10), (a2, b2, c8, d11), (a2, b2, c8, d12), (a2, b2, c9, d1), (a2, b2, c9, d2), (a2, b2, c9, d3), (a2, b2, c9, d4), (a2, b2, c9, d5), (a2, b2, c9, d6), (a2, b2, c9, d7), (a2, b2, c9, d8), (a2, b2, c9, d9), (a2, b2, c9, d10), (a2, b2, c9, d11), (a2, b2, c9, d12), (a2, b2, c10, d1), (a2, b2, c10, d2), (a2, b2, c10, d3), (a2, b2, c10, d4), (a2, b2, c10, d5), (a2, b2, c10, d6), (a2, b2, c10, d7), (a2, b2, c10, d8), (a2, b2, c10, d9), (a2, b2, c10, d10), (a2, b2, c10, d11), (a2, b2, c10, d12), (a2, b2, c11, d1), (a2, b2, c11, d2), (a2, b2, c11, d3), (a2, b2, c11, d4), (a2, b2, c11, d5), (a2, b2, c11, d6), (a2, b2, c11, d7), (a2, b2, c11, d8), (a2, b2, c11, d9), (a2, b2, c11, d10), (a2, b2, c11, d11), (a2, b2, c11, d12), (a2, b2, c12, d1), (a2, b2, c12, d2), (a2, b2, c12, d3), (a2, b2, c12, d4), (a2, b2, c12, d5), (a2, b2, c12, d6), (a2, b2, c12, d7), (a2, b2, c12, d8), (a2, b2, c12, d9), (a2, b2, c12, d10), (a2, b2, c12, d11), (a2, b2, c12, d12), (a2, b2, c13, d1), (a2, b2, c13, d2), (a2, b2, c13, d3), (a2, b2, c13, d4), (a2, b2, c13, d5), (a2, b2, c13, d6), (a2, b2, c13, d7), (a2, b2, c13, d8), (a2, b2, c13, d9), (a2, b2, c13, d10), (a2, b2, c13, d11), (a2, b2, c13, d12), (a2, b3, c1, d1), (a2, b3, c1, d2), (a2, b3, c1, d3), (a2, b3, c1, d4), (a2, b3, c1, d5), (a2, b3, c1, d6), (a2, b3, c1, d7), (a2, b3, c1, d8), (a2, b3, c1, d9), (a2, b3, c1, d10), (a2, b3, c1, d11), (a2, b3, c1, d12), (a2, b3, c2, d1), (a2, b3, c2, d2), (a2, b3, c2, d3), (a2, b3, c2, d4), (a2, b3, c2, d5), (a2, b3, c2, d6), (a2, b3, c2, d7), (a2, b3, c2, d8), (a2, b3, c2, d9), (a2, b3, c2, d10), (a2, b3, c2, d11), (a2, b3, c2, d12), (a2, b3, c3, d1), (a2, b3, c3, d2), (a2, b3, c3, d3), (a2, b3, c3, d4), (a2, b3, c3, d5), (a2, b3, c3, d6), (a2, b3, c3, d7), (a2, b3, c3, d8), (a2, b3, c3, d9), (a2, b3, c3, d10), (a2, b3, c3, d11), (a2, b3, c3, d12), (a2, b3, c4, d1), (a2, b3, c4, d2), (a2, b3, c4, d3), (a2, b3, c4, d4), (a2, b3, c4, d5), (a2, b3, c4, d6), (a2, b3, c4, d7), (a2, b3, c4, d8), (a2, b3, c4, d9), (a2, b3, c4, d10), (a2, b3, c4, d11), (a2, b3, c4, d12), (a2, b3, c5, d1), (a2, b3, c5, d2), (a2, b3, c5, d3), (a2, b3, c5, d4), (a2, b3, c5, d5), (a2, b3, c5, d6), (a2, b3, c5, d7), (a2, b3, c5, d8), (a2, b3, c5, d9), (a2, b3, c5, d10), (a2, b3, c5, d11), (a2, b3, c5, d12), (a2, b3, c6, d1), (a2, b3, c6, d2), (a2, b3, c6, d3), (a2, b3, c6, d4), (a2, b3, c6, d5), (a2, b3, c6, d6), (a2, b3, c6, d7), (a2, b3, c6, d8), (a2, b3, c6, d9), (a2, b3, c6, d10), (a2, b3, c6, d11), (a2, b3, c6, d12), (a2, b3, c7, d1), (a2, b3, c7, d2), (a2, b3, c7, d3), (a2, b3, c7, d4), (a2, b3, c7, d5), (a2, b3, c7, d6), (a2, b3, c7, d7), (a2, b3, c7, d8), (a2, b3, c7, d9), (a2, b3, c7, d10), (a2, b3, c7, d11), (a2, b3, c7, d12), (a2, b3, c8, d1), (a2, b3, c8, d2), (a2, b3, c8, d3), (a2, b3, c8, d4), (a2, b3, c8, d5), (a2, b3, c8, d6), (a2, b3, c8, d7), (a2, b3, c8, d8), (a2, b3, c8, d9), (a2, b3, c8, d10), (a2, b3, c8, d11), (a2, b3, c8, d12), (a2, b3, c9, d1), (a2, b3, c9, d2), (a2, b3, c9, d3), (a2, b3, c9, d4), (a2, b3, c9, d5), (a2, b3, c9, d6), (a2, b3, c9, d7), (a2, b3, c9, d8), (a2, b3, c9, d9), (a2, b3, c9, d10), (a2, b3, c9, d11), (a2, b3, c9, d12), (a2, b3, c10, d1), (a2, b3, c10, d2), (a2, b3, c10, d3), (a2, b3, c10, d4), (a2, b3, c10, d5), (a2, b3, c10, d6), (a2, b3, c10, d7), (a2, b3, c10, d8), (a2, b3, c10, d9), (a2, b3, c10, d10), (a2, b3, c10, d11), (a2, b3, c10, d12), (a2, b3, c11, d1), (a2, b3, c11, d2), (a2, b3, c11, d3), (a2, b3, c11, d4), (a2, b3, c11, d5), (a2, b3, c11, d6), (a2, b3, c11, d7), (a2, b3, c11, d8), (a2, b3, c11, d9), (a2, b3, c11, d10), (a2, b3, c11, d11), (a2, b3, c11, d12), (a2, b3, c12, d1), (a2, b3, c12, d2), (a2, b3, c12, d3), (a2, b3, c12, d4), (a2, b3, c12, d5), (a2, b3, c12, d6), (a2, b3, c12, d7), (a2, b3, c12, d8), (a2, b3, c12, d9), (a2, b3, c12, d10), (a2, b3, c12, d11), (a2, b3, c12, d12), (a2, b3, c13, d1), (a2, b3, c13, d2), (a2, b3, c13, d3), (a2, b3, c13, d4), (a2, b3, c13, d5), (a2, b3, c13, d6), (a2, b3, c13, d7), (a2, b3, c13, d8), (a2, b3, c13, d9), (a2, b3, c13, d10), (a2, b3, c13, d11), (a2, b3, c13, d12), (a2, b4, c1, d1), (a2, b4, c1, d2), (a2, b4, c1, d3), (a2, b4, c1, d4), (a2, b4, c1, d5), (a2, b4, c1, d6), (a2, b4, c1, d7), (a2, b4, c1, d8), (a2, b4, c1, d9), (a2, b4, c1, d10), (a2, b4, c1, d11), (a2, b4, c1, d12), (a2, b4, c2, d1), (a2, b4, c2, d2), (a2, b4, c2, d3), (a2, b4, c2, d4), (a2, b4, c2, d5), (a2, b4, c2, d6), (a2, b4, c2, d7), (a2, b4, c2, d8), (a2, b4, c2, d9), (a2, b4, c2, d10), (a2, b4, c2, d11), (a2, b4, c2, d12), (a2, b4, c3, d1), (a2, b4, c3, d2), (a2, b4, c3, d3), (a2, b4, c3, d4), (a2, b4, c3, d5), (a2, b4, c3, d6), (a2, b4, c3, d7), (a2, b4, c3, d8), (a2, b4, c3, d9), (a2, b4, c3, d10), (a2, b4, c3, d11), (a2, b4, c3, d12), (a2, b4, c4, d1), (a2, b4, c4, d2), (a2, b4, c4, d3), (a2, b4, c4, d4), (a2, b4, c4, d5), (a2, b4, c4, d6), (a2, b4, c4, d7), (a2, b4, c4, d8), (a2, b4, c4, d9), (a2, b4, c4, d10), (a2, b4, c4, d11), (a2, b4, c4, d12), (a2, b4, c5, d1), (a2, b4, c5, d2), (a2, b4, c5, d3), (a2, b4, c5, d4), (a2, b4, c5, d5), (a2, b4, c5, d6), (a2, b4, c5, d7), (a2, b4, c5, d8), (a2, b4, c5, d9), (a2, b4, c5, d10), (a2, b4, c5, d11), (a2, b4, c5, d12), (a2, b4, c6, d1), (a2, b4, c6, d2), (a2, b4, c6, d3), (a2, b4, c6, d4), (a2, b4, c6, d5), (a2, b4, c6, d6), (a2, b4, c6, d7), (a2, b4, c6, d8), (a2, b4, c6, d9), (a2, b4, c6, d10), (a2, b4, c6, d11), (a2, b4, c6, d12), (a2, b4, c7, d1), (a2, b4, c7, d2), (a2, b4, c7, d3), (a2, b4, c7, d4), (a2, b4, c7, d5), (a2, b4, c7, d6), (a2, b4, c7, d7), (a2, b4, c7, d8), (a2, b4, c7, d9), (a2, b4, c7, d10), (a2, b4, c7, d11), (a2, b4, c7, d12), (a2, b4, c8, d1), (a2, b4, c8, d2), (a2, b4, c8, d3), (a2, b4, c8, d4), (a2, b4, c8, d5), (a2, b4, c8, d6), (a2, b4, c8, d7), (a2, b4, c8, d8), (a2, b4, c8, d9), (a2, b4, c8, d10), (a2, b4, c8, d11), (a2, b4, c8, d12), (a2, b4, c9, d1), (a2, b4, c9, d2), (a2, b4, c9, d3), (a2, b4, c9, d4), (a2, b4, c9, d5), (a2, b4, c9, d6), (a2, b4, c9, d7), (a2, b4, c9, d8), (a2, b4, c9, d9), (a2, b4, c9, d10), (a2, b4, c9, d11), (a2, b4, c9, d12), (a2, b4, c10, d1), (a2, b4, c10, d2), (a2, b4, c10, d3), (a2, b4, c10, d4), (a2, b4, c10, d5), (a2, b4, c10, d6), (a2, b4, c10, d7), (a2, b4, c10, d8),

(a2, b4, c10, d9), (a2, b4, c10, d10), (a2, b4, c10, d11), (a2, b4, c10, d12), (a2, b4, c11, d1), (a2, b4, c11, d2), (a2, b4, c11, d3), (a2, b4, c11, d4), (a2, b4, c11, d5), (a2, b4, c11, d6), (a2, b4, c11, d7), (a2, b4, c11, d8), (a2, b4, c11, d9), (a2, b4, c11, d10), (a2, b4, c11, d11), (a2, b4, c11, d12), (a2, b4, c12, d1), (a2, b4, c12, d2), (a2, b4, c12, d3), (a2, b4, c12, d4), (a2, b4, c12, d5), (a2, b4, c12, d6), (a2, b4, c12, d7), (a2, b4, c12, d8), (a2, b4, c12, d9), (a2, b4, c12, d10), (a2, b4, c12, d11), (a2, b4, c12, d12), (a2, b4, c13, d1), (a2, b4, c13, d2), (a2, b4, c13, d3), (a2, b4, c13, d4), (a2, b4, c13, d5), (a2, b4, c13, d6), (a2, b4, c13, d7), (a2, b4, c13, d8), (a2, b4, c13, d9), (a2, b4, c13, d10), (a2, b4, c13, d11), (a2, b4, c13, d12), (a2, b5, c1, d1), (a2, b5, c1, d2), (a2, b5, c1, d3), (a2, b5, c1, d4), (a2, b5, c1, d5), (a2, b5, c1, d6), (a2, b5, c1, d7), (a2, b5, c1, d8), (a2, b5, c1, d9), (a2, b5, c1, d10), (a2, b5, c1, d11), (a2, b5, c1, d12), (a2, b5, c2, d1), (a2, b5, c2, d2), (a2, b5, c2, d3), (a2, b5, c2, d4), (a2, b5, c2, d5), (a2, b5, c2, d6), (a2, b5, c2, d7), (a2, b5, c2, d8), (a2, b5, c2, d9), (a2, b5, c2, d10), (a2, b5, c2, d11), (a2, b5, c2, d12), (a2, b5, c3, d1), (a2, b5, c3, d2), (a2, b5, c3, d3), (a2, b5, c3, d4), (a2, b5, c3, d5), (a2, b5, c3, d6), (a2, b5, c3, d7), (a2, b5, c3, d8), (a2, b5, c3, d9), (a2, b5, c3, d10), (a2, b5, c3, d11), (a2, b5, c3, d12), (a2, b5, c4, d1), (a2, b5, c4, d2), (a2, b5, c4, d3), (a2, b5, c4, d4), (a2, b5, c4, d5), (a2, b5, c4, d6), (a2, b5, c4, d7), (a2, b5, c4, d8), (a2, b5, c4, d9), (a2, b5, c4, d10), (a2, b5, c4, d11), (a2, b5, c4, d12), (a2, b5, c5, d1), (a2, b5, c5, d2), (a2, b5, c5, d3), (a2, b5, c5, d4), (a2, b5, c5, d5), (a2, b5, c5, d6), (a2, b5, c5, d7), (a2, b5, c5, d8), (a2, b5, c5, d9), (a2, b5, c5, d10), (a2, b5, c5, d11), (a2, b5, c5, d12), (a2, b5, c6, d1), (a2, b5, c6, d2), (a2, b5, c6, d3), (a2, b5, c6, d4), (a2, b5, c6, d5), (a2, b5, c6, d6), (a2, b5, c6, d7), (a2, b5, c6, d8), (a2, b5, c6, d9), (a2, b5, c6, d10), (a2, b5, c6, d11), (a2, b5, c6, d12), (a2, b5, c7, d1), (a2, b5, c7, d2), (a2, b5, c7, d3), (a2, b5, c7, d4), (a2, b5, c7, d5), (a2, b5, c7, d6), (a2, b5, c7, d7), (a2, b5, c7, d8), (a2, b5, c7, d9), (a2, b5, c7, d10), (a2, b5, c7, d11), (a2, b5, c7, d12), (a2, b5, c8, d1), (a2, b5, c8, d2), (a2, b5, c8, d3), (a2, b5, c8, d4), (a2, b5, c8, d5), (a2, b5, c8, d6), (a2, b5, c8, d7), (a2, b5, c8, d8), (a2, b5, c8, d9), (a2, b5, c8, d10), (a2, b5, c8, d11), (a2, b5, c8, d12), (a2, b5, c9, d1), (a2, b5, c9, d2), - (a2, b5, c9, d3), (a2, b5, c9, d4), (a2, b5, c9, d5), (a2, b5, c9, d6), (a2, b5, c9, d7), (a2, b5, c9, d8), (a2, b5, c9, d9), (a2, b5, c9, d10), (a2, b5, c9, d11), (a2, b5, c9, d12), (a2, b5, c10, d1), (a2, b5, c10, d2), (a2, b5, c10, d3), (a2, b5, c10, d4), (a2, b5, c10, d5), (a2, b5, c10, d6), (a2, b5, c10, d7), (a2, b5, c10, d8), (a2, b5, c10, d9), (a2, b5, c10, d10), (a2, b5, c10, d11), (a2, b5, c10, d12), (a2, b5, c11, d1), (a2, b5, c11, d2), (a2, b5, c11, d3), (a2, b5, c11, d4), (a2, b5, c11, d5), (a2, b5, c11, d6), (a2, b5, c11, d7), (a2, b5, c11, d8), (a2, b5, c11, d9), (a2, b5, c11, d10), (a2, b5, c11, d11), (a2, b5, c11, d12), (a2, b5, c12, d1), (a2, b5, c12, d2), (a2, b5, c12, d3), (a2, b5, c12, d4), (a2, b5, c12, d5), (a2, b5, c12, d6), (a2, b5, c12, d7), (a2, b5, c12, d8), (a2, b5, c12, d9), (a2, b5, c12, d10), (a2, b5, c12, d11), (a2, b5, c12, d12), (a2, b5, c13, d1), (a2, b5, c13, d2), (a2, b5, c13, d3), (a2, b5, c13, d4), (a2, b5, c13, d5), (a2, b5, c13, d6), (a2, b5, c13, d7), (a2, b5, c13, d8), (a2, b5, c13, d9), (a2, b5, c13, d10), (a2, b5, c13, d11), (a2, b5, c13, d12), (a2, b6, c1, d1), (a2, b6, c1, d2), (a2, b6, c1, d3), (a2, b6, c1, d4), (a2, b6, c1, d5), (a2, b6, c1, d6), (a2, b6, c1, d7), (a2, b6, c1, d8), (a2, b6, c1, d9), (a2, b6, c1, d10), (a2, b6, c1, d11), (a2, b6, c1, d12), (a2, b6, c2, d1), (a2, b6, c2, d2), (a2, b6, c2, d3), (a2, b6, c2, d4), (a2, b6, c2, d5), (a2, b6, c2, d6), (a2, b6, c2, d7), (a2, b6, c2, d8), (a2, b6, c2, d9), (a2, b6, c2, d10), (a2, b6, c2, d11), (a2, b6, c2, d12), (a2, b6, c3, d1), (a2, b6, c3, d2), (a2, b6, c3, d3), (a2, b6, c3, d4), (a2, b6, c3, d5), (a2, b6, c3, d6), (a2, b6, c3, d7), (a2, b6, c3, d8), (a2, b6, c3, d9), (a2, b6, c3, d10), (a2, b6, c3, d11), (a2, b6, c3, d12), (a2, b6, c4, d1), (a2, b6, c4, d2), (a2, b6, c4, d3), (a2, b6, c4, d4), (a2, b6, c4, d5), (a2, b6, c4, d6), (a2, b6, c4, d7), (a2, b6, c4, d8), (a2, b6, c4, d9), (a2, b6, c4, d10), (a2, b6, c4, d11), (a2, b6, c4, d12), (a2, b6, c5, d1), (a2, b6, c5, d2), (a2, b6, c5, d3), (a2, b6, c5, d4), (a2, b6, c5, d5), (a2, b6, c5, d6), (a2, b6, c5, d7), (a2, b6, c5, d8), (a2, b6, c5, d9), (a2, b6, c5, d10), (a2, b6, c5, d11), (a2, b6, c5, d12), (a2, b6, c6, d1), (a2, b6, c6, d2), (a2, b6, c6, d3), (a2, b6, c6, d4), (a2, b6, c6, d5), (a2, b6, c6, d6), (a2, b6, c6, d7), (a2, b6, c6, d8), (a2, b6, c6, d9), (a2, b6, c6, d10), (a2, b6, c6, d11), (a2, b6, c6, d12), (a2, b6, c7, d1), (a2, b6, c7, d2), (a2, b6, c7, d3), (a2, b6, c7, d4), (a2, b6, c7, d5), (a2, b6, c7, d6), (a2, b6, c7, d7), (a2, b6, c7, d8), (a2, b6, c7, d9), (a2, b6, c7, d10), (a2, b6, c7, d11), (a2, b6, c7, d12), (a2, b6, c8, d1), (a2, b6, c8, d2), (a2, b6, c8, d3), (a2, b6, c8, d4), (a2, b6, c8, d5), (a2, b6, c8, d6), (a2, b6, c8, d7), (a2, b6, c8, d8), (a2, b6, c8, d9), (a2, b6, c8, d10), (a2, b6, c8, d11), (a2, b6, c8, d12), (a2, b6, c9, d1), (a2, b6, c9, d2), (a2, b6, c9, d3), (a2, b6, c9, d4), (a2, b6, c9, d5), (a2, b6, c9, d6), (a2, b6, c9, d7), (a2, b6, c9, d8), (a2, b6, c9, d9), (a2, b6, c9, d10), (a2, b6, c9, d11), (a2, b6, c9, d12), (a2, b6, c10, d1), (a2, b6, c10, d2), (a2, b6, c10, d3), (a2, b6, c10, d4), (a2, b6, c10, d5), (a2, b6, c10, d6), (a2, b6, c10, d7), (a2, b6, c10, d8), (a2, b6, c10, d9), (a2, b6, c10, d10), (a2, b6, c10, d11), (a2, b6, c10, d12), (a2, b6, c11, d1), (a2, b6, c11, d2), (a2, b6, c11, d3), (a2, b6, c11, d4), (a2, b6, c11, d5), (a2, b6, c11, d6), (a2, b6, c11, d7), (a2, b6, c11, d8), (a2, b6, c11, d9), (a2, b6, c11, d10), (a2, b6, c11, d11), (a2, b6, c11, d12), (a2, b6, c12, d1), (a2, b6, c12, d2), (a2, b6, c12, d3), (a2, b6, c12, d4), (a2, b6, c12, d5), (a2, b6, c12, d6), (a2, b6, c12, d7), (a2, b6, c12, d8), (a2, b6, c12, d9), (a2, b6, c12, d10), (a2, b6, c12, d11), (a2, b6, c12, d12), (a2, b6, c13, d1), (a2, b6, c13, d2), (a2, b6, c13, d3), (a2, b6, c13, d4), (a2, b6, c13, d5), (a2, b6, c13, d6), (a2, b6, c13, d7), (a2, b6, c13, d8), (a2, b6, c13, d9), (a2, b6, c13, d10), (a2, b6, c13, d11), (a2, b6, c13, d12), (a2, b7, c1, d1), (a2, b7, c1, d2), (a2, b7, c1, d3), (a2, b7, c1, d4), (a2, b7, c1, d5), (a2, b7, c1, d6), (a2, b7, c1, d7), (a2, b7, c1, d8), (a2, b7, c1, d9), (a2, b7, c1, d10), (a2, b7, c1, d11), (a2, b7, c1, d12), (a2, b7, c2, d1), (a2, b7, c2, d2), (a2, b7, c2, d3), (a2, b7, c2, d4), (a2, b7, c2, d5), (a2, b7, c2, d6), (a2, b7, c2, d7), (a2, b7, c2, d8), (a2, b7, c2, d9), (a2, b7, c2, d10), (a2, b7, c2, d11), (a2, b7, c2, d12), (a2, b7, c3, d1), (a2, b7, c3, d2), (a2, b7, c3, d3), (a2, b7, c3, d4), (a2, b7, c3, d5), (a2, b7, c3, d6), (a2, b7, c3, d7), (a2, b7, c3, d8), (a2, b7, c3, d9), (a2, b7, c3, d10), (a2, b7, c3, d11), (a2, b7, c3, d12), (a2, b7, c4, d1), (a2, b7, c4, d2), (a2, b7, c4, d3), (a2, b7, c4, d4), (a2, b7, c4, d5), (a2, b7, c4, d6), (a2, b7, c4, d7), (a2, b7, c4, d8), (a2, b7, c4, d9), (a2, b7, c4, d10), (a2, b7, c4, d11), (a2, b7, c4,

d12), (a2, b7, c5, d1), (a2, b7, c5, d2), (a2, b7, c5, d3), (a2, b7, c5, d4), (a2, b7, c5, d5), (a2, b7, c5, d6), (a2, b7, c5, d7), (a2, b7, c5, d8), (a2, b7, c5, d9), (a2, b7, c5, d10), (a2, b7, c5, d11), (a2, b7, c5, d12), (a2, b7, c6, d1), (a2, b7, c6, d2), (a2, b7, c6, d3), (a2, b7, c6, d4), (a2, b7, c6, d5), (a2, b7, c6, d6), (a2, b7, c6, d7), (a2, b7, c6, d8), (a2, b7, c6, d9), (a2, b7, c6, d10), (a2, b7, c6, d11), (a2, b7, c6, d12), (a2, b7, c7, d1), (a2, b7, c7, d2), (a2, b7, c7, d3), (a2, b7, c7, d4), (a2, b7, c7, d5), (a2, b7, c7, d6), (a2, b7, c7, d7), (a2, b7, c7, d8), (a2, b7, c7, d9), (a2, b7, c7, d10), (a2, b7, c7, d11), (a2, b7, c7, d12), (a2, b7, c8, d1), (a2, b7, c8, d2), (a2, b7, c8, d3), (a2, b7, c8, d4), (a2, b7, c8, d5), (a2, b7, c8, d6), (a2, b7, c8, d7), (a2, b7, c8, d8), (a2, b7, c8, d9), (a2, b7, c8, d10), (a2, b7, c8, d11), (a2, b7, c8, d12), (a2, b7, c9, d1), (a2, b7, c9, d2), (a2, b7, c9, d3), (a2, b7, c9, d4), (a2, b7, c9, d5), (a2, b7, c9, d6), (a2, b7, c9, d7), (a2, b7, c9, d8), (a2, b7, c9, d9), (a2, b7, c9, d10), (a2, b7, c9, d11), (a2, b7, c9, d12), (a2, b7, c10, d1), (a2, b7, c10, d2), (a2, b7, c10, d3), (a2, b7, c10, d4), (a2, b7, c10, d5), (a2, b7, c10, d6), (a2, b7, c10, d7), (a2, b7, c10, d8), (a2, b7, c10, d9), (a2, b7, c10, d10), (a2, b7, c10, d11), (a2, b7, c10, d12), (a2, b7, c11, d1), (a2, b7, c11, d2), (a2, b7, c11, d3), (a2, b7, c11, d4), (a2, b7, c11, d5), (a2, b7, c11, d6), (a2, b7, c11, d7), (a2, b7, c11, d8), (a2, b7, c11, d9), (a2, b7, c11, d10), (a2, b7, c11, d11), (a2, b7, c11, d12), (a2, b7, c12, d1), (a2, b7, c12, d2), (a2, b7, c12, d3), (a2, b7, c12, d4), (a2, b7, c12, d5), (a2, b7, c12, d6), (a2, b7, c12, d7), (a2, b7, c12, d8), (a2, b7, c12, d9), (a2, b7, c12, d10), (a2, b7, c12, d11), (a2, b7, c12, d12), (a2, b7, c13, d1), (a2, b7, c13, d2), (a2, b7, c13, d3), (a2, b7, c13, d4), (a2, b7, c13, d5), (a2, b7, c13, d6), (a2, b7, c13, d7), (a2, b7, c13, d8), (a2, b7, c13, d9), (a2, b7, c13, d10), (a2, b7, c13, d11), (a2, b7, c13, d12), (a2, b8, c1, d1), (a2, b8, c1, d2), (a2, b8, c1, d3), (a2, b8, c1, d4), (a2, b8, c1, d5), (a2, b8, c1, d6), (a2, b8, c1, d7), (a2, b8, c1, d8), (a2, b8, c1, d9), (a2, b8, c1, d10), (a2, b8, c1, d11), (a2, b8, c1, d12), (a2, b8, c2, d1), (a2, b8, c2, d2), (a2, b8, c2, d3), (a2, b8, c2, d4), (a2, b8, c2, d5), (a2, b8, c2, d6), (a2, b8, c2, d7), (a2, b8, c2, d8), (a2, b8, c2, d9), (a2, b8, c2, d10), (a2, b8, c2, d11), (a2, b8, c2, d12), (a2, b8, c3, d1), (a2, b8, c3, d2), (a2, b8, c3, d3), (a2, b8, c3, d4), (a2, b8, c3, d5), (a2, b8, c3, d6), (a2, b8, c3, d7), (a2, b8, c3, d8), (a2, b8, c3, d9), (a2, b8, c3, d10), (a2, b8, c3, d11), (a2, b8, c3, d12), (a2, b8, c4, d1), (a2, b8, c4, d2), (a2, b8, c4, d3), (a2, b8, c4, d4), (a2, b8, c4, d5), (a2, b8, c4, d6), (a2, b8, c4, d7), (a2, b8, c4, d8), (a2, b8, c4, d9), (a2, b8, c4, d10), (a2, b8, c4, d11), (a2, b8, c4, d12), (a2, b8, c5, d1), (a2, b8, c5, d2), (a2, b8, c5, d3), (a2, b8, c5, d4), (a2, b8, c5, d5), (a2, b8, c5, d6), (a2, b8, c5, d7), (a2, b8, c5, d8), (a2, b8, c5, d9), (a2, b8, c5, d10), (a2, b8, c5, d11), (a2, b8, c5, d12), (a2, b8, c6, d1), (a2, b8, c6, d2), (a2, b8, c6, d3), (a2, b8, c6, d4), (a2, b8, c6, d5), (a2, b8, c6, d6), (a2, b8, c6, d7), (a2, b8, c6, d8), (a2, b8, c6, d9), (a2, b8, c6, d10), (a2, b8, c6, d11), (a2, b8, c6, d12), (a2, b8, c7, d1), (a2, b8, c7, d2), (a2, b8, c7, d3), (a2, b8, c7, d4), (a2, b8, c7, d5), (a2, b8, c7, d6), (a2, b8, c7, d7), (a2, b8, c7, d8), (a2, b8, c7, d9), (a2, b8, c7, d10), (a2, b8, c7, d11), (a2, b8, c7, d12), (a2, b8, c8, d1), (a2, b8, c8, d2), (a2, b8, c8, d3), (a2, b8, c8, d4), (a2, b8, c8, d5), (a2, b8, c8, d6), (a2, b8, c8, d7), (a2, b8, c8, d8), (a2, b8, c8, d9), (a2, b8, c8, d10), (a2, b8, c8, d11), (a2, b8, c8, d12), (a2, b8, c9, d1), (a2, b8, c9, d2), (a2, b8, c9, d3), (a2, b8, c9, d4), (a2, b8, c9, d5), (a2, b8, c9, d6), (a2, b8, c9, d7), (a2, b8, c9, d8), (a2, b8, c9, d9), (a2, b8, c9, d10), (a2, b8, c9, d11), (a2, b8, c9, d12), (a2, b8, c10, d1), (a2, b8, c10, d2), (a2, b8, c10, d3), (a2, b8, c10, d4), (a2, b8, c10, d5), (a2, b8, c10, d6), (a2, b8, c10, d7), (a2, b8, c10, d8), (a2, b8, c10, d9), (a2, b8, c10, d10), (a2, b8, c10, d11), (a2, b8, c10, d12), (a2, b8, c11, d1), (a2, b8, c11, d2), (a2, b8, c11, d3), (a2, b8, c11, d4), (a2, b8, c11, d5), (a2, b8, c11, d6), (a2, b8, c11, d7), (a2, b8, c11, d8), (a2, b8, c11, d9), (a2, b8, c11, d10), (a2, b8, c11, d11), (a2, b8, c11, d12), (a2, b8, c12, d1), (a2, b8, c12, d2), (a2, b8, c12, d3), (a2, b8, c12, d4), (a2, b8, c12, d5), (a2, b8, c12, d6), (a2, b8, c12, d7), (a2, b8, c12, d8), (a2, b8, c12, d9), (a2, b8, c12, d10), (a2, b8, c12, d11), (a2, b8, c12, d12), (a2, b8, c13, d1), (a2, b8, c13, d2), (a2, b8, c13, d3), (a2, b8, c13, d4), (a2, b8, c13, d5), (a2, b8, c13, d6), (a2, b8, c13, d7), (a2, b8, c13, d8), (a2, b8, c13, d9), (a2, b8, c13, d10), (a2, b8, c13, d11), (a2, b8, c13, d12), (a2, b9, c1, d1), (a2, b9, c1, d2), (a2, b9, c1, d3), (a2, b9, c1, d4), (a2, b9, c1, d5), (a2, b9, c1, d6), (a2, b9, c1, d7), (a2, b9, c1, d8), (a2, b9, c1, d9), (a2, b9, c1, d10), (a2, b9, c1, d11) (a2, b9, c1, d12), (a2, b9, c2, d1), (a2, b9, c2, d2), (a2, b9, c2, d3), (a2, b9, c2, d4), (a2, b9, c2, d5), (a2, b9, c2, d6), (a2, b9, c2, d7), (a2, b9, c2, d8), (a2, b9, c2, d9), (a2, b9, c2, d10), (a2, b9, c2, d11), (a2, b9, c2, d12), (a2, b9, c3, d1), (a2, b9, c3, d2), (a2, b9, c3, d3), (a2, b9, c3, d4), (a2, b9, c3, d5), (a2, b9, c3, d6), (a2, b9, c3, d7), (a2, b9, c3, d8), (a2, b9, c3, d9), (a2, b9, c3, d10), (a2, b9, c3, d11), (a2, b9, c3, d12), (a2, b9, c4, d1), (a2, b9, c4, d2), (a2, b9, c4, d3), (a2, b9, c4, d4), (a2, b9, c4, d5), (a2, b9, c4, d6), (a2, b9, c4, d7), (a2, b9, c4, d8), (a2, b9, c4, d9), (a2, b9, c4, d10), (a2, b9, c4, d11), (a2, b9, c4, d12), (a2, b9, c5, d1), (a2, b9, c5, d2), (a2, b9, c5, d3), (a2, b9, c5, d4), (a2, b9, c5, d5), (a2, b9, c5, d6), (a2, b9, c5, d7), (a2, b9, c5, d8), (a2, b9, c5, d9), (a2, b9, c5, d10), (a2, b9, c5, d11), (a2, b9, c5, d12), (a2, b9, c6, d1), (a2, b9, c6, d2), (a2, b9, c6, d3), (a2, b9, c6, d4), (a2, b9, c6, d5), (a2, b9, c6, d6), (a2, b9, c6, d7), (a2, b9, c6, d8), (a2, b9, c6, d9), (a2, b9, c6, d10), (a2, b9, c6, d11), (a2, b9, c6, d12), (a2, b9, c7, d1), (a2, b9, c7, d2), (a2, b9, c7, d3), (a2, b9, c7, d4), (a2, b9, c7, d5), (a2, b9, c7, d6), (a2, b9, c7, d7), (a2, b9, c7, d8), (a2, b9, c7, d9), (a2, b9, c7, d10), (a2, b9, c7, d11), (a2, b9, c7, d12), (a2, b9, c8, d1), (a2, b9, c8, d2), (a2, b9, c8, d3), (a2, b9, c8, d4), (a2, b9, c8, d5), (a2, b9, c8, d6), (a2, b9, c8, d7), (a2, b9, c8, d8), (a2, b9, c8, d9), (a2, b9, c8, d10), (a2, b9, c8, d11), (a2, b9, c8, d12), (a2, b9, c9, d1), (a2, b9, c9, d2), (a2, b9, c9, d3), (a2, b9, c9, d4), (a2, b9, c9, d5), (a2, b9, c9, d6), (a2, b9, c9, d7), (a2, b9, c9, d8), (a2, b9, c9, d9), (a2, b9, c9, d10), (a2, b9, c9, d11), (a2, b9, c9, d12), (a2, b9, c10, d1), (a2, b9, c10, d2), (a2, b9, c10, d3), (a2, b9, c10, d4), (a2, b9, c10, d5), (a2, b9, c10, d6), (a2, b9, c10, d7), (a2, b9, c10, d8), (a2, b9, c10, d9), (a2, b9, c10, d10), (a2, b9, c10, d11), (a2, b9, c10, d12), (a2, b9, c11, d1), (a2, b9, c11, d2), (a2, b9, c11, d3), (a2, b9, c11, d4), (a2, b9, c11, d5), (a2, b9, c11, d6), (a2, b9, c11, d7), (a2, b9, c11, d8), (a2, b9, c11, d9), (a2, b9, c11, d10), (a2, b9, c11, d11), (a2, b9, c11, d12), (a2, b9, c12, d1), (a2, b9, c12, d2), (a2, b9, c12, d3), (a2, b9, c12,

d4), (a2, b9, c12, d5), (a2, b9, c12, d6), (a2, b9, c12, d7), (a2, b9, c12, d8), (a2, b9, c12, d9), (a2, b9, c12, d10), (a2, b9, c12, d11), (a2, b9, c12, d12), (a2, b9, c13, d1), (a2, b9, c13, d2), (a2, b9, c13, d3), (a2, b9, c13, d4), (a2, b9, c13, d5), (a2, b9, c13, d6), (a2, b9, c13, d7), (a2, b9, c13, d8), (a2, b9, c13, d9), (a2, b9, c13, d10), (a2, b9, c13, d11), (a2, b9, c13, d12), (a2, b10, c1, d1), (a2, b10, c1, d2), (a2, b10, c1, d3), (a2, b10, c1, d4), (a2, b10, c1, d5), (a2, b10, c1, d6), (a2, b10, c1, d7), (a2, b10, c1, d8), (a2, b10, c1, d9), (a2, b10, c1, d10), (a2, b10, c1, d11), (a2, b10, c1, d12), (a2, b10, c2, d1), (a2, b10, c2, d2), (a2, b10, c2, d3), (a2, b10, c2, d4), (a2, b10, c2, d5), (a2, b10, c2, d6), (a2, b10, c2, d7), (a2, b10, c2, d8), (a2, b10, c2, d9), (a2, b10, c2, d10), (a2, b10, c2, d11), (a2, b10, c2, d12), (a2, b10, c3, d1), (a2, b10, c3, d2), (a2, b10, c3, d3), (a2, b10, c3, d4), (a2, b10, c3, d5), (a2, b10, c3, d6), (a2, b10, c3, d7), (a2, b10, c3, d8), (a2, b10, c3, d9), (a2, b10, c3, d10), (a2, b10, c3, d11), (a2, b10, c3, d12), (a2, b10, c4, d1), (a2, b10, c4, d2), (a2, b10, c4, d3), (a2, b10, c4, d4), (a2, b10, c4, d5), (a2, b10, c4, d6), (a2, b10, c4, d7), (a2, b10, c4, d8), (a2, b10, c4, d9), (a2, b10, c4, d10), (a2, b10, c4, d11), (a2, b10, c4, d12), (a2, b10, c5, d1), (a2, b10, c5, d2), (a2, b10, c5, d3), (a2, b10, c5, d4), (a2, b10, c5, d5), (a2, b10, c5, d6), (a2, b10, c5, d7), (a2, b10, c5, d8), (a2, b10, c5, d9), (a2, b10, c5, d10), (a2, b10, c5, d11), (a2, b10, c5, d12), (a2, b10, c6, d1), (a2, b10, c6, d2), (a2, b10, c6, d3), (a2, b10, c6, d4), (a2, b10, c6, d5), (a2, b10, c6, d6), (a2, b10, c6, d7), (a2, b10, c6, d8), (a2, b10, c6, d9), (a2, b10, c6, d10), (a2, b10, c6, d11), (a2, b10, c6, d12), (a2, b10, c7, d1), (a2, b10, c7, d2), (a2, b10, c7, d3), (a2, b10, c7, d4), (a2, b10, c7, d5), (a2, b10, c7, d6), (a2, b10, c7, d7), (a2, b10, c7, d8), (a2, b10, c7, d9), (a2, b10, c7, d10), (a2, b10, c7, d11), (a2, b10, c7, d12), (a2, b10, c8, d1), (a2, b10, c8, d2), (a2, b10, c8, d3), (a2, b10, c8, d4), (a2, b10, c8, d5), (a2, b10, c8, d6), (a2, b10, c8, d7), (a2, b10, c8, d8), (a2, b10, c8, d9), (a2, b10, c8, d10), (a2, b10, c8, d11), (a2, b10, c8, d12), (a2, b10, c9, d1), (a2, b10, c9, d2), (a2, b10, c9, d3), (a2, b10, c9, d4), (a2, b10, c9, d5), (a2, b10, c9, d6), (a2, b10, c9, d7), (a2, b10, c9, d8), (a2, b10, c9, d9), (a2, b10, c9, d10), (a2, b10, c9, d11), (a2, b10, c9, d12), (a2, b10, c10, d1), (a2, b10, c10, d2), (a2, b10, c10, d3), (a2, b10, c10, d4), (a2, b10, c10, d5), (a2, b10, c10, d6), (a2, b10, c10, d7), (a2, b10, c10, d8), (a2, b10, c10, d9), (a2, b10, c10, d10), (a2, b10, c10, d11), (a2, b10, c10, d12), (a2, b10, c11, d1), (a2, b10, c11, d2), (a2, b10, c11, d3), (a2, b10, c11, d4), (a2, b10, c11, d5), (a2, b10, c11, d6), (a2, b10, c11, d7), (a2, b10, c11, d8), (a2, b10, c11, d9), (a2, b10, c11, d10), (a2, b10, c11, d11), (a2, b10, c11, d12), (a2, b10, c12, d1), (a2, b10, c12, d2), (a2, b10, c12, d3), (a2, b10, c12, d4), (a2, b10, c12, d5), (a2, b10, c12, d6), (a2, b10, c12, d7), (a2, b10, c12, d8), (a2, b10, c12, d9), (a2, b10, c12, d10), (a2, b10, c12, d11), (a2, b10, c12, d12), (a2, b10, c13, d1), (a2, b10, c13, d2), (a2, b10, c13, d3), (a2, b10, c13, d4), (a2, b10, c13, d5), (a2, b10, c13, d6), (a2, b10, c13, d7), (a2, b10, c13, d8), (a2, b10, c13, d9), (a2, b10, c13, d10), (a2, b10, c13, d11), (a2, b10, c13, d12), (a2, b11, c1, d1), (a2, b11, c1, d2), (a2, b11, c1, d3), (a2, b11, c1, d4), (a2, b11, c1, d5), (a2, b11, c1, d6), (a2, b11, c1, d7), (a2, b11, c1, d8), (a2, b11, c1, d9), (a2, b11, c1, d10), (a2, b11, c1, d11), (a2, b11, c1, d12), (a2, b11, c2, d1), (a2, b11, c2, d2), (a2, b11, c2, d3), (a2, b11, c2, d4), (a2, b11, c2, d5), (a2, b11, c2, d6), (a2, b11, c2, d7), (a2, b11, c2, d8), (a2, b11, c2, d9), (a2, b11, c2, d10), (a2, b11, c2, d11), (a2, b11, c2, d12), (a2, b11, c3, d1), (a2, b11, c3, d2), (a2, b11, c3, d3), (a2, b11, c3, d4), (a2, b11, c3, d5), (a2, b11, c3, d6), (a2, b11, c3, d7), (a2, b11, c3, d8), (a2, b11, c3, d9), (a2, b11, c3, d10), (a2, b11, c3, d11), (a2, b11, c3, d12), (a2, b11, c4, d1), (a2, b11, c4, d2), (a2, b11, c4, d3), (a2, b11, c4, d4), (a2, b11, c4, d5), (a2, b11, c4, d6), (a2, b11, c4, d7), (a2, b11, c4, d8), (a2, b11, c4, d9), (a2, b11, c4, d10), (a2, b11, c4, d11), (a2, b11, c4, d12), (a2, b11, c5, d1), (a2, b11, c5, d2), (a2, b11, c5, d3), (a2, b11, c5, d4), (a2, b11, c5, d5), (a2, b11, c5, d6), (a2, b11, c5, d7), (a2, b11, c5, d8), (a2, b11, c5, d9), (a2, b11, c5, d10), (a2, b11, c5, d11), (a2, b11, c5, d12), (a2, b11, c6, d1), (a2, b11, c6, d2), (a2, b11, c6, d3), (a2, b11, c6, d4), (a2, b11, c6, d5), (a2, b11, c6, d6), (a2, b11, c6, d7), (a2, b11, c6, d8), (a2, b11, c6, d9), (a2, b11, c6, d10), (a2, b11, c6, d11), (a2, b11, c6, d12), (a2, b11, c7, d1), (a2, b11, c7, d2), (a2, b11, c7, d3), (a2, b11, c7, d4), (a2, b11, c7, d5), (a2, b11, c7, d6), (a2, b11, c7, d7), (a2, b11, c7, d8), (a2, b11, c7, d9), (a2, b11, c7, d10), (a2, b11, c7, d11), (a2, b11, c7, d12), (a2, b11, c8, d1), (a2, b11, c8, d2), (a2, b11, c8, d3), (a2, b11, c8, d4), (a2, b11, c8, d5), (a2, b11, c8, d6), (a2, b11, c8, d7), (a2, b11, c8, d8), (a2, b11, c8, d9), (a2, b11, c8, d10), (a2, b11, c8, d11), (a2, b11, c8, d12), (a2, b11, c9, d1), (a2, b11, c9, d2), (a2, b11, c9, d3), (a2, b11, c9, d4), (a2, b11, c9, d5), (a2, b11, c9, d6), (a2, b11, c9, d7), (a2, b11, c9, d8), (a2, b11, c9, d9), (a2, b11, c9, d10), (a2, b11, c9, d11), (a2, b11, c9, d12), (a2, b11, c10, d1), (a2, b11, c10, d2), (a2, b11, c10, d3), (a2, b11, c10, d4), (a2, b11, c10, d5), (a2, b11, c10, d6), (a2, b11, c10, d7), (a2, b11, c10, d8), (a2, b11, c10, d9), (a2, b11, c10, d10), (a2, b11, c10, d11), (a2, b11, c10, d12), (a2, b11, c11, d1), (a2, b11, c11, d2), (a2, b11, c11, d3), (a2, b11, c11, d4), (a2, b11, c11, d5), (a2, b11, c11, d6), (a2, b11, c11, d7), (a2, b11, c11, d8), (a2, b11, c11, d9), (a2, b11, c11, d10), (a2, b11, c11, d11), (a2, b11, c11, d12), (a2, b11, c12, d1), (a2, b11, c12, d2), (a2, b11, c12, d3), (a2, b11, c12, d4), (a2, b11, c12, d5), (a2, b11, c12, d6), (a2, b11, c12, d7), (a2, b11, c12, d8), (a2, b11, c12, d9), (a2, b11, c12, d10), (a2, b11, c12, d11), (a2, b11 c12, d12), (a2, b11, c13, d1), (a2, b11, c13, d2), (a2, b11, c13, d3), (a2, b11, c13, d4), (a2, b11, c13, d5), (a2, b11, c13, d6), (a2, b11, c13, d7), (a2, b11, c13, d8), (a2, b11, c13, d9), (a2, b11, c13, d10), (a2, b11, c13, d11), (a2, b11, c13, d12), (a2, b12, c1, d1), (a2, b12, c1, d2), (a2, b12, c1, d3), (a2, b12, c1, d4), (a2, b12, c1, d5), (a2, b12, c1, d6), (a2, b12, c1, d7), (a2, b12, c1, d8), (a2, b12, c1, d9), (a2, b12, c1, d10), (a2, b12, c1, d11), (a2, b12, c1, d12), (a2, b12, c2, d1), (a2, b12, c2, d2), (a2, b12, c2, d3), (a2, b12, c2, d4), (a2, b12, c2, d5), (a2, b12, c2, d6), (a2, b12, c2, d7), (a2, b12, c2, d8), (a2, b12, c2, d9), (a2, b12, c2, d10), (a2, b12, c2, d11), (a2, b12, c2, d12), (a2, b12, c3, d1), (a2, b12, c3, d2), (a2, b12, c3, d3), (a2, b12, c3, d4), (a2, b12, c3, d5), (a2, b12, c3, d6), (a2, b12, c3, d7), (a2, b12, c3, d8), (a2, b12, c3, d9), (a2, b12, c3, d10), (a2, b12, c3, d11), (a2, b12, c3, d12), (a2, b12, c4, d1), (a2, b12, c4, d2), (a2, b12, c4, d3), (a2, b12, c4, d4), (a2, b12, c4, d5), (a2, b12, c4,

d6), (a2, b12, c4, d7), (a2, b12, c4, d8), (a2, b12, c4, d9), (a2, b12, c4, d10), (a2, b12, c4, d11), (a2, b12, c4, d12), (a2, b12, c5, d1), (a2, b12, c5, d2), (a2, b12, c5, d3), (a2, b12, c5, d4), (a2, b12, c5, d5), (a2, b12, c5, d6), (a2, b12, c5, d7), (a2, b12, c5, d8), (a2, b12, c5, d9), (a2, b12, c5, d10), (a2, b12, c5, d11), (a2, b12, c5, d12), (a2, b12, c6, d1), (a2, b12, c6, d2), (a2, b12, c6, d3), (a2, b 12, c6, d4), (a2, b 12, c6, d5), (a2, b 12, c6, d6), (a2, b 12, c6, d7), (a2, b12, c6, d8), (a2, b12, c6, d9), (a2, b12, c6, d10), (a2, b12, c6, d11), (a2, b12, c6, d12), (a2, b12, c7, d1), (a2, b12, c7, d2), (a2, b12, c7, d3), (a2, b12, c7, d4), (a2, b12, c7, d5), (a2, b12, c7, d6), (a2, b12, c7, d7), (a2, b12, c7, d8), (a2, b12, c7, d9), (a2, b12, c7, d10), (a2, b12, c7, d11), (a2, b12, c7, d12), (a2, b12, c8, d1), (a2, b12, c8, d2), (a2, b12, c8, d3), (a2, b12, c8, d4), (a2, b12, c8, d5), (a2, b12, c8, d6), (a2, b12, c8, d7), (a2, b12, c8, d8), (a2, b12, c8, d9), (a2, b12, c8, d10), (a2, b12, c8, d11), (a2, b12, c8, d12), (a2, b12, c9, d1), (a2, b12, c9, d2), (a2, b12, c9, d3), (a2, b12, c9, d4), (a2, b12, c9, d5), (a2, b12, c9, d6), (a2, b12, c9, d7), (a2, b12, c9, d8), (a2, b12, c9, d9), (a2, b12, c9, d10), (a2, b12, c9, d11), (a2, b12, c9, d12), (a2, b12, c10, d1), (a2, b12, c10, d2), (a2, b12, c10, d3), (a2, b12, c10, d4), (a2, b12, c10, d5), (a2, b12, c10, d6), (a2, b12, c10, d7), (a2, b12, c10, d8), (a2, b12, c10, d9), (a2, b12, c10, d10), (a2, b12, c10, d11), (a2, b12, c10, d12), (a2, b12, c11, d1), (a2, b12, c11, d2), (a2, b12, c11, d3), (a2, b12, c11, d4), (a2, b12, c11, d5), (a2, b12, c11, d6), (a2, b12, c11, d7), (a2, b12, c11, d8), (a2, b12, c11, d9), (a2, b12, c11, d10), (a2, b12, c11, d11), (a2, b12, c11, d12), (a2, b12, c12, d1), (a2, b12, c12, d2), (a2, b12, c12, d3), (a2, b12, c12, d4), (a2, b12, c12, d5), (a2, b12, c12, d6), (a2, b12, c12, d7), (a2, b12, c12, d8), (a2, b12, c12, d9), (a2, b12, c12, d10), (a2, b12, c12, d11), (a2, b12, c12, d12), (a2, b12, c13, d1), (a2, b12, c13, d2), (a2, b12, c13, d3), (a2, b12, c13, d4), (a2, b12, c13, d5), (a2, b12, c13, d6), (a2, b12, c13, d7), (a2, b12, c13, d8), (a2, b12, c13, d9), (a2, b12, c13, d10), (a2, b12, c13, d11), (a2, b12, c13, d12), (a2, b13, c1, d1), (a2, b13, c1, d2), (a2, b13, c1, d3), (a2, b13, c1, d4), (a2, b13, c1, d5), (a2, b13, c1, d6), (a2, b13, c1, d7), (a2, b13, c1, d8), (a2, b13, c1, d9), (a2, b13, c1, d10), (a2, b13, c1, d11), (a2, b13, c1, d12), (a2, b13, c2, d1), (a2, b13, c2, d2), (a2, b13, c2, d3), (a2, b13, c2, d4), (a2, b13, c2, d5), (a2, b13, c2, d6), (a2, b13, c2, d7), (a2, b13, c2, d8), (a2, b13, c2, d9), (a2, b13, c2, d10), (a2, b13, c2, d11), (a2, b13, c2, d12), (a2, b13, c3, d1), (a2, b13, c3, d2), (a2, b13, c3, d3), (a2, b13, c3, d4), (a2, b13, c3, d5), (a2, b13, c3, d6), (a2, b13, c3, d7), (a2, b13, c3, d8), (a2, b13, c3, d9), (a2, b13, c3, d10), (a2, b13, c3, d11), (a2, b13, c3, d12), (a2, b13, c4, d1), (a2, b13, c4, d2), (a2, b13, c4, d3), (a2, b13, c4, d4), (a2, b13, c4, d5), (a2, b13, c4, d6), (a2, b13, c4, d7), (a2, b13, c4, d8), (a2, b13, c4, d9), (a2, b13, c4, d10), (a2, b13, c4, d11), (a2, b13, c4, d12), (a2, b13, c5, d1), (a2, b13, c5, d2), (a2, b13, c5, d3), (a2, b13, c5, d4), (a2, b13, c5, d5), (a2, b13, c5, d6), (a2, b13, c5, d7), (a2, b13, c5, d8), (a2, b13, c5, d9), (a2, b13, c5, d10), (a2, b13, c5, d11), (a2, b13, c5, d12), (a2, b13, c6, d1), (a2, b13, c6, d2), (a2, b13, c6, d3), (a2, b13, c6, d4), (a2, b13, c6, d5), (a2, b13, c6, d6), (a2, b13, c6, d7), (a2, b13, c6, d8), (a2, b13, c6, d9), (a2, b13, c6, d10), (a2, b13, c6, d11), (a2, b13, c6, d12), (a2, b13, c7, d1), (a2, b13, c7, d2), (a2, b13, c7, d3), (a2, b13, c7, d4), (a2, b13, c7, d5), (a2, b13, c7, d6), (a2, b13, c7, d7), (a2, b13, c7, d8), (a2, b13, c7, d9), (a2, b13, c7, d10), (a2, b13, c7, d11), (a2, b13, c7, d12), (a2, b13, c8, d1), (a2, b13, c8, d2), (a2, b13, c8, d3), (a2, b13, c8, d4), (a2, b13, c8, d5), (a2, b13, c8, d6), (a2, b13, c8, d7), (a2, b13, c8, d8), (a2, b13, c8, d9), (a2, b13, c8, d10), (a2, b13, c8, d11), (a2, b13, c8, d12), (a2, b13, c9, d1), (a2, b13, c9, d2), (a2, b13, c9, d3), (a2, b13, c9, d4), (a2, b13, c9, d5), (a2, b13, c9, d6), (a2, b13, c9, d7), (a2, b13, c9, d8), (a2, b13, c9, d9), (a2, b13, c9, d10), (a2, b13, c9, d11), (a2, b13, c9, d12), (a2, b13, c10, d1), (a2, b13, c10, d2), (a2, b13, c10, d3), (a2, b13, c10, d4), (a2, b13, c10, d5), (a2, b13, c10, d6), (a2, b13, c10, d7), (a2, b13, c10, d8), (a2, b13, c10, d9), (a2, b13, c10, d10), (a2, b13, c10, d11), (a2, b13, c10, d12), (a2, b13, c11, d1), (a2, b13, c11, d2), (a2, b13, c11, d3), (a2, b13, c11, d4), (a2, b13, c11, d5), (a2, b13, c11, d6), (a2, b13, c11, d7), (a2, b13, c11, d8), (a2, b13, c11, d9), (a2, b13, c11, d10), (a2, b13, c11, d11), (a2, b13, c11, d12), (a2, b13, c12, d1), (a2, b13, c12, d2), (a2, b13, c12, d3), (a2, b13, c12, d4), (a2, b13, c12, d5), (a2, b13, c12, d6), (a2, b13, c12, d7), (a2, b13, c12, d8), (a2, b13, c12, d9), (a2, b13, c12, d10), (a2, b13, c12, d11), (a2, b13, c12, d12), (a2, b13, c13, d1), (a2, b13, c13, d2), (a2, b13, c13, d3), (a2, b13, c13, d4), (a2, b13, c13, d5), (a2, b13, c13, d6), (a2, b13, c13, d7), (a2, b13, c13, d8), (a2, b13, c13, d9), (a2, b13, c13, d10), (a2, b13, c13, d11), (a2, b13, c13, d12), (a2, b14, c1, d1), (a2, b14, c1, d2), (a2, b14, c1, d3), (a2, b14, c1, d4), (a2, b14, c1, d5), (a2, b14, c1, d6), (a2, b14, c1, d7), (a2, b14, c1, d8), (a2, b14, c1, d9), (a2, b14, c1, d10), (a2, b14, c1, d11), (a2, b14, c1, d12), (a2, b14, c2, d1), (a2, b14, c2, d2), (a2, b14, c2, d3), (a2, b14, c2, d4), (a2, b14, c2, d5), (a2, b14, c2, d6), (a2, b14, c2, d7), (a2, b14, c2, d8), (a2, b14, c2, d9), (a2, b14, c2, d10), (a2, b14, c2, d11), (a2, b14, c2,-d12), (a2, b14, c3, d1), (a2, b14, c3, d2), (a2, b14, c3, d3), (a2, b14, c3, d4), (a2, b14, c3, d5), (a2, b14, c3, d6), (a2, b14, c3, d7), (a2, b14, c3, d8), (a2, b14, c3, d9), (a2, b14, c3, d10), (a2, b14, c3, d11), (a2, b14, c3, d12), (a2, b14, c4, d1), (a2, b14, c4, d2), (a2, b14, c4, d3), (a2, b14, c4, d4), (a2, b14, c4, d5), (a2, b14, c4, d6), (a2, b14, c4, d7), (a2, b14, c4, d8), (a2, b14, c4, d9), (a2, b14, c4, d10), (a2, b14, c4, d11), (a2, b14, c4, d12), (a2, b14, c5, d1), (a2, b14, c5, d2), (a2, b14, c5, d3), (a2, b14, c5, d4), (a2, b14, c5, d5), (a2, b14, c5, d6), (a2, b14, c5, d7), (a2, b14, c5, d8), (a2, b14, c5, d9), (a2, b14, c5, d10), (a2, b14, c5, d11), (a2, b14, c5, d12), (a2, b14, c6, d1), (a2, b14, c6, d2), (a2, b14, c6, d3), (a2, b14, c6, d4), (a2, b14, c6, d5), (a2, b14, c6, d6), (a2, b14, c6, d7), (a2, b14, c6, d8), (a2, b14, c6, d9), (a2, b14, c6, d10), (a2, b14, c6, d11), (a2, b14, c6, d12), (a2, b14, c7, d1), (a2, b14, c7, d2), (a2, b14, c7, d3), (a2, b14, c7, d4), (a2, b14, c7, d5), (a2, b14, c7, d6), (a2, b14, c7, d7), (a2, b14, c7, d8), (a2, b14, c7, d9), (a2, b14, c7, d10), (a2, b14, c7, d11), (a2, b14, c7, d12), (a2, b14, c8, d1), (a2, b14, c8, d2), (a2, b14, c8, d3), (a2, b14, c8, d4), (a2, b14, c8, d5), (a2, b14, c8, d6), (a2, b14, c8, d7), (a2, b14, c8, d8), (a2, b14, c8, d9), (a2, b14, c8, d10), (a2, b14, c8, d11), (a2, b14, c8, d12), (a2, b14, c9, d1), (a2, b14, c9, d2), (a2, b14, c9, d3), (a2, b14, c9, d4), (a2, b14, c9, d5), (a2, b14, c9, d6), (a2, b14, c9, d7), (a2, b14, c9, d8), (a2, b14, c9,

d9), (a2, b14, c9, b10), (a2, b14, c9, d11), (a2, b14, c9, d12), (a2, b14, c10, d1), (a2, b14, c10, d2), (a2, b14, c10, d3), (a2, b14, c10, d4), (a2, b14, c10, d5), (a2, b14, c10, d6), (a2, b14, c10, d7), (a2, b14, c10, d8), (a2, b14, c10, d9), (a2, b14, c10, d10), (a2, b14, c10, d11), (a2, b14, c10, d12), (a2, b14, c11, d1), (a2, b14, c11, d2), (a2, b14, c11, d3), (a2, b14, c11, d4), (a2, b14, c11, d5), (a2, b14, c11, d6), (a2, b14, c11, d7), (a2, b14, c11, d8), (a2, b14, c11, d9), (a2, b14, c11, d10), (a2, b14, c11, d11), (a2, b14, c11, d12), (a2, b14, c12, d1), (a2, b14, c12, d2), (a2, b14, c12, d3), (a2, b14, c12, d4), (a2, b14, c12, d5), (a2, b14, c12, d6), (a2, b14, c12, d7), (a2, b14, c12, d8), (a2, b14, c12, d9), (a2, b14, c12, d10), (a2, b14, c12, d11), (a2, b14, c12, d12), (a2, b14, c13, d1), (a2, b14, c13, d2), (a2, b14, c13, d3), (a2, b14, c13, d4), (a2, b14, c13, d5), (a2, b14, c13, d6), (a2, b14, c13, d7), (a2, b14, c13, d8), (a2, b14, c13, d9), (a2, b14, c13, d10), (a2, b14, c13, d11), (a2, b14, c13, d12), (a2, b15, c1, d1), (a2, b15, c1, d2), (a2, b15, c1, d3), (a2, b15, c1, d4), (a2, b15, c1, d5), (a2, b15, c1, d6), (a2, b15, c1, d7), (a2, b15, c1, d8), (a2, b15, c1, d9), (a2, b15, c1, d10), (a2, b15, c1, d11), (a2, b15, c1, d12), (a2, b15, c2, d1), (a2, b15, c2, d2), (a2, b15, c2, d3), (a2, b15, c2, d4), (a2, b15, c2, d5), (a2, b15, c2, d6), (a2, b15, c2, d7), (a2, b15, c2, d8), (a2, b15, c2, d9), (a2, b15, c2, d10), (a2, b15, c2, d11), (a2, b15, c2, d12), (a2, b15, c3, d1), (a2, b15, c3, d2), (a2, b15, c3, d3), (a2, b15, c3, d4), (a2, b15, c3, d5), (a2, b15, c3, d6), (a2, b15, c3, d7), (a2, b15, c3, d8), (a2, b15, c3, d9), (a2, b15, c3, d10), (a2, b15, c3, d11), (a2, b15, c3, d12), (a2, b15, c4, d1), (a2, b15, c4, d2), (a2, b15, c4, d3), (a2, b15, c4, d4), (a2, b15, c4, d5), (a2, b15, c4, d6), (a2, b15, c4, d7), (a2, b15, c4, d8), (a2, b15, c4, d9), (a2, b15, c4, d10), (a2, b15, c4, d11), (a2, b15, c4, d12), (a2, b15, c5, d1), (a2, b15, c5, d2), (a2, b15, c5, d3), (a2, b15, c5, d4), (a2, b15, c5, d5), (a2, b15, c5, d6), (a2, b15, c5, d7), (a2, b15, c5, d8), (a2, b15, c5, d9), (a2, b15, c5, d10), (a2, b15, c5, d11), (a2, b15, c5, d12), (a2, b15, c6, d1), (a2, b15, c6, d2), (a2, b15, c6, d3), (a2, b15, c6, d4), (a2, b15, c6, d5), (a2, b15, c6, d6), (a2, b15, c6, d7), (a2, b15, c6, d8), (a2, b15, c6, d9), (a2, b15, c6, d10), (a2, b15, c6, d11), (a2, b15, c6, d12), (a2, b15, c7, d1), (a2, b15, c7, d2), (a2, b15, c7, d3), (a2, b15, c7, d4), (a2, b15, c7, d5), (a2, b15, c7, d6), (a2, b15, c7, d7), (a2, b15, c7, d8), (a2, b15, c7, d9), (a2,-b15, c7, d10), (a2, b15, c7, d11), (a2, b15, c7, d12), (a2, b15, c8, d1), (a2, b15, c8, d2), (a2, b15, c8, d3), (a2, b15, c8, d4), (a2, b15, c8, d5), (a2, b15, c8, d6), (a2, b15, c8, d7), (a2, b15, c8, d8), (a2, b15, c8, d9), (a2, b15, c8, d10), (a2, b15, c8, d11), (a2, b15, c8, d12), (a2, b15, c9, d1), (a2, b15, c9, d2), (a2, b15, c9, d3), (a2, b15, c9, d4), (a2, b15, c9, d5), (a2, b15, c9, d6), (a2, b15, c9, d7), (a2, b15, c9, d8), (a2, b15, c9, d9), (a2, b15, c9, d10), (a2, b15, c9, d11), (a2, b15, c9, d12), (a2, b15, c10, d1), (a2, b15, c10, d2), (a2, b15, c10, d3), (a2, b15, c10, d4), (a2, b15, c10, d5), (a2, b15, c10, d6), (a2, b15, c10, d7), (a2, b15, c10, d8), (a2, b15, c10, d9), (a2, b15, c10, d10), (a2, b15, c10, d11), (a2, b15, c10, d12), (a2, b15, c11, d1), (a2, b15, c11, d2), (a2, b15, c11, d3), (a2, b15, c11, d4), (a2, b15, c11, d5), (a2, b15, c11, d6), (a2, b15, c11, d7), (a2, b15, c11, d8), (a2, b15, c11, d9), (a2, b15, c11, d10), (a2, b15, c11, d11), (a2, b15, c11, d12), (a2, b15, c12, d1), (a2, b15, c12, d2), (a2, b15, c12, d3), (a2, b15, c12, d4), (a2, b15, c12, d5), (a2, b15, c12, d6), (a2, b15, c12, d7), (a2, b15, c12, d8), (a2, b15, c12, d9), (a2, b15, c12, d10), (a2, b15, c12, d11), (a2, b15, c12, d12), (a2, b15, c13, d1), (a2, b15, c13, d2), (a2, b15, c13, d3), (a2, b15, c13, d4), (a2, b15, c13, d5), (a2, b15, c13, d6), (a2, b15, c13, d7), (a2, b15, c13, d8), (a2, b15, c13, d9), (a2, b15, c13, d10), (a2, b15, c13, d11), (a2, b15, c13, d12), (a3, b1, c1, d1), (a3, b1, c1, d2), (a3, b1, c1, d3), (a3, b1, c1, d4), (a3, b1, c1, d5), (a3, b1, c1, d6), (a3, b1, c1, d7), (a3, b1, c1, d8), (a3, b1, c1, d9), (a3, b1, c1, d10), (a3, b1, c1, d11), (a3, b1, c1, d12), (a3, b1, c2, d1), (a3, b1, c2, d2), (a3, b1, c2, d3), (a3, b1, c2, d4), (a3, b1, c2, d5), (a3, b1, c2, d6), (a3, b1, c2, d7), (a3, b1, c2, d8), (a3, b1, c2, d9), (a3, b1, c2, d10), (a3, b1, c2, d11), (a3, b1, c2, d12), (a3, b1, c3, d1), (a3, b1, c3, d2), (a3, b1, c3, d3), (a3, b1, c3, d4), (a3, b1, c3, d5), (a3, b1, c3, d6), (a3, b1, c3, d7), (a3, b1, c3, d8), (a3, b1, c3, d9), (a3, b1, c3, d10), (a3, b1, c3, d11), (a3, b1, c3, d12), (a3, b1, c4, d1), (a3, b1, c4, d2), (a3, b1, c4, d3), (a3, b1, c4, d4), (a3, b1, c4, d5), (a3, b1, c4, d6), (a3, b1, c4, d7), (a3, b1, c4, d8), (a3, b1, c4, d9), (a3, b1, c4, d10), (a3, b1, c4, d11), (a3, b1, c4, d12), (a3, b1, c5, d1), (a3, b1, c5, d2), (a3, b1, c5, d3), (a3, b1, c5, d4), (a3, b1, c5, d5), (a3, b1, c5, d6), (a3, b1, c5, d7), (a3, b1, c5, d8), (a3, b1, c5, d9), (a3, b1, c5, d10), (a3, b1, c5, d11), (a3, b1, c5, d12), (a3, b1, c6, d1), (a3, b1, c6, d2), (a3, b1, c6, d3), (a3, b1, c6, d4), (a3, b1, c6, d5), (a3, b1, c6, d6), (a3, b1, c6, d7), (a3, b1, c6, d8), (a3, b1, c6, d9), (a3, b1, c6, d10), (a3, b1, c6, d11), (a3, b1, c6, d12), (a3, b1, c7, d1), (a3, b1, c7, d2), (a3, b1, c7, d3), (a3, b1, c7, d4), (a3, b1, c7, d5), (a3, b1, c7, d6), (a3, b1, c7, d7), (a3, b1, c7, d8), (a3, b1 c7, d9), (a3, b1 c7, d10), (a3, b1, c7, d11), (a3, b1, c7, d12), (a3, b1, c8, d1), (a3, b1, c8, d2), (a3, b1, c8, d3), (a3, b1, c8, d4), (a3, b1, c8, d5), (a3, b1, c8, d6), (a3, b1, c8, d7), (a3, b1, c8, d8), (a3, b1, c8, d9), (a3, b1, c8, d10), (a3, b1, c8, d11), (a3, b1, c8, d12), (a3, b1, c9, d1), (a3, b1, c9, d2), (a3, b1, c9, d3), (a3, b1, c9, d4), (a3, b1, c9, d5), (a3, b1, c9, d6), (a3, b1, c9, d7), (a3, b1, c9, d8), (a3, b1, c9, d9), (a3, b1, c9, d10), (a3, b1, c9, d11), (a3, b1, c9, d12), (a3, b1, c10, d1), (a3, b1, c10, d2), (a3, b1, c10, d3), (a3, b1, c10, d4), (a3, b1, c10, d5), (a3, b1, c10, d6), (a3, b1, c10, d7), (a3, b1, c10, d8), (a3, b1, c10, d9), (a3, b1, c10, d10), (a3, b1, c10, d11), (a3, b1, c10, d12), (a3, b1, c11, d1), (a3, b1, c11, d2), (a3, b1, c11, d3), (a3, b1, c11, d4), (a3, b1, c11, d5), (a3, b1, c11, d6), (a3, b1, c11, d7), (a3, b1, c11, d8), (a3, b1, c11, d9), (a3, b1, c11, d10), (a3, b1, c11, d11), (a3, b1, c11, d12), (a3, b1, c12, d1), (a3, b1, c12, d2), (a3, b1, c12, d3), (a3, b1, c12, d4), (a3, b1, c12, d5), (a3, b1, c12, d6), (a3, b1, c12, d7), (a3, b1, c12, d8), (a3, b1, c12, d9), (a3, b1, c12, d10), (a3, b1, c12, d11), (a3, b1, c12, d12), (a3, b1, c13, d1), (a3, b1, c13, d2), (a3, b1, c13, d3), (a3, b1, c13, d4), (a3, b1, c13, d5), (a3, b1, c13, d6), (a3, b1, c13, d7), (a3, b1, c13, d8), (a3, b1, c13, d9), (a3, b1, c13, d10), (a3, b1, c13, d11), (a3, b1, c13, d12), (a3, b2, c1, d1), (a3, b2, c1, d2), (a3, b2, c1, d3), (a3, b2, c1, d4), (a3, b2, c1, d5), (a3, b2, c1, d6), (a3, b2, c1, d7), (a3, b2, c1, d8), (a3, b2, c1, d9), (a3, b2, c1, d10), (a3, b2, c1, d11), (a3, b2, c1, d12), (a3, b2, c2, d1), (a3, b2, c2, d2), (a3, b2, c2, d3), (a3, b2, c2, d4), (a3, b2, c2, d5), (a3, b2, c2, d6), (a3, b2, c2, d7), (a3, b2, c2, d8), (a3,

b2, c2, d9), (a3, b2, c2, d10), (a3, b2, c2, d11), (a3, b2, c2, d12), (a3, b2, c3, d1), (a3, b2, c3, d2), (a3, b2, c3, d3), (a3, b2, c3, d4), (a3, b2, c3, d5), (a3, b2, c3, d6), (a3, b2, c3, d7), (a3, b2, c3, d8), (a3, b2, c3, d9), (a3, b2, c3, d10), (a3, b2, c3, d11), (a3, b2, c3, d12), (a3, b2, c4, d1), (a3, b2, c4, d2), (a3, b2, c4, d3), (a3, b2, c4, d4), (a3, b2, c4, d5), (a3, b2, c4, d6), (a3, b2, c4, d7), (a3, b2, c4, d8), (a3, b2, c4, d9), (a3, b2, c4, d10), (a3, b2, c4, d11), (a3, b2, c4, d12), (a3, b2, c5, d1), (a3, b2, c5, d2), (a3, b2, c5, d3), (a3, b2, c5, d4), (a3, b2, c5, d5), (a3, b2, c5, d6), (a3, b2, c5, d7), (a3, b2, c5, d8), (a3, b2, c5, d9), (a3, b2, c5, d10), (a3, b2, c5, d11), (a3, b2, c5, d12), (a3, b2, c6, d1), (a3, b2, c6, d2), (a3, b2, c6, d3), (a3, b2, c6, d4), (a3, b2, c6, d5), (a3, b2, c6, d6), (a3, b2, c6, d7), (a3, b2, c6, d8), (a3, b2, c6, d9), (a3, b2, c6, d10), (a3, b2, c6, d11), (a3, b2, c6, d12), (a3, b2, c7, d1), (a3, b2, c7, d2), (a3, b2, c7, d3), (a3, b2, c7, d4), (a3, b2, c7, d5), (a3, b2, c7, d6), (a3, b2, c7, d7), (a3, b2, c7, d8), (a3, b2, c7, d9), (a3, b2, c7, d10), (a3, b2, c7, d11), (a3, b2, c7, d12), (a3, b2, c8, d1), (a3, b2, c8, d2), (a3, b2, c8, d3), (a3, b2, c8, d4), (a3, b2, c8, d5), (a3, b2, c8, d6), (a3, b2, c8, d7), (a3, b2, c8, d8), (a3, b2, c8, d9), (a3, b2, c8, d10), (a3, b2, c8, d11), (a3, b2, c8, d12), (a3, b2, c9, d1), (a3, b2, c9, d2), (a3, b2, c9, d3), (a3, b2, c9, d4), (a3, b2, c9, d5), (a3, b2, c9, d6), (a3, b2, c9, d7), (a3, b2, c9, d8), (a3, b2, c9, d9), (a3, b2, c9, d10), (a3, b2, c9, d11), (a3, b2, c9, d12), (a3, b2, c10, d1), (a3, b2, c10, d2), (a3, b2, c10, d3), (a3, b2, c10, d4), (a3, b2, c10, d5), (a3, b2, c10, d6), (a3, b2, c10, d7), (a3, b2, c10, d8), (a3, b2, c10, d9), (a3, b2, c10, d10), (a3, b2, c10, d11), (a3, b2, c10, d12), (a3, b2, c11, d1), (a3, b2, c11, d2), (a3, b2, c11, d3), (a3, b2, c11, d4), (a3, b2, c11, d5), (a3, b2, c11, d6), (a3, b2, c11, d7), (a3, b2, c11, d8), (a3, b2, c11, d9), (a3, b2, c11, d10), (a3, b2, c11, d11), (a3, b2, c11, d12), (a3, b2, c12, d1), (a3, b2, c12, d2), (a3, b2, c12, d3), (a3, b2, c12, d4), (a3, b2, c12, d5), (a3, b2, c12, d6), (a3, b2, c12, d7), (a3, b2, c12, d8), (a3, b2, c12, d9), (a3, b2, c12, d10), (a3, b2, c12, d11), (a3, b2, c12, d12), (a3, b2, c13, d1), (a3, b2, c13, d2), (a3, b2, c13, d3), (a3, b2, c13, d4), (a3, b2, c13, d5), (a3, b2, c13, d6), (a3, b2, c13, d7), (a3, b2, c13, d8), (a3, b2, c13, d9), (a3, b2, c13, d10), (a3, b2, c13, d11), (a3, b2, c13, d12), (a3, b3, c1, d1), (a3, b3, c1, d2), (a3, b3, c1, d3), (a3, b3, c1, d4), (a3, b3, c1, d5), (a3, b3, c1, d6), (a3, b3, c1, d7), (a3, b3, c1, d8), (a3, b3, c1, d9), (a3, b3, c1, d10), (a3, b3, c1, d11), (a3, b3, c1, d12), (a3, b3, c2, d1), (a3, b3, c2, d2), (a3, b3, c2, d3), (a3, b3, c2, d4), (a3, b3, c2, d5), (a3, b3, c2, d6), (a3, b3, c2, d7), (a3, b3, c2, d8), (a3, b3, c2, d9), (a3, b3, c2, d10), (a3, b3, c2, d11), (a3, b3, c2, d12), (a3, b3, c3, d1), (a3, b3, c3, d2), (a3, b3, c3, d3), (a3, b3, c3, d4), (a3, b3, c3, d5), (a3, b3, c3, d6), (a3, b3, c3, d7), (a3, b3, c3, d8), (a3, b3, c3, d9), (a3, b3, c3, d10), (a3, b3, c3, d11), (a3, b3, c3, d12), (a3, b3, c4, d1), (a3, b3, c4, d2), (a3, b3, c4, d3), (a3, b3, c4, d4), (a3, b3, c4, d5), (a3, b3, c4, d6), (a3, b3, c4, d7), (a3, b3, c4, d8), (a3, b3, c4, d9), (a3, b3, c4, d10), (a3, b3, c4, d11), (a3, b3, c4, d12), (a3, b3, c5, d1), (a3, b3, c5, d2), (a3, b3, c5, d3), (a3, b3, c5, d4), (a3, b3, c5, d5), (a3, b3, c5, d6), (a3, b3, c5, d7), (a3, b3, c5, d8), (a3, b3, c5, d9), (a3, b3, c5, d10), (a3, b3, c5, d11), (a3, b3, c5, d12), (a3, b3, c6, d1), (a3, b3, c6, d2), (a3, b3, c6, d3), (a3, b3, c6, d4), (a3, b3, c6, d5), (a3, b3, c6, d6), (a3, b3, c6, d7), (a3, b3, c6, d8), (a3, b3, c6, d9), (a3, b3, c6, d10), (a3, b3, c6, d11), (a3, b3, c6, d12), (a3, b3, c7, d1), (a3, b3, c7, d2), (a3, b3, c7, d3), (a3, b3, c7, d4), (a3, b3, c7, d5), (a3, b3, c7, d6), (a3, b3, c7, d7), (a3, b3, c7, d8), (a3, b3, c7, d9), (a3, b3, c7, d10), (a3, b3, c7, d11), (a3, b3, c7, d12), (a3, b3, c8, d1), (a3, b3, c8, d2), (a3, b3, c8, d3), (a3, b3, c8, d4), (a3, b3, c8, d5), (a3, b3, c8, d6), (a3, b3, c8, d7), (a3, b3, c8, d8), (a3, b3, c8, d9), (a3, b3, c8, d10), (a3, b3, c8, d11), (a3, b3, c8, d12), (a3, b3, c9, d1), (a3, b3, c9, d2), (a3, b3, c9, d3), (a3, b3, c9, d4), (a3, b3, c9, d5), (a3, b3, c9, d6), (a3, b3, c9, d7), (a3, b3, c9, d8), (a3, b3, c9, d9), (a3, b3, c9, d10), (a3, b3, c9, d11), (a3, b3, c9, d12), (a3, b3, c10, d1), (a3, b3, c10, d2), (a3, b3, c10, d3), (a3, b3, c10, d4), (a3, b3, c10, d5), (a3, b3, c10, d6), (a3, b3, c10, d7), (a3, b3, c10, d8), (a3, b3, c10, d9), (a3, b3, c10, d10), (a3, b3, c10, d11), (a3, b3, c10, d12), (a3, b3, c11, d1), (a3, b3, c11, d2), (a3, b3, c11, d3), (a3, b3, c11, d4), (a3, b3, c11, d5), (a3, b3, c11, d6), (a3, b3, c11, d7), (a3, b3, c11, d8), (a3, b3, c11, d9), (a3, b3, c11, d10), (a3, b3, c11, d11), (a3, b3, c11, d12), (a3, b3, c12, d1), (a3, b3, c12, d2), (a3, b3, c12, d3), (a3, b3, c12, d4), (a3, b3, c12, d5), (a3, b3, c12, d6), (a3, b3, c12, d7), (a3, b3, c12, d8), (a3, b3, c12, d9), (a3, b3, c12, d10), (a3, b3, c12, d11), (a3, b3, c12, d12), (a3, b3, c13, d1), (a3, b3, c13, d2), (a3, b3, c13, d3), (a3, b3, c13, d4), (a3, b3, c13, d5), (a3, b3, c13, d6), (a3, b3, c13, d7), (a3, b3, c13, d8), (a3, b3, c13, d9), (a3, b3, c13, d10), (a3, b3, c13, d11), (a3, b3, c13, d12), (a3, b4, c1 d1), (a3, b4, c1, d2), (a3, b4, c1, d3), (a3, b4, c1, d4), (a3, b4, c1, d5), (a3, b4, c1, d6), (a3, b4, c1, d7), (a3, b4, c1, d8), (a3, b4, c1, d9), (a3, b4, c1 d10), (a3, b4, c1, d11), (a3, b4, c1, d12), (a3, b4, c2, d1), (a3, b4, c2, d2), (a3, b4, c2, d3), (a3, b4, c2, d4), (a3, b4, c2, d5), (a3, b4, c2, d6), (a3, b4, c2, d7), (a3, b4, c2, d8), (a3, b4, c2, d9), (a3, b4, c2, d10), (a3, b4, c2, d11), (a3, b4, c2, d12), (a3, b4, c3, d1), (a3, b4, c3, d2), (a3, b4, c3, d3), (a3, b4, c3, d4), (a3, b4, c3, d5), (a3, b4, c3, d6), (a3, b4, c3, d7), (a3, b4, c3, d8), (a3, b4, c3, d9), (a3, b4, c3, d10), (a3, b4, c3, d11), (a3, b4, c3, d12), (a3, b4, c4, d1), (a3, b4, c4, d2), (a3, b4, c4, d3), (a3, b4, c4, d4), (a3, b4, c4, d5), (a3, b4, c4, d6), (a3, b4, c4, d7), (a3, b4, c4, d8), (a3, b4, c4, d9), (a3, b4, c4, d10), (a3, b4, c4, d11), (a3, b4, c4, d12), (a3, b4, c5, d1), (a3, b4, c5, d2), (a3, b4, c5, d3), (a3, b4, c5, d4), (a3, b4, c5, d5), (a3, b4, c5, d6), (a3, b4, c5, d7), (a3, b4, c5, d8), (a3, b4, c5, d9), (a3, b4, c5, d10), (a3, b4, c5, d11), (a3, b4, c5, d12), (a3, b4, c6, d1), (a3, b4, c6, d2), (a3, b4, c6, d3), (a3, b4, c6, d4), (a3, b4, c6, d5), (a3, b4, c6, d6), (a3, b4, c6, d7), (a3, b4, c6, d8), (a3, b4, c6, d9), (a3, b4, c6, d10), (a3, b4, c6, d11), (a3, b4, c6, d12), (a3, b4, c7, d1), (a3, b4, c7, d2), (a3, b4, c7, d3), (a3, b4, c7, d4), (a3, b4, c7, d5), (a3, b4, c7, d6), (a3, b4, c7, d7), (a3, b4, c7, d8), (a3, b4, c7, d9), (a3, b4, c7, d10), (a3, b4, c7, d11), (a3, b4, c7, d12), (a3, b4, c8, d1), (a3, b4, c8, d2), (a3, b4, c8, d3), (a3, b4, c8, d4), (a3, b4, c8, d5), (a3, b4, c8, d6), (a3, b4, c8, d7), (a3, b4, c8, d8), (a3, b4, c8, d9), (a3, b4, c8, d10), (a3, b4, c8, d11), (a3, b4, c8, d12), (a3, b4, c9, d1), (a3, b4, c9, d2), (a3, b4, c9, d3), (a3, b4, c9, d4), (a3, b4, c9, d5), (a3, b4, c9, d6), (a3, b4, c9, d7), (a3, b4, c9, d8), (a3, b4, c9, d9), (a3, b4, c9, d10), (a3, b4, c9, d11), (a3, b4, c9, d12), (a3, b4, c10, d1), (a3, b4, c10, d2), (a3, b4, c10,

d3), (a3, b4, c10, d4), (a3, b4, c10, d5), (a3, b4, c10, d6), (a3, b4, c10, d7), (a3, b4, c10, d8), (a3, b4, c10, d9), (a3, b4, c10, d10), (a3, b4, c10, d11), (a3, b4, c10, d12), (a3, b4, c11, d1), (a3, b4, c11, d2), (a3, b4, c11, d3), (a3, b4, c11, d4), (a3, b4, c11, d5), (a3, b4, c11, d6), (a3, b4, c11, d7), (a3, b4, c11, d8), (a3, b4, c11, d9), (a3, b4, c11, d10), (a3, b4, c11, d11), (a3, b4, c11, d12), (a3, b4, c12, d1), (a3, b4, c12, d2), (a3, b4, c12, d3), (a3, b4, c12, d4), (a3, b4, c12, d5), (a3, b4, c12, d6), (a3, b4, c12, d7), (a3, b4, c12, d8), (a3, b4, c12, d9), (a3, b4, c12, d10), (a3, b4, c12, d11), (a3, b4, c12, d12), (a3, b4, c13, d1), (a3, b4, c13, d2), (a3, b4, c13, d3), (a3, b4, c13, d4), (a3, b4, c13, d5), (a3, b4, c13, d6), (a3, b4, c13, d7), (a3, b4, c13, d8), (a3, b4, c13, d9), (a3, b4, c13, d10), (a3, b4, c13, d11), (a3, b4, c13, d12), (a3, b5, c1, d1), (a3, b5, c1, d2), (a3, b5, c1, d3), (a3, b5, c1, d4), (a3, b5, c1, d5), (a3, b5, c1, d6), (a3, b5, c1, d7), (a3, b5, c1, d8), (a3, b5, c1, d9), (a3, b5, c1 d10), (a3, b5, c1, d11), (a3, b5, c1 d12), (a3, b5, c2, d1), (a3, b5, c2, d2), (a3, b5, c2, d3), (a3, b5, c2, d4), (a3, b5, c2, d5), (a3, b5, c2, d6), (a3, b5, c2, d7), (a3, b5, c2, d8), (a3, b5, c2, d9), (a3, b5, c2, d10), (a3, b5, c2, d11), (a3, b5, c2, d12), (a3, b5, c3, d1), (a3, b5, c3, d2), (a3, b5, c3, d3), (a3, b5, c3, d4), (a3, b5, c3, d5), (a3, b5, c3, d6), (a3, b5, c3, d7), (a3, b5, c3, d8), (a3, b5, c3, d9), (a3, b5, c3, d10), (a3, b5, c3, d11), (a3, b5, c3, d12), (a3, b5, c4, d1), (a3, b5, c4, d2), (a3, b5, c4, d3), (a3, b5, c4, d4), (a3, b5, c4, d5), (a3, b5, c4, d6), (a3, b5, c4, d7), (a3, b5, c4, d8), (a3, b5, c4, d9), (a3, b5, c4, d10), (a3, b5, c4, d11), (a3, b5, c4, d12), (a3, b5, c5, d1), (a3, b5, c5, d2), (a3, b5, c5, d3), (a3, b5, c5, d4), (a3, b5, c5, d5), (a3, b5, c5, d6), (a3, b5, c5, d7), (a3, b5, c5, d8), (a3, b5, c5, d9), (a3, b5, c5, d10), (a3, b5, c5, d11), (a3, b5, c5, d12), (a3, b5, c6, d1), (a3, b5, c6, d2), (a3, b5, c6, d3), (a3, b5, c6, d4), (a3, b5, c6, d5), (a3, b5, c6, d6), (a3, b5, c6, d7), (a3, b5, c6, d8), (a3, b5, c6, d9), (a3, b5, c6, d10), (a3, b5, c6, d11), (a3, b5, c6, d12), (a3, b5, c7, d1), (a3, b5, c7, d2), (a3, b5, c7, d3), (a3, b5, c7, d4), (a3, b5, c7, d5), (a3, b5, c7, d6), (a3, b5, c7, d7), (a3, b5, c7, d8), (a3, b5, c7, d9), (a3, b5, c7, d10), (a3, b5, c7, d11), (a3, b5, c7, d12), (a3, b5, c8, d1), (a3, b5, c8, d2), (a3, b5, c8, d3), (a3, b5, c8, d4), (a3, b5, c8, d5), (a3, b5, c8, d6), (a3, b5, c8, d7), (a3, b5, c8, d8), (a3, b5, c8, d9), (a3, b5, c8, d10), (a3, b5, c8, d11), (a3, b5, c8, d12), (a3, b5, c9, d1), (a3, b5, c9, d2), (a3, b5, c9, d3), (a3, b5, c9, d4), (a3, b5, c9, d5), (a3, b5, c9, d6), (a3, b5, c9, d7), (a3, b5, c9, d8), (a3, b5, c9, d9), (a3, b5, c9, d10), (a3, b5, c9, d11), (a3, b5, c9, d12), (a3, b5, c10, d1), (a3, b5, c10, d2), (a3, b5, c10, d3), (a3, b5, c10, d4), (a3, b5, c10, d5), (a3, b5, c10, d6), (a3, b5, c10, d7), (a3, b5, c10, d8), (a3, b5, c10, d9), (a3, b5, c10, d10), (a3, b5, c10, d11), (a3, b5, c10, d12), (a3, b5, c11, d1), (a3, b5, c11, d2), (a3, b5, c11, d3), (a3, b5, c11, d4), (a3, b5, c11, d5), (a3, b5, c11, d6), (a3, b5, c11, d7), (a3, b5, c11, d8), (a3, b5, c11, d9), (a3, b5, c11, d10), (a3, b5, c11, d11), (a3, b5, c11, d12), (a3, b5, c12, d1), (a3, b5, c12, d2), (a3, b5, c12, d3), (a3, b5, c12, d4), (a3, b5, c12, d5), (a3, b5, c12, d6), (a3, b5, c12, d7), (a3, b5, c12, d8), (a3, b5, c12, d9), (a3, b5, c12, d10), (a3, b5, c12, d11), (a3, b5, c12, d12), (a3, b5, c13, d1), (a3, b5, c13, d2), (a3, b5, c13, d3), (a3, b5, c13, d4), (a3, b5, c13, d5), (a3, b5, c13, d6), (a3, b5, c13, d7), (a3, b5, c13, d8), (a3, b5, c13, d9), (a3, b5, c13, d10), (a3, b5, c13, d11), (a3, b5, c13, d12), (a3, b6, c1, d1), (a3, b6, c1, d2), (a3, b6, c1, d3), (a3, b6, c1, d4), (a3, b6, c1, d5), (a3, b6, c1, d6), (a3, b6, c1, d7), (a3, b6, c1, d8), (a3, b6, c1, d9), (a3, b6, c1, d10), (a3, b6, c1, d11), (a3, b6, c1, d12), (a3, b6, c2, d1), (a3, b6, c2, d2), (a3, b6, c2, d3), (a3, b6, c2, d4), (a3, b6, c2, d5), (a3, b6, c2, d6), (a3, b6, c2, d7), (a3, b6, c2, d8), (a3, b6, c2, d9), (a3, b6, c2, d10), (a3, b6, c2, d11), (a3, b6, c2, d12), (a3, b6, c3, d1), (a3, b6, c3, d2), (a3, b6, c3, d3), (a3, b6, c3, d4), (a3, b6, c3, d5), (a3, b6, c3, d6), (a3, b6, c3, d7), (a3, b6, c3, d8), (a3, b6, c3, d9), (a3, b6, c3, d10), (a3, b6, c3, d11), (a3, b6, c3, d12), (a3, b6, c4, d1), (a3, b6, c4, d2), (a3, b6, c4, d3), (a3, b6, c4, d4), (a3, b6, c4, d5), (a3, b6, c4, d6), (a3, b6, c4, d7), (a3, b6, c4, d8), (a3, b6, c4, d9), (a3, b6, c4, d10), (a3, b6, c4, d11), (a3, b6, c4, d12), (a3, b6, c5, d1), (a3, b6, c5, d2), (a3, b6, c5, d3), (a3, b6, c5, d4), (a3, b6, c5, d5), (a3, b6, c5, d6), (a3, b6, c5, d7), (a3, b6, c5, d8), (a3, b6, c5, d9), (a3, b6, c5, d10), (a3, b6, c5, d11), (a3, b6, c5, d12), (a3, b6, c6, d1), (a3, b6, c6, d2), (a3, b6, c6, d3), (a3, b6, c6, d4), (a3, b6, c6, d5), (a3, b6, c6, d6), (a3, b6, c6, d7), (a3, b6, c6, d8), (a3, b6, c6, d9), (a3, b6, c6, d10), (a3, b6, c6, d11), (a3, b6, c6, d12), (a3, b6, c7, d1), (a3, b6, c7, d2), (a3, b6, c7, d3), (a3, b6, c7, d4), (a3, b6, c7, d5), (a3, b6, c7, d6), (a3, b6, c7, d7), (a3, b6, c7, d8), (a3, b6, c7, d9), (a3, b6, c7, d10), (a3, b6, c7, d11), (a3, b6, c7, d12), (a3, b6, c8, d1), (a3, b6, c8, d2), (a3, b6, c8, d3), (a3, b6, c8, d4), (a3, b6, c8, d5), (a3, b6, c8, d6), (a3, b6, c8, d7), (a3, b6, c8, d8), (a3, b6, c8, d9), (a3, b6, c8, d10), (a3, b6, c8, d11), (a3, b6, c8, d12), (a3, b6, c9, d1), (a3, b6, c9, d2), (a3, b6, c9, d3), (a3, b6, c9, d4), (a3, b6, c9, d5), (a3, b6, c9, d6), (a3, b6, c9, d7), (a3, b6, c9, d8), (a3, b6, c9, d9), (a3, b6, c9, d10), (a3, b6, c9, d11), (a3, b6, c9, d12), (a3, b6, c10, d1), (a3, b6, c10, d2), (a3, b6, c10, d3), (a3, b6, c10, d4), (a3, b6, c10, d5), (a3, b6, c10, d6), (a3, b6, c10, d7), (a3, b6, c10, d8), (a3, b6, c10, d9), (a3, b6, c10, d10), (a3, b6, c10, d11), (a3, b6, c10, d12), (a3, b6, c11, d1), (a3, b6, c11, d2), (a3, b6, c11, d3), (a3, b6, c11, d4), (a3, b6, c11, d5), (a3, b6, c11, d6), (a3, b6, c11, d7), (a3, b6, c11, d8), (a3, b6, c11, d9), (a3, b6, c11, d10), (a3, b6, c11, d11), (a3, b6, c11, d12), (a3, b6, c12, d1), (a3, b6, c12, d2), (a3, b6, c12, d3), (a3, b6, c12, d4), (a3, b6, c12, d5), (a3, b6, c12, d6), (a3, b6, c12, d7), (a3, b6, c12, d8), (a3, b6, c12, d9), (a3, b6, c12, d10), (a3, b6, c12, d11), (a3, b6, c12, d12), (a3, b6, c13, d1), (a3, b6, c13, d2), (a3, b6, c13, d3), (a3, b6, c13, d4), (a3, b6, c13, d5), (a3, b6, c13, d6), (a3, b6, c13, d7), (a3, b6, c13, d8), (a3, b6, c13, d9), (a3, b6, c13, d10), (a3, b6, c13, d11), (a3, b6, c13, d12), (a3, b7, c1, d1), (a3, b7, c1, d2), (a3, b7, c1, d3), (a3, b7, c1, d4), (a3, b7, c1, d5), (a3, b7, c1, d6), (a3, b7, c1, d7), (a3, b7, c1, d8), (a3, b7, c1, d9), (a3, b7, c1, d10), (a3, b7, c1, d11), (a3, b7, c1, d12), (a3, b7, c2, d1), (a3, b7, c2, d2), (a3, b7, c2, d3), (a3, b7, c2, d4), (a3, b7, c2, d5), (a3, b7, c2, d6), (a3, b7, c2, d7), (a3, b7, c2, d8), (a3, b7, c2, d9), (a3, b7, c2, d10), (a3, b7, c2, d11), (a3, b7, c2, d12), (a3, b7, c3, d1), (a3, b7, c3, d2), (a3, b7, c3, d3), (a3, b7, c3, d4), (a3, b7, c3, d5), (a3, b7, c3, d6), (a3, b7, c3, d7), (a3, b7, c3, d8), (a3, b7, c3, d9), (a3, b7, c3, d10), (a3, b7, c3, d11), (a3, b7, c3, d12), (a3, b7, c4, d1), (a3, b7, c4, d2), (a3, b7, c4, d3), (a3, b7, c4, d4), (a3, b7, c4, d5), (a3, b7,

c4, d6), (a3, b7, c4, d7), (a3, b7, c4, d8), (a3, b7, c4, d9), (a3, b7, c4, d10), (a3, b7, c4, d11), (a3, b7, c4, d12), (a3, b7, c5, d1), (a3, b7, c5, d2), (a3, b7, c5, d3), (a3, b7, c5, d4), (a3, b7, c5, d5), (a3, b7, c5, d6), (a3, b7, c5, d7), (a3, b7, c5, d8), (a3, b7, c5, d9), (a3, b7, c5, d10), (a3, b7, c5, d11), (a3, b7, c5, d12), (a3, b7, c6, d1), (a3, b7, c6, d2), (a3, b7, c6, d3), (a3, b7, c6, d4), (a3, b7, c6, d5), (a3, b7, c6, d6), (a3, b7, c6, d7), (a3, b7, c6, d8), (a3, b7, c6, d9), (a3, b7, c6, d10), (a3, b7, c6, d11), (a3, b7, c6, d12), (a3, b7, c7, d1), (a3, b7, c7, d2), (a3, b7, c7, d3), (a3, b7, c7, d4), (a3, b7, c7, d5), (a3, b7, c7, d6), (a3, b7, c7, d7), (a3, b7, c7, d8), (a3, b7, c7, d9), (a3, b7, c7, d10), (a3, b7, c7, d11), (a3, b7, c7, d12), (a3, b7, c8, d1), (a3, b7, c8, d2), (a3, b7, c8, d3), (a3, b7, c8, d4), (a3, b7, c8, d5), (a3, b7, c8, d6), (a3, b7, c8, d7), (a3, b7, c8, d8), (a3, b7, c8, d9), (a3, b7, c8, d10), (a3, b7, c8, d11), (a3, b7, c8, d12), (a3, b7, c9, d1), (a3, b7, c9, d2), (a3, b7, c9, d3), (a3, b7, c9, d4), (a3, b7, c9, d5), (a3, b7, c9, d6), (a3, b7, c9, d7), (a3, b7, c9, d8), (a3, b7, c9, d9), (a3, b7, c9, d10), (a3, b7, c9, d11), (a3, b7, c9, d12), (a3, b7, c10, d1), (a3, b7, c10, d2), (a3, b7, c10, d3), (a3, b7, c10, d4), (a3, b7, c10, d5), (a3, b7, c10, d6), (a3, b7, c10, d7), (a3, b7, c10, d8), (a3, b7, c10, d9), (a3, b7, c10, d10), (a3, b7, c10, d11), (a3, b7, c10, d12), (a3, b7, c11, d1), (a3, b7, c11, d2), (a3, b7, c11, d3), (a3, b7, c11, d4), (a3, b7, c11, d5), (a3, b7, c11, d6), (a3, b7, c11, d7), (a3, b7, c11, d8), (a3, b7, c11, d9), (a3, b7, c11, d10), (a3, b7, c11, d11), (a3, b7, c11, d12), (a3, b7, c12, d1), (a3, b7, c12, d2), (a3, b7, c12, d3), (a3, b7, c12, d4), (a3, b7, c12, d5), (a3, b7, c12, d6), (a3, b7, c12, d7), (a3, b7, c12, d8), (a3, b7, c12, d9), (a3, b7, c12, d10), (a3, b7, c12, d11), (a3, b7, c12, d12), (a3, b7, c13, d1), (a3, b7, c13, d2), (a3, b7, c13, d3), (a3, b7, c13, d4), (a3, b7, c13, d5), (a3, b7, c13, d6), (a3, b7, c13, d7), (a3, b7, c13, d8), (a3, b7, c13, d9), (a3, b7, c13, d10), (a3, b7, c13, d11), (a3, b7, c13, d12), (a3, b8, c1, d1), (a3, b8, c1, d2), (a3, b8, c1, d3), (a3, b8, c1, d4), (a3, b8, c1, d5), (a3, b8, c1, d6), (a3, b8, c1, d7), (a3, b8, c1, d8), (a3, b8, c1, d9), (a3, b8, c1, d10), (a3, b8, c1, d11), (a3, b8, c1, d12), (a3, b8, c2, d1), (a3, b8, c2, d2), (a3, b8, c2, d3), (a3, b8, c2, d4), (a3, b8, c2, d5), (a3, b8, c2, d6), (a3, b8, c2, d7), (a3, b8, c2, d8), (a3, b8, c2, d9), (a3, b8, c2, d10), (a3, b8, c2, d11), (a3, b8, c2, d12), (a3, b8, c3, d1), (a3, b8, c3, d2), (a3, b8, c3, d3), (a3, b8, c3, d4), (a3, b8, c3, d5), (a3, b8, c3, d6), (a3, b8, c3, d7), (a3, b8, c3, d8), (a3, b8, c3, d9), (a3, b8, c3, d10), (a3, b8, c3, d11), (a3, b8, c3, d12), (a3, b8, c4, d1), (a3, b8, c4, d2), (a3, b8, c4, d3), (a3, b8, c4, d4), (a3, b8, c4, d5), (a3, b8, c4, d6), (a3, b8, c4, d7), (a3, b8, c4, d8), (a3, b8, c4, d9), (a3, b8, c4, d10), (a3, b8, c4, d11), (a3, b8, c4, d12), (a3, b8, c5, d1), (a3, b8, c5, d2), (a3, b8, c5, d3), (a3, b8, c5, d4), (a3, b8, c5, d5), (a3, b8, c5, d6), (a3, b8, c5, d7), (a3, b8, c5, d8), (a3, b8, c5, d9), (a3, b8, c5, d10), (a3, b8, c5, d11), (a3, b8, c5, d12), (a3, b8, c6, d1), (a3, b8, c6, d2), (a3, b8, c6, d3), (a3, b8, c6, d4), (a3, b8, c6, d5), (a3, b8, c6, d6), (a3, b8, c6, d7), (a3, b8, c6, d8), (a3, b8, c6, d9), (a3, b8, c6, d10), (a3, b8, c6, d11), (a3, b8, c6, d12), (a3, b8, c7, d1), (a3, b8, c7, d2), (a3, b8, c7, d3), (a3, b8, c7, d4), (a3, b8, c7, d5), (a3, b8, c7, d6), (a3, b8, c7, d7), (a3, b8, c7, d8), (a3, b8, c7, d9), (a3, b8, c7, d10), (a3, b8, c7, d11), (a3, b8, c7, d12), - (a3, b8, c8, d1), (a3, b8, c8, d2), (a3, b8, c8, d3), (a3, b8, c8, d4), (a3, b8, c8, d5), (a3, b8, c8, d6), (a3, b8, c8, d7), (a3, b8, c8, d8), (a3, b8, c8, d9), (a3, b8, c8, d10), (a3, b8, c8, d11), (a3, b8, c8, d12), (a3, b8, c9, d1), (a3, b8, c9, d2), (a3, b8, c9, d3), (a3, b8, c9, d4), (a3, b8, c9, d5), (a3, b8, c9, d6), (a3, b8, c9, d7), (a3, b8, c9, d8), (a3, b8, c9, d9), (a3, b8, c9, d10), (a3, b8, c9, d11), (a3, b8, c9, d12), (a3, b8, c10, d1), (a3, b8, c10, d2), (a3, b8, c10, d3), (a3, b8, c10, d4), (a3, b8, c10, d5), (a3, b8, c10, d6), (a3, b8, c10, d7), (a3, b8, c10, d8), (a3, b8, c10, d9), (a3, b8, c10, d10), (a3, b8, c10, d11), (a3, b8, c10, d12), (a3, b8, c11, d1), (a3, b8, c11, d2), (a3, b8, c11, d3), (a3, b8, c11, d4), (a3, b8, c11, d5), (a3, b8, c11, d6), (a3, b8, c11, d7), (a3, b8, c11, d8), (a3, b8, c11, d9), (a3, b8, c11, d10), (a3, b8, c11, d11), (a3, b8, c11, d12), (a3, b8, c12, d1), (a3, b8, c12, d2), (a3, b8, c12, d3), (a3, b8, c12, d4), (a3, b8, c12, d5), (a3, b8, c12, d6), (a3, b8, c12, d7), (a3, b8, c12, d8), (a3, b8, c12, d9), (a3, b8, c12, d10), (a3, b8, c12, d11), (a3, b8, c12, d12), (a3, b8, c13, d1), (a3, b8, c13, d2), (a3, b8, c13, d3), (a3, b8, c13, d4), (a3, b8, c13, d5), (a3, b8, c13, d6), (a3, b8, c13, d7), (a3, b8, c13, d8), (a3, b8, c13, d9), (a3, b8, c13, d10), (a3, b8, c13, d11), (a3, b8, c13, d12), (a3, b9, c1, d1), (a3, b9, c1, d2), (a3, b9, c1, d3), (a3, b9, c1, d4), (a3, b9, c1, d5), (a3, b9, c1, d6), (a3, b9, c1, d7), (a3, b9, c1, d8), (a3, b9, c1, d9), (a3, b9, c1, d10), (a3, b9, c1, d11), (a3, b9, c1, d12), (a3, b9, c2, d1), (a3, b9, c2, d2), (a3, b9, c2, d3), (a3, b9, c2, d4), (a3, b9, c2, d5), (a3, b9, c2, d6), (a3, b9, c2, d7), (a3, b9, c2, d8), (a3, b9, c2, d9), (a3, b9, c2, d10), (a3, b9, c2, d11), (a3, b9, c2, d12), (a3, b9, c3, d1), (a3, b9, c3, d2), (a3, b9, c3, d3), (a3, b9, c3, d4), (a3, b9, c3, d5), (a3, b9, c3, d6), (a3, b9, c3, d7), (a3, b9, c3, d8), (a3, b9, c3, d9), (a3, b9, c3, d10), (a3, b9, c3, d11), (a3, b9, c3, d12), (a3, b9, c4, d1), (a3, b9, c4, d2), (a3, b9, c4, d3), (a3, b9, c4, d4), (a3, b9, c4, d5), (a3, b9, c4, d6), (a3, b9, c4, d7), (a3, b9, c4, d8), (a3, b9, c4, d9), (a3, b9, c4, d10), (a3, b9, c4, d11), (a3, b9, c4, d12), (a3, b9, c5, d1), (a3, b9, c5, d2), (a3, b9, c5, d3), (a3, b9, c5, d4), (a3, b9, c5, d5), (a3, b9, c5, d6), (a3, b9, c5, d7), (a3, b9, c5, d8), (a3, b9, c5, d9), (a3, b9, c5, d10), (a3, b9, c5, d11), (a3, b9, c5, d12), (a3, b9, c6, d1), (a3, b9, c6, d2), (a3, b9, c6, d3), (a3, b9, c6, d4), (a3, b9, c6, d5), (a3, b9, c6, d6), (a3, b9, c6, d7), (a3, b9, c6, d8), (a3, b9, c6, d9), (a3, b9, c6, d10), (a3, b9, c6, d11), (a3, b9, c6, d12), (a3, b9, c7, d1), (a3, b9, c7, d2), (a3, b9, c7, d3), (a3, b9, c7, d4), (a3, b9, c7, d5), (a3, b9, c7, d6), (a3, b9, c7, d7), (a3, b9, c7, d8), (a3, b9, c7, d9), (a3, b9, c7, d10), (a3, b9, c7, d11), (a3, b9, c7, d12), (a3, b9, c8, d1), (a3, b9, c8, d2), (a3, b9, c8, d3), (a3, b9, c8, d4), (a3, b9, c8, d5), (a3, b9, c8, d6), (a3, b9, c8, d7), (a3, b9, c8, d8), (a3, b9, c8, d9), (a3, b9, c8, d10), (a3, b9, c8, d11), (a3, b9, c8, d12), (a3, b9, c9, d1), (a3, b9, c9, d2), (a3, b9, c9, d3), (a3, b9, c9, d4), (a3, b9, c9, d5), (a3, b9, c9, d6), (a3, b9, c9, d7), (a3, b9, c9, d8), (a3, b9, c9, d9), (a3, b9, c9, d10), (a3, b9, c9, d11), (a3, b9, c9, d12), (a3, b9, c10, d1), (a3, b9, c10, d2), (a3, b9, c10, d3), (a3, b9, c10, d4), (a3, b9, c10, d5), (a3, b9, c10, d6), (a3, b9, c10, d7), (a3, b9, c10, d8), (a3, b9, c10, d9), (a3, b9, c10, d10), (a3, b9, c10, d11), (a3, b9, c10, d12), (a3, b9, c11, d1), (a3, b9, c11, d2), (a3, b9, c11, d3), (a3, b9, c11, d4), (a3, b9, c11, d5), (a3, b9, c11, d6), (a3, b9, c11, d7), (a3, b9, c11, d8), (a3, b9, c11, d9), (a3, b9, c11, d10), (a3, b9,

c11, d11), (a3, b9, c11, d12), (a3, b9, c12, d1), (a3, b9, c12, d2), (a3, b9, c12, d3), (a3, b9, c12, d4), (a3, b9, c12, d5), (a3, b9, c12, d6), (a3, b9, c12, d7), (a3, b9, c12, d8), (a3, b9, c12, d9), (a3, b9, c12, d10), (a3, b9, c12, d11), (a3, b9, c12, d12), (a3, b9, c13, d1), (a3, b9, c13, d2), (a3, b9, c13, d3), (a3, b9, c13, d4), (a3, b9, c13, d5), (a3, b9, c13, d6), (a3, b9, c13, d7), (a3, b9, c13, d8), (a3, b9, c13, d9), (a3, b9, c13, d10), (a3, b9, c13, d11), (a3, b9, c13, d12), (a3, b10, c1, d1), (a3, b10, c1, d2), (a3, b10, c1, d3), (a3, b10, c1, d4), (a3, b10, c1, d5), (a3, b10, c1, d6), (a3, b10, c1, d7), (a3, b10, c1, d8), (a3, b10, c1, d9), (a3, b10, c1, d10), (a3, b10, c1, d11), (a3, b10, c1, d12), (a3, b10, c2, d1), (a3, b10, c2, d2), (a3, b10, c2, d3), (a3, b10, c2, d4), (a3, b10, c2, d5), (a3, b10, c2, d6), (a3, b10, c2, d7), (a3, b10, c2, d8), (a3, b10, c2, d9), (a3, b10, c2, d10), (a3, b10, c2, d11), (a3, b10, c2, d12), (a3, b10, c3, d1), (a3, b10, c3, d2), (a3, b10, c3, d3), (a3, b10, c3, d4), (a3, b10, c3, d5), (a3, b10, c3, d6), (a3, b10, c3, d7), (a3, b10, c3, d8), (a3, b10, c3, d9), (a3, b10, c3, d10), (a3, b10, c3, d11), (a3, b10, c3, d12), (a3, b10, c4, d1), (a3, b10, c4, d2), (a3, b10, c4, d3), (a3, b10, c4, d4), (a3, b10, c4, d5), (a3, b10, c4, d6), (a3, b10, c4, d7), (a3, b10, c4, d8), (a3, b10, c4, d9), (a3, b10, c4, d10), (a3, b10, c4, d11), (a3, b10, c4, d12), (a3, b10, c5, d1), (a3, b10, c5, d2), (a3, b10, c5, d3), (a3, b10, c5, d4), (a3, b10, c5, d5), (a3, b10, c5, d6), (a3, b10, c5, d7), (a3, b10, c5, d8), (a3, b10, c5, d9), (a3, b10, c5, d10), (a3, b10, c5, d11), (a3, b10, c5, d12), (a3, b10, c6, d1), (a3, b10, c6, d2), (a3, b10, c6, d3), (a3, b10, c6, d4), (a3, b10, c6, d5), (a3, b10, c6, d6), (a3, b10, c6, d7), (a3, b10, c6, d8), (a3, b10, c6, d9), (a3, b10, c6, d10), (a3, b10, c6, d11), (a3, b10, c6, d12), (a3, b10, c7, d1), (a3, b10, c7, d2), (a3, b10, c7, d3), (a3, b10, c7, d4), (a3, b10, c7, d5), (a3, b10, c7, d6), (a3, b10, c7, d7), (a3, b10, c7, d8), (a3, b10, c7, d9), (a3, b10, c7, d10), (a3, b10, c7, d11), (a3, b10, c7, d12), (a3, b10, c8, d1), (a3, b10, c8, d2), (a3, b10, c8, d3), (a3, b10, c8, d4), (a3, b10, c8, d5), (a3, b10, c8, d6), (a3, b10, c8, d7), (a3, b10, c8, d8), (a3, b10, c8, d9), (a3, b10, c8, d10), (a3, b10, c8, d11), (a3, b10, c8, d12), (a3, b10, c9, d1), (a3, b10, c9, d2), (a3, b10, c9, d3), (a3, b10, c9, d4), (a3, b10, c9, d5), (a3, b10, c9, d6), (a3, b10, c9, d7), (a3, b10, c9, d8), (a3, b10, c9, d9), (a3, b10, c9, d10), (a3, b10, c9, d11), (a3, b10, c9, d12), (a3, b10, c10, d1), (a3, b10, c10, d2), (a3, b10, c10, d3), (a3, b10, c10, d4), (a3, b10, c10, d5), (a3, b10, c10, d6), (a3, b10, c10, d7), (a3, b10, c10, d8), (a3, b10, c10, d9), (a3, b10, c10, d10), (a3, b10, c10, d11), (a3, b10, c10, d12), (a3, b10, e11, d1), (a3, b10, c11, d2), (a3, b10, c11, d3), (a3, b10, c11, d4), (a3, b10, e11, d5), (a3, b10, c11, d6), (a3, b10, c11, d7), (a3, b10, c11, d8), (a3, b10, c11, d9), (a3, b10, c11, d10), (a3, b10, c11, d11), (a3, b10, c11, d12), (a3, b10, c12, d1), (a3, b10, c12, d2), (a3, b10, c12, d3), (a3, b10, c12, d4), (a3, b10, c12, d5), (a3, b10, c12, d6), (a3, b10, c12, d7), (a3, b10, c12, d8), (a3, b10, c12, d9), (a3, b10, c12, d10), (a3, b10, c12, d11), (a3, b10, c12, d12), (a3, b10, c13, d1), (a3, b10, c13, d2), (a3, b10, c13, d3), (a3, b10, c13, d4), (a3, b10, c13, d5), (a3, b10, c13, d6), (a3, b10, c13, d7), (a3, b10, c13, d8), (a3, b10, c13, d9), (a3, b10, c13, d10), (a3, b10, c13, d11), (a3, b10, c13, d12), (a3, b11, c1, d1), (a3, b11, c1, d2), (a3, b11, c1, d3), (a3, b11, c1, d4), (a3, b11, c1, d5), (a3, b11, c1, d6), (a3, b11, c1, d7), (a3, b11, c1, d8), (a3, b11, c1, d9), (a3, b11, c1, d10), (a3, b11, c1, d11), (a3, b11, c1, d12), (a3, b11, c2, d1), (a3, b11, c2, d2), (a3, b11, c2, d3), (a3, b11, c2, d4), (a3, b11, c2, d5), (a3, b11, c2, d6), (a3, b11, c2, d7), (a3, b11, c2, d8), (a3, b11, c2, d9), (a3, b11, c2, d10), (a3, b11, c2, d11), (a3, b11, c2, d12), (a3, b11, c3, d1), (a3, b11, c3, d2), (a3, b11, c3, d3), (a3, b11, c3, d4), (a3, b11, c3, d5), (a3, b11, c3, d6), (a3, b11, c3, d7), (a3, b11, c3, d8), (a3, b11, c3, d9), (a3, b11, c3, d10), (a3, b11, c3, d11), (a3, b11, c3, d12), (a3, b11, c4, d1), (a3, b11, c4, d2), (a3, b11, c4, d3), (a3, b11, c4, d4), (a3, b11, c4, d5), (a3, b11, c4, d6), (a3, b11, c4, d7), (a3, b11, c4, d8), (a3, b11, c4, d9), (a3, b11, c4, d10), (a3, b11, c4, d11), (a3, b11, c4, d12), (a3, b11, c5, d1), (a3, b11, c5, d2), (a3, b11, c5, d3), (a3, b11, c5, d4), (a3, b11, c5, d5), (a3, b11, c5, d6), (a3, b11, c5, d7), (a3, b11, c5, d8), (a3, b11, c5, d9), (a3, b11, c5, d10), (a3, b11, c5, d11), (a3, b11, c5, d12), (a3, b11, c6, d1), (a3, b11, c6, d2), (a3, b11, c6, d3), (a3, b11, c6, d4), (a3, b11, c6, d5), (a3, b11, c6, d6), (a3, b11, c6, d7), (a3, b11, c6, d8), (a3, b11, c6, d9), (a3, b11, c6, d10), (a3, b11, c6, d11), (a3, b11, c6, d12), (a3, b11, c7, d1), (a3, b11, c7, d2), (a3, b11, c7, d3), (a3, b11, c7, d4), (a3, b11, c7, d5), (a3, b11, c7, d6), (a3, b11, c7, d7), (a3, b11, c7, d8), (a3, b11, c7, d9), (a3, b11, c7, d10), (a3, b11, c7, d11), (a3, b11, c7, d12), (a3, b11, c8, d1), (a3, b11, c8, d2), (a3, b11, c8, d3), (a3, b11, c8, d4), (a3, b11, c8, d5), (a3, b11, c8, d6), (a3, b11, c8, d7), (a3, b11, c8, d8), (a3, b11, c8, d9), (a3, b11, c8, d10), (a3, b11, c8, d11), (a3, b11, c8, d12), (a3, b11, c9, d1), (a3, b11, c9, d2), (a3, b11, c9, d3), (a3, b11, c9, d4), (a3, b11, c9, d5), (a3, b11, c9, d6), (a3, b11, c9, d7), (a3, b11, c9, d8), (a3, b11, c9; d9), (a3, b11, c9, d10), (a3, b11, c9, d11), (a3, b11, c9, d12), (a3, b11, c10, d1), (a3, b11, c10, d2), (a3, b11, c10, d3), (a3, b11, c10, d4), (a3, b11, c10, d5), (a3, b11, c10, d6), (a3, b11, c10, d7), (a3, b11, c10, d8), (a3, b11, c10, d9), (a3, b11, c10, d10), (a3, b11, c10, d11), (a3, b11, c10, d12), (a3, b11, c11, d1), (a3, b11, c11, d2), (a3, b11, c11, d3), (a3, b11, c11, d4), (a3, b11, c11, d5), (a3, b11, c11, d6), (a3, b11, c11, d7), (a3, b11, c11, d8), (a3, b11, c11, d9), (a3, b11, c11, d10), (a3, b11, c11, d11), (a3, b11, c11, d12), (a3, b11, c12, d1), (a3, b11, c12, d2), (a3, b11, c12, d3), (a3, b11, c12, d4), (a3, b11, c12, d5), (a3, b11, c12, d6), (a3, b11, c12, d7), (a3, b11, c12, d8), (a3, b11, c12, d9), (a3, b11, c12, d10), (a3, b11, c12, d11), (a3, b11, c12, d12), (a3, b11, c13, d1), (a3, b11, c13, d2), (a3, b11, c13, d3), (a3, b11, c13, d4), (a3, b11, c13, d5), (a3, b11, c13, d6), (a3, b11, c13, d7), (a3, b11, c13, d8), (a3, b11, c13, d9), (a3, b11, c13, d10), (a3, b11, c13, d11), (a3, b11, c13, d12), (a3, b12, c1, d1), (a3, b12, c1, d2), (a3, b12, c1, d3), (a3, b12, c1, d4), (a3, b12, c1, d5), (a3, b12, c1, d6), (a3, b12, c1, d7), (a3, b12, c1, d8), (a3, b12, c1, d9), (a3, b12, c1, d10), (a3, b12, c1, d11), (a3, b12, c1, d12), (a3, b12, c2, d1), (a3, b12, c2, d2), (a3, b12, c2, d3), (a3, b12, c2, d4), (a3, b12, c2, d5), (a3, b12, c2, d6), (a3, b12, c2, d7), (a3, b12, c2, d8), (a3, b12, c2, d9), (a3, b12, c2, d10), (a3, b12, c2, d11), (a3, b12, c2, d12), (a3, b12, c3, d1), (a3, b12, c3, d2), (a3, b12, c3, d3), (a3, b12, c3, d4), (a3, b12, c3, d5), (a3, b12, c3, d6), (a3, b12, c3, d7), (a3, b12, c3, d8), (a3, b12, c3, d9), (a3, b12, c3, d10), (a3, b12, c3, d11), (a3, b12, c3, d12), (a3, b12, c4,

d1), (a3, b12, c4, d2), (a3, b12, c4, d3), (a3, b12, c4, d4), (a3, b12, c4, d5), (a3, b12, c4, d6), (a3, b12, c4, d7), (a3, b12, c4, d8), (a3, b12, c4, d9), (a3, b12, c4, d10), (a3, b12, c4, d11), (a3, b12, c4, d12), (a3, b12, c5, d1), (a3, b12, c5, d2), (a3, b12, c5, d3), (a3, b12, c5, d4), (a3, b12, c5, d5), (a3, b12, c5, d6), (a3, b12, c5, d7), (a3, b12, c5, d8), (a3, b12, c5, d9), (a3, b12, c5, d10), (a3, b12, c5, d11), (a3, b12, c5, d12), (a3, b12, c6, d1), (a3, b12, c6, d2), (a3, b12, c6, d3), (a3, b12, c6, d4), (a3, b12, c6, d5), (a3, b12, c6, d6), (a3, b12, c6, d7), (a3, b12, c6, d8), (a3, b12, c6, d9), (a3, b12, c6, d10), (a3, b12, c6, d11), (a3, b12, c6, d12), (a3, b12, c7, d1), (a3, b12, c7, d2), (a3, b12, c7, d3), (a3, b12, c7, d4), (a3, b12, c7, d5), (a3, b12, c7, d6), (a3, b12, c7, d7), (a3, b12, c7, d8), (a3, b12, c7, d9), (a3, b12, c7, d10), (a3, b12, c7, d11), (a3, b12, c7, d12), (a3, b12, c8, d1), (a3, b12, c8, d2), (a3, b12, c8, d3), (a3, b12, c8, d4), (a3, b12, c8, d5), (a3, b12, c8, d6), (a3, b12, c8, d7), (a3, b12, c8, d8), (a3, b12, c8, d9), (a3, b12, c8, d10), (a3, b12, c8, d11), (a3, b12, c8, d12), (a3, b12, c9, d1), (a3, b12, c9, d2), (a3, b12, c9, d3), (a3, b12, c9, d4), (a3, b12, c9, d5), (a3, b12, c9, d6), (a3, b12, c9, d7), (a3, b12, c9, d8), (a3, b12, c9, d9), (a3, b12, c9, d10), (a3, b12, c9, d11), (a3, b12, c9, d12), (a3, b12, c10, d1), (a3, b12, c10, d2), (a3, b12, c10, d3), (a3, b12, c10, d4), (a3, b12, c10, d5), (a3, b12, c10, d6), (a3, b12, c10, d7), (a3, b12, c10, d8), (a3, b12, c10, d9), (a3, b12, c10, d10), (a3, b12, c10, d11), (a3, b12, c10, d12), (a3, b12, c11, d1), (a3, b12, c11, d2), (a3, b12, c11, d3), (a3, b12, c11, d4), (a3, b12, c11, d5), (a3, b12, c11, d6), (a3, b12, c11, d7), (a3, b12, c11, d8), (a3, b12, c11, d9), (a3, b12, c11, d10), (a3, b12, c11, d11), (a3, b12, c11, d12), (a3, b12, c12, d1), (a3, b12, c12, d2), (a3, b12, c12, d3), (a3, b12, c12, d4), (a3, b12, c12, d5), (a3, b12, c12, d6), (a3, b12, c12, d7), (a3, b12, c12, d8), (a3, b12, c12, d9), (a3, b12, c12, d10), (a3, b12, c12, d11), (a3, b12, c12, d12), (a3, b12, c13, d1), (a3, b12, c13, d2), (a3, b12, c13, d3), (a3, b12, c13, d4), (a3, b12, c13, d5), (a3, b12, c13, d6), (a3, b12, c13, d7), (a3, b12, c13, d8), (a3, b12, c13, d9), (a3, b12, c13, d10), (a3, b12, c13, d11), (a3, b12, c13, d12), (a3, b13, c1, d1), (a3, b13, c1, d2), (a3, b13, c1, d3), (a3, b13, c1, d4), (a3, b13, c1, d5), (a3, b13, c1, d6), (a3, b13, c1, d7), (a3, b13, c1, d8), (a3, b13, c1, d9), (a3, b13, c1, d10), (a3, b13, c1, d11), (a3, b13, c1, d12), (a3, b13, c2, d1), (a3, b13, c2, d2), (a3, b13, c2, d3), (a3, b13, c2, d4), (a3, b13, c2, d5), (a3, b13, c2, d6), (a3, b13, c2, d7), (a3, b13, c2, d8), (a3, b13, c2, d9), (a3, b13, c2, d10), (a3, b13, c2, d11), (a3, b13, c2, d12), (a3, b13, c3, d1), (a3, b13, c3, d2), (a3, b13, c3, d3), (a3, b13, c3, d4), (a3, b13, c3, d5), (a3, b13, c3, d6), (a3, b13, c3, d7), (a3, b13, c3, d8), (a3, b13, c3, d9), (a3, b13, c3, d10), (a3, b13, c3, d11), (a3, b13, c3, d12), (a3, b13, c4, d1), (a3, b13, c4, d2), (a3, b13, c4, d3), (a3, b13, c4, d4), (a3, b13, c4, d5), (a3, b13, c4, d6), (a3, b13, c4, d7), (a3, b13, c4, d8), (a3, b13, c4, d9), (a3, b13, c4, d10), (a3, b13, c4, d11), (a3, b13, c4, d12), (a3, b13, c5, d1), (a3, b13, c5, d2), (a3, b13, c5, d3), (a3, b13, c5, d4), (a3, b13, c5, d5), (a3, b13, c5, d6), (a3, b13, c5, d7), (a3, b13, c5, d8), (a3, b13, c5, d9), (a3, b13, c5, d10), (a3, b13, c5, d11), (a3, b13, c5, d12), (a3, b13, c6, d1), (a3, b13, c6, d2), (a3, b13, c6, d3), (a3, b13, c6, d4), (a3, b13, c6, d5), (a3, b13, c6, d6), (a3, b13, c6, d7), (a3, b13, c6, d8), (a3, b13, c6, d9), (a3, b13, c6, d10), (a3, b13, c6, d11), (a3, b13, c6, d12), (a3, b13, c7, d1), (a3, b13, c7, d2), (a3, b13, c7, d3), (a3, b13, c7, d4), (a3, b13, c7, d5), (a3, b13, c7, d6), (a3, b13, c7, d7), (a3, b13, c7, d8), (a3, b13, c7, d9), (a3, b13, c7, d10), (a3, b13, c7, d11), (a3, b13, c7, d12), (a3, b13, c8, d1), (a3, b13, c8, d2), (a3, b13, c8, d3), (a3, b13, c8, d4), (a3, b13, c8, d5), (a3, b13, c8, d6), (a3, b13, c8, d7), (a3, b13, c8, d8), (a3, b13, c8, d9), (a3, b13, c8, d10), (a3, b13, c8, d11), (a3, b13, c8, d12), (a3, b13, c9, d1), (a3, b13, c9, d2), (a3, b13, c9, d3), (a3, b13, c9, d4), (a3, b13, c9, d5), (a3, b13, c9, d6), (a3, b13, c9, d7), (a3, b13, c9, d8), (a3, b13, c9, d9), (a3, b13, c9, d10), (a3, b13, c9, d11), (a3, b13, c9, d12), (a3, b13, c10, d1), (a3, b13, c10, d2), (a3, b13, c10, d3), (a3, b13, c10, d4), (a3, b13, c10, d5), (a3, b13, c10, d6), (a3, b13, c10, d7), (a3, b13, c10, d8), (a3, b13, c10, d9), (a3, b13, c10, d10), (a3, b13, c10, d11), (a3, b13, c10, d12), (a3, b13, c11, d1), (a3, b13, c11, d2), (a3, b13, c11, d3), (a3, b13, c11, d4), (a3, b13, c11, d5), (a3, b13, c11, d6), (a3, b13, c11, d7), (a3, b13, c11, d8), (a3, b13, c11, d9), (a3, b13, c11, d10), (a3, b13, c11, d11), (a3, b13, c11, d12), (a3, b13, c12, d1), (a3, b13, c12, d2), (a3, b13, c12, d3), (a3, b13, c12, d4), (a3, b13, c12, d5), (a3, b13, c12, d6), (a3, b13, c12, d7), (a3, b13, c12, d8), (a3, b13, c12, d9), (a3, b13, c12, d10), (a3, b13, c12, d11), (a3, b13, c12, d12), (a3, b13, c13, d1), (a3, b13, c13, d2), (a3, b13, c13, d3), (a3, b13, c13, d4), (a3, b13, c13, d5), (a3, b13, c13, d6), (a3, b13, c13, d7), (a3, b13, c13, d8), (a3, b13, c13, d9), (a3, b13, c13, d10), (a3, b13, c13, d11), (a3, b13, c13, d12), (a3, b14, c1, d1), (a3, b14, c1, d2), (a3, b14, c1, d3), (a3, b14, c1, d4), (a3, b14, c1, d5), (a3, b14, c1, d6), (a3, b14, c1, d7), (a3, b14, c1, d8), (a3, b14, c1, d9), (a3, b14, c1, d10), (a3, b14, c1, d11), (a3, b14, c1, d12), (a3, b14, c2, d1), (a3, b14, c2, d2), (a3, b14, c2, d3), (a3, b14, c2, d4), (a3, b14, c2, d5), (a3, b14, c2, d6), (a3, b14, c2, d7), (a3, b14, c2, d8), (a3, b14, c2, d9), (a3, b14, c2, d10), (a3, b14, c2, d11), (a3, b14, c2, d12), (a3, b14, c3, d1), (a3, b14, c3, d2), (a3, b14, c3, d3), (a3, b14, c3, d4), (a3, b14, c3, d5), (a3, b14, c3, d6), (a3, b14, c3, d7), (a3, b14, c3, d8), (a3, b14, c3, d9), (a3, b14, c3, d10), (a3, b14, c3, d11), (a3, b14, c3, d12), (a3, b14, c4, d1), (a3, b14, c4, d2), (a3, b14, c4, d3), (a3, b14, c4, d4), (a3, b14, c4, d5), (a3, b14, c4, d6), (a3, b14, c4, d7), (a3, b14, c4, d8), (a3, b14, c4, d9), (a3, b14, c4, d10), (a3, b14, c4, d11), (a3, b14, c4, d12), (a3, b14, c5, d1), (a3, b14, c5, d2), (a3, b14, c5, d3), (a3, b14, c5, d4), (a3, b14, c5, d5), (a3, b14, c5, d6), (a3, b14, c5, d7), (a3, b14, c5, d8), (a3, b14, c5, d9), (a3, b14, c5, d10), (a3, b14, c5, d11), (a3, b14, c5, d12), (a3, b14, c6, d1), (a3, b14, c6, d2), (a3, b14, c6, d3), (a3, b14, c6, d4), (a3, b14, c6, d5), (a3, b14, c6, d6), (a3, b14, c6, d7), (a3, b14, c6, d8), (a3, b14, c6, d9), (a3, b14, c6, d10), (a3, b14, c6, d11), (a3, b14, c6, d12), (a3, b14, c7, d1), (a3, b14, c7, d2), (a3, b14, c7, d3), (a3, b14, c7, d4), (a3, b14, c7, d5), (a3, b14, c7, d6), (a3, b14, c7, d7), (a3, b14, c7, d8), (a3, b14, c7, d9), (a3, b14; c7, d10), (a3, b14, c7, d11), (a3, b14, c7, d12), (a3, b14, c8, d1), (a3, b14, c8, d2), (a3, b14, c8, d3), (a3, b14, c8, d4), (a3, b14, c8, d5), (a3, b14, c8, d6), (a3, b14, c8, d7), (a3, b14, c8, d8), (a3, b14, c8, d9), (a3, b14, c8, d10), (a3, b14, c8, d11), (a3, b14, c8, d12), (a3, b14, c9, d1), (a3, b14, c9, d2), (a3, b14, c9, d3), (a3,

b14, c9, d4), (a3, b14, c9, d5), (a3, b14, c9, d6), (a3, b14, c9, d7), (a3, b14, c9, d8), (a3, b14, c9, d9), (a3, b14, c9, d10), (a3, b14, c9, d11), (a3, b14, c9, d12), (a3, b14, c10, d1), (a3, b14, c10, d2), (a3, b14, c10, d3), (a3, b14, c10, d4), (a3, b14, c10, d5), (a3, b14, c10, d6), (a3, b14, c10, d7), (a3, b14, c10, d8), (a3, b14, c10, d9), (a3, b14, c10, d10), (a3, b14, c10, d11), (a3, b14, c10, d12), (a3, b14, c11, d1), (a3, b14, c11, d2), (a3, b14, c11, d3), (a3, b14, c11, d4), (a3, b14, e11, d5), (a3, b14, c11, d6), (a3, b14, c11, d7), (a3, b14, c11, d8), (a3, b14, c11, d9), (a3, b14, c11, d10), (a3, b14, c11, d11), (a3, b14, c11, d12), (a3, b14, c12, d1), (a3, b14, c12, d2), (a3, b14, c12, d3), (a3, b14, c12, d4), (a3, b14, c12, d5), (a3, b14, c12, d6), (a3, b14, c12, d7), (a3, b14, c12, d8), (a3, b14, c12, d9), (a3, b14, c12, d10), (a3, b14, c12, d11), (a3, b14, c12, d12), (a3, b14, c13, d1), (a3, b14, c13, d2), (a3, b14, c13, d3), (a3, b14, c13, d4), (a3, b14, c13, d5), (a3, b14, c13, d6), (a3, b14, c13, d7), (a3, b14, c13, d8), (a3, b14, c13, d9), (a3, b14, c13, d10), (a3, b14, c13, d11), (a3, b14, c13, d12), (a3, b15, c1, d1), (a3, b15, c1, d2), (a3, b15, c1, d3), (a3, b15, c1, d4), (a3, b15, c1, d5), (a3, b15, c1, d6), (a3, b15, c1, d7), (a3, b15, c1, d8), (a3, b15, c1, d9), (a3, b15, c1, d10), (a3, b15, c1, d11), (a3, b15, c1, d12), (a3, b15, c2, d1), (a3, b15, c2, d2), (a3, b15, c2, d3), (a3, b15, c2, d4), (a3, b15, c2, d5), (a3, b15, c2, d6), (a3, b15, c2, d7), (a3, b15, c2, d8), (a3, b15, c2, d9), (a3, b15, c2, d10), (a3, b15, c2, d11), (a3, b15, c2, d12), (a3, b15, c3, d1), (a3, b15, c3, d2), (a3, b15, c3, d3), (a3, b15, c3, d4), (a3, b15, c3, d5), (a3, b15, c3, d6), (a3, b15, c3, d7), (a3, b15, c3, d8), (a3, b15, c3, d9), (a3, b15, c3, d10), (a3, b15, c3, d11), (a3, b15, c3, d12), (a3, b15, c4, d1), (a3, b15, c4, d2), (a3, b15, c4, d3), (a3, b15, c4, d4), (a3, b15, c4, d5), (a3, b15, c4, d6), (a3, b15, c4, d7), (a3, b15, c4, d8), (a3, b15, c4, d9), (a3, b15, c4, d10), (a3, b15, c4, d11), (a3, b15, c4, d12), (a3, b15, c5, d1), (a3, b15, c5, d2), (a3, b15, c5, d3), (a3, b15, c5, d4), (a3, b15, c5, d5), (a3, b15, c5, d6), (a3, b15, c5, d7), (a3, b15, c5, d8), (a3, b15, c5, d9), (a3, b15, c5, d10), (a3, b15, c5, d11), (a3, b15, c5, d12), (a3, b15, c6, d1), (a3, b15, c6, d2), (a3, b15, c6, d3), (a3, b15, c6, d4), (a3, b15, c6, d5), (a3, b15, c6, d6), (a3, b15, c6, d7), (a3, b15, c6, d8), (a3, b15, c6, d9), (a3, b15, c6, d10), (a3, b15, c6, d11), (a3, b15, c6, d12), (a3, b15, c7, d1), (a3, b15, c7, d2), (a3, b15, c7, d3), (a3, b15, c7, d4), (a3, b15, c7, d5), (a3, b15, c7, d6), (a3, b15, c7, d7), (a3, b15, c7, d8), (a3, b15, c7, d9), (a3, b15, c7, d10), (a3, b15, c7, d11), (a3, b15, c7, d12), (a3, b15, c8, d1), (a3, b15, c8, d2), (a3, b15, c8, d3), (a3, b15, c8, d4), (a3, b15, c8, d5), (a3, b15, c8, d6), (a3, b15, c8, d7), (a3, b15, c8, d8), (a3, b15, c8, d9), (a3, b15, c8, d10), (a3, b15, c8, d11), (a3, b15, c8, d12), (a3, b15, c9, d1), (a3, b15, c9, d2), (a3, b15, c9, d3), (a3, b15, c9, d4), (a3, b15, c9, d5), (a3, b15, c9, d6), (a3, b15, c9, d7), (a3, b15, c9, d8), (a3, b15, c9, d9), (a3, b15, c9, d10), (a3, b15, c9, d11), (a3, b15, c9, d12), (a3, b15, c10, d1), (a3, b15, c10, d2), (a3, b15, c10, d3), (a3, b15, c10, d4), (a3, b15, c10, d5), (a3, b15, c10, d6), (a3, b15, c10, d7), (a3, b15, c10, d8), (a3, b15, c10, d9), (a3, b15, c10, d10), (a3, b15, c10, d11), (a3, b15, c10, d12), (a3, b15, c11, d1), (a3, b15, c11, d2), (a3, b15, c11, d3), (a3, b15, c11, d4), (a3, b15, c11, d5), (a3, b15, c11, d6), (a3, b15, c11, d7), (a3, b15, c11, d8), (a3, b15, c11, d9), (a3, b15, c11, d10), (a3, b15, c11, d11), (a3, b15, c11, d12), (a3, b15, c12, d1), (a3, b15, c12, d2), (a3, b15, c12, d3), (a3, b15, c12, d4), (a3, b15, c12, d5), (a3, b15, c12, d6), (a3, b15, c12, d7), (a3, b15, c12, d8), (a3, b15, c12, d9), (a3, b15, c12, d10), (a3, b15, c12, d11), (a3, b15, c12, d12), (a3, b15, c13, d1), (a3, b15, c13, d2), (a3, b15, c13, d3), (a3, b15, c13, d4), (a3, b15, c13, d5), (a3, b15, c13, d6), (a3, b15, c13, d7), (a3, b15, c13, d8), (a3, b15, c13, d9), (a3, b15, c13, d10), (a3, b15, c13, d11), (a3, b15, c13, d12), (a4, b1, c1, d1), (a4, b1, c1, d2), (a4, b1, c1, d3), (a4, b1, c1, d4), (a4, b1, c1, d5), (a4, b1, c1, d6), (a4, b1, c1, d7), (a4, b1, c1, d8), (a4, b1, c1, d9), (a4, b1, c1, d10), (a4, b1, c1, d11), (a4, b1, c1, d12), (a4, b1, c2, d1), (a4, b1, c2, d2), (a4, b1, c2, d3), (a4, b1, c2, d4), (a4, b1, c2, d5), (a4, b1, c2, d6), (a4, b1, c2, d7), (a4, b1, c2, d8), (a4, b1, c2, d9), (a4, b1, c2, d10), (a4, b1, c2, d11), (a4, b1, c2, d12), (a4, b1, c3, d1), (a4, b1, c3, d2), (a4, b1, c3, d3), (a4, b1, c3, d4), (a4, b1, c3, d5), (a4, b1, c3, d6), (a4, b1, c3, d7), (a4, b1, c3, d8), (a4, b1, c3, d9), (a4, b1, c3, d10), (a4, b1, c3, d11), (a4, b1, c3, d12), (a4, b1, c4, d1), (a4, b1, c4, d2), (a4, b1, c4, d3), (a4, b1, c4, d4), (a4, b1, c4, d5), (a4, b1, c4, d6), (a4, b1, c4, d7), (a4, b1, c4, d8), (a4, b1, c4, d9), (a4, b1, c4, d10), (a4, b1, c4, d11), (a4, b1, c4, d12), (a4, b1, c5, d1), (a4, b1, c5, d2), (a4, b1, c5, d3), (a4, b1, c5, d4), (a4, b1, c5, d5), (a4, b1, c5, d6), (a4, b1, c5, d7), (a4, b1, c5, d8), (a4, b1, c5, d9), (a4, b1, c5, d10), (a4, b1, c5, d11), (a4, b1, c5, d12), (a4, b1, c6, d1), (a4, b1, c6, d2), (a4, b1, c6, d3), (a4, b1, c6, d4), (a4, b1, c6, d5), (a4, b1, c6, d6), (a4, b1, c6, d7), (a4, b1, c6, d8), (a4, b1, c6, d9), (a4, b1, c6, d10), (a4, b1, c6, d11), (a4, b1, c6, d12), (a4, b1, c7, d1), (a4, b1, c7, d2), (a4, b1, c7, d3), (a4, b1, c7, d4), (a4, b1, c7, d5), (a4, b1, c7, d6), (a4, b1, c7, d7), (a4, b1, c7, d8), (a4, b1, c7, d9), (a4, b1, c7, d10), (a4, b1, c7, d11), (a4, b1, c7, d12), (a4, b1, c8, d1), (a4, b1, c8, d2), (a4, b1, c8, d3), (a4, b1, c8, d4), (a4, b1, c8, d5), (a4, b1, c8, d6), (a4, b1, c8, d7), (a4, b1, c8, d8), (a4, b1, c8, d9), (a4, b1, c8, d10), (a4, b1, c8, d11), (a4, b1, c8, d12), (a4, b1, c9, d1), (a4, b1, c9, d2), (a4, b1, c9, d3), (a4, b1, c9, d4), (a4, b1, c9, d5), (a4, b1, c9, d6), (a4, b1, c9, d7), (a4, b1, c9, d8), (a4, b1, c9, d9), (a4, b1, c9, d10), (a4, b1, c9, d11), (a4, b1, c9, d12), (a4, b1, c10, d1), (a4, b1, c10, d2), (a4, b1, c10, d3), (a4, b1, c10, d4), (a4, b1, c10, d5), (a4, b1, c10, d6), (a4, b1, c10, d7), (a4, b1, c10, d8), (a4, b1, c10, d9), (a4, b1, c10, d10), (a4, b1, c10, d11), (a4, b1, c10, d12), (a4, b1, c11, d1), (a4, b1, c11, d2), (a4, b1, c11, d3), (a4, b1, c11, d4), (a4, b1, c11, d5), (a4, b1, c11, d6), (a4, b1, c11, d7), (a4, b1, c11, d8), (a4, b1, c11, d9), (a4, b1, c11, d10), (a4, b1, c11, d11), (a4, b1, c11, d12), (a4, b1, c12, d1), (a4, b1, c12, d2), (a4, b1, c12, d3), (a4, b1, c12, d4), (a4, b1, c12, d5), (a4, b1, c12, d6), (a4, b1, c12, d7), (a4, b1, c12, d8), (a4, b1, c12, d9), (a4, b1, c12, d10), (a4, b1, c12, d11), (a4, b1, c12, d12), (a4, b1, c13, d1), (a4, b1, c13, d2), (a4, b1, c13, d3), (a4, b1, c13, d4), (a4, b1, c13, d5), (a4, b1, c13, d6), (a4, b1, c13, d7), (a4, b1, c13, d8), (a4, b1, c13, d9), (a4, b1, c13, d10), (a4, b1, c13, d11), (a4, b1, c13, d12), (a4, b2, c1, d1), (a4, b2, c1, d2), (a4, b2, c1, d3), (a4, b2, c1, d4), (a4, b2, c1, d5), (a4, b2, c1, d6), (a4, b2, c1, d7), (a4, b2, c1, d8), (a4, b2, c1, d9), (a4, b2, c1, d10), (a4, b2, c1, d11), (a4, b2, c1, d12), (a4, b2, c2, d1), (a4, b2, c2, d2), (a4,

b2, c2, d3), (a4, b2, c2, d4), (a4, b2, c2, d5), (a4, b2, c2, d6), (a4, b2, c2, d7), (a4, b2, c2, d8), (a4, b2, c2, d9), (a4, b2, c2, d10), (a4, b2, c2, d11), (a4, b2, c2, d12), (a4, b2, c3, d1), (a4, b2, c3, d2), (a4, b2, c3, d3), (a4, b2, c3, d4), (a4, b2, c3, d5), (a4, b2, c3, d6), (a4, b2, c3, d7), (a4, b2, c3, d8), (a4, b2, c3, d9), (a4, b2, c3, d10), (a4, b2, c3, d11), (a4, b2, c3, d12), (a4, b2, c4, d1), (a4, b2, c4, d2), (a4, b2, c4, d3), (a4, b2, c4, d4), (a4, b2, c4, d5), (a4, b2, c4, d6), (a4, b2, c4, d7), (a4, b2, c4, d8), (a4, b2, c4, d9), (a4, b2, c4, d10), (a4, b2, c4, d11), (a4, b2, c4, d12), (a4, b2, c5, d1), (a4, b2, c5, d2), (a4, b2, c5, d3), (a4, b2, c5, d4), (a4, b2, c5, d5), (a4, b2, c5, d6), (a4, b2, c5, d7), (a4, b2, c5, d8), (a4, b2, c5, d9), (a4, b2, c5, d10), (a4, b2, c5, d11), (a4, b2, c5, d12), (a4, b2, c6, d1), (a4, b2, c6, d2), (a4, b2, c6, d3), (a4, b2, c6, d4), (a4, b2, c6, d5), (a4, b2, c6, d6), (a4, b2, c6, d7), (a4, b2, c6, d8), (a4, b2, c6, d9), (a4, b2, c6, d10), (a4, b2, c6, d11), (a4, b2, c6, d12), (a4, b2, c7, d1), (a4, b2, c7, d2), (a4, b2, c7, d3), (a4, b2, c7, d4), (a4, b2, c7, d5), (a4, b2, c7, d6), (a4, b2, c7, d7), (a4, b2, c7, d8), (a4, b2, c7, d9), (a4, b2, c7, d10), (a4, b2, c7, d11), (a4, b2, c7, d12), (a4, b2, c8, d1), (a4, b2, c8, d2), (a4, b2, c8, d3), (a4, b2, c8, d4), (a4, b2, c8, d5), (a4, b2, c8, d6), (a4, b2, c8, d7), (a4, b2, c8, d8), (a4, b2, c8, d9), (a4, b2, c8, d10), (a4, b2, c8, d11), (a4, b2, c8, d12), (a4, b2, c9, d1), (a4, b2, c9, d2), (a4, b2, c9, d3), (a4, b2, c9, d4), (a4, b2, c9, d5), (a4, b2, c9, d6), (a4, b2, c9, d7), (a4, b2, c9, d8), (a4, b2, c9, d9), (a4, b2, c9, d10), (a4, b2, c9, d11), (a4, b2, c9, d12), (a4, b2, c10, d1), (a4, b2, c10, d2), (a4, b2, c10, d3), (a4, b2, c10, d4), (a4, b2, c10, d5), (a4, b2, c10, d6), (a4, b2, c10, d7), (a4, b2, c10, d8), (a4, b2, c10, d9), (a4, b2, c10; d10), (a4, b2, c10, d11), (a4, b2, c10, d12), (a4, b2, c11, d1), (a4, b2, c11, d2), (a4, b2, c11, d3), (a4, b2, c11, d4), (a4, b2, c11, d5), (a4, b2, c11, d6), (a4, b2, c11, d7), (a4, b2, c11, d8), (a4, b2, c11, d9), (a4, b2, c11, d10), (a4, b2, c11, d11), (a4, b2, c11, d12), (a4, b2, c12, d1), (a4, b2, c12, d2), (a4, b2, c12, d3), (a4, b2, c12, d4), (a4, b2, c12, d5), (a4, b2, c12, d6), (a4, b2, c12, d7), (a4, b2, c12, d8), (a4, b2, c12, d9), (a4, b2, c12, d10), (a4, b2, c12, d11), (a4, b2, c12, d12), (a4, b2, c13, d1), (a4, b2, c13, d2), (a4, b2, c13, d3), (a4, b2, c13, d4), (a4, b2, c13, d5), (a4, b2, c13, d6), (a4, b2, c13, d7), (a4, b2, c13, d8), (a4, b2, c13, d9), (a4, b2, c13, d10), (a4, b2, c13, d11), (a4, b2, c13, d12), (a4, b3, c1, d1), (a4, b3, c1, d2), (a4, b3, c1, d3), (a4, b3, c1, d4), (a4, b3, c1, d5), (a4, b3, c1, d6), (a4, b3, c1, d7), (a4, b3, c1, d8), (a4, b3, c1, d9), (a4, b3, c1, d10), (a4, b3, c1, d11), (a4, b3, c1, d12), (a4, b3, c2, d1), (a4, b3, c2, d2), (a4, b3, c2, d3), (a4, b3, c2, d4), (a4, b3, c2, d5), (a4, b3, c2, d6), (a4, b3, c2, d7), (a4, b3, c2, d8), (a4, b3, c2, d9), (a4, b3, c2, d10), (a4, b3, c2, d11), (a4, b3, c2, d12), (a4, b3, c3, d1), (a4, b3, c3, d2), (a4, b3, c3, d3), (a4, b3, c3, d4), (a4, b3, c3, d5), (a4, b3, c3, d6), (a4, b3, c3, d7), (a4, b3, c3, d8), (a4, b3, c3, d9), (a4, b3, c3, d10), (a4, b3, c3, d11), (a4, b3, c3, d12), (a4, b3, c4, d1), (a4, b3, c4, d2), (a4, b3, c4, d3), (a4, b3, c4, d4), (a4, b3, c4, d5), (a4, b3, c4, d6), (a4, b3, c4, d7), (a4, b3, c4, d8), (a4, b3, c4, d9), (a4, b3, c4, d10), (a4, b3, c4, d11), (a4, b3, c4, d12), (a4, b3, c5, d1), (a4, b3, c5, d2), (a4, b3, c5, d3), (a4, b3, c5, d4), (a4, b3, c5, d5), (a4, b3, c5, d6), (a4, b3, c5, d7), (a4, b3, c5, d8), (a4, b3, c5, d9), (a4, b3, c5, d10), (a4, b3, c5, d11), (a4, b3, c5, d12), (a4, b3, c6, d1), (a4, b3, c6, d2), (a4, b3, c6, d3), (a4, b3, c6, d4), (a4, b3, c6, d5), (a4, b3, c6, d6), (a4, b3, c6, d7), (a4, b3, c6, d8), (a4, b3, c6, d9), (a4, b3, c6, d10), (a4, b3, c6, d11), (a4, b3, c6, d12), (a4, b3, c7, d1), (a4, b3, c7, d2), (a4, b3, c7, d3), (a4, b3, c7, d4), (a4, b3, c7, d5), (a4, b3, c7, d6), (a4, b3, c7, d7), (a4, b3, c7, d8), (a4, b3, c7, d9), (a4, b3, c7, d10), (a4, b3, c7, d11), (a4, b3, c7, d12), (a4, b3, c8, d1), (a4, b3, c8, d2), (a4, b3, c8, d3), (a4, b3, c8, d4), (a4, b3, c8, d5), (a4, b3, c8, d6), (a4, b3, c8, d7), (a4, b3, c8, d8), (a4, b3, c8, d9), (a4, b3, c8, d10), (a4, b3, c8, d11), (a4, b3, c8, d12), (a4, b3, c9, d1), (a4, b3, c9, d2), (a4, b3, c9, d3), (a4, b3, c9, d4), (a4, b3, c9, d5), (a4, b3, c9, d6), (a4, b3, c9, d7), (a4, b3, c9, d8), (a4, b3, c9, d9), (a4, b3, c9, d10), (a4, b3, c9, d11), (a4, b3, c9, d12), (a4, b3, c10, d1), (a4, b3, c10, d2), (a4, b3, c10, d3), (a4, b3, c10, d4), (a4, b3, c10, d5), (a4, b3, c10, d6), (a4, b3, c10, d7), (a4, b3, c10, d8), (a4, b3, c10, d9), (a4, b3, c10, d10), (a4, b3, c10, d11), (a4, b3, c10, d12), (a4, b3, c11, d1), (a4, b3, c11, d2), (a4, b3, c11, d3), (a4, b3, c11, d4), (a4, b3, c11, d5), (a4, b3, c11, d6), (a4, b3, c11, d7), (a4, b3, c11, d8), (a4, b3, c11, d9), (a4, b3, c11, d10), (a4, b3, c11, d11), (a4, b3, c11, d12), (a4, b3, c12, d1), (a4, b3, c12, d2), (a4, b3, c12, d3), (a4, b3, c12, d4), (a4, b3, c12, d5), (a4, b3, c12, d6), (a4, b3, c12, d7), (a4, b3, c12, d8), (a4, b3, c12, d9), (a4, b3, c12, d10), (a4, b3, c12, d11), (a4, b3, c12, d12), (a4, b3, c13, d1), (a4, b3, c13, d2), (a4, b3, c13, d3), (a4, b3, c13, d4), (a4, b3, c13, d5), (a4, b3, c13, d6), (a4, b3, c13, d7), (a4, b3, c13, d8), (a4, b3, c13, d9), (a4, b3, c13, d10), (a4, b3, c13, d11), (a4, b3, c13, d12), (a4, b4, c1, d1), (a4, b4, c1, d2), (a4, b4, c1, d3), (a4, b4, c1, d4), (a4, b4, c1, d5), (a4, b4, c1, d6), (a4, b4, c1, d7), (a4, b4, c1, d8), (a4, b4, c1, d9), (a4, b4, c1, d10), (a4, b4, c1, d11), (a4, b4, c1, d12), (a4, b4, c2, d1), (a4, b4, c2, d2), (a4, b4, c2, d3), (a4, b4, c2, d4), (a4, b4, c2, d5), (a4, b4, c2, d6), (a4, b4, c2, d7), (a4, b4, c2, d8), (a4, b4, c2, d9), (a4, b4, c2, d10), (a4, b4, c2, d11), (a4, b4, c2, d12), (a4, b4, c3, d1), (a4, b4, c3, d2), (a4, b4, c3, d3), (a4, b4, c3, d4), (a4, b4, c3, d5), (a4, b4, c3, d6), (a4, b4, c3, d7), (a4, b4, c3, d8), (a4, b4, c3, d9), (a4, b4, c3, d10), (a4, b4, c3, d11), (a4, b4, c3, d12), (a4, b4, c4, d1), (a4, b4, c4, d2), (a4, b4, c4, d3), (a4, b4, c4, d4), (a4, b4, c4, d5), (a4, b4, c4, d6), (a4, b4, c4, d7), (a4, b4, c4, d8), (a4, b4, c4, d9), (a4, b4, c4, d10), (a4, b4, c4, d11), (a4, b4, c4, d12), (a4, b4, c5, d1), (a4, b4, c5, d2), (a4, b4, c5, d3), (a4, b4, c5, d4), (a4, b4, c5, d5), (a4, b4, c5, d6), (a4, b4, c5, d7), (a4, b4, c5, d8), (a4, b4, c5, d9), (a4, b4, c5, d10), (a4, b4, c5, d11), (a4, b4, c5, d12), (a4, b4, c6, d1), (a4, b4, c6, d2), (a4, b4, c6, d3), (a4, b4, c6, d4), (a4, b4, c6, d5), (a4, b4, c6, d6), (a4, b4, c6, d7), (a4, b4, c6, d8), (a4, b4, c6, d9), (a4, b4, c6, d10), (a4, b4, c6, d11), (a4, b4, c6, d12), (a4, b4, c7, d1), (a4, b4, c7, d2), (a4, b4, c7, d3), (a4, b4, c7, d4), (a4, b4, c7, d5), (a4, b4, c7, d6), (a4, b4, c7, d7), (a4, b4, c7, d8), (a4, b4, c7, d9), (a4, b4, c7, d10), (a4, b4, c7, d11), (a4, b4, c7, d12), (a4, b4, c8, d1), (a4, b4, c8, d2), (a4, b4, c8, d3), (a4, b4, c8, d4), (a4, b4, c8, d5), (a4, b4, c8, d6), (a4, b4, c8, d7), (a4, b4, c8, d8), (a4, b4, c8, d9), (a4, b4, c8, d10), (a4, b4, c8, d11), (a4, b4, c8, d12), (a4, b4, c9, d1), (a4, b4, c9, d2), (a4, b4, c9, d3), (a4, b4, c9, d4), (a4, b4, c9, d5), (a4, b4, c9, d6), (a4, b4, c9, d7), (a4, b4, c9, d8), (a4, b4, c9, d9),

(a4, b4, c9, d10), (a4, b4, c9, d11), (a4, b4, c9, d12), (a4, b4, c10, d1), (a4, b4, c10, d2), (a4, b4, c10, d3), (a4, b4, c10, d4), (a4, b4, c10, d5), (a4, b4, c10, d6), (a4, b4, c10, d7), (a4, b4, c10, d8), (a4, b4, c10, d9), (a4, b4, c10, d10), (a4, b4, c10, d11), (a4, b4, c10, d12), (a4, b4, c11, d1), (a4, b4, c11, d2), (a4, b4, c11, d3), (a4, b4, c11, d4), (a4, b4, c11, d5), (a4, b4, c11, d6), (a4, b4, c11, d7), (a4, b4, c11, d8), (a4, b4, c11, d9), (a4, b4, c11, d10), (a4, b4, c11, d11), (a4, b4, c11, d12), (a4, b4, c12, d1), (a4, b4, c12, d2), (a4, b4, c12, d3), (a4, b4, c12, d4), (a4, b4, c12, d5), (a4, b4, c12, d6), (a4, b4, c12, d7), (a4, b4, c12, d8), (a4, b4, c12, d9), (a4, b4, c12, d10), (a4, b4, c12, d11), (a4, b4, c12, d12), (a4, b4, c13, d1), (a4, b4, c13, d2), (a4, b4, c13, d3), (a4, b4, c13, d4), (a4, b4, c13, d5), (a4, b4, c13, d6), (a4, b4, c13, d7), (a4, b4, c13, d8), (a4, b4, c13, d9), (a4, b4, c13, d10), (a4, b4, c13, d11), (a4, b4, c13, d12), (a4, b5, c1, d1), (a4, b5, c1, d2), (a4, b5, c1, d3), (a4, b5, c1, d4), (a4, b5, c1, d5), (a4, b5, c1, d6), (a4, b5, c1, d7), (a4, b5, c1, d8), (a4, b5, c1, d9), (a4, b5, c1, d10), (a4, b5, c1, d11), (a4, b5, c1, d12), (a4, b5, c2, d1), (a4, b5, c2, d2), (a4, b5, c2, d3), (a4, b5, c2, d4), (a4, b5, c2, d5), (a4, b5, c2, d6), (a4, b5, c2, d7), (a4, b5, c2, d8), (a4, b5, c2, d9), (a4, b5, c2, d10), (a4, b5, c2, d11), (a4, b5, c2, d12), (a4, b5, c3, d1), (a4, b5, c3, d2), (a4, b5, c3, d3), (a4, b5, c3, d4), (a4, b5, c3, d5), (a4, b5, c3, d6), (a4, b5, c3, d7), (a4, b5, c3, d8), (a4, b5, c3, d9), (a4, b5, c3, d10), (a4, b5, c3, d11), (a4, b5, c3, d12), (a4, b5, c4, d1), (a4, b5, c4, d2), (a4, b5, c4, d3), (a4, b5, c4, d4), (a4, b5, c4, d5), (a4, b5, c4, d6), (a4, b5, c4, d7), (a4, b5, c4, d8), (a4, b5, c4, d9), (a4, b5, c4, d10), (a4, b5, c4, d11), (a4, b5, c4, d12), (a4, b5, c5, d1), (a4, b5, c5, d2), (a4, b5, c5, d3), (a4, b5, c5, d4), (a4, b5, c5, d5), (a4, b5, c5, d6), (a4, b5, c5, d7), (a4, b5, c5, d8), (a4, b5, c5, d9), (a4, b5, c5, d10), (a4, b5, c5, d11), (a4, b5, c5, d12), (a4, b5, c6, d1), (a4, b5, c6, d2), (a4, b5, c6, d3), (a4, b5, c6, d4), (a4, b5, c6, d5), (a4, b5, c6, d6), (a4, b5, c6, d7), (a4, b5, c6, d8), (a4, b5, c6, d9), (a4, b5, c6, d10), (a4, b5, c6, d11), (a4, b5, c6, d12), (a4, b5, c7, d1), (a4, b5, c7, d2), (a4, b5, c7, d3), (a4, b5, c7, d4), (a4, b5, c7, d5), (a4, b5, c7, d6), (a4, b5, c7, d7), (a4, b5, c7, d8), (a4, b5, c7, d9), (a4, b5, c7, d10), (a4, b5, c7, d11), (a4, b5, c7, d12), (a4, b5, c8, d1), (a4, b5, c8, d2), (a4, b5, c8, d3), (a4, b5, c8, d4), (a4, b5, c8, d5), (a4, b5, c8, d6), (a4, b5, c8, d7), (a4, b5, c8, d8), (a4, b5, c8, d9), (a4, b5, c8, d10), (a4, b5, c8, d11), (a4, b5, c8, d12), (a4, b5, c9, d1), (a4, b5, c9, d2), (a4, b5, c9, d3), (a4, b5, c9, d4), (a4, b5, c9, d5), (a4, b5, c9, d6), (a4, b5, c9, d7), (a4, b5, c9, d8), (a4, b5, c9, d9), (a4, b5, c9, d10), (a4, b5, c9, d11), (a4, b5, c9, d12), (a4, b5, c10, d1), (a4, b5, c10, d2), (a4, b5, c10, d3), (a4, b5, c10, d4), (a4, b5, c10, d5), (a4, b5, c10, d6), (a4, b5, c10, d7), (a4, b5, c10, d8), (a4, b5, c10, d9), (a4, b5, c10, d10), (a4, b5, c10, d11), (a4, b5, c10, d12), (a4, b5, c11, d1), (a4, b5, c11, d2), (a4, b5, c11, d3), (a4, b5, c11, d4), (a4, b5, c11, d5), (a4, b5, c11, d6), (a4, b5, c11, d7), (a4, b5, c11, d8), (a4, b5, c11, d9), (a4, b5, c11, d10), (a4, b5, c11, d11), (a4, b5, c11, d12), (a4, b5, c12, d1), (a4, b5, c12, d2), (a4, b5, c12, d3), (a4, b5, c12, d4), (a4, b5, c12, d5), (a4, b5, c12, d6), (a4, b5, c12, d7), (a4, b5, c12, d8), (a4, b5, c12, d9), (a4, b5, c12, d10), (a4, b5, c12, d11), (a4, b5, c12, d12), (a4, b5, c13, d1), (a4, b5, c13, d2), (a4, b5, c13, d3), (a4, b5, c13, d4), (a4, b5, c13, d5), (a4, b5, c13, d6), (a4, b5, c13, d7), (a4, b5, c13, d8), (a4, b5, c13, d9), (a4, b5, c13, d10), (a4, b5, c13, d11), (a4, b5, c13, d12), (a4, b6, c1, d1), (a4, b6, c1, d2), (a4, b6, c1, d3), (a4, b6, c1, d4), (a4, b6, c1, d5), (a4, b6, c1, d6), (a4, b6, c1, d7), (a4, b6, c1, d8), (a4, b6, c1, d9), (a4, b6, c1, d10), (a4, b6, c1, d11), (a4, b6, c1, d12), (a4, b6, c2, d1), (a4, b6, c2, d2), (a4, b6, c2, d3), (a4, b6, c2, d4), (a4, b6, c2, d5), (a4, b6, c2, d6), (a4, b6, c2, d7), (a4, b6, c2, d8), (a4, b6, c2, d9), (a4, b6, c2, d10), (a4, b6, c2, d11), (a4, b6, c2, d12), (a4, b6, c3, d1), (a4, b6, c3, d2), (a4, b6, c3, d3), (a4, b6, c3, d4), (a4, b6, c3, d5), (a4, b6, c3, d6), (a4, b6, c3, d7), (a4, b6, c3, d8), (a4, b6, c3, d9), (a4, b6, c3, d10), (a4, b6, c3, d11), (a4, b6, c3, d12), (a4, b6, c4, d1), (a4, b6, c4, d2), (a4, b6, c4, d3), (a4, b6, c4, d4), (a4, b6, c4, d5), (a4, b6, c4, d6), (a4, b6, c4, d7), (a4; b6, c4, d8), (a4, b6, c4, d9), (a4, b6, c4, d10), (a4, b6, c4, d11), (a4, b6, c4, d12), (a4, b6, c5, d1), (a4, b6, c5, d2), (a4, b6, c5, d3), (a4, b6, c5, d4), (a4, b6, c5, d5), (a4, b6, c5, d6), (a4, b6, c5, d7), (a4, b6, c5, d8), (a4, b6, c5, d9), (a4, b6, c5, d10), (a4, b6, c5, d11), (a4, b6, c5, d12), (a4, b6, c6, d1), (a4, b6, c6, d2), (a4, b6, c6, d3), (a4, b6, c6, d4), (a4, b6, c6, d5), (a4, b6, c6, d6), (a4, b6, c6, d7), (a4, b6, c6, d8), (a4, b6, c6, d9), (a4, b6, c6, d10), (a4, b6, c6, d11), (a4, b6, c6, d12), (a4, b6, c7, d1), (a4, b6, c7, d2), (a4, b6, c7, d3), (a4, b6, c7, d4), (a4, b6, c7, d5), (a4, b6, c7, d6), (a4, b6, c7, d7), (a4, b6, c7, d8), (a4, b6, c7, d9), (a4, b6, c7, d10), (a4, b6, c7, d11), (a4, b6, c7, d12), (a4, b6, c8, d1), (a4, b6, c8, d2), (a4, b6, c8, d3), (a4, b6, c8, d4), (a4, b6, c8, d5), (a4, b6, c8, d6), (a4, b6, c8, d7), (a4, b6, c8, d8), (a4, b6, c8, d9), (a4, b6, c8, d10), (a4, b6, c8, d11), (a4, b6, c8, d12), (a4, b6, c9, d1), (a4, b6, c9, d2), (a4, b6, c9, d3), (a4, b6, c9, d4), (a4, b6, c9, d5), (a4, b6, c9, d6), (a4, b6, c9, d7), (a4, b6, c9, d8), (a4, b6, c9, d9), (a4, b6, c9, d10), (a4, b6, c9, d11), (a4, b6, c9, d12), (a4, b6, c10, d1), (a4, b6, c10, d2), (a4, b6, c10, d3), (a4, b6, c10, d4), (a4, b6, c10, d5), (a4, b6, c10, d6), (a4, b6, c10, d7), (a4, b6, c10, d8), (a4, b6, c10, d9), (a4, b6, c10, d10), (a4, b6, c10, d11), (a4, b6, c10, d12), (a4, b6, c11, d1), (a4, b6, c11, d2), (a4, b6, c11, d3), (a4, b6, c11, d4), (a4, b6, c11, d5), (a4, b6, c11, d6), (a4, b6, c11, d7), (a4, b6, c11, d8), (a4, b6, c11, d9), (a4, b6, c11, d10), (a4, b6, c11, d11), (a4, b6, c11, d12), (a4, b6, c12, d1), (a4, b6, c12, d2), (a4, b6, c12, d3), (a4, b6, c12, d4), (a4, b6, c12, d5), (a4, b6, c12, d6), (a4, b6, c12, d7), (a4, b6, c12, d8), (a4, b6, c12, d9), (a4, b6, c12, d10), (a4, b6, c12, d11), (a4, b6, c12, d12), (a4, b6, c13, d1), (a4, b6, c13, d2), (a4, b6, c13, d3), (a4, b6, c13, d4), (a4, b6, c13, d5), (a4, b6, c13, d6), (a4, b6, c13, d7), (a4, b6, c13, d8), (a4, b6, c13, d9), (a4, b6, c13, d10), (a4, b6, c13, d11), (a4, b6, c13, d12), (a4, b7, c1, d1), (a4, b7, c1, d2), (a4, b7, c1, d3), (a4, b7, c1, d4), (a4, b7, c1, d5), (a4, b7, c1, d6), (a4, b7, c1, d7), (a4, b7, c1, d8), (a4, b7, c1, d9), (a4, b7, c1, d10), (a4, b7, c1, d11), (a4, b7, c1, d12), (a4, b7, c2, d1), (a4, b7, c2, d2), (a4, b7, c2, d3), (a4, b7, c2, d4), (a4, b7, c2, d5), (a4, b7, c2, d6), (a4, b7, c2, d7), (a4, b7, c2, d8), (a4, b7, c2, d9), (a4, b7, c2, d10), (a4, b7, c2, d11), (a4, b7, c2, d12), (a4, b7, c3, d1), (a4, b7, c3, d2), (a4, b7, c3, d3), (a4, b7, c3, d4), (a4, b7, c3, d5), (a4, b7, c3, d6), (a4, b7, c3, d7), (a4, b7, c3, d8), (a4, b7, c3, d9), (a4, b7, c3, d10), (a4, b7, c3, d11), (a4, b7, c3, d12),

(a4, b7, c4, d1), (a4, b7, c4, d2), (a4, b7, c4, d3), (a4, b7, c4, d4), (a4, b7, c4, d5), (a4, b7, c4, d6), (a4, b7, c4, d7), (a4, b7, c4, d8), (a4, b7, c4, d9), (a4, b7, c4, d10), (a4, b7, c4, d11), (a4, b7, c4, d12), (a4, b7, c5, d1), (a4, b7, c5, d2), (a4, b7, c5, d3), (a4, b7, c5, d4), (a4, b7, c5, d5), (a4, b7, c5, d6), (a4, b7, c5, d7), (a4, b7, c5, d8), (a4, b7, c5, d9), (a4, b7, c5, d10), (a4, b7, c5, d11), (a4, b7, c5, d12), (a4, b7, c6, d1), (a4, b7, c6, d2), (a4, b7, c6, d3), (a4, b7, c6, d4), (a4, b7, c6, d5), (a4, b7, c6, d6), (a4, b7, c6, d7), (a4, b7, c6, d8), (a4, b7, c6, d9), (a4, b7, c6, d10), (a4, b7, c6, d11), (a4, b7, c6, d12), (a4, b7, c7, d1), (a4, b7, c7, d2), (a4, b7, c7, d3), (a4, b7, c7, d4), (a4, b7, c7, d5), (a4, b7, c7, d6), (a4, b7, c7, d7), (a4, b7, c7, d8), (a4, b7, c7, d9), (a4, b7, c7, d10), (a4, b7, c7, d11), (a4, b7, c7, d12), (a4, b7, c8, d1), (a4, b7, c8, d2), (a4, b7, c8, d3), (a4, b7, c8, d4), (a4, b7, c8, d5), (a4, b7, c8, d6), (a4, b7, c8, d7), (a4, b7, c8, d8), (a4, b7, c8, d9), (a4, b7, c8, d10), (a4, b7, c8, d11), (a4, b7, c8, d12), (a4, b7, c9, d1), (a4, b7, c9, d2), (a4, b7, c9, d3), (a4, b7, c9, d4), (a4, b7, c9, d5), (a4, b7, c9, d6), (a4, b7, c9, d7), (a4, b7, c9, d8), (a4, b7, c9, d9), (a4, b7, c9, d10), (a4, b7, c9, d11), (a4, b7, c9, d12), (a4, b7, c10, d1), (a4, b7, c10, d2), (a4, b7, c10, d3), (a4, b7, c10, d4), (a4, b7, c10, d5), (a4, b7, c10, d6), (a4, b7, c10, d7), (a4, b7, c10, d8), (a4, b7, c10, d9), (a4, b7, c10, d10), (a4, b7, c10, d11), (a4, b7, c10, d12), (a4, b7, c11, d1), (a4, b7, c11, d2), (a4, b7, c11, d3), (a4, b7, c11, d4), (a4, b7, c11, d5), (a4, b7, c11, d6), (a4, b7, c11, d7), (a4, b7, c11, d8), (a4, b7, c11, d9), (a4, b7, c11, d10), (a4, b7, c11, d11), (a4, b7, c11, d12), (a4, b7, c12, d1), (a4, b7, c12, d2), (a4, b7, c12, d3), (a4, b7, c12, d4), (a4, b7, c12, d5), (a4, b7, c12, d6), (a4, b7, c12, d7), (a4, b7, c12, d8), (a4, b7, c12, d9), (a4, b7, c12, d10), (a4, b7, c12, d11), (a4, b7, c12, d12), (a4, b7, c13, d1), (a4, b7, c13, d2), (a4, b7, c13, d3), (a4, b7, c13, d4), (a4, b7, c13, d5), (a4, b7, c13, d6), (a4, b7, c13, d7), (a4, b7, c13, d8), (a4, b7, c13, d9), (a4, b7, c13, d10), (a4, b7, c13, d11), (a4, b7, c13, d12), (a4, b8, c1, d1), (a4, b8, c1, d2), (a4, b8, c1, d3), (a4, b8, c1, d4), (a4, b8, c1, d5), (a4, b8, c1, d6), (a4, b8, c1, d7), (a4, b8, c1, d8), (a4, b8, c1, d9), (a4, b8, c1, d10), (a4, b8, c1, d11), (a4, b8, c1, d12), (a4, b8, c2, d1), (a4, b8, c2, d2), (a4, b8, c2, d3), (a4, b8, c2, d4), (a4, b8, c2, d5), (a4, b8, c2, d6), (a4, b8, c2, d7), (a4, b8, c2, d8), (a4, b8, c2, d9), (a4, b8, c2, d10), (a4, b8, c2, d11), (a4, b8, c2, d12), (a4, b8, c3, d1), (a4, b8, c3, d2), (a4, b8, c3, d3), (a4, b8, c3, d4), (a4, b8, c3, d5), (a4, b8, c3, d6), (a4, b8, c3, d7), (a4, b8, c3, d8), (a4, b8, c3, d9), (a4, b8, c3, d10), (a4, b8, c3, d11), (a4, b8, c3, d12), (a4, b8, c4, d1), (a4, b8, c4, d2), (a4, b8, c4, d3), (a4, b8, c4, d4), (a4, b8, c4, d5), (a4, b8, c4, d6), (a4, b8, c4, d7), (a4, b8, c4, d8), (a4, b8, c4, d9), (a4, b8, c4, d10), (a4, b8, c4, d11), (a4, b8, c4, d12), (a4, b8, c5, d1), (a4, b8, c5, d2), (a4, b8, c5, d3), (a4, b8, c5, d4), (a4, b8, c5, d5), (a4, b8, c5, d6), (a4, b8, c5, d7), (a4, b8, c5, d8), (a4, b8, c5, d9), (a4, b8, c5, d10), (a4, b8, c5, d11), (a4, b8, c5, d12), (a4, b8, c6, d1), (a4, b8, c6, d2), (a4, b8, c6, d3), (a4, b8, c6, d4), (a4, b8, c6, d5), (a4, b8, c6, d6), (a4, b8, c6, d7), (a4, b8, c6, d8), (a4, b8, c6, d9), (a4, b8, c6, d10), (a4, b8, c6, d11), (a4, b8, c6, d12), (a4, b8, c7, d1), (a4, b8, c7, d2), (a4, b8, c7, d3), (a4, b8, c7, d4), (a4, b8, c7, d5), (a4, b8, c7, d6), (a4, b8, c7, d7), (a4, b8, c7, d8), (a4, b8, c7, d9), (a4, b8, c7, d10), (a4, b8, c7, d11), (a4, b8, c7, d12), (a4, b8, c8, d1), (a4, b8, c8, d2), (a4, b8, c8, d3), (a4, b8, c8, d4), (a4, b8, c8, d5), (a4, b8, c8, d6), (a4, b8, c8, d7), (a4, b8, c8, d8), (a4, b8, c8, d9), (a4, b8, c8, d10), (a4, b8, c8, d11), (a4, b8, c8, d12), (a4, b8, c9, d1), (a4, b8, c9, d2), (a4, b8, c9, d3), (a4, b8, c9, d4), (a4, b8, c9, d5), (a4, b8, c9, d6), (a4, b8, c9, d7), (a4, b8, c9, d8), (a4, b8, c9, d9), (a4, b8, c9, d10), (a4, b8, c9, d11), (a4, b8, c9, d12), (a4, b8, c10, d1, (a4, b8, c10, d2), (a4, b8, c10, d3), (a4, b8, c10, d4), (a4, b8, c10, d5), (a4, b8, c10, d6), (a4, b8, c10, d7), (a4, b8, c10, d8), (a4, b8, c10, d9), (a4, b8, c10, d10), (a4, b8, c10, d11), (a4, b8, c10, d12), (a4, b8, c11, d1), (a4, b8, c11, d2), (a4, b8, c11, d3), (a4, b8, c11, d4), (a4, b8, c11, d5), (a4, b8, c11, d6), (a4, b8, c11, d7), (a4, b8, c11, d8), (a4, b8, c11, d9), (a4, b8, c11, d10), (a4, b8, c11, d11), (a4, b8, c11, d12), (a4, b8, c12, d1), (a4, b8, c12, d2), (a4, b8, c12, d3), (a4, b8, c12, d4), (a4, b8, c12, d5), (a4, b8, c12, d6), (a4, b8, c12, d7), (a4, b8, c12, d8), (a4, b8, c12, d9), (a4, b8, c12, d10), (a4, b8, c12, d11), (a4, b8, c12, d12), (a4, b8, c13, d1), (a4, b8, c13, d2), (a4, b8, c13, d3), (a4, b8, c13, d4), (a4, b8, c13, d5), (a4, b8, c13, d6), (a4, b8, c13, d7), (a4, b8, c13, d8), (a4, b8, c13, d9), (a4, b8, c13, d10), (a4, b8, c13, d11), (a4, b8, c13, d12), (a4, b9, c1, d1), (a4, b9, c1, d2), (a4, b9, c1, d3), (a4, b9, c1, d4), (a4, b9, c1, d5), (a4, b9, c1, d6), (a4, b9, c1, d7), (a4, b9, c1, d8), (a4, b9, c1, d9), (a4, b9, c1, d10), (a4, b9, c1, d11), (a4, b9, c1, d12), (a4, b9, c2, d1), (a4, b9, c2, d2), (a4, b9, c2, d3), (a4, b9, c2, d4), (a4, b9, c2, d5), (a4, b9, c2, d6), (a4, b9, c2, d7), (a4, b9, c2, d8), (a4, b9, c2, d9), (a4, b9, c2, d10), (a4, b9, c2, d11), (a4, b9, c2, d12), (a4, b9, c3, d1), (a4, b9, c3, d2), (a4, b9, c3, d3), (a4, b9, c3, d4), (a4, b9, c3, d5), (a4, b9, c3, d6), (a4, b9, c3, d7), (a4, b9, c3, d8), (a4, b9, c3, d9), (a4, b9, c3, d10), (a4, b9, c3, d11), (a4, b9, c3, d12), (a4, b9, c4, d1), (a4, b9, c4, d2), (a4, b9, c4, d3), (a4, b9, c4, d4), (a4, b9, c4, d5), (a4, b9, c4, d6), (a4, b9, c4, d7), (a4, b9, c4, d8), (a4, b9, c4, d9), (a4, b9, c4, d10), (a4, b9, c4, d11), (a4, b9, c4, d12), (a4, b9, c5, d1), (a4, b9, c5, d2), (a4, b9, c5, d3), (a4, b9, c5, d4), (a4, b9, c5, d5), (a4, b9, c5, d6), (a4, b9, c5, d7), (a4, b9, c5, d8), (a4, b9, c5, d9), (a4, b9, c5, d10), (a4, b9, c5, d11), (a4, b9, c5, d12), (a4, b9, c6, d1), (a4, b9, c6, d2), (a4, b9, c6, d3), (a4, b9, c6, d4), (a4, b9, c6, d5), (a4, b9, c6, d6), (a4, b9, c6, d7), (a4, b9, c6, d8), (a4, b9, c6, d9), (a4, b9, c6, d10), (a4, b9, c6, d11), (a4, b9, c6, d12), (a4, b9, c7, d1), (a4, b9, c7, d2), (a4, b9, c7, d3), (a4, b9, c7, d4), (a4, b9, c7, d5), (a4, b9, c7, d6), (a4, b9, c7, d7), (a4, b9, c7, d8), (a4, b9, c7, d9), (a4, b9, c7, d10), (a4, b9, c7, d11), (a4, b9, c7, d12), (a4, b9, c8, d1), (a4, b9, c8, d2), (a4, b9, c8, d3), (a4, b9, c8, d4), (a4, b9, c8, d5), (a4, b9, c8, d6), (a4, b9, c8, d7), (a4, b9, c8, d8), (a4, b9, c8, d9), (a4, b9, c8, d10), (a4, b9, c8, d11), (a4, b9, c8, d12), (a4, b9, c9, d1), (a4, b9, c9, d2), (a4, b9, c9, d3), (a4, b9, c9, d4), (a4, b9, c9, d5), (a4, b9, c9, d6), (a4, b9, c9, d7), (a4, b9, c9, d8), (a4, b9, c9, d9), (a4, b9, c9, d10), (a4, b9, c9, d11), (a4, b9, c9, d12), (a4, b9, c10, d1), (a4, b9, c10, d2), (a4, b9, c10, d3), (a4, b9, c10, d4), (a4, b9, c10, d5), (a4, b9, c10, d6), (a4, b9, c10, d7), (a4, b9, c10, d8), (a4, b9, c10, d9), (a4, b9, c10, d10), (a4, b9, c10, d11), (a4, b9, c10, d12), (a4, b9, c11, d1), (a4, b9, c11, d2), (a4, b9, c11, d3), (a4, b9, c11, d4), (a4, b9, c11, d5), (a4,

b9, c11, d6), (a4, b9, c11, d7), (a4, b9, c11, d8), (a4, b9, c11, d9), (a4, b9, c11, d10), (a4, b9, c11, d11), (a4, b9, c11, d12), (a4, b9, c12, d1), (a4, b9, c12, d2), (a4, b9, c12, d3), (a4, b9, c12, d4), (a4, b9, c12, d5), (a4, b9, c12, d6), (a4, b9, c12, d7), (a4, b9, c12, d8), (a4, b9, c12, d9), (a4, b9, c12, d10), (a4, b9, c12, d11), (a4, b9, c12, d12), (a4, b9, c13, d1), (a4, b9, c13, d2), (a4, b9, c13, d3), (a4, b9, c13, d4), (a4, b9, c13, d5), (a4, b9, c13, d6), (a4, b9, c13, d7), (a4, b9, c13, d8), (a4, b9, c13, d9), (a4, b9, c13, d10), (a4, b9, c13, d11), (a4, b9, c13, d12), (a4, b10, c1, d1), (a4, b10, c1, d2), (a4, b10, c1, d3), (a4, b10, c1, d4), (a4, b10, c1, d5), (a4, b10, c1, d6), (a4, b10, c1, d7), (a4, b10, c1, d8), (a4, b10, c1, d9), (a4, b10, c1, d10), (a4, b10, c1, d11), (a4, b10, c1, d12), (a4, b10, c2, d1), (a4, b10, c2, d2), (a4, b10, c2, d3), (a4, b10, c2, d4), (a4, b10, c2, d5), (a4, b10, c2, d6), (a4, b10, c2, d7), (a4, b10, c2, d8), (a4, b10, c2, d9), (a4, b10, c2, d10), (a4, b10, c2, d11), (a4, b10, c2, d12), (a4, b10, c3, d1), (a4, b10, c3, d2), (a4, b10, c3, d3), (a4, b10, c3, d4), (a4, b10, c3, d5), (a4, b10, c3, d6), (a4, b10, c3, d7), (a4, b10, c3, d8), (a4, b10, c3, d9), (a4, b10, c3, d10), (a4, b10, c3, d11), (a4, b10, c3, d12), (a4, b10, c4, d1), (a4, b10, c4, d2), (a4, b10, c4, d3), (a4, b10, c4, d4), (a4, b10, c4, d5), (a4, b10, c4, d6), (a4, b10, c4, d7), (a4, b10, c4, d8), (a4, b10, c4, d9), (a4, b10, c4, d10), (a4, b10, c4, d11), (a4, b10, c4, d12), (a4, b10, c5, d1), (a4, b10, c5, d2), (a4, b10, c5, d3), (a4, b10, c5, d4), (a4, b10, c5, d5), (a4, b10, c5, d6), (a4, b10, c5, d7), (a4, b10, c5, d8), (a4, b10, c5, d9), (a4, b10, c5, d10), (a4, b10, c5, d11), (a4, b10, c5, d12), (a4, b10, c6, d1), (a4, b10, c6, d2), (a4, b10, c6, d3), (a4, b10, c6, d4), (a4, b10, c6, d5), (a4, b10, c6, d6), (a4, b10, c6, d7), (a4, b10, c6, d8), (a4, b10, c6, d9), (a4, b10, c6, d10), (a4, b10, c6, d11), (a4, b10, c6, d12), (a4, b10, c7, d1), (a4, b10, c7, d2), (a4, b10, c7, d3), (a4, b10, c7, d4), (a4, b10, c7, d5), (a4, b10, c7, d6), (a4, b10, c7, d7), (a4, b10, c7, d8), (a4, b10, c7, d9), (a4, b10, c7, d10), (a4, b10, c7, d11), (a4, b10, c7, d12), (a4, b10, c8, d1), (a4, b10, c8, d2), (a4, b10, c8, d3), (a4, b10, c8, d4), (a4, b10, c8, d5), (a4, b10, c8, d6), (a4, b10, c8, d7), (a4, b10, c8, d8), (a4, b10, c8, d9), (a4, b10, c8, d10), (a4, b10, c8, d11), (a4, b10, c8, d12), (a4, b10, c9, d1), (a4, b10, c9, d2), (a4, b10, c9, d3), (a4, b10, c9, d4), (a4, b10, c9, d5), (a4, b10, c9, d6), (a4, b10, c9, d7), (a4, b10, c9, d8), (a4, b10, c9, d9), (a4, b10, c9, d10), (a4, b10, c9, d11), (a4, b10, c9, d12), (a4, b10, c10, d1), (a4, b10, c10, d2), (a4, b10, c10, d3), (a4, b10, c10, d4), (a4, b10, c10, d5), (a4, b10, c10, d6), (a4, b10, c10, d7), (a4, b10, c10, d8), (a4, b10, c10, d9), (a4, b10, c10, d10), (a4, b10, c10, d11), (a4, b10, c10, d12), (a4, b10, c11, d1), (a4, b10, c11, d2), (a4, b10, c11, d3), (a4, b10, c11, d4), (a4, b10, c11, d5), (a4, b10, c11, d6), (a4, b10, c11, d7), (a4, b10, c11, d8), (a4, b10, c11, d9), (a4, b10, c11, d10), (a4, b10, c11, d11), (a4, b10, c11, d12), (a4, b10, c12, d1), (a4, b10, c12, d2), (a4, b10, c12, d3), (a4, b10, c12, d4), (a4, b10, c12, d5), (a4, b10, c12, d6), (a4, b10, c12, d7), (a4, b10, c12, d8), (a4, b10, c12, d9), (a4, b10, c12, d10), (a4, b10, c12, d11), (a4 b10, c12, d12), (a4, b10, c13, d1), (a4, b10, c13, d2), (a4, b10, c13, d3), (a4, b10, c13, d4), (a4, b10, c13, d5), (a4, b10 c13, d6), (a4, b10, c13, d7), (a4, b10, c13, d8), (a4, b10, c13, d9), (a4, b10, c13, d10), (a4, b10, c13, d11), (a4, b10, c13, d12), (a4, b11, c1, d1), (a4, b11, c1, d2), (a4, b11, c1, d3), (a4, b11, c1, d4), (a4, b11, c1, d5), (a4, b11, c1, d6), (a4, b11, c1, d7), (a4, b11, c1, d8), (a4, b11, c1, d9), (a4, b11, c1, d10), (a4, b11, c1, d11), (a4, b11, c1, d12), (a4, b11, c2, d1), (a4, b11, c2, d2), (a4, b11, c2, d3), (a4, b11, c2, d4), (a4, b11, c2, d5), (a4, b11, c2, d6), (a4, b11, c2, d7), (a4, b11, c2, d8), (a4, b11, c2, d9), (a4, b11, c2, d10), (a4, b11, c2, d11), (a4, b11, c2, d12), (a4, b11, c3, d1), (a4, b11, c3, d2), (a4, b11, c3, d3), (a4, b11, c3, d4), (a4, b11, c3, d5), (a4, b11, c3, d6), (a4, b11, c3, d7), (a4, b11, c3, d8), (a4, b11, c3, d9), (a4, b11, c3, d10), (a4, b11, c3, d11), (a4, b11, c3, d12), (a4, b11, c4, d1), (a4, b11, c4, d2), (a4, b11, c4, d3), (a4, b11, c4, d4), (a4, b11, c4, d5), (a4, b11, c4, d6), (a4, b11, c4, d7), (a4, b11, c4, d8), (a4, b11, c4, d9), (a4, b11, c4, d10), (a4, b11, c4, d11), (a4, b11, c4, d12), (a4, b11, c5, d1), (a4, b11, c5, d2), (a4, b11, c5, d3), (a4, b11, c5, d4), (a4, b11, c5, d5), (a4, b11, c5, d6), (a4, b11, c5, d7), (a4, b11, c5, d8), (a4, b11, c5, d9), (a4, b11, c5, d10), (a4, b11, c5, d11), (a4, b11, c5, d12), (a4, b11, c6, d1), (a4, b11, c6, d2), (a4, b11, c6, d3), (a4, b11, c6, d4), (a4, b11, c6, d5), (a4, b11, c6, d6), (a4, b11, c6, d7), (a4, b11, c6, d8), (a4, b11, c6, d9), (a4, b11, c6, d10), (a4, b11, c6, d11), (a4, b11, c6, d12), (a4, b11, c7, d1), (a4, b11, c7, d2), (a4, b11, c7, d3), (a4, b11, c7, d4), (a4, b11, c7, d5), (a4, b11, c7, d6), (a4, b11, c7, d7), (a4, b11, c7, d8), (a4, b11, c7, d9), (a4, b11, c7, d10), (a4, b11, c7, d11), (a4, b11, c7, d12), (a4, b11, c8, d1), (a4, b11, c8, d2), (a4, b11, c8, d3), (a4, b11, c8, d4), (a4, b11, c8, d5), (a4, b11, c8, d6), (a4, b11, c8, d7), (a4, b11, c8, d8), (a4, b11, c8, d9), (a4, b11, c8, d10), (a4, b11, c8, d11), (a4, b11, c8, d12), (a4, b11, c9, d1), (a4, b11, c9, d2), (a4, b11, c9, d3), (a4, b11, c9, d4), (a4, b11, c9, d5), (a4, b11, c9, d6), (a4, b11, c9, d7), (a4, b11, c9, d8), (a4, b11, c9, d9), (a4, b11, c9, d10), (a4, b11, c9, d11), (a4, b11, c9, d12), (a4, b11, c10, d1), (a4, b11, c10, d2), (a4, b11, c10, d3), (a4, b11, c10, d4), (a4, b11, c10, d5), (a4, b11, c10, d6), (a4, b11, c10, d7), (a4, b11, c10, d8), (a4, b11, c10, d9), (a4, b11, c10, d10), (a4, b11, c10, d11), (a4, b11, c10, d12), (a4, b11, c11, d1), (a4, b11, c11, d2), (a4, b11, c11, d3), (a4, b11, c11, d4), (a4, b11, c11, d5), (a4, b11, c11, d6), (a4, b11, c11, d7), (a4, b11, c11, d8), (a4, b11, c11, d9), (a4, b11, c11, d10), (a4, b11, c11, d11), (a4, b11, c11, d12), (a4, b11, c12, d1), (a4, b11, c12, d2), (a4, b11, c12, d3), (a4, b11, c12, d4), (a4, b11, c12, d5), (a4, b11, c12, d6), (a4, b11, c12, d7), (a4, b11, c12, d8), (a4, b11, c12, d9), (a4, b11, c12, d10), (a4, b11, c12, d11), (a4, b11, c12, d12), (a4, b11, c13, d1), (a4, b11, c13, d2), (a4, b11, c13, d3), (a4, b11, c13, d4), (a4, b11, c13, d5), (a4, b11, c13, d6), (a4, b11, c13, d7), (a4, b11, c13, d8), (a4, b11, c13, d9), (a4, b11, c13, d10), (a4, b11, c13, d11), (a4, b11, c13, d12), (a4, b12, c1, d1), (a4, b12, c1, d2), (a4, b12, c1, d3), (a4, b12, c1, d4), (a4, b12, c1, d5), (a4, b12, c1, d6), (a4, b12, c1, d7), (a4, b12, c1, d8), (a4, b12, c1, d9), (a4, b12, c1, d10), (a4, b12, c1, d11), (a4, b12, c1, d12), (a4, b12, c2, d1), (a4, b12, c2, d2), (a4, b12, c2, d3), (a4, b12, c2, d4), (a4, b12, c2, d5), (a4, b12, c2, d6), (a4, b12, c2, d7), (a4, b12, c2, d8), (a4, b12, c2, d9), (a4, b 12, c2, d10), (a4, b12, c2, d11), (a4, b12, c2, d12), (a4, b12, c3, d1), (a4, b12, c3, d2), (a4, b12, c3, d3), (a4, b12, c3, d4), (a4, b12, c3, d5), (a4, b12, c3, d6), (a4, b12, c3, d7), (a4, b12, c3, d8), (a4,

b12, c3, d9), (a4, b12, c3, d10), (a4, b12, c3, d11), (a4, b12, c3, d12), (a4, b12, c4, d1), (a4, b12, c4, d2), (a4, b12, c4, d3), (a4, b12, c4, d4), (a4, b12, c4, d5), (a4, b12, c4, d6), (a4, b 12, c4, d7), (a4, b12, c4, d8), (a4, b12, c4, d9), (a4, b12, c4, d10), (a4, b12, c4, d11), (a4, b12, c4, d12), (a4, b12, c5, d1), (a4, b12, c5, d2), (a4, b12, c5, d3), (a4, b12, c5, d4), (a4, b12, c5, d5), (a4, b12, c5, d6), (a4, b12, c5, d7), (a4, b12, c5, d8), (a4, b12, c5, d9), (a4, b12, c5, d10), (a4, b12, c5, d11), (a4, b12, c5, d12), (a4, b12, c6, d1), (a4, b12, c6, d2), (a4, b12, c6, d3), (a4, b12, c6, d4), (a4, b12, c6, d5), (a4, b12, c6, d6), (a4, b12, c6, d7), (a4, b12, c6, d8), (a4, b12, c6, d9), (a4, b12, c6, d10), (a4, b12, c6, d11), (a4, b12, c6, d12), (a4, b12, c7, d1), (a4, b12, c7, d2), (a4, b12, c7, d3), (a4, b12, c7, d4), (a4, b12, c7, d5), (a4, b12, c7, d6), (a4, b12, c7, d7), (a4, b12, c7, d8), (a4, b12, c7, d9), (a4, b12, c7, d10), (a4, b12, c7, d11), (a4, b12, c7, d12), (a4, b12, c8, d1), (a4, b12, c8, d2), (a4, b12, c8, d3), (a4, b12, c8, d4), (a4, b12, c8, d5), (a4, b12, c8, d6), (a4, b12, c8, d7), (a4, b12, c8, d8), (a4, b12, c8, d9), (a4, b12, c8, d10), (a4, b12, c8, d11), (a4, b12, c8, d12), (a4, b12, c9, d1), (a4, b12, c9, d2), (a4, b12, c9, d3), (a4, b12, c9, d4), (a4, b12, c9, d5), (a4, b12, c9, d6), (a4, b12, c9, d7), (a4, b12, c9, d8), (a4, b12, c9, d9), (a4, b12, c9, d10), (a4, b12, c9, d11), (a4, b12, c9, d12), (a4, b12, c10, d1), (a4, b12, c10, d2), (a4, b12, c10, d3), (a4, b12, c10, d4), (a4, b12, c10, d5), (a4, b12, c10, d6), (a4, b12, c10, d7), (a4, b12, c10, d8), (a4, b12, c10, d9), (a4, b12, c10, d10), (a4, b12, c10, d11), (a4, b12, c10, d12), (a4, b12, c11, d1), (a4, b12, c11, d2), (a4, b12, c11, d3), (a4, b12, c11, d4), (a4, b12, c11, d5), (a4, b12, c11, d6), (a4, b12, c11, d7), (a4, b12, c11, d8), (a4, b12, c11, d9), (a4, b12, c11, d10), (a4, b12, c11, d11), (a4, b12, c11, d12), (a4, b12, c12, d1), (a4, b12, c12, d2), (a4, b12, c12, d3), (a4, b12, c12, d4), (a4, b12, c12, d5), (a4, b12, c12, d6), (a4, b12, c12, d7), (a4, b12, c12, d8), (a4, b12, c12, d9), (a4, b12, c12, d10), (a4, b12, c12, d11), (a4, b12, c12, d12), (a4, b12, c13, d1), (a4, b12, c13, d2), (a4, b12, c13, d3), (a4, b12, c13, d4), (a4, b12, c13, d5), (a4, b12, c13, d6), (a4, b12, c13, d7), (a4, b12, c13, d8), (a4, b12, c13, d9), (a4, b12, c13, d10), (a4, b12, c13, d11), (a4, b12, c13, d12), (a4, b13, c1, d1), (a4, b13, c1, d2), (a4, b13, c1, d3), (a4, b13, c1, d4), (a4, b13, c1, d5), (a4, b13, c1, d6), (a4, b13, c1, d7), (a4, b13, c1, d8), (a4, b13, c1, d9), (a4, b13, c1, d10), (a4, b13, c1, d11), (a4, b13, c1, d12), (a4, b13, c2, d1), (a4, b13, c2, d2), (a4, b13, c2, d3), (a4, b13, c2, d4), (a4, b13, c2, d5), (a4, b13, c2, d6), (a4, b13, c2, d7), (a4, b13, c2, d8), (a4, b13, c2, d9), (a4, b13, c2, d10), (a4, b13, c2, d11), (a4, b13, c2, d12), (a4, b13, c3, d1), (a4, b13, c3, d2), (a4, b13, c3, d3), (a4, b13, c3, d4), (a4, b13, c3, d5), (a4, b13, c3, d6), (a4, b13, c3, d7), (a4, b13, c3, d8), (a4, b13, c3, d9), (a4, b13, c3, d10), (a4, b13, c3, d11), (a4, b13, c3, d12), (a4, b13, c4, d1), (a4, b13, c4, d2), (a4, b13, c4, d3), (a4, b13, c4, d4), (a4, b13, c4, d5), (a4, b13, c4, d6), (a4, b13, c4, d7), (a4, b13, c4, d8), (a4, b13, c4, d9), (a4, b13, c4, d10), (a4, b13, c4, d11), (a4, b13, c4, d12), (a4, b13, c5, d1), (a4, b13, c5, d2), (a4, b13, c5, d3), (a4, b13, c5, d4), (a4, b13, c5, d5), (a4, b13, c5, d6), (a4, b13, c5, d7), (a4, b13, c5, d8), (a4, b13, c5, d9), (a4, b13, c5, d10), (a4, b13, c5, d11), (a4, b13, c5, d12), (a4, b13, c6, d1), (a4, b13, c6, d2), (a4, b13, c6, d3), (a4, b13, c6, d4), (a4, b13, c6, d5), (a4, b13, c6, d6), (a4, b13, c6, d7), (a4, b13, c6, d8), (a4, b13, c6, d9), (a4, b13, c6, d10), (a4, b13, c6, d11), (a4, b13, c6, d12), (a4, b13, c7, d1), (a4, b13, c7, d2), (a4, b13, c7, d3), (a4, b13, c7, d4), (a4, b13, c7, d5), (a4, b13, c7, d6), (a4, b13, c7, d7), (a4, b13, c7, d8), (a4, b13, c7, d9), (a4, b13, c7, d10), (a4, b13, c7, d11), (a4, b13, c7, d12), (a4, b13, c8, d1), (a4, b13, c8, d2), (a4, b13, c8, d3), (a4, b13, c8, d4), (a4, b13, c8, d5), (a4, b13, c8, d6), (a4, b13, c8, d7), (a4, b13, c8, d8), (a4, b13, c8, d9), (a4, b13, c8, d10), (a4, b13, c8, d11), (a4, b13, c8, d12), (a4, b13, c9, d1), (a4, b13, c9, d2), (a4, b13, c9, d3), (a4, b13, c9, d4), (a4, b13, c9, d5), (a4, b13, c9, d6), (a4, b13, c9, d7), (a4, b13, c9, d8), (a4, b13, c9, d9), (a4, b13, c9, d10), (a4, b13, c9, d11), (a4, b13, c9, d12), (a4, b13, c10, d1), (a4, b13, c10, d2), (a4, b13, c10, d3), (a4, b13, c10, d4), (a4, b13, c10, d5), (a4, b13, c10, d6), (a4, b13, c10, d7), (a4, b13, c10, d8), (a4, b13, c10, d9), (a4, b13, c10, d10), (a4, b13, c10, d11), (a4, b13, c10, d12), (a4, b13, c11, d1), (a4, b13, c11, d2), (a4, b13, c11, d3), (a4, b13, c11, d4), (a4, b13, c11, d5), (a4, b13, c11, d6), (a4, b13, c11, d7), (a4, b13, c11, d8), (a4, b13, c11, d9), (a4, b13, c11, d10), (a4, b13, c11, d11), (a4, b13, c11, d12), (a4, b13, c12, d1), (a4, b13, c12, d2), (a4, b13, c12, d3), (a4, b13, c12, d4), (a4, b13, c12, d5), (a4, b13, c12, d6), (a4, b13, c12, d7), (a4, b13, c12, d8), (a4, b13, c12, d9), (a4, b13, c12, d10), (a4, b13, c12, d11), (a4, b13, c12, d12), (a4, b13, c13, d1), (a4, b13, c13, d2), (a4, b13, c13, d3), (a4, b13, c13, d4), (a4, b13, c13, d5), (a4, b13, c13, d6), (a4, b13, c13, d7), (a4, b13, c13, d8), (a4, b13, c13, d9), (a4, b13, c13, d10), (a4, b13, c13, d11), (a4, b13, c13, d12), (a4, b14, c1, d1), (a4, b14, c1, d2), (a4, b14, c1, d3), (a4, b14, c1, d4), (a4, b14, c1, d5), (a4, b14, c1, d6), (a4, b14, c1, d7), (a4, b14, c1, d8), (a4, b14, c1, d9), (a4, b14, c1, d10), (a4, b14, c1, d11), (a4, b14, c1, d12), (a4, b14, c2, d1), (a4, b14, c2, d2), (a4, b14, c2, d3), (a4, b14, c2, d4), (a4, b14, c2, d5), (a4, b14, c2, d6), (a4, b14, c2, d7), (a4, b14, c2, d8), (a4, b14, c2, d9), (a4, b14, c2, d10), (a4, b14, c2, d11), (a4, b14, c2, d12), (a4, b14, c3, d1), (a4, b14, c3, d2), (a4, b14, c3, d3), (a4, b14, c3, d4), (a4, b14, c3, d5), (a4, b14, c3, d6), (a4, b14, c3, d7), (a4, b14, c3, d8), (a4, b14, c3, d9), (a4, b14, c3, d10), (a4, b14, c3, d11), (a4, b14, c3, d12), (a4, b14, c4, d1), (a4, b14, c4, d2), (a4, b14, c4, d3), (a4, b14, c4, d4), (a4, b14, c4, d5), (a4, b14, c4, d6), (a4, b14, c4, d7), (a4, b14, c4, d8), (a4, b14, c4, d9), (a4, b14, c4, d10), (a4, b14, c4, d11), (a4, b14, c4, d12), (a4, b14, c5, d1), (a4, b14, c5, d2), (a4, b14, c5, d3), (a4, b14, c5, d4), (a4, b14, c5, d5), (a4, b14, c5, d6), (a4, b14, c5, d7), (a4, b14, c5, d8), (a4, b14, c5, d9), (a4, b14, c5, d10), (a4, b14, c5, d11), (a4, b14, c5, d12), (a4, b14, c6, d1), (a4, b14, c6, d2), (a4, b14, c6, d3), (a4, b14, c6, d4), (a4, b14, c6, d5), (a4, b14, c6, d6), (a4, b14, c6, d7), (a4, b14, c6, d8), (a4, b14, c6, d9), (a4, b14, c6, d10), (a4, b14, c6, d11), (a4, b14, c6, d12), (a4, b14, c7, d1), (a4, b14, c7, d2), (a4, b14, c7, d3), (a4, b14, c7, d4), (a4, b14, c7, d5), (a4, b14, c7, d6), (a4, b14, c7, d7), (a4, b14, c7, d8), (a4, b14, c7, d9), (a4, b14, c7, d10), (a4, b14, c7, d11), (a4, b14, c7, d12), (a4, b14, c8, d1), (a4, b14, c8, d2), (a4, b14, c8, d3), (a4, b14, c8, d4), (a4, b14, c8, d5), (a4, b14, c8, d6), (a4, b14, c8, d7), (a4, b14, c8, d8), (a4, b14, c8, d9), (a4, b14, c8, d10), (a4,

b14, c8, d11), (a4, b14, c8, d12), (a4, b14, c9, d1), (a4, b14, c9, d2), (a4, b14, c9, d3), (a4, b14, c9, d4), (a4, b14, c9, d5), (a4, b14, c9, d6), (a4, b14, c9, d7), (a4, b14, c9, d8), (a4, b14, c9, d9), (a4, b14, c9, d10), (a4, b14, c9, d11), (a4, b14, c9, d12), (a4, b14, c10, d1), (a4, b14, c10, d2), (a4, b14, c10, d3), (a4, b14, c10, d4), (a4, b14, c10, d5), (a4, b14, c10, d6), (a4, b14, c10, d7), (a4, b14, c10, d8), (a4, b14, c10, d9), (a4, b14, c10, d10), (a4, b14, c10, d11), (a4, b14, c10, d12), (a4, b14, c11, d1), (a4, b14, c11, d2), (a4, b14, c11, d3), (a4, b14, c11, d4), (a4, b14, c11, d5), (a4, b14, c11, d6), (a4, b14, c11, d7), (a4, b14, c11, d8), (a4, b14, c11, d9), (a4, b14, c11, d10), (a4, b14, c11, d11), (a4, b14, c11, d12), (a4, b14, c12, d1), (a4, b14, c12, d2), (a4, b14, c12, d3), (a4, b14, c12, d4), (a4, b14, c12, d5), (a4, b14, c12, d6), (a4, b14, c12, d7), (a4, b14, c12, d8), (a4, b14, c12, d9), (a4, b14, c12, d10), (a4, b14, c12, d11), (a4, b14, c12, d12), (a4, b14, c13, d1), (a4, b14, c13, d2), (a4, b14, c13, d3), (a4, b14, c13, d4), (a4, b14, c13, d5), (a4, b14, c13, d6), (a4, b14, c13, d7), (a4, b14, c13, d8), (a4, b14, c13, d9), (a4, b14, c13, d10), (a4, b14, c13, d11), (a4, b14, c13, d12), (a4, b15, c1, d1), (a4, b15, c1, d2), (a4, b15, c1, d3), (a4, b15, c1, d4), (a4, b15, c1, d5), (a4, b15, c1, d6), (a4, b15, c1, d7), (a4, b15, c1, d8), (a4, b15, c1, d9), (a4, b15, c1, d10), (a4, b15, c1, d11), (a4, b15, c1, d12), (a4, b15, c2, d1), (a4, b15, c2, d2), (a4, b15, c2, d3), (a4, b15, c2, d4), (a4, b15, c2, d5), (a4, b15, c2, d6), (a4, b15, c2, d7), (a4, b15, c2, d8), (a4, b15, c2, d9), (a4, b15, c2, d10), (a4, b15, c2, d11), (a4, b15, c2, d12), (a4, b15, c3, d1), (a4, b15, c3, d2), (a4, b15, c3, d3), (a4, b15, c3, d4), (a4, b15, c3, d5), (a4, b15, c3, d6), (a4, b15, c3, d7), (a4, b15, c3, d8), (a4, b15, c3, d9), (a4, b15, c3, d10), (a4, b15, c3, d11), (a4, b15, c3, d12), (a4, b15, c4, d1), (a4, b15, c4, d2), (a4, b15, c4, d3), (a4, b15, c4, d4), (a4, b15, c4, d5), (a4, b15, c4, d6), (a4, b15, c4, d7), (a4, b15, c4, d8), (a4, b15, c4, d9), (a4, b15, c4, d10), (a4, b15, c4, d11), (a4, b15, c4, d12), (a4, b15, c5, d1), (a4, b15, c5, d2), (a4, b15, c5, d3), (a4, b15, c5, d4), (a4, b15, c5, d5), (a4, b15, c5, d6), (a4, b15, c5, d7), (a4, b15, c5, d8), (a4, b15, c5, d9), (a4, b15, c5, d10), (a4, b15, c5, d11), (a4, b15, c5, d12), (a4, b15, c6, d1), (a4, b15, c6, d2), (a4, b15, c6, d3), (a4, b15, c6, d4), (a4, b15, c6, d5), (a4, b15, c6, d6), (a4, b15, c6, d7), (a4, b15, c6, d8), (a4, b15, c6, d9), (a4, b15, c6, d10), (a4, b15, c6, d11), (a4, b15, c6, d12), (a4, b15, c7, d1), (a4, b15, c7, d2), (a4, b15, c7, d3), (a4, b15, c7, d4), (a4, b15, c7, d5), (a4, b15, c7, d6), (a4, b15, c7, d7), (a4, b15, c7, d8), (a4, b15, c7, d9), (a4, b15, c7, d10), (a4, b15, c7, d11), (a4, b15, c7, d12), (a4, b15, c8, d1), (a4, b15, c8, d2), (a4, b15, c8, d3), (a4, b15, c8, d4), (a4, b15, c8, d5), (a4, b15, c8, d6), (a4, b15, c8, d7), (a4, b15, c8, d8), (a4, b15, c8, d9), (a4, b15, c8, d10), (a4, b15, c8, d11), (a4, b15, c8, d12), (a4, b15, c9, d1), (a4, b15, c9, d2), (a4, b15, c9, d3), (a4, b15, c9, d4), (a4, b15, c9, d5), (a4, b15, c9, d6), (a4, b15, c9, d7), (a4, b15, c9, d8), (a4, b15, c9, d9), (a4, b15, c9, d10), (a4, b15, c9, d11), (a4, b15, c9, d12), (a4, b15, c10, d1), (a4, b15, c10, d2), (a4, b15, c10, d3), (a4, b15, c10, d4), (a4, b15, c10, d5), (a4, b15, c10, d6), (a4, b15, c10, d7), (a4, b15, c10, d8), (a4, b15, c10, d9), (a4, b15, c10, d10), (a4, b15, c10, d11), (a4, b15, c10, d12), (a4, b15, c11, d1), (a4, b15, c11, d2), (a4, b15, c11, d3), (a4, b15, c11, d4), (a4, b15, c11, d5), (a4, b15, c11, d6), (a4, b15, c11, d7), (a4, b15, c11, d8), (a4, b15, c11, d9), (a4, b15, c11, d10), (a4, b15, c11, d11), (a4, b15, c11, d12), (a4, b15, c12, d1), (a4, b15, c12, d2), (a4, b15, c12, d3), (a4, b15, c12, d4), (a4, b15, c12, d5), (a4, b15, c12, d6), (a4, b15, c12, d7), (a4, b15, c12, d8), (a4, b15, c12, d9), (a4, b15, c12, d10), (a4, b15, c12, d11), (a4, b15, c12, d12), (a4, b15, c13, d1), (a4, b15, c13, d2), (a4, b15, c13, d3), (a4, b15, c13, d4), (a4, b15, c13, d5), (a4, b15, c13, d6), (a4, b15, c13, d7), (a4, b15, c13, d8), (a4, b15, c13, d9), (a4, b15, c13, d10), (a4, b15, c13, d11), (a4, b15, c13, d12), (a5, b1, c1, d1), (a5, b1, c1, d2), (a5, b1, c1, d3), (a5, b1, c1, d4), (a5, b1, c1, d5), (a5, b1, c1, d6), (a5, b1, c1, d7), (a5, b1, c1, d8), (a5, b1, c1, d9), (a5, b1, c1, d10), (a5, b1, c1, d11), (a5, b1, c1, d12), (a5, b1, c2, d1), (a5, b1, c2, d2), (a5, b1, c2, d3), (a5, b1, c2, d4), (a5, b1, c2, d5), (a5, b1, c2, d6), (a5, b1, c2, d7), (a5, b1, c2, d8), (a5, b1, c2, d9), (a5, b1, c2, d10), (a5, b1, c2, d11), (a5, b1, c2, d12), (a5, b1, c3, d1), (a5, b1, c3, d2), (a5, b1, c3, d3), (a5, b1, c3, d4), (a5, b1, c3, d5), (a5, b1, c3, d6), (a5, b1, c3, d7), (a5, b1, c3, d8), (a5, b1, c3, d9), (a5, b1, c3, d10), (a5, b1, c3, d11), (a5, b1, c3, d12), (a5, b1, c4, d1), (a5, b1, c4, d2), (a5, b1, c4, d3), (a5, b1, c4, d4), (a5, b1, c4, d5), (a5, b1, c4, d6), (a5, b1, c4, d7), (a5, b1, c4, d8), (a5, b1, c4, d9), (a5, b1, c4, d10), (a5, b1, c4, d11), (a5, b1, c4, d12), (a5, b1, c5, d1), (a5, b1, c5, d2), (a5, b1, c5, d3), (a5, b1, c5, d4), (a5, b1, c5, d5), (a5, b1, c5, d6), (a5, b1, c5, d7), (a5, b1, c5, d8), (a5, b1, c5, d9), (a5, b1, c5, d10), (a5, b1, c5, d11), (a5, b1, c5, d12), (a5, b1, c6, d1), (a5, b1, c6, d2), (a5, b1, c6, d3), (a5, b1, c6, d4), (a5, b1, c6, d5), (a5, b1, c6, d6), (a5, b1, c6, d7), (a5, b1, c6, d8), (a5, b1, c6, d9), (a5, b1, c6, d10), (a5, b1, c6, d11), (a5, b1, c6, d12), (a5, b1, c7, d1), (a5, b1, c7, d2), (a5, b1, c7, d3), (a5, b1, c7, d4), (a5, b1, c7, d5), (a5, b1, c7, d6), (a5, b1, c7, d7), (a5, b1, c7, d8), (a5, b1, c7, d9), (a5, b1, c7, d10), (a5, b1, c7, d11), (a5, b1, c7, d12), (a5, b1, c8, d1), (a5, b1, c8, d2), (a5, b1, c8, d3), (a5, b1, c8, d4), (a5, b1, c8, d5), (a5, b1, c8, d6), (a5, b1, c8, d7), (a5, b1, c8, d8), (a5, b1, c8, d9), (a5, b1, c8, d10), (a5, b1, c8, d11), (a5, b1, c8, d12), (a5, b1, c9, d1), (a5, b1, c9, d2), (a5, b1, c9, d3), (a5, b1, c9, d4), (a5, b1, c9, d5), (a5, b1, c9, d6), (a5, b1, c9, d7), (a5, b1, c9, d8), (a5, b1, c9, d9), (a5, b1, c9, d10), (a5, b1, c9, d11), (a5, b1, c9, d12), (a5, b1, c10, d1), (a5, b1, c10, d2), (a5, b1, c10, d3), (a5, b1, c10, d4), (a5, b1, c10, d5), (a5, b1, c10, d6), (a5, b1, c10, d7), (a5, b1, c10, d8), (a5, b1, c10, d9), (a5, b1, c10, d10), (a5, b1, c10, d11), (a5, b1, c10, d12), (a5, b1, c11, d1), (a5, b1, c11, d2), (a5, b1, c11, d3), (a5, b1, c11, d4), (a5, b1, c11, d5), (a5, b1, c11, d6), (a5, b1, c11, d7), (a5, b1, c11, d8), (a5, b1, c11, d9), (a5, b1, c11, d10), (a5, b1, c11, d11), (a5, b1, c11, d12), (a5, b1, c12, d1), (a5, b1, c12, d2), (a5, b1, c12, d3), (a5, b1, c12, d4), (a5, b1, c12, d5), (a5, b1, c12, d6), (a5, b1, c12, d7), (a5, b1, c12, d8), (a5, b1, c12, d9), (a5, b1, c12, d10), (a5, b1, c12, d11), (a5, b1, c12, d12), (a5, b1, c13, d1), (a5, b1, c13, d2), (a5, b1, c13, d3), (a5, b1, c13, d4), (a5, b1, c13, d5), (a5, b1, c13, d6), (a5, b1, c13, d7), (a5, b1, c13, d8), (a5, b1, c13, d9), (a5, b1, c13, d10), (a5, b1, c13, d11), (a5, b1, c13, d12), (a5, b2, c1, d1), (a5, b2, c1, d2), (a5, b2, c1, d3), (a5, b2, c1, d4), (a5, b2, c1, d5), (a5, b2, c1, d6), (a5, b2, c1, d7), (a5, b2, c1, d8), (a5, b2, c1, d9), (a5,

b2, c1, d10), (a5, b2, c1, d11), (a5, b2, c1, d12), (a5, b2, c2, d1), (a5, b2, c2, d2), (a5, b2, c2, d3), (a5, b2, c2, d4), (a5, b2, c2, d5), (a5, b2, c2, d6), (a5, b2, c2, d7), (a5, b2, c2, d8), (a5, b2, c2, d9), (a5, b2, c2, d10), (a5, b2, c2, d11), (a5, b2, c2, d12), (a5, b2, c3, d1), (a5, b2, c3, d2), (a5, b2, c3, d3), (a5, b2, c3, d4), (a5, b2, c3, d5), (a5, b2, c3, d6), (a5, b2, c3, d7), (a5, b2, c3, d8), (a5, b2, c3, d9), (a5, b2, c3, d10), (a5, b2, c3, d11), (a5, b2, c3, d12), (a5, b2, c4, d1), (a5, b2, c4, d2), (a5, b2, c4, d3), (a5, b2, c4, d4), (a5, b2, c4, d5), (a5, b2, c4, d6), (a5, b2, c4, d7), (a5, b2, c4, d8), (a5, b2, c4, d9), (a5, b2, c4, d10), (a5, b2, c4, d11), (a5, b2, c4, d12), (a5, b2, c5, d1), (a5, b2, c5, d2), (a5, b2, c5, d3), (a5, b2, c5, d4), (a5, b2, c5, d5), (a5, b2, c5, d6), (a5, b2, c5, d7), (a5, b2, c5, d8), (a5, b2, c5, d9), (a5, b2, c5, d10), (a5, b2, c5, d11), (a5, b2, c5, d12), (a5, b2, c6, d1), (a5, b2, c6, d2), (a5, b2, c6, d3), (a5, b2, c6, d4), (a5, b2, c6, d5), (a5, b2, c6, d6), (a5, b2, c6, d7), (a5, b2, c6, d8), (a5, b2, c6, d9), (a5, b2, c6, d10), (a5, b2, c6, d11), (a5, b2, c6, d12), (a5, b2, c7, d1), (a5, b2, c7, d2), (a5, b2, c7, d3), (a5, b2, c7, d4), (a5, b2, c7, d5), (a5, b2, c7, d6), (a5, b2, c7, d7), (a5, b2, c7, d8), (a5, b2, c7, d9), (a5, b2, c7, d10), (a5, b2, c7, d11), (a5, b2, c7, d12), (a5, b2, c8, d1), (a5, b2, c8, d2), (a5, b2, c8, d3), (a5, b2, c8, d4), (a5, b2, c8, d5), (a5, b2, c8, d6), (a5, b2, c8, d7), (a5, b2, c8, d8), (a5, b2, c8, d9), (a5, b2, c8, d10), (a5, b2, c8, d11), (a5, b2, c8, d12), (a5, b2, c9, d1), (a5, b2, c9, d2), (a5, b2, c9, d3), (a5, b2, c9, d4), (a5, b2, c9, d5), (a5, b2, c9, d6), (a5, b2, c9, d7), (a5, b2, c9, d8), (a5, b2, c9, d9), (a5, b2, c9, d10), (a5, b2, c9, d11), (a5, b2, c9, d12), (a5, b2, c10, d1), (a5, b2, c10, d2), (a5, b2, c10, d3), (a5, b2, c10, d4), (a5, b2, c10, d5), (a5, b2, c10, d6), (a5, b2, c10, d7), (a5, b2, c10, d8), (a5, b2, c10, d9), (a5, b2, c10, d10), (a5, b2, c10, d11), (a5, b2, c10, d12), (a5, b2, c11, d1), (a5, b2, c11, d2), (a5, b2, c11, d3), (a5, b2, c11, d4), (a5, b2, c11, d5), (a5, b2, c11, d6), (a5, b2, c11, d7), (a5, b2, c11, d8), (a5, b2, c11, d9), (a5, b2, c11, d10), (a5, b2, c11, d11), (a5, b2, c11, d12), (a5, b2, c12, d1), (a5, b2, c12, d2), (a5, b2, c12, d3), (a5, b2, c12, d4), (a5, b2, c12, d5), (a5, b2, c12, d6), (a5, b2, c12, d7), (a5, b2, c12, d8), (a5, b2, c12, d9), (a5, b2, c12, d10), (a5, b2, c12, d11), (a5, b2, c12, d12), (a5, b2, c13, d1), (a5, b2, c13, d2), (a5, b2, c13, d3), (a5, b2, c13, d4), (a5, b2, c13, d5), (a5, b2, c13, d6), (a5, b2, c13, d7), (a5, b2, c13, d8), (a5, b2, c13, d9), (a5, b2, c13, d10), (a5, b2, c13, d11), (a5, b2, c13, d12), (a5, b3, c1, d1), (a5, b3, c1, d2), (a5, b3, c1, d3), (a5, b3, c1, d4), (a5, b3, c1, d5), (a5, b3, c1, d6), (a5, b3, c1, d7), (a5, b3, c1, d8), (a5, b3, c1, d9), (a5, b3, c1, d10), (a5, b3, c1, d11), (a5, b3, c1, d12), (a5, b3, c2, d1), (a5, b3, c2, d2), (a5, b3, c2, d3), (a5, b3, c2, d4), (a5, b3, c2, d5), (a5, b3, c2, d6), (a5, b3, c2, d7), (a5, b3, c2, d8), (a5, b3, c2, d9), (a5, b3, c2, d10), (a5, b3, c2, d11), (a5, b3, c2, d12), (a5, b3, c3, d1), (a5, b3, c3, d2), (a5, b3, c3, d3), (a5, b3, c3, d4), (a5, b3, c3, d5), (a5, b3, c3, d6), (a5, b3, c3, d7), (a5, b3, c3, d8), (a5, b3, c3, d9), (a5, b3, c3, d10), (a5, b3, c3, d11), (a5, b3, c3, d12), (a5, b3, c4, d1), (a5, b3, c4, d2), (a5, b3, c4, d3), (a5, b3, c4, d4), (a5, b3, c4, d5), (a5, b3, c4, d6), (a5, b3, c4, d7), (a5, b3, c4, d8), (a5, b3, c4, d9), (a5, b3, c4, d10), (a5, b3, c4, d11), (a5, b3, c4, d12), (a5, b3, c5, d1), (a5, b3, c5, d2), (a5, b3, c5, d3), (a5, b3, c5, d4), (a5, b3, c5, d5), (a5, b3, c5, d6), (a5, b3, c5, d7), (a5, b3, c5, d8), (a5, b3, c5, d9), (a5, b3, c5, d10), (a5, b3, c5, d11), (a5, b3, c5, d12), (a5, b3, c6, d1), (a5, b3, c6, d2), (a5, b3, c6, d3), (a5, b3, c6, d4), (a5, b3, c6, d5), (a5, b3, c6, d6), (a5, b3, c6, d7), (a5, b3, c6, d8), (a5, b3, c6, d9), (a5, b3, c6, d10), (a5, b3, c6, d11), (a5, b3, c6, d12), (a5, b3, c7, d1), (a5, b3, c7, d2), (a5, b3, c7, d3), (a5, b3, c7, d4), (a5, b3, c7, d5), (a5, b3, c7, d6), (a5, b3, c7, d7), (a5, b3, c7, d8), (a5, b3, c7, d9), (a5, b3, c7, d10), (a5, b3, c7, d11), (a5, b3, c7, d12), (a5, b3, c8, d1), (a5, b3, c8, d2), (a5, b3, c8, d3), (a5, b3, c8, d4), (a5, b3, c8, d5), (a5, b3, c8, d6), (a5, b3, c8, d7), (a5, b3, c8, d8), (a5, b3, c8, d9), (a5, b3, c8, d10), (a5, b3, c8, d11), (a5, b3, c8, d12), (a5, b3, c9, d1), (a5, b3, c9, d2), (a5, b3, c9, d3), (a5, b3, c9, d4), (a5, b3, c9, d5), (a5, b3, c9, d6), (a5, b3, c9, d7), (a5, b3, c9, d8), (a5, b3, c9, d9), (a5, b3, c9, d10), (a5, b3, c9, d11), (a5, b3, c9, d12), (a5, b3, c10, d1), (a5, b3, c10, d2), (a5, b3, c10, d3), (a5, b3, c10, d4), (a5, b3, c10, d5), (a5, b3, c10, d6), (a5, b3, c10, d7), (a5, b3, c10, d8), (a5, b3, c10, d9), (a5, b3, c10, d10), (a5, b3, c10, d11), (a5, b3, c10, d12), (a5, b3, c11, d1), (a5, b3, c11, d2), (a5, b3, c11, d3), (a5, b3, c11, d4), (a5, b3, c11, d5), (a5, b3, c11, d6), (a5, b3, c11, d7), (a5, b3, c11, d8), (a5, b3, c11, d9), (a5, b3, c11, d10), (a5, b3, c11, d11), (a5, b3, c11, d12), (a5, b3, c12, d1), (a5, b3, c12, d2), (a5, b3, c12, d3), (a5, b3, c12, d4), (a5, b3, c12, d5), (a5, b3, c12, d6), (a5, b3, c12, d7), (a5, b3, c12, d8), (a5, b3, c12, d9), (a5, b3, c12, d10), (a5, b3, c12, d11), (a5, b3, c12, d12), (a5, b3, c13, d1), (a5, b3, c13, d2), (a5, b3, c13, d3), (a5, b3, c13, d4), (a5, b3, c13, d5), (a5, b3, c13, d6), (a5, b3, c13, d7), (a5, b3, c13, d8), (a5, b3, c13, d9), (a5, b3, c13, d10), (a5, b3, c13, d11), (a5, b3, c13, d12), (a5, b4, c1, d1), (a5, b4, c1, d2), (a5, b4, c1, d3), (a5, b4, c1, d4), (a5, b4, c1, d5), (a5, b4, c1, d6), (a5, b4, c1, d7), (a5, b4, c1, d8), (a5, b4, c1, d9), (a5, b4, c1, d10), (a5, b4, c1, d11), (a5, b4, c1, d12), (a5, b4, c2, d1), (a5, b4, c2, d2), (a5, b4, c2, d3), (a5, b4, c2, d4), (a5, b4, c2, d5), (a5, b4, c2, d6), (a5, b4, c2, d7), (a5, b4, c2, d8), (a5, b4, c2, d9), (a5, b4, c2, d10), (a5, b4, c2, d11), (a5, b4, c2, d12), (a5, b4, c3, d1), (a5, b4, c3, d2), (a5, b4, c3, d3), (a5, b4, c3, d4), (a5, b4, c3, d5), (a5, b4, c3, d6), (a5, b4, c3, d7), (a5, b4, c3, d8), (a5, b4, c3, d9), (a5, b4, c3, d10), (a5, b4, c3, d11), (a5, b4, c3, d12), (a5, b4, c4, d1), (a5, b4, c4, d2), (a5, b4, c4, d3), (a5, b4, c4, d4), (a5, b4, c4, d5), (a5, b4, c4, d6), (a5, b4, c4, d7), (a5, b4, c4, d8), (a5, b4, c4, d9), (a5, b4, c4, d10), (a5, b4, c4, d11), (a5, b4, c4, d12), (a5, b4, c5, d1), (a5, b4, c5, d2), (a5, b4, c5, d3), (a5, b4, c5, d4), (a5, b4, c5, d5), (a5, b4, c5, d6), (a5, b4, c5, d7), (a5, b4, c5, d8), (a5, b4, c5, d9), (a5, b4, c5, d10), (a5, b4, c5, d11), (a5, b4, c5, d12), (a5, b4, c6, d1), (a5, b4, c6, d2), (a5, b4, c6, d3), (a5, b4, c6, d4), (a5, b4, c6, d5), (a5, b4, c6, d6), (a5, b4, c6, d7), (a5, b4, c6, d8), (a5, b4, c6, d9), (a5, b4, c6, d10), (a5, b4, c6, d11), (a5, b4, c6, d12), (a5, b4, c7, d1), (a5, b4, c7, d2), (a5, b4, c7, d3), (a5, b4, c7, d4), (a5, b4, c7, d5), (a5, b4, c7, d6), (a5, b4, c7, d7), (a5, b4, c7, d8), (a5, b4, c7, d9), (a5, b4, c7, d10), (a5, b4, c7, d11), (a5, b4, c7, d12), (a5, b4, c8, d1), (a5, b4, c8, d2), (a5, b4, c8, d3), (a5, b4, c8, d4), (a5, b4, c8, d5), (a5, b4, c8, d6), (a5, b4, c8, d7), (a5, b4, c8, d8), (a5, b4, c8, d9), (a5, b4, c8, d10), (a5, b4, c8, d11), (a5, b4, c8, d12), (a5, b4, c9, d1), (a5, b4, c9, d2), (a5, b4, c9, d3), (a5, b4, c9,

d4), (a5, b4, c9, d5), (a5, b4, c9, d6), (a5, b4, c9, d7), (a5, b4, c9, d8), (a5, b4, c9, d9), (a5, b4, c9, d10), (a5, b4, c9, d11), (a5, b4, c9, d12), (a5, b4, c10, d1), (a5, b4, c10, d2), (a5, b4, c10, d3), (a5, b4, c10, d4), (a5, b4, c10, d5), (a5, b4, c10, d6), (a5, b4, c10, d7), (a5, b4, c10, d8), (a5, b4, c10, d9), (a5, b4, c10, d10), (a5, b4, c10, d11), (a5, b4, c10, d12), (a5, b4, c11, d1), (a5, b4, c11, d2), (a5, b4, c11, d3), (a5, b4, c11, d4), (a5, b4, c11, d5), (a5, b4, c11, d6), (a5, b4, c11, d7), (a5, b4, c11, d8), (a5, b4, c11, d9), (a5, b4, c11, d10), (a5, b4, c11, d11), (a5, b4, c11, d12), (a5, b4, c12, d1), (a5, b4, c12, d2), (a5, b4, c12, d3), (a5, b4, c12, d4), (a5, b4, c12, d5), (a5, b4, c12, d6), (a5, b4, c12, d7), (a5, b4, c12, d8), (a5, b4, c12, d9), (a5, b4, c12, d10), (a5, b4, c12, d11), (a5, b4, c12, d12), (a5, b4, c13, d1), (a5, b4, c13, d2), (a5, b4, c13, d3), (a5, b4, c13, d4), (a5, b4, c13, d5), (a5, b4, c13, d6), (a5, b4, c13, d7), (a5, b4, c13, d8), (a5, b4, c13, d9), (a5, b4, c13, d10), (a5, b4, c13, d11), (a5, b4, c13, d12), (a5, b5, c1, d1), (a5, b5, c1, d2), (a5, b5, c1, d3), (a5, b5, c1, d4), (a5, b5, c1, d5), (a5, b5, c1, d6), (a5, b5, c1, d7), (a5, b5, c1, d8), (a5, b5, c1, d9), (a5, b5, c1, d10), (a5, b5, c1, d11), (a5, b5, c1, d12), (a5, b5, c2, d1), (a5, b5, c2, d2), (a5, b5, c2, d3), (a5, b5, c2, d4), (a5, b5, c2, d5), (a5, b5, c2, d6), (a5, b5, c2, d7), (a5, b5, c2, d8), (a5, b5, c2, d9), (a5, b5, c2, d10), (a5, b5, c2, d11), (a5, b5, c2, d12), (a5, b5, c3, d1), (a5, b5, c3, d2), (a5, b5, c3, d3), (a5, b5, c3, d4), (a5, b5, c3, d5), (a5, b5, c3, d6), (a5, b5, c3, d7), (a5, b5, c3, d8), (a5, b5, c3, d9), (a5, b5, c3, d10), (a5, b5, c3, d11), (a5, b5, c3, d12), (a5, b5, c4, d1), (a5, b5, c4, d2), (a5, b5, c4, d3), (a5, b5, c4, d4), (a5, b5, c4, d5), (a5, b5, c4, d6), (a5, b5, c4, d7), (a5, b5, c4, d8), (a5, b5, c4, d9), (a5, b5, c4, d10), (a5, b5, c4, d11), (a5, b5, c4, d12), (a5, b5, c5, d1), (a5, b5, c5, d2), (a5, b5, c5, d3), (a5, b5, c5, d4), (a5, b5, c5, d5), (a5, b5, c5, d6), (a5, b5, c5, d7), (a5, b5, c5, d8), (a5, b5, c5, d9), (a5, b5, c5, d10), (a5, b5, c5, d11), (a5, b5, c5, d12), (a5, b5, c6, d1), (a5, b5, c6, d2), (a5, b5, c6, d3), (a5, b5, c6, d4), (a5, b5, c6, d5), (a5, b5, c6, d6), (a5, b5, c6, d7), (a5, b5, c6, d8), (a5, b5, c6, d9), (a5, b5, c6, d10), (a5, b5, c6, d11), (a5, b5, c6, d12), (a5, b5, c7, d1), (a5, b5, c7, d2), (a5, b5, c7, d3), (a5, b5, c7, d4), (a5, b5, c7, d5), (a5, b5, c7, d6), (a5, b5, c7, d7), (a5, b5, c7, d8), (a5, b5, c7, d9), (a5, b5, c7, d10), (a5, b5, c7, d11), (a5, b5, c7, d12), (a5, b5, c8, d1), (a5, b5, c8, d2), (a5, b5, c8, d3), (a5, b5, c8, d4), (a5, b5, c8, d5), (a5, b5, c8, d6), (a5, b5, c8, d7), (a5, b5, c8, d8), (a5, b5, c8, d9), (a5, b5, c8, d10), (a5, b5, c8, d11), (a5, b5, c8, d12), (a5, b5, c9, d1), (a5, b5, c9, d2), (a5, b5, c9, d3), (a5, b5, c9, d4), (a5, b5, c9, d5), (a5, b5, c9, d6), (a5, b5, c9, d7), (a5, b5, c9, d8), (a5, b5, c9, d9), (a5, b5, c9, d10), (a5, b5, c9, d11), (a5, b5, c9, d12), (a5, b5, c10, d1), (a5, b5, c10, d2), (a5, b5, c10, d3), (a5, b5, c10, d4), (a5, b5, c10, d5), (a5, b5, c10, d6), (a5, b5, c10, d7), (a5, b5, c10, d8), (a5, b5, c10, d9), (a5, b5, c10, d10), (a5, b5, c10, d11), (a5, b5, c10, d12), (a5, b5, c11, d1), (a5, b5, c11, d2), (a5, b5, c11, d3), (a5, b5, c11, d4), (a5, b5, c11, d5), (a5, b5, c11, d6), (a5, b5, c11, d7), (a5, b5, c11, d8), (a5, b5, c11, d9), (a5, b5, c11, d10), (a5, b5, c11, d11), (a5, b5, c11, d12), (a5, b5, c12, d1), (a5, b5, c12, d2), (a5, b5, c12, d3), (a5, b5, c12, d4), (a5, b5, c12, d5), (a5, b5, c12, d6), (a5, b5, c12, d7), (a5, b5, c12, d8), (a5, b5, c12, d9), (a5, b5, c12, d10), (a5, b5, c12, d11), (a5, b5, c12, d12), (a5, b5, c13, d1), (a5, b5, c13, d2), (a5, b5, c13, d3), (a5, b5, c13, d4), (a5, b5, c13, d5), (a5, b5, c13, d6), (a5, b5, c13, d7), (a5, b5, c13, d8), (a5, b5, c13, d9), (a5, b5, c13, d10), (a5, b5, c13, d11), (a5, b5, c13, d12), (a5, b6, c1, d1), (a5, b6, c1, d2), (a5, b6, c1, d3), (a5, b6, c1, d4), (a5, b6, c1, d5), (a5, b6, c1, d6), (a5, b6, c1, d7), (a5, b6, c1, d8), (a5, b6, c1, d9), (a5, b6, c1, d10), (a5, b6, c1, d11), (a5, b6, c1, d12), (a5, b6, c2, d1), (a5, b6, c2, d2), (a5, b6, c2, d3), (a5, b6, c2, d4), (a5, b6, c2, d5), (a5, b6, c2, d6), (a5, b6, c2, d7), (a5, b6, c2, d8), (a5, b6, c2, d9), (a5, b6, c2, d10), (a5, b6, c2, d11), (a5, b6, c2, d12), (a5, b6, c3, d1), (a5, b6, c3, d2), (a5, b6, c3, d3), (a5, b6, c3, d4), (a5, b6, c3, d5), (a5, b6, c3, d6), (a5, b6, c3, d7), (a5, b6, c3, d8), (a5, b6, c3, d9), (a5, b6, c3, d10), (a5, b6, c3, d11), (a5, b6, c3, d12), (a5, b6, c4, d1), (a5, b6, c4, d2), (a5, b6, c4, d3), (a5, b6, c4, d4), (a5, b6, c4, d5), (a5, b6, c4, d6), (a5, b6, c4, d7), (a5, b6, c4, d8), (a5, b6, c4, d9), (a5, b6, c4, d10), (a5, b6, c4, d11), (a5, b6, c4, d12), (a5, b6, c5, d1), (a5, b6, c5, d2), (a5, b6, c5, d3), (a5, b6, c5, d4), (a5, b6, c5, d5), (a5, b6, c5, d6), (a5, b6, c5, d7), (a5, b6, c5, d8), (a5, b6, c5, d9), (a5, b6, c5, d10), (a5, b6, c5, d11), (a5, b6, c5, d12), (a5, b6, c6, d1), (a5, b6, c6, d2), (a5, b6, c6, d3), (a5, b6, c6, d4), (a5, b6, c6, d5), (a5, b6, c6, d6), (a5, b6, c6, d7), (a5, b6, c6, d8), (a5, b6, c6, d9), (a5, b6, c6, d10), (a5, b6, c6, d11), (a5, b6, c6, d12), (a5, b6, c7, d1), (a5, b6, c7, d2), (a5, b6, c7, d3), (a5, b6, c7, d4), (a5, b6, c7, d5), (a5, b6, c7, d6), (a5, b6, c7, d7), (a5, b6, c7, d8), (a5, b6, c7, d9), (a5, b6, c7, d10), (a5, b6, c7, d11), (a5, b6, c7, d12), (a5, b6, c8, d1), (a5, b6, c8, d2), (a5, b6, c8, d3), (a5, b6, c8, d4), (a5, b6, c8, d5), (a5, b6, c8, d6), (a5, b6, c8, d7), (a5, b6, c8, d8), (a5, b6, c8, d9), (a5, b6, c8, d10), (a5, b6, c8, d11), (a5, b6, c8, d12), (a5, b6, c9, d1), (a5, b6, c9, d2), (a5, b6, c9, d3), (a5, b6, c9, d4), (a5, b6, c9, d5), (a5, b6, c9, d6), (a5, b6, c9, d7), (a5, b6, c9, d8), (a5, b6, c9, d9), (a5, b6, c9, d10), (a5, b6, c9, d11), (a5, b6, c9, d12), (a5, b6, c10, d1), (a5, b6, c10, d2), (a5, b6, c10, d3), (a5, b6, c10, d4), (a5, b6, c10, d5), (a5, b6, c10, d6), (a5, b6, c10, d7), (a5, b6, c10, d8), (a5, b6, c10, d9), (a5, b6, c10, d10), (a5, b6, c10, d11), (a5, b6, c10, d12), (a5, b6, c11, d1), (a5, b6, c11, d2), (a5, b6, c11, d3), (a5, b6, c11, d4), (a5, b6, c11, d5), (a5, b6, c11, d6), (a5, b6, c11, d7), (a5, b6, c11, d8), (a5, b6, c11, d9), (a5, b6, c11, d10), (a5, b6, c11, d11), (a5, b6, c11, d12), (a5, b6, c12, d1), (a5, b6, c12, d2), (a5, b6, c12, d3), (a5, b6, c12, d4), (a5, b6, c12, d5), (a5, b6, c12, d6), (a5, b6, c12, d7), (a5, b6, c12, d8), (a5, b6, c12, d9), (a5, b6, c12, d10), (a5, b6, c12, d11), (a5, b6, c12, d12), (a5, b6, c13, d1), (a5, b6, c13, d2), (a5, b6, c13, d3), (a5, b6, c13, d4), (a5, b6, c13, d5), (a5, b6, c13, d6), (a5, b6, c13, d7), (a5, b6, c13, d8), (a5, b6, c13, d9), (a5, b6, c13, d10), (a5, b6, c13, d11), (a5, b6, c13, d12), (a5, b7, c1, d1), (a5, b7, c1, d2), (a5, b7, c1, d3), (a5, b7, c1, d4), (a5, b7, c1, d5), (a5, b7, c1, d6), (a5, b7, c1, d7), (a5, b7, c1, d8), (a5, b7, c1, d9), (a5, b7, c1, d10), (a5, b7, c1, d11), (a5, b7, c1, d12), (a5, b7, c2, d1), (a5, b7, c2, d2), (a5, b7, c2, d3), (a5, b7, c2, d4), (a5, b7, c2, d5), (a5, b7, c2, d6), (a5, b7, c2, d7), (a5, b7, c2, d8), (a5, b7, c2, d9), (a5, b7, c2, d10), (a5, b7, c2, d11), (a5, b7, c2, d12), (a5, b7, c3, d1), (a5, b7, c3, d2), (a5, b7, c3, d3), (a5, b7, c3, d4), (a5, b7, c3, d5), (a5, b7, c3, d6), (a5,

b7, c3, d7), (a5, b7, c3, d8), (a5, b7, c3, d9), (a5, b7, c3, d10), (a5, b7, c3, d11), (a5, b7, c3, d12), (a5, b7, c4, d1), (a5, b7, c4, d2), (a5, b7, c4, d3), (a5, b7, c4, d4), (a5, b7, c4, d5), (a5, b7, c4, d6), (a5, b7, c4, d7), (a5, b7, c4, d8), (a5, b7, c4, d9), (a5, b7, c4, d10), (a5, b7, c4, d11), (a5, b7, c4, d12), (a5, b7, c5, d1), (a5, b7, c5, d2), (a5, b7, c5, d3), (a5, b7, c5, d4), (a5, b7, c5, d5), (a5, b7, c5, d6), (a5, b7, c5, d7), (a5, b7, c5, d8), (a5, b7, c5, d9), (a5, b7, c5, d10), (a5, b7, c5, d11), (a5, b7, c5, d12), (a5, b7, c6, d1), (a5, b7, c6, d2), (a5, b7, c6, d3), (a5, b7, c6, d4), (a5, b7, c6, d5), (a5, b7, c6, d6), (a5, b7, c6, d7), (a5, b7, c6, d8), (a5, b7, c6, d9), (a5, b7, c6, d10), (a5, b7, c6, d11), (a5, b7, c6, d12), (a5, b7, c7, d1), (a5, b7, c7, d2), (a5, b7, c7, d3), (a5, b7, c7, d4), (a5, b7, c7, d5), (a5, b7, c7, d6), (a5, b7, c7, d7), (a5, b7, c7, d8), (a5, b7, c7, d9), (a5, b7, c7, d10), (a5, b7, c7, d11), (a5, b7, c7, d12), (a5, b7, c8, d1), (a5, b7, c8, d2), (a5, b7, c8, d3), (a5, b7, c8, d4), (a5, b7, c8, d5), (a5, b7, c8, d6), (a5, b7, c8, d7), (a5, b7, c8, d8), (a5, b7, c8, d9), (a5, b7, c8, d10), (a5, b7, c8, d11), (a5, b7, c8, d12), (a5, b7, c9, d1), (a5, b7, c9, d2), (a5, b7, c9, d3), (a5, b7, c9, d4), (a5, b7, c9, d5), (a5, b7, c9, d6), (a5, b7, c9, d7), (a5, b7, c9, d8), (a5, b7, c9, d9), (a5, b7, c9, d10), (a5, b7, c9, d11), (a5, b7, c9, d12), (a5, b7, c10, d1), (a5, b7, c10, d2), (a5, b7, c10, d3), (a5, b7, c10, d4), (a5, b7, c10, d5), (a5, b7, c10, d6), (a5, b7, c10, d7), (a5, b7, c10, d8), (a5, b7, c10, d9), (a5, b7, c10, d10), (a5, b7, c10, d11), (a5, b7, c10, d12), (a5, b7, c11, d1), (a5, b7, c11, d2), (a5, b7, c11, d3), (a5, b7, c11, d4), (a5, b7, c11, d5), (a5, b7, c11, d6), (a5, b7, c11, d7), (a5, b7, c11, d8), (a5, b7, c11, d9), (a5, b7, c11, d10), (a5, b7, c11, d11), (a5, b7, c11, d12), (a5, b7, c12, d1), (a5, b7, c12, d2), (a5, b7, c12, d3), (a5, b7, c12, d4), (a5, b7, c12, d5), (a5, b7, c12, d6), (a5, b7, c12, d7), (a5, b7, c12, d8), (a5, b7, c12, d9), (a5, b7, c12, d10), (a5, b7, c12, d11), (a5, b7, c12, d12), (a5, b7, c13, d1), (a5, b7, c13, d2), (a5, b7, c13, d3), (a5, b7, c13, d4), (a5, b7, c13, d5), (a5, b7, c13, d6), (a5, b7, c13, d7), (a5, b7, c13, d8), (a5, b7, c13, d9), (a5, b7, c13, d10), (a5, b7, c13, d11), (a5, b7, c13, d12), (a5, b8, c1, d1), (a5, b8, c1, d2), (a5, b8, c1, d3), (a5, b8, c1, d4), (a5, b8, c1, d5), (a5, b8, c1, d6), (a5, b8, c1, d7), (a5, b8, c1, d8), (a5, b8, c1, d9), (a5, b8, c1, d10), (a5, b8, c1, d11), (a5, b8, c1, d12), (a5, b8, c2, d1), (a5, b8, c2, d2), (a5, b8, c2, d3), (a5, b8, c2, d4), (a5, b8, c2, d5), (a5, b8, c2, d6), (a5, b8, c2, d7), (a5, b8, c2, d8), (a5, b8, c2, d9), (a5, b8, c2, d10), (a5, b8, c2, d11), (a5, b8, c2, d12), (a5, b8, c3, d1), (a5, b8, c3, d2), (a5, b8, c3, d3), (a5, b8, c3, d4), (a5, b8, c3, d5), (a5, b8, c3, d6), (a5, b8, c3, d7), (a5, b8, c3, d8), (a5, b8, c3, d9), (a5, b8, c3, d10), (a5, b8, c3, d11), (a5, b8, c3, d12), (a5, b8, c4, d1), (a5, b8, c4, d2), (a5, b8, c4, d3), (a5, b8, c4, d4), (a5, b8, c4, d5), (a5, b8, c4, d6), (a5, b8, c4, d7), (a5, b8, c4, d8), (a5, b8, c4, d9), (a5, b8, c4, d10), (a5, b8, c4, d11), (a5, b8, c4, d12), (a5, b8, c5, d1), (a5, b8, c5, d2), (a5, b8, c5, d3), (a5, b8, c5, d4), (a5, b8, c5, d5), (a5, b8, c5, d6), (a5, b8, c5, d7), (a5, b8, c5, d8), (a5, b8, c5, d9), (a5, b8, c5, d10), (a5, b8, c5, d11), (a5, b8, c5, d12), (a5, b8, c6, d1), (a5, b8, c6, d2), (a5, b8, c6, d3), (a5, b8, c6, d4), (a5, b8, c6, d5), (a5, b8, c6, d6), (a5, b8, c6, d7), (a5, b8, c6, d8), (a5, b8, c6, d9), (a5, b8, c6, d10), (a5, b8, c6, d11), (a5, b8, c6, d12), (a5, b8, c7, d1), (a5, b8, c7, d2), (a5, b8, c7, d3), (a5, b8, c7, d4), (a5, b8, c7, d5), (a5, b8, c7, d6), (a5, b8, c7, d7), (a5, b8, c7, d8), (a5, b8, c7, d9), (a5, b8, c7, d10), (a5, b8, c7, d11), (a5, b8, c7, d12), (a5, b8, c8, d1), (a5, b8, c8, d2), (a5, b8, c8, d3), (a5, b8, c8, d4), (a5, b8, c8, d5), (a5, b8, c8, d6), (a5, b8, c8, d7), (a5, b8, c8, d8), (a5, b8, c8, d9), (a5, b8, c8, d10), (a5, b8, c8, d11), (a5, b8, c8, d12), (a5, b8, c9, d1), (a5, b8, c9, d2), (a5, b8, c9, d3), (a5, b8, c9, d4), (a5, b8, c9, d5), (a5, b8, c9, d6), (a5, b8, c9, d7), (a5, b8, c9, d8), (a5, b8, c9, d9), (a5, b8, c9, d10), (a5, b8, c9, d11), (a5, b8, c9, d12), (a5, b8, c10, d1), (a5, b8, c10, d2), (a5, b8, c10, d3), (a5, b8, c10, d4), (a5, b8, c10, d5), (a5, b8, c10, d6), (a5, b8, c10, d7), (a5, b8, c10, d8), (a5, b8, c10, d9), (a5, b8, c10, d10), (a5, b8, c10, d11), (a5, b8, c10, d12), (a5, b8, c11, d1), (a5, b8, c11, d2), (a5, b8, c11, d3), (a5, b8, c11, d4), (a5, b8, c11, d5), (a5, b8, c11, d6), (a5, b8, c11, d7), (a5, b8, c11, d8), (a5, b8, c11, d9), (a5, b8, c11, d10), (a5, b8, c11, d11), (a5, b8, c11, d12), (a5, b8, c12, d1), (a5, b8, c12, d2), (a5, b8, c12, d3), (a5, b8, c12, d4), (a5, b8, c12, d5), (a5, b8, c12, d6), (a5, b8, c12, d7), (a5, b8, c12, d8), (a5, b8, c12, d9), (a5, b8, c12, d10), (a5, b8, c12, d11), (a5, b8, c12, d12), (a5, b8, c13, d1), (a5, b8, c13, d2), (a5, b8, c13, d3), (a5, b8, c13, d4), (a5, b8, c13, d5), (a5, b8, c13, d6), (a5, b8, c13, d7), (a5, b8, c13, d8), (a5, b8, c13, d9), (a5, b8, c13, d10), (a5, b8, c13, d11), (a5, b8, c13, d12), (a5, b9, c1, d1), (a5, b9, c1, d2), (a5, b9, c1, d3), (a5, b9, c1, d4), (a5, b9, c1, d5), (a5, b9, c1, d6), (a5, b9, c1, d7), (a5, b9, c1, d8), (a5, b9, c1, d9), (a5, b9, c1, d10), (a5, b9, c1, d11), (a5, b9, c1, d12), (a5, b9, c2, d1), (a5, b9, c2, d2), (a5, b9, c2, d3), (a5, b9, c2, d4), (a5, b9, c2, d5), (a5, b9, c2, d6), (a5, b9, c2, d7), (a5, b9, c2, d8), (a5, b9, c2, d9), (a5, b9, c2, d10), (a5, b9, c2, d11), (a5, b9, c2, d12), (a5, b9, c3, d1), (a5, b9, c3, d2), (a5, b9, c3, d3), (a5, b9, c3, d4), (a5, b9, c3, d5), (a5, b9, c3, d6), (a5, b9, c3, d7), (a5, b9, c3, d8), (a5, b9, c3, d9), (a5, b9, c3, d10), (a5, b9, c3, d11), (a5, b9, c3, d12), (a5, b9, c4, d1), (a5, b9, c4, d2), (a5, b9, c4, d3), (a5, b9, c4, d4), (a5, b9, c4, d5), (a5, b9, c4, d6), (a5, b9, c4, d7), (a5, b9, c4, d8), (a5, b9, c4, d9), (a5, b9, c4, d10), (a5, b9, c4, d11), (a5, b9, c4, d12), (a5, b9, c5, d1), (a5, b9, c5, d2), (a5, b9, c5, d3), (a5, b9, c5, d4), (a5, b9, c5, d5), (a5, b9, c5, d6), (a5, b9, c5, d7), (a5, b9, c5, d8), (a5, b9, c5, d9), (a5, b9, c5, d10), (a5, b9, c5, d11), (a5, b9, c5, d12), (a5, b9, c6, d1), (a5, b9, c6, d2), (a5, b9, c6, d3), (a5, b9, c6, d4), (a5, b9, c6, d5), (a5, b9, c6, d6), (a5, b9, c6, d7), (a5, b9, c6, d8), (a5, b9, c6, d9), (a5, b9, c6, d10), (a5, b9, c6, d11), (a5, b9, c6, d12), (a5, b9, c7, d1), (a5, b9, c7, d2), (a5, b9, c7, d3), (a5, b9, c7, d4), (a5, b9, c7, d5), (a5, b9, c7, d6), (a5, b9, c7, d7), (a5, b9, c7, d8), (a5, b9, c7, d9), (a5, b9, c7, d10), (a5, b9, c7, d11), (a5, b9, c7, d12), (a5, b9, c8, d1), (a5, b9, c8, d2), (a5, b9, c8, d3), (a5, b9, c8, d4), (a5, b9, c8, d5), (a5, b9, c8, d6), (a5, b9, c8, d7), (a5, b9, c8, d8), (a5, b9, c8, d9), (a5, b9, c8, d10), (a5, b9, c8, d11), (a5, b9, c8, d12), (a5, b9, c9, d1), (a5, b9, c9, d2), (a5, b9, c9, d3), (a5, b9, c9, d4), (a5, b9, c9, d5), (a5, b9, c9, d6), (a5, b9, c9, d7), (a5, b9, c9, d8), (a5, b9, c9, d9), (a5, b9, c9, d10), (a5, b9, c9, d11), (a5, b9, c9, d12), (a5, b9, c10, d1), (a5, b9, c10, d2), (a5, b9, c10, d3), (a5, b9, c10, d4), (a5, b9, c10, d5), (a5, b9, c10, d6), (a5, b9, c10, d7), (a5, b9, c10, d8), (a5, b9, c10, d9), (a5, b9, c10, d10), (a5, b9, c10, d11), (a5, b9, c10,

d12), (a5, b9, c11, d1), (a5, b9, c11, d2), (a5, b9, c11, d3), (a5, b9, c11, d4), (a5, b9, c11, d5), (a5, b9, c11, d6), (a5, b9, c11, d7), (a5, b9, c11, d8), (a5, b9, c11, d9), (a5, b9, c11, d10), (a5, b9, c11, d11), (a5, b9, c11, d12), (a5, b9, c12, d1), (a5, b9, c12, d2), (a5, b9, c12, d3), (a5, b9, c12, d4), (a5, b9, c12, d5), (a5, b9, c12, d6), (a5, b9, c12, d7), (a5, b9, c12, d8), (a5, b9, c12, d9), (a5, b9, c12, d10), (a5, b9, c12, d11), (a5, b9, c12, d12), (a5, b9, c13, d1), (a5, b9, c13, d2), (a5, b9, c13, d3), (a5, b9, c13, d4), (a5, b9, c13, d5), (a5, b9, c13, d6), (a5, b9, c13, d7), (a5, b9, c13, d8), (a5, b9, c13, d9), (a5, b9, c13, d10), (a5, b9, c13, d11), (a5, b9, c13, d12), (a5, b10, c1, d1), (a5, b10, c1, d2), (a5, b10, c1, d3), (a5, b10, c1, d4), (a5, b10, c1, d5), (a5, b10, c1, d6), (a5, b10, c1, d7), (a5, b10, c1, d8), (a5, b10, c1, d9), (a5, b10, c1, d10), (a5, b10, c1, d11), (a5, b10, c1, d12), (a5, b10, c2, d1), (a5, b10, c2, d2), (a5, b10, c2, d3), (a5, b10, c2, d4), (a5, b10, c2, d5), (a5, b10, c2, d6), (a5, b10, c2, d7), (a5, b10, c2, d8), (a5, b10, c2, d9), (a5, b10, c2, d10), (a5, b10, c2, d11), (a5, b10, c2, d12), (a5, b10, c3, d1), (a5, b10, c3, d2), (a5, b10, c3, d3), (a5, b10, c3, d4), (a5, b10, c3, d5), (a5, b10, c3, d6), (a5, b10, c3, d7), (a5, b10, c3, d8), (a5, b10, c3, d9), (a5, b10, c3, d10), (a5, b10, c3, d11), (a5, b10, c3, d12), (a5, b10, c4, d1), (a5, b10, c4, d2), (a5, b10, c4, d3), (a5, b10, c4, d4), (a5, b10, c4, d5), (a5, b10, c4, d6), (a5, b10, c4, d7), (a5, b10, c4, d8), (a5, b10, c4, d9), (a5, b10, c4, d10), (a5, b10, c4, d11), (a5, b10, c4, d12), (a5, b10, c5, d1), (a5, b10, c5, d2), (a5, b10, c5, d3), (a5, b10, c5, d4), (a5, b10, c5, d5), (a5, b10, c5, d6), (a5, b10, c5, d7), (a5, b10, c5, d8), (a5, b10, c5, d9), (a5, b10, c5, d10), (a5, b10, c5, d11), (a5, b10, c5, d12), (a5, b10, c6, d1), (a5, b10, c6, d2), (a5, b10, c6, d3), (a5, b10, c6, d4), (a5, b10, c6, d5), (a5, b10, c6, d6), (a5, b10, c6, d7), (a5, b10, c6, d8), (a5, b10, c6, d9), (a5, b10, c6, d10), (a5, b10, c6, d11), (a5, b10, c6, d12), (a5, b10, c7, d1), (a5, b10, c7, d2), (a5, b10, c7, d3), (a5, b10, c7, d4), (a5, b10, c7, d5), (a5, b10, c7, d6), (a5, b10, c7, d7), (a5, b10, c7, d8), (a5, b10, c7, d9), (a5, b10, c7, d10), (a5, b10, c7, d11), (a5, b10, c7, d12), (a5, b10, c8, d1), (a5, b10, c8, d2), (a5, b10, c8, d3), (a5, b10, c8, d4), (a5, b10, c8, d5), (a5, b10, c8, d6), (a5, b10, c8, d7), (a5, b10, c8, d8), (a5, b10, c8, d9), (a5, b10, c8, d10), (a5, b10, c8, d11), (a5, b10, c8, d12), (a5, b10, c9, d1), (a5, b10, c9, d2), (a5, b10, c9, d3), (a5, b10, c9, d4), (a5, b10, c9, d5), (a5, b10, c9, d6), (a5, b10, c9, d7), (a5, b10, c9, d8), (a5, b10, c9, d9), (a5, b10, c9, d10), (a5, b10, c9, d11), (a5, b10, c9, d12), (a5, b10, c10, d1), (a5, b10, c10, d2), (a5, b10, c10, d3), (a5, b10, c10, d4), (a5, b10, c10, d5), (a5, b10, c10, d6), (a5, b10, c10, d7), (a5, b10, c10, d8), (a5, b10, c10, d9), (a5, b10, c10, d10), (a5, b10, c10, d11), (a5, b10, c10, d12), (a5, b10, c11, d1), (a5, b10, c11, d2), (a5, b10, c11, d3), (a5, b10, c11, d4), (a5, b10, c11, d5), (a5, b10, c11, d6), (a5, b10, c11, d7), (a5, b10, c11, d8), (a5, b10, c11, d9), (a5, b10, c11, d10), (a5, b10, c11, d11), (a5, b10, c11, d12), (a5, b10, c12, d1), (a5, b10, c12, d2), (a5, b10, c12, d3), (a5, b10, c12, d4), (a5, b10, c12, d5), (a5, b10, c12, d6), (a5, b10, c12, d7), (a5, b10, c12, d8), (a5, b10, c12, d9), (a5, b10, c12, d10), (a5, b10, c12, d11), (a5, b10, c12, d12), (a5, b10, c13, d1), (a5, b10, c13, d2), (a5, b10, c13, d3), (a5, b10, c13, d4), (a5, b10, c13, d5), (a5, b10, c13, d6), (a5, b10, c13, d7), (a5, b10, c13, d8), (a5, b10, c13, d9), (a5, b10, c13, d10), (a5, b10, c13, d11), (a5, b10, c13, d12), (a5, b11, c1, d1), (a5, b11, c1, d2), (a5, b11, c1, d3), (a5, b11, c1, d4), (a5, b11, c1, d5), (a5, b11, c1, d6), (a5, b11, c1, d7), (a5, b11, c1, d8), (a5, b11, c1, d9), (a5, b11, c1, d10), (a5, b11, c1, d11), (a5, b11, c1, d12), (a5, b11, c2, d1), (a5, b11, c2, d2), (a5, b11, c2, d3), (a5, b11, c2, d4), (a5, b11, c2, d5), (a5, b11, c2, d6), (a5, b11, c2, d7), (a5, b11, c2, d8), (a5, b11, c2, d9), (a5, b11, c2, d10), (a5, b11, c2, d11), (a5, b11, c2, d12), (a5, b11, c3, d1), (a5, b11, c3, d2), (a5, b11, c3, d3), (a5, b11, c3, d4), (a5, b11, c3, d5), (a5, b11, c3, d6), (a5, b11, c3, d7), (a5, b11, c3, d8), (a5, b11, c3, d9), (a5, b11, c3, d10), (a5, b11, c3, d11), (a5, b11, c3, d12), (a5, b11, c4, d1), (a5, b11, c4, d2), (a5, b11, c4, d3), (a5, b11, c4, d4), (a5, b11, c4, d5), (a5, b11, c4, d6), (a5, b11, c4, d7), (a5, b11, c4, d8), (a5, b11, c4, d9), (a5, b11, c4, d10), (a5, b11, c4, d11), (a5, b11, c4, d12), (a5, b11, c5, d1), (a5, b11, c5, d2), (a5, b11, c5, d3), (a5, b11, c5, d4), (a5, b11, c5, d5), (a5, b11, c5, d6), (a5, b11, c5, d7), (a5, b11, c5, d8), (a5, b11, c5, d9), (a5, b11, c5, d10), (a5, b11, c5, d11), (a5, b11, c5, d12), (a5, b11, c6, d1), (a5, b11, c6, d2), (a5, b11, c6, d3), (a5, b11, c6, d4), (a5, b11, c6, d5), (a5, b11, c6, d6), (a5, b11, c6, d7), (a5, b11, c6, d8), (a5, b11, c6, d9), (a5, b11, c6, d10), (a5, b11, c6, d11), (a5, b11, c6, d12), (a5, b11, c7, d1), (a5, b11, c7, d2), (a5, b11, c7, d3), (a5, b11, c7, d4), (a5, b11, c7, d5), (a5, b11, c7, d6), (a5, b11, c7, d7), (a5, b11, c7, d8), (a5, b11, c7, d9), (a5, b11, c7, d10), (a5, b11, c7, d11), (a5, b11, c7, d12), (a5, b11, c8, d1), (a5, b11, c8, d2), (a5, b11, c8, d3), (a5, b11, c8, d4), (a5, b11, c8, d5), (a5, b11, c8, d6), (a5, b11, c8, d7), (a5, b11, c8, d8), (a5, b11, c8, d9), (a5, b11, c8, d10), (a5, b11, c8, d11), (a5, b11, c8, d12), (a5, b11, c9, d1), (a5, b11, c9, d2), (a5, b11, c9, d3), (a5, b11, c9, d4), (a5, b11, c9, d5), (a5, b11, c9, d6), (a5, b11, c9, d7), (a5, b11, c9, d8), (a5, b11, c9, d9), (a5, b11, c9, d10), (a5, b11, c9, d11), (a5, b11, c9, d12), (a5, b11, c10, d1), (a5, b11, c10, d2), (a5, b11, c10, d3), (a5, b11, c10, d4), (a5, b11, c10, d5), (a5, b11, c10, d6), (a5, b11, c10, d7), (a5, b11, c10, d8), (a5, b11, c10, d9), (a5, b11, c10, d10), (a5, b11, c10, d11), (a5, b11, c10, d12), (a5, b11, c11, d1), (a5, b11, c11, d2), (a5, b11, c11, d3), (a5, b11, c11, d4), (a5, b11, c11, d5), (a5, b11, c11, d6), (a5, b11, c11, d7), (a5, b11, c11, d8), (a5, b11, c11, d9), (a5, b11, c11, d10), (a5, b11, c11, d11), (a5, b11, c11, d12), (a5, b11, c12, d1), (a5, b11, c12, d2), (a5, b11, c12, d3), (a5, b11, c12, d4), (a5, b11, c12, d5), (a5, b11, c12, d6), (a5, b11, c12, d7), (a5, b11, c12, d8), (a5, b11, c12, d9), (a5, b11, c12, d10), (a5, b11, c12, d11), (a5, b11, c12, d12), (a5, b11, c13, d1), (a5, b11, c13, d2), (a5, b11, c13, d3), (a5, b11, c13, d4), (a5, b11, c13, d5), (a5, b11, c13, d6), (a5, b11, c13, d7), (a5, b11, c13, d8), (a5, b11, c13, d9), (a5, b11, c13, d10), (a5, b11, c13, d11), (a5, b11, c13, d12), (a5, b12, c1, d1), (a5, b12, c1, d2), (a5, b12, c1, d3), (a5, b12, c1, d4), (a5, b12, c1, d5), (a5, b12, c1, d6), (a5, b12, c1, d7), (a5, b12, c1, d8), (a5, b12, c1, d9), (a5, b12, c1, d10), (a5, b12, c1, d11), (a5, b12, c1, d12), (a5, b12, c2, d1), (a5, b12, c2, d2), (a5, b12, c2, d3), (a5, b12, c2, d4), (a5, b12, c2, d5), (a5, b12, c2, d6), (a5, b12, c2, d7), (a5, b12, c2, d8), (a5, b12, c2, d9), (a5, b12, c2, d10), (a5, b12, c2, d11), (a5, b12, c2, d12), (a5, b12, c3, d1), (a5, b12, c3, d2),

(a5, b12, c3, d3), (a5, b12, c3, d4), (a5, b12, c3, d5), (a5, b12, c3, d6), (a5, b12, c3, d7), (a5, b12, c3, d8), (a5, b12, c3, d9), (a5, b12, c3, d10), (a5, b12, c3, d11), (a5, b12, c3, d12), (a5, b12, c4, d1), (a5, b12, c4, d2), (a5, b12, c4, d3), (a5, b12, c4, d4), (a5, b12, c4, d5), (a5, b12, c4, d6), (a5, b12, c4, d7), (a5, b12, c4, d8), (a5, b12, c4, d9), (a5, b12, c4, d10), (a5, b12, c4, d11), (a5, b12, c4, d12), (a5, b12, c5, d1), (a5, b12, c5, d2), (a5, b12, c5, d3), (a5, b12, c5, d4), (a5, b12, c5, d5), (a5, b12, c5, d6), (a5, b12, c5, d7), (a5, b12, c5, d8), (a5, b12, c5, d9), (a5, b12, c5, d10), (a5, b12, c5, d11), (a5, b12, c5, d12), (a5, b12, c6, d1), (a5, b12, c6, d2), (a5, b12, c6, d3), (a5, b12, c6, d4), (a5, b12, c6, d5), (a5, b12, c6, d6), (a5, b12, c6, d7), (a5, b12, c6, d8), (a5, b12, c6, d9), (a5, b12, c6, d10), (a5, b12, c6, d11), (a5, b12, c6, d12), (a5, b12, c7, d1), (a5, b12, c7, d2), (a5, b12, c7, d3), (a5, b12, c7, d4), (a5, b12, c7, d5), (a5, b12, c7, d6), (a5, b12, c7, d7), (a5, b12, c7, d8), (a5, b12, c7, d9), (a5, b12, c7, d10), (a5, b12, c7, d11), (a5, b12, c7, d12), (a5, b12, c8, d1), (a5, b12, c8, d2), (a5, b12, c8, d3), (a5, b12, c8, d4), (a5, b12, c8, d5), (a5, b12, c8, d6), (a5, b12, c8, d7), (a5, b12, c8, d8), (a5, b12, c8, d9), (a5, b12, c8, d10), (a5, b12, c8, d11), (a5, b12, c8, d12), (a5, b12, c9, d1), (a5, b12, c9, d2), (a5, b12, c9, d3), (a5, b12, c9, d4), (a5, b12, c9, d5), (a5, b12, c9, d6), (a5, b12, c9, d7), (a5, b12, c9, d8), (a5, b12, c9, d9), (a5, b12, c9, d10), (a5, b12, c9, d11), (a5, b12, c9, d12), (a5, b12, c10, d1), (a5, b12, c10, d2), (a5, b12, c10, d3), (a5, b12, c10, d4), (a5, b12, c10, d5), (a5, b12, c10, d6), (a5, b12, c10, d7), (a5, b12, c10, d8), (a5, b12, c10, d9), (a5, b12, c10, d10), (a5, b12, c10, d11), (a5, b12, c10, d12), (a5, b12, c11, d1), (a5, b12, c11, d2), (a5, b12, c11, d3), (a5, b12, c11, d4), (a5, b12, c11, d5), (a5, b12, c11, d6), (a5, b12, c11, d7), (a5, b12, c11, d8), (a5, b12, c11, d9), (a5, b12, c11, d10), (a5, b12, c11, d11), (a5, b12, c11, d12), (a5, b12, c12, d1), (a5, b12, c12, d2), (a5, b12, c12, d3), (a5, b12, c12, d4), (a5, b12, c12, d5), (a5, b12, c12, d6), (a5, b12, c12, d7), (a5, b12, c12, d8), (a5, b12, c12, d9), (a5, b12, c12, d10), (a5, b12, c12, d11), (a5, b12, c12, d12), (a5, b12, c13, d1), (a5, b12, c13, d2), (a5, b12, c13, d3), (a5, b12, c13, d4), (a5, b12, c13, d5), (a5, b12, c13, d6), (a5, b12, c13, d7), (a5, b12, c13, d8), (a5, b12, c13, d9), (a5, b12, c13, d10), (a5, b12, c13, d11), (a5, b12, c13, d12), (a5, b13, c1, d1), (a5, b13, c1, d2), (a5, b13, c1, d3), (a5, b13, c1, d4), (a5, b13, c1, d5), (a5, b13, c1, d6), (a5, b13, c1, d7), (a5, b13, c1, d8), (a5, b13, c1, d9), (a5, b13, c1, d10), (a5, b13, c1, d11), (a5, b13, c1, d12), (a5, b13, c2, d1), (a5, b13, c2, d2), (a5, b13, c2, d3), (a5, b13, c2, d4), (a5, b13, c2, d5), (a5, b13, c2, d6), (a5, b13, c2, d7), (a5, b13, c2, d8), (a5, b13, c2, d9), (a5, b13, c2, d10), (a5, b13, c2, d11), (a5, b13, c2, d12), (a5, b13, c3, d1), (a5, b13, c3, d2), (a5, b13, c3, d3), (a5, b13, c3, d4), (a5, b13, c3, d5), (a5, b13, c3, d6), (a5, b13, c3, d7), (a5, b13, c3, d8), (a5, b13, c3, d9), (a5, b13, c3, d10), (a5, b13, c3, d11), (a5, b13, c3, d12), (a5, b13, c4, d1), (a5, b13, c4, d2), (a5, b13, c4, d3), (a5, b13, c4, d4), (a5, b13, c4, d5), (a5, b13, c4, d6), (a5, b13, c4, d7), (a5, b13, c4, d8), (a5, b13, c4, d9), (a5, b13, c4, d10), (a5, b13, c4, d11), (a5, b13, c4, d12), (a5, b13, c5, d1), (a5, b13, c5, d2), (a5, b13, c5, d3), (a5, b13, c5, d4), (a5, b13, c5, d5), (a5, b13, c5, d6), (a5, b13, c5, d7), (a5, b13, c5, d8), (a5, b13, c5, d9), (a5, b13, c5, d10), (a5, b13, c5, d11), (a5, b13, c5, d12), (a5, b13, c6, d1), (a5, b13, c6, d2), (a5, b13, c6, d3), (a5, b13, c6, d4), (a5, b13, c6, d5), (a5, b13, c6, d6), (a5, b13, c6, d7), (a5, b13, c6, d8), (a5, b13, c6, d9), (a5, b13, c6, d10), (a5, b13, c6, d11), (a5, b13, c6, d12), (a5, b13, c7, d1), (a5, b13, c7, d2), (a5, b13, c7, d3), (a5, b13, c7, d4), (a5, b13, c7, d5), (a5, b13, c7, d6), (a5, b13, c7, d7), (a5, b13, c7, d8), (a5, b13, c7, d9), (a5, b13, c7, d10), (a5, b13, c7, d11), (a5, b13, c7, d12), (a5, b13, c8, d1), (a5, b13, c8, d2), (a5, b13, c8, d3), (a5, b13, c8, d4), (a5, b13, c8, d5), (a5, b13, c8, d6), (a5, b13, c8, d7), (a5, b13, c8, d8), (a5, b13, c8, d9), (a5, b13, c8, d10), (a5, b13, c8, d11), (a5, b13, c8, d12), (a5, b13, c9, d1), (a5, b13, c9, d2), (a5, b13, c9, d3), (a5, b13, c9, d4), (a5, b13, c9, d5), (a5, b13, c9, d6), (a5, b13, c9, d7), (a5, b13, c9, d8), (a5, b13, c9, d9), (a5, b13, c9, d10), (a5, b13, c9, d11), (a5, b13, c9, d12), (a5, b13, c10, d1), (a5, b13, c10, d2), (a5, b13, c10, d3), (a5, b13, c10, d4), (a5, b13, c10, d5), (a5, b13, c10, d6), (a5, b13, c10, d7), (a5, b13, c10, d8), (a5, b13, c10, d9), (a5, b13, c10, d10), (a5, b13, c10, d11), (a5, b13, c10, d12), (a5, b13, c11, d1), (a5, b13, c11, d2), (a5, b13, c11, d3), (a5, b13, c11, d4), (a5, b13, c11, d5), (a5, b13, c11, d6), (a5, b13, c11, d7), (a5, b13, c11, d8), (a5, b13, c11, d9), (a5, b13, c11, d10), (a5, b13, c11, d11), (a5, b13, c11, d12), (a5, b13, c12, d1), (a5, b13, c12, d2), (a5, b13, c12, d3), (a5, b13, c12, d4), (a5, b13, c12, d5), (a5, b13, c12, d6), (a5, b13, c12, d7), (a5, b13, c12, d8), (a5, b13, c12, d9), (a5, b13, c12, d10), (a5, b13, c12, d11), (a5, b13, c12, d12), (a5, b13, c13, d1), (a5, b13, c13, d2), (a5, b13, c13, d3), (a5, b13, c13, d4), (a5, b13, c13, d5), (a5, b13, c13, d6), (a5, b13, c13, d7), (a5, b13, c13, d8), (a5, b13, c13, d9), (a5, b13, c13, d10), (a5, b13, c13, d11), (a5, b13, c13, d12), (a5, b14, c1, d1), (a5, b14, c1, d2), (a5, b14, c1, d3), (a5, b14, c1, d4), (a5, b14, c1, d5), (a5, b14, c1, d6), (a5, b14, c1, d7), (a5, b14, c1, d8), (a5, b14, c1, d9), (a5, b14, c1 d10), (a5, b14, c1, d11), (a5, b14, c1, d12), (a5, b14, c2, d1), (a5, b14, c2, d2), (a5, b14, c2, d3), (a5, b14, c2, d4), (a5, b14, c2, d5), (a5, b14, c2, d6), (a5, b14, c2, d7), (a5, b14, c2, d8), (a5, b14, c2, d9), (a5, b14, c2, d10), (a5, b14, c2, d11), (a5, b14, c2, d12), (a5, b14, c3, d1), (a5, b14, c3, d2), (a5, b14, c3, d3), (a5, b14, c3, d4), (a5, b14, c3, d5), (a5, b14, c3, d6), (a5, b14, c3, d7), (a5, b14, c3, d8), (a5, b14, c3, d9), (a5, b14, c3, d10), (a5, b14, c3, d11), (a5, b14, c3, d12), (a5, b14, c4, d1), (a5, b14, c4, d2), (a5, b14, c4, d3), (a5, b14, c4, d4), (a5, b14, c4, d5), (a5, b14, c4, d6), (a5, b14, c4, d7), (a5, b14, c4, d8), (a5, b14, c4, d9), (a5, b14, c4, d10), (a5, b14, c4, d11), (a5, b14, c4, d12), (a5, b14, c5, d1), (a5, b14, c5, d2), (a5, b14, c5, d3), (a5, b14, c5, d4), (a5, b14, c5, d5), (a5, b14, c5, d6), (a5, b14, c5, d7), (a5, b14, c5, d8), (a5, b14, c5, d9), (a5, b14, c5, d10), (a5, b14, c5, d11), (a5, b14, c5, d12), (a5, b14, c6, d1), (a5, b14, c6, d2), (a5, b14, c6, d3), (a5, b14, c6, d4), (a5, b14, c6, d5), (a5, b14, c6, d6), (a5, b14, c6, d7), (a5, b14, c6, d8), (a5, b14, c6, d9), (a5, b14, c6, d10), (a5, b14, c6, d11), (a5, b14, c6, d12), (a5, b14, c7, d1), (a5, b14, c7, d2), (a5, b14, c7, d3), (a5, b14, c7, d4), (a5, b14, c7, d5), (a5, b14, c7, d6), (a5, b14, c7, d7), (a5, b14, c7, d8), (a5, b14, c7, d9), (a5, b14, c7, d10), (a5, b14, c7, d11), (a5, b14, c7, d12), (a5, b14, c8, d1), (a5, b14, c8, d2), (a5, b14, c8, d3), (a5, b14, c8, d4), (a5, b14, c8, d5),

(a5, b14, c8, d6), (a5, b14, c8, d7), (a5, b14, c8, d8), (a5, b14, c8, d9), (a5, b14, c8, d10), (a5, b14, c8, d11), (a5, b14, c8, d12), (a5, b14, c9, d1), (a5, b14, c9, d2), (a5, b14, c9, d3), (a5, b14, c9, d4), (a5, b14, c9, d5), (a5, b14, c9, d6), (a5, b14, c9, d7), (a5, b14, c9, d8), (a5, b14, c9, d9), (a5, b14, c9, d10), (a5, b14, c9, d11), (a5, b14, c9, d12), (a5, b14, c10, d1), (a5, b14, c10, d2), (a5, b14, c10, d3), (a5, b14, c10, d4), (a5, b14, c10, d5), (a5, b14, c10, d6), (a5, b14, c10, d7), (a5, b14, c10, d8), (a5, b14, c10, d9), (a5, b14, c10, d10), (a5, b14, c10, d11), (a5, b14, c10, d12), (a5, b14, c11, d1), (a5, b14, c11, d2), (a5, b14, c11, d3), (a5, b14, c11, d4), (a5, b14, c11, d5), (a5, b14, c11, d6), (a5, b14, c11, d7), (a5, b14, c11, d8), (a5, b14, c11, d9), (a5, b14, c11, d10), (a5, b14, c11, d11), (a5, b14, c11, d12), (a5, b14, c12, d1), (a5, b14, c12, d2), (a5, b14, c12, d3), (a5, b14, c12, d4), (a5, b14, c12, d5), (a5, b14, c12, d6), (a5, b14, c12, d7), (a5, b14, c12, d8), (a5, b14, c12, d9), (a5, b14, c12, d10), (a5, b14, c12, d11), (a5, b14, c12, d12), (a5, b14, c13, d1), (a5, b14, c13, d2), (a5, b14, c13, d3), (a5, b14, c13, d4), (a5, b14, c13, d5), (a5, b14, c13, d6), (a5, b14, c13, d7), (a5, b14, c13, d8), (a5, b14, c13, d9), (a5, b14, c13, d10), (a5, b14, c13, d11), (a5, b14, c13, d12), (a5, b15, c1, d1), (a5, b15, c1, d2), (a5, b15, c1, d3), (a5, b15 c1, d4), (a5, b15, c1, d5), (a5, b15, c1, d6), (a5, b15, c1, d7), (a5, b15, c1, d8), (a5, b15, c1, d9), (a5, b15, c1, d10), (a5; b15, c1, d11), (a5, b15, c1, d12), (a5, b15, c2, d1), (a5, b15, c2, d2), (a5, b15, c2, d3), (a5, b15, c2, d4), (a5, b15, c2, d5), (a5, b15, c2, d6), (a5, b15, c2, d7), (a5, b15, c2, d8), (a5, b15, c2, d9), (a5, b15, c2, d10), (a5, b15, c2, d11), (a5, b15, c2, d12), (a5, b15, c3, d1), (a5, b15, c3, d2), (a5, b15, c3, d3), (a5, b15, c3, d4), (a5, b15, c3, d5), (a5, b15, c3, d6), (a5, b15, c3, d7), (a5, b15, c3, d8), (a5, b15, c3, d9), (a5, b15, c3, d10), (a5, b15, c3, d11), (a5, b15, c3, d12), (a5, b15, c4, d1), (a5, b15, c4, d2), (a5, b15, c4, d3), (a5, b15, c4, d4), (a5, b15, c4, d5), (a5, b15, c4, d6), (a5, b15, c4, d7), (a5, b15, c4, d8), (a5, b15, c4, d9), (a5, b15, c4, d10), (a5, b15, c4, d11), (a5, b15, c4, d12), (a5, b15, c5, d1), (a5, b15, c5, d2), (a5, b15, c5, d3), (a5, b15, c5, d4), (a5, b15, c5, d5), (a5, b15, c5, d6), (a5, b15, c5, d7), (a5, b15, c5, d8), (a5, b15, c5, d9), (a5, b15, c5, d10), (a5, b15, c5, d11), (a5, b15, c5, d12), (a5, b15, c6, d1), (a5, b15, c6, d2), (a5, b15, c6, d3), (a5, b15, c6, d4), (a5, b15, c6, d5), (a5, b15, c6, d6), (a5, b15, c6, d7), (a5, b15, c6, d8), (a5, b15, c6, d9), (a5, b15, c6, d10), (a5, b15, c6, d11), (a5, b15, c6, d12), (a5, b15, c7, d1), (a5, b15, c7, d2), (a5, b15, c7, d3), (a5, b15, c7, d4), (a5, b15, c7, d5), (a5, b15, c7, d6), (a5, b15, c7, d7), (a5, b15, c7, d8), (a5, b15, c7, d9), (a5, b15, c7, d10), (a5, b15, c7, d11), (a5, b15, c7, d12), (a5, b15, c8, d1), (a5, b15, c8, d2), (a5, b15, c8, d3), (a5, b15, c8, d4), (a5, b15 c8, d5), (a5, b15, c8, d6), (a5, b15, c8, d7), (a5, b15, c8, d8), (a5, b15, c8, d9), (a5, b15, c8, d10), (a5, b15, c8, d11), (a5, b15, c8, d12), (a5, b15, c9, d1), (a5, b15, c9, d2), (a5, b15, c9, d3), (a5, b15, c9, d4), (a5, b15, c9, d5), (a5, b15, c9, d6), (a5, b15, c9, d7), (a5, b15, c9, d8), (a5, b15, c9, d9), (a5, b15, c9, d10), (a5, b15, c9, d11), (a5, b15, c9, d12), (a5, b15, c10, d1), (a5, b15, c10, d2), (a5, b15, c10, d3), (a5, b15, c10, d4), (a5, b15, c10, d5), (a5, b15, c10, d6), (a5, b15, c10, d7), (a5, b15, c10, d8), (a5, b15, c10, d9), (a5, b15, c10, d10), (a5, b15, c10, d11), (a5, b15, c10, d12), (a5, b15, c11, d1), (a5, b15, c11, d2), (a5, b15, c11, d3), (a5, b15, c11, d4), (a5, b15, c11, d5), (a5, b15, c11, d6), (a5, b15, c11, d7), (a5, b15, c11, d8), (a5, b15, c11, d9), (a5, b15, c11, d10), (a5, b15, c11, d11), (a5, b15, c11, d12), (a5, b15, c12, d1), (a5, b15, c12, d2), (a5, b15, c12, d3), (a5, b15, c12, d4), (a5, b15, c12, d5), (a5, b15, c12, d6), (a5, b15, c12, d7), (a5, b15, c12, d8), (a5, b15, c12, d9), (a5, b15, c12, d10), (a5, b15, c12, d11), (a5, b15, c12, d12), (a5, b15, c13, d1), (a5, b15, c13, d2), (a5, b15, c13, d3), (a5, b15, c13, d4), (a5, b15, c13, d5), (a5, b15, c13, d6), (a5, b15, c13, d7), (a5, b15, c13, d8), (a5, b15, c13, d9), (a5, b15, c13, d10), (a5, b15, c13, d11), (a5, b15, c13, d12), (a6, b1, c1, d1), (a6, b1, c1, d2), (a6, b1, c1, d3), (a6, b1, c1, d4), (a6, b1, c1, d5), (a6, b1, c1, d6), (a6, b1, c1, d7), (a6, b1, c1 d8), (a6, b1, c1, d9), (a6, b1, c1, d10), (a6, b1, c1, d11), (a6, b1, c1, d12), (a6, b1, c2, d1), (a6, b1, c2, d2), (a6, b1, c2, d3), (a6, b1, c2, d4), (a6, b1, c2, d5), (a6, b1, c2, d6), (a6, b1, c2, d7), (a6, b1, c2, d8), (a6, b1, c2, d9), (a6, b1, c2, d10), (a6, b1, c2, d11), (a6, b1, c2, d12), (a6, b1, c3, d1), (a6, b1, c3, d2), (a6, b1, c3, d3), (a6, b1, c3, d4), (a6, b1, c3, d5), (a6, b1, c3, d6), (a6, b1, c3, d7), (a6, b1, c3, d8), (a6, b1, c3, d9), (a6, b1, c3, d10), (a6, b1, c3, d11), (a6, b1, c3, d12), (a6, b1, c4, d1), (a6, b1, c4, d2), (a6, b1, c4, d3), (a6, b1, c4, d4), (a6, b1, c4, d5), (a6, b1, c4, d6), (a6, b1, c4, d7), (a6, b1, c4, d8), (a6, b1, c4, d9), (a6, b1, c4, d10), (a6, b1, c4, d11), (a6, b1, c4, d12), (a6, b1, c5, d1), (a6, b1, c5, d2), (a6, b1, c5, d3), (a6, b1, c5, d4), (a6, b1, c5, d5), (a6, b1, c5, d6), (a6, b1, c5, d7), (a6, b1, c5, d8), (a6, b1, c5, d9), (a6, b1, c5, d10), (a6, b1, c5, d11), (a6, b1, c5, d12), (a6, b1, c6, d1), (a6, b1, c6, d2), (a6, b1, c6, d3), (a6, b1, c6, d4), (a6, b1, c6, d5), (a6, b1, c6, d6), (a6, b1, c6, d7), (a6, b1, c6, d8), (a6, b1, c6, d9), (a6, b1, c6, d10), (a6, b1, c6, d11)., (a6, b1, c6, d12), (a6, b1, c7, d1), (a6, b1, c7, d2), (a6, b1, c7, d3), (a6, b1, c7, d4), (a6, b1, c7, d5), (a6, b1, c7, d6), (a6, b1, c7, d7), (a6, b1, c7, d8), (a6, b1, c7, d9), (a6, b1, c7, d10), (a6, b1, c7, d11), (a6, b1, c7, d12), (a6, b1, c8, d1), (a6, b1, c8, d2), (a6, b1, c8, d3), (a6, b1, c8, d4), (a6, b1, c8, d5), (a6, b1, c8, d6), (a6, b1, c8, d7), (a6, b1, c8, d8), (a6, b1, c8, d9), (a6, b1, c8, d10), (a6, b1, c8, d11), (a6, b1, c8, d12), (a6, b1, c9, d1), (a6, b1, c9, d2), (a6, b1, c9, d3), (a6, b1, c9, d4), (a6, b1, c9, d5), (a6, b1, c9, d6), (a6, b1, c9, d7), (a6, b1, c9, d8), (a6, b1, c9, d9), (a6, b1, c9, d10) (a6, b1, c9, d11), (a6, b1, c9, d12), (a6, b1, c10, d1), (a6, b1, c10, d2), (a6, b1, c10, d3), (a6, b1, c10, d4), (a6, b1, c10, d5), (a6, b1, c10, d6), (a6, b1, c10, d7), (a6, b1, c10, d8), (a6, b1, c10, d9), (a6, b1, c10, d10), (a6, b1, c10, d11), (a6, b1, c10, d12), (a6, b1, c11, d1), (a6, b1, c11, d2), (a6, b1, c11, d3), (a6, b1, c11, d4), (a6, b1, c11, d5), (a6, b1, c11, d6), (a6, b1, c11, d7), (a6, b1, c11, d8), (a6, b1, c11, d9), (a6, b1, c11, d10), (a6, b1, c11, d11) (a6, b1, c1 d12), (a6, b1, c12, d1), (a6, b1, c12, d2), (a6, b1, c12, d3), (a6, b1, c12, d4), (a6, b1, c12, d5), (a6, b1, c12, d6), (a6, b1, c12, d7), (a6, b1, c12, d8), (a6, b1, c12, d9), (a6, b1, c12, d10), (a6, b1, c12, d11), (a6, b1, c12, d12), (a6, b1, c13, d1), (a6, b1, c13, d2), (a6, b1, c13, d3), (a6, b1, c13, d4), (a6, b1, c13, d5), (a6, b1, c13, d6), (a6, b1, c13, d7), (a6, b1, c13, d8), (a6, b1, c13, d9), (a6, b1, c13, d10), (a6, b1, c13, d11), (a6, b1, c13, d12), (a6, b2, c1, d1), (a6, b2, c1, d2), (a6, b2, c1, d3), (a6, b2,

c1, d4), (a6, b2, c1, d5), (a6, b2, c1, d6), (a6, b2, c1, d7), (a6, b2, c1, d8), (a6, b2, c1, d9), (a6, b2, c1, d10), (a6, b2, c1, d11), (a6, b2, c1, d12), (a6, b2, c2, d1), (a6, b2, c2, d2), (a6, b2, c2, d3), (a6, b2, c2, d4), (a6, b2, c2, d5), (a6, b2, c2, d6), (a6, b2, c2, d7), (a6, b2, c2, d8), (a6, b2, c2, d9), (a6, b2, c2, d10), (a6, b2, c2, d1l) (a6, b2, c2, d12), (a6, b2, c3, d1), (a6, b2, c3, d2), (a6, b2, c3, d3), (a6, b2, c3, d4), (a6, b2, c3, d5), (a6, b2, c3, d6), (a6, b2, c3, d7), (a6, b2, c3, d8), (a6, b2, c3, d9), (a6, b2, c3, d10), (a6, b2, c3, d11), (a6, b2, c3, d12), (a6, b2, c4, d1), (a6, b2, c4, d2), (a6, b2, c4, d3), (a6, b2, c4, d4), (a6, b2, c4, d5), (a6, b2, c4, d6), (a6, b2, c4, d7), (a6, b2, c4, d8), (a6, b2, c4, d9), (a6, b2, c4, d10), (a6, b2, c4, d11) (a6, b2, c4, d12), (a6, b2, c5, d1), (a6, b2, c5, d2), (a6, b2, c5, d3), (a6, b2, c5, d4), (a6, b2, c5, d5), (a6, b2, c5, d6), (a6, b2, c5, d7), (a6, b2, c5, d8), (a6, b2, c5, d9), (a6, b2, c5, d10), (a6, b2, c5, d11), (a6, b2, c5, d12), (a6, b2, c6, d1), (a6, b2, c6, d2), (a6, b2, c6, d3), (a6, b2, c6, d4), (a6, b2, c6, d5), (a6, b2, c6, d6), (a6, b2, c6, d7), (a6, b2, c6, d8), (a6, b2, c6, d9), (a6, b2, c6, d10), (a6, b2, c6, d11), (a6, b2, c6, d12), (a6, b2, c7, d1), (a6, b2, c7, d2), (a6, b2, c7, d3), (a6, b2, c7, d4), (a6, b2, c7, d5), (a6, b2, c7, d6), (a6, b2, c7, d7), (a6, b2, c7, d8), (a6, b2, c7, d9), (a6, b2, c7, d10), (a6, b2, c7, d11), (a6, b2, c7, d12), (a6, b2, c8; d1), (a6, b2, c8, d2), (a6, b2, c8, d3), (a6, b2, c8, d4), (a6, b2, c8, d5), (a6, b2, c8, d6), (a6, b2, c8, d7), (a6, b2, c8, d8), (a6, b2, c8, d9), (a6, b2, c8, d10), (a6, b2, c8, d11) (a6, b2, c8, d12), (a6, b2, c9, d1), (a6, b2, c9, d2), (a6, b2, c9, d3), (a6, b2, c9, d4), (a6, b2, c9, d5), (a6, b2, c9, d6), (a6, b2, c9, d7), (a6, b2, c9, d8), (a6, b2, c9, d9), (a6, b2, c9, d10), (a6, b2, c9, d11), (a6, b2, c9, d12), (a6, b2, c10, d1), (a6, b2, c10, d2), (a6, b2, c10, d3), (a6, b2, c10, d4), (a6, b2, c10, d5), (a6, b2, c10, d6), (a6, b2, c10, d7), (a6, b2, c10, d8), (a6, b2, c10, d9), (a6, b2, c10, d10), (a6, b2, c10, d11), (a6, b2, c10, d12), (a6, b2, c11, d1), (a6, b2, c11, d2), (a6, b2, c11, d3), (a6, b2, c11, d4), (a6, b2, c11, d5), (a6, b2, c11, d6), (a6, b2, c11 d7), (a6, b2, c11, d8), (a6, b2, c11 d9), (a6, b2, c11, d10), (a6, b2, c11 d11), (a6, b2, c11 d12), (a6, b2, c12, d1), (a6, b2, c12, d2), (a6, b2, c12, d3), (a6, b2, c12, d4), (a6, b2, c12, d5), (a6, b2, c12, d6), (a6, b2, c12, d7), (a6, b2, c12, d8), (a6, b2, c12, d9), (a6, b2, c12, d10), (a6, b2, c12, d11), (a6, b2, c12, d12), (a6, b2, c13, d1), (a6, b2, c13, d2), (a6, b2, c13, d3), (a6, b2, c13, d4), (a6, b2, c13, d5), (a6, b2, c13, d6), (a6, b2, c13, d7), (a6, b2, c13, d8), (a6, b2, c13, d9), (a6, b2, c13, d10), (a6, b2, c13, d11), (a6, b2, c13, d12), (a6, b3, c1, d1), (a6, b3, c1, d2), (a6, b3, c1, d3), (a6, b3, c1, d4), (a6, b3, c1, d5), (a6, b3, c1, d6), (a6, b3, c1, d7), (a6, b3, el, d8), (a6, b3, c1, d9), (a6, b3, c1, d10), (a6, b3, c1, d11), (a6, b3, c1, d12), (a6, b3, c2, d1), (a6, b3, c2, d2), (a6, b3, c2, d3), (a6, b3, c2, d4), (a6, b3, c2, d5), (a6, b3, c2, d6), (a6, b3, c2, d7), (a6, b3, c2, d8), (a6, b3, c2, d9), (a6, b3, c2, d10), (a6, b3, c2, d11), (a6, b3, c2, d12), (a6, b3, c3, d1), (a6, b3, c3, d2), (a6, b3, c3, d3), (a6, b3, c3, d4), (a6, b3, c3, d5), (a6, b3, c3, d6), (a6, b3, c3, d7), (a6, b3, c3, d8), (a6, b3, c3, d9), (a6, b3, c3, d10), (a6, b3, c3, d11), (a6, b3, c3, d12), (a6, b3, c4, d1), (a6, b3, c4, d2), (a6, b3, c4, d3), (a6, b3, c4, d4), (a6, b3, c4, d5), (a6, b3, c4, d6), (a6, b3, c4, d7), (a6, b3, c4, d8), (a6, b3, c4, d9), (a6, b3, c4, d10), (a6, b3, c4, d11), (a6, b3, c4, d12), (a6, b3, c5, d1), (a6, b3, c5, d2), (a6, b3, c5, d3), (a6, b3, c5, d4), (a6, b3, c5, d5), (a6, b3, c5, d6), (a6, b3, c5, d7), (a6, b3, c5, d8), (a6, b3, c5, d9), (a6, b3, c5, d10), (a6, b3, c5, d11), (a6, b3, c5, d12), (a6, b3, c6, d1), (a6, b3, c6, d2), (a6, b3, c6, d3), (a6, b3, c6, d4), (a6, b3, c6, d5), (a6, b3, c6, d6), (a6, b3, c6, d7), (a6, b3, c6, d8), (a6, b3, c6, d9), (a6, b3, c6, d10), (a6, b3, c6, d11), (a6, b3, c6, d12), (a6, b3, c7, d1), (a6, b3, c7, d2), (a6, b3, c7, d3), (a6, b3, c7, d4), (a6, b3, c7, d5), (a6, b3, c7, d6), (a6, b3, c7, d7), (a6, b3, c7, d8), (a6, b3, c7, d9), (a6, b3, c7, d10), (a6, b3, c7, d11), (a6, b3, c7, d12), (a6, b3, c8, d1 (a6, b3, c8, d2), (a6, b3, c8, d3), (a6, b3, c8, d4), (a6, b3, c8, d5), (a6, b3, c8, d6), (a6, b3, c8, d7), (a6, b3, c8, d8), (a6, b3, c8, d9), (a6, b3, c8, d10), (a6, b3, c8, d11) (a6, b3, c8, d12), (a6, b3, c9, d1), (a6, b3, c9, d2), (a6, b3, c9, d3), (a6, b3, c9, d4), (a6, b3, c9, d5), (a6, b3, c9, d6), (a6, b3, c9, d7), (a6, b3, c9, d8), (a6, b3, c9, d9), (a6, b3, c9, d10), (a6, b3, c9, d11) (a6, b3, c9, d12), (a6, b3, c10, d1), (a6, b3, c10, d2), (a6, b3, c10, d3), (a6, b3, c10, d4), (a6, b3, c10, d5), (a6, b3, c10, d6), (a6, b3, c10, d7), (a6, b3, c10, d8), (a6, b3, c10, d9), (a6, b3, c10, d10), (a6, b3, c10, d1 (a6, b3, c10, d12), (a6, b3, c11, d1 (a6, b3, c11, d2), (a6, b3, c11, d3), (a6, b3, c11, d4), (a6, b3, c11, d5), (a6, b3, c11, d6), (a6, b3, c11, d7), (a6, b3, c11, d8), (a6, b3, c11, d9), (a6, b3, c11, d10), (a6, b3, c11, d11), (a6, b3, c11, d12), (a6, b3, c12, d1), (a6, b3, c12, d2), (a6, b3, c12, d3), (a6, b3, c12, d4), (a6, b3, c12, d5), (a6, b3, c12, d6), (a6, b3, c12, d7), (a6, b3, c12, d8), (a6, b3, c12, d9), (a6, b3, c12, d10), (a6, b3, c12, d11), (a6, b3, c12, d12), (a6, b3, c13, d1), (a6, b3, c13, d2), (a6, b3, c13, d3), (a6, b3, c13, d4), (a6, b3, c13, d5), (a6, b3, c13, d6), (a6, b3, c13, d7), (a6, b3, c13, d8), (a6, b3, c13, d9), (a6, b3, c13, d10), (a6, b3, c13, d11), (a6, b3, c13, d12), (a6, b4, c1, d1), (a6, b4, c1, d2), (a6, b4, c1, d3), (a6, b4, c1, d4), (a6, b4, c1, d5), (a6, b4, c1, d6), (a6, b4, c1, d7), (a6, b4, c1, d8), (a6, b4, c1, d9), (a6, b4, c1 d10), (a6, b4, c1 d11) (a6, b4, c1 d12), (a6, b4, c2, d1), (a6, b4, c2, d2), (a6, b4, c2, d3), (a6, b4, c2, d4), (a6, b4, c2, d5), (a6, b4, c2, d6), (a6, b4, c2, d7), (a6, b4, c2, d8), (a6, b4, c2, d9), (a6, b4, c2, d10), (a6, b4, c2, d11), (a6, b4, c2, d12), (a6, b4, c3, d1), (a6, b4, c3, d2), (a6, b4, c3, d3), (a6, b4, c3, d4), (a6, b4, c3, d5), (a6, b4, c3, d6), (a6, b4, c3, d7), (a6, b4, c3, d8), (a6, b4, c3, d9), (a6, b4, c3, d10), (a6, b4, c3, d11), (a6, b4, c3, d12), (a6, b4, c4, d1), (a6, b4, c4, d2), (a6, b4, c4, d3), (a6, b4, c4, d4), (a6, b4, c4, d5), (a6, b4, c4, d6), (a6, b4, c4, d7), (a6, b4, c4, d8), (a6, b4, c4, d9), (a6, b4, c4, d10), (a6, b4, c4, d11), (a6, b4, c4, d12), (a6, b4, c5, d1), (a6, b4, c5, d2), (a6, b4, c5, d3), (a6, b4, c5, d4), (a6, b4, c5, d5), (a6, b4, c5, d6), (a6, b4, c5, d7), (a6, b4, c5, d8), (a6, b4, c5, d9), (a6, b4, c5, d10), (a6, b4, c5, d11), (a6, b4, c5, d12), (a6, b4, c6, d1), (a6, b4, c6, d2), (a6, b4, c6, d3), (a6, b4, c6, d4), (a6, b4, c6, d5), (a6, b4, c6, d6), (a6, b4, c6, d7), (a6, b4, c6, d8), (a6, b4, c6, d9), (a6, b4, c6, d10), (a6, b4, c6, d11), (a6, b4, c6, d12), (a6, b4, c7, d1), (a6, b4, c7, d2), (a6, b4, c7, d3), (a6, b4, c7, d4), (a6, b4, c7, d5), (a6, b4, c7, d6), (a6, b4, c7, d7), (a6, b4, c7, d8), (a6, b4, c7, d9), (a6, b4, c7., d10), (a6, b4, c7, d11), (a6, b4, c7, d12), (a6, b4, c8, d1), (a6, b4, c8, d2), (a6, b4, c8, d3), (a6, b4, c8, d4), (a6, b4, c8, d5), (a6, b4, c8, d6), (a6, b4, c8, d7), (a6, b4, c8, d8), (a6, b4, c8, d9), (a6, b4, c8, d10), (a6, b4, c8, d11), (a6, b4,

c8, d12), (a6, b4, c9, d1), (a6, b4, c9, d2), (a6, b4, c9, d3), (a6, b4, c9, d4), (a6, b4, c9, d5), (a6, b4, c9, d6), (a6, b4, c9, d7), (a6, b4, c9, d8), (a6, b4, c9, d9), (a6, b4, c9, d10), (a6, b4, c9, d11), (a6, b4, c9, d12), (a6, b4, c10, d1), (a6, b4, c10, d2), (a6, b4, c10, d3), (a6, b4, c10, d4), (a6, b4, c10, d5), (a6, b4, c10, d6), (a6, b4, c10, d7), (a6, b4, c10, d8), (a6, b4, c10, d9), (a6, b4, c10, d10), (a6, b4, c10, d11), (a6, b4, c10, d12), (a6, b4, c11, d1), (a6, b4, c11, d2), (a6, b4, c11, d3), (a6, b4, c11, d4), (a6, b4, c11, d5), (a6, b4, c11, d6), (a6, b4, c11, d7), (a6, b4, c11, d8), (a6, b4, c11, d9), (a6, b4, c11, d10), (a6, b4, c11, d11), (a6, b4, c11, d12), (a6, b4, c12, d1), (a6, b4, c12, d2), (a6, b4, c12, d3), (a6, b4, c12, d4), (a6, b4, c12, d5), (a6, b4, c12, d6), (a6, b4, c12, d7), (a6, b4, c12, d8), (a6, b4, c12, d9), (a6, b4, c12, d10), (a6, b4, c12, d11), (a6, b4, c12, d12), (a6, b4, c13, d1), (a6, b4, c13, d2), (a6, b4, c13, d3), (a6, b4, c13, d4), (a6, b4, c13, d5), (a6, b4, c13, d6), (a6, b4, c13, d7), (a6, b4, c13, d8), (a6, b4, c13, d9), (a6, b4, c13, d10), (a6, b4, c13, d11), (a6, b4, c13, d12), (a6, b5, c1, d1), (a6, b5, c1, d2), (a6, b5, c1, d3), (a6, b5, c1, d4), (a6, b5, c1, d5), (a6, b5, c1, d6), (a6, b5, c1, d7), (a6, b5, c1, d8), (a6, b5, c1, d9), (a6, b5, c1, d10), (a6, b5, c1, d11), (a6, b5, c1, d12), (a6, b5, c2, d1), (a6, b5, c2, d2), (a6, b5, c2, d3), (a6, b5, c2, d4), (a6, b5, c2, d5), (a6, b5, c2, d6), (a6, b5, c2, d7), (a6, b5, c2, d8), (a6, b5, c2, d9), (a6, b5, c2, d10), (a6, b5, c2, d11), (a6, b5, c2, d12), (a6, b5, c3, d1), (a6, b5, c3, d2), (a6, b5, c3, d3), (a6, b5, c3, d4), (a6, b5, c3, d5), (a6, b5, c3, d6), (a6, b5, c3, d7), (a6, b5, c3, d8), (a6, b5, c3, d9), (a6, b5, c3, d10), (a6, b5, c3, d11), (a6, b5, c3, d12), (a6, b5, c4, d1), (a6, b5, c4, d2), (a6, b5, c4, d3), (a6, b5, c4, d4), (a6, b5, c4, d5), (a6, b5, c4, d6), (a6, b5, c4, d7), (a6, b5, c4, d8), (a6, b5, c4, d9), (a6, b5, c4, d10), (a6, b5, c4, d11), (a6, b5, c4, d12), (a6, b5, c5, d1), (a6, b5, c5, d2), (a6, b5, c5, d3), (a6, b5, c5, d4), (a6, b5, c5, d5), (a6, b5, c5, d6), (a6, b5, c5, d7), (a6, b5, c5, d8), (a6, b5, c5, d9), (a6, b5, c5, d10), (a6, b5, c5, d11), (a6, b5, c5, d12), (a6, b5, c6, d1), (a6, b5, c6, d2), (a6, b5, c6, d3), (a6, b5, c6, d4), (a6, b5, c6, d5), (a6, b5, c6, d6), (a6, b5, c6, d7), (a6, b5, c6, d8), (a6, b5, c6, d9), (a6, b5, c6, d10), (a6, b5, c6, d11), (a6, b5, c6, d12), (a6, b5, c7, d1), (a6, b5, c7, d2), (a6, b5, c7, d3), (a6, b5, c7, d4), (a6, b5, c7, d5), (a6, b5, c7, d6), (a6, b5, c7, d7), (a6, b5, c7, d8), (a6, b5, c7, d9), (a6, b5, c7, d10), (a6, b5, c7, d11), (a6, b5, c7, d12), (a6, b5, c8, d1), (a6, b5, c8, d2), (a6, b5, c8, d3), (a6, b5, c8, d4), (a6, b5, c8, d5), (a6, b5, c8, d6), (a6, b5, c8, d7), (a6, b5, c8, d8), (a6, b5, c8, d9), (a6, b5, c8, d10), (a6, b5, c8, d11), (a6, b5, c8, d12), (a6, b5, c9, d1), (a6, b5, c9, d2), (a6, b5, c9, d3), (a6, b5, c9, d4), (a6, b5, c9, d5), (a6, b5, c9, d6), (a6, b5, c9, d7), (a6, b5, c9, d8), (a6, b5, c9, d9), (a6, b5, c9, d10), (a6, b5, c9, d11), (a6, b5, c9, d12), (a6, b5, c10, d1), (a6, b5, c10, d2), (a6, b5, c10, d3), (a6, b5, c10, d4), (a6, b5, c10, d5), (a6, b5, c10, d6), (a6, b5, c10, d7), (a6, b5, c10, d8), (a6, b5, c10, d9), (a6, b5, c10, d10), (a6, b5, c10, d11), (a6, b5, c10, d12), (a6, b5, c11, d1), (a6, b5, c11, d2), (a6, b5, c11, d3), .(a6, b5, c11, d4), (a6, b5, c11, d5), (a6, b5, c11, d6), (a6, b5, c11, d7), (a6, b5, c11, d8), (a6, b5, c11, d9), (a6, b5, c11, d10), (a6, b5, c11, d11), (a6, b5, c11, d12), (a6, b5, c12, d1), (a6, b5, c12, d2), (a6, b5, c12, d3), (a6, b5, c12, d4), (a6, b5, c12, d5), (a6, b5, c12, d6), (a6, b5, c12, d7), (a6, b5, c12, d8), (a6, b5, c12, d9), (a6, b5, c12, d10), (a6, b5, c12, d11), (a6, b5, c12, d12), (a6, b5, c13, d1), (a6, b5, c13, d2), (a6, b5, c13, d3), (a6, b5, c13, d4), (a6, b5, c13, d5), (a6, b5, c13, d6), (a6, b5, c13, d7), (a6, b5, c13, d8), (a6, b5, c13, d9), (a6, b5, c13, d10), (a6, b5, c13, d11), (a6, b5, c13, d12), (a6, b6, c1, d1), (a6, b6, c1, d2), (a6, b6, c1, d3), (a6, b6, c1, d4), (a6, b6, c1, d5), (a6, b6, c1, d6), (a6, b6, c1, d7), (a6, b6, c1, d8), (a6, b6, c1, d9), (a6, b6, c1, d10), (a6, b6, c1, d11), (a6, b6, c1, d12), (a6, b6, c2, d1), (a6, b6, c2, d2), (a6, b6, c2, d3), (a6, b6, c2, d4), (a6, b6, c2, d5), (a6, b6, c2, d6), (a6, b6, c2, d7), (a6, b6, c2, d8), (a6, b6, c2, d9), (a6, b6, c2, d10), (a6, b6, c2, d11), (a6, b6, c2, d12), (a6, b6, c3, d1), (a6, b6, c3, d2), (a6, b6, c3, d3), (a6, b6, c3, d4), (a6, b6, c3, d5), (a6, b6, c3, d6), (a6, b6, c3, d7), (a6, b6, c3, d8), (a6, b6, c3, d9), (a6, b6, c3, d10), (a6, b6, c3, d11), (a6, b6, c3, d12), (a6, b6, c4, d1), (a6, b6, c4, d2), (a6, b6, c4, d3), (a6, b6, c4, d4), (a6, b6, c4, d5), (a6, b6, c4, d6), (a6, b6, c4, d7), (a6, b6, c4, d8), (a6, b6, c4, d9), (a6, b6, c4, d10), (a6, b6, c4, d11), (a6, b6, c4, d12), (a6, b6, c5, d1), (a6, b6, c5, d2), (a6, b6, c5, d3), (a6, b6, c5, d4), (a6, b6, c5, d5), (a6, b6, c5, d6), (a6, b6, c5, d7), (a6, b6, c5, d8), (a6, b6, c5, d9), (a6, b6, c5, d10), (a6, b6, c5, d11), (a6, b6, c5, d12), (a6, b6, c6, d1), (a6, b6, c6, d2), (a6, b6, c6, d3), (a6, b6, c6, d4), (a6, b6, c6, d5), (a6, b6, c6, d6), (a6, b6, c6, d7), (a6, b6, c6, d8), (a6, b6, c6, d9), (a6, b6, c6, d10), (a6, b6, c6, d11), (a6, b6, c6, d12), (a6, b6, c7, d1), (a6, b6, c7, d2), (a6, b6, c7, d3), (a6, b6, c7, d4), (a6, b6, c7, d5), (a6, b6, c7, d6), (a6, b6, c7, d7), (a6, b6, c7, d8), (a6, b6, c7, d9), (a6, b6, c7, d10), (a6, b6, c7, d11), (a6, b6, c7, d12), (a6, b6, c8, d1), (a6, b6, c8, d2), (a6, b6, c8, d3), (a6, b6, c8, d4), (a6, b6, c8, d5), (a6, b6, c8, d6), (a6, b6, c8, d7), (a6, b6, c8, d8), (a6, b6, c8, d9), (a6, b6, c8, d10), (a6, b6, c8, d11) (a6, b6, c8, d12), (a6, b6, c9, d1), (a6, b6, c9, d2), (a6, b6, c9, d3), (a6, b6, c9, d4), (a6, b6, c9, d5), (a6, b6, c9, d6), (a6, b6, c9, d7), (a6, b6, c9, d8), (a6, b6, c9, d9), (a6, b6, c9, d10), (a6, b6, c9, d11), (a6, b6, c9, d12), (a6, b6, c10, d1), (a6, b6, c10, d2), (a6, b6, c10, d3), (a6, b6, c10, d4), (a6, b6, c10, d5), (a6, b6, c10, d6), (a6, b6, c10, d7), (a6, b6, c10, d8), (a6, b6, c10, d9), (a6, b6, c10, d10), (a6, b6, c10, d11), (a6, b6, c10, d12), (a6, b6, c11, d1), (a6, b6, c11, d2), (a6, b6, c11, d3), (a6, b6, c11, d4), (a6, b6, c11, d5), (a6, b6, c11, d6), (a6, b6, c11, d7), (a6, b6, c11, d8), (a6, b6, c11, d9), (a6, b6, c11, d10), (a6, b6, c11, d11), (a6, b6, c11, d12), (a6, b6, c12, d1), (a6, b6, c12, d2), (a6, b6, c12, d3), (a6, b6, c12, d4), (a6, b6, c12, d5), (a6, b6, c12, d6), (a6, b6, c12, d7), (a6, b6, c12, d8), (a6, b6, c12, d9), (a6, b6, c12, d10), (a6, b6, c12, d11), (a6, b6, c12, d12), (a6, b6, c13, d1), (a6, b6, c13, d2), (a6, b6, c13, d3), (a6, b6, c13, d4), (a6, b6, c13, d5), (a6, b6, c13, d6), (a6, b6, c13, d7), (a6, b6, c13, d8), (a6, b6, c13, d9), (a6, b6, c13, d10), (a6, b6, c13, d11), (a6, b6, c13, d12), (a6, b7, c1, d1), (a6, b7, c1, d2), (a6, b7, c1, d3), (a6, b7, c1 d4), (a6, b7, c1, d5), (a6, b7, c1 d6), (a6, b7, c1, d7), (a6, b7, c1, d8), (a6, b7, c1 d9), (a6, b7, c1 d10), (a6, b7, c1, d11), (a6, b7, c1, d12), (a6, b7, c2, d1), (a6, b7, c2, d2), (a6, b7, c2, d3), (a6, b7, c2, d4), (a6, b7, c2, d5), (a6, b7, c2, d6), (a6, b7, c2, d7), (a6, b7, c2, d8), (a6, b7, c2, d9), (a6, b7, c2, d10), (a6, b7, c2, d11), (a6, b7, c2, d12), (a6, b7, c3, d1), (a6, b7, c3, d2), (a6, b7,

c3, d3), (a6, b7, c3, d4), (a6, b7, c3, d5), (a6, b7, c3, d6), (a6, b7, c3, d7), (a6, b7, c3, d8), (a6, b7, c3, d9), (a6, b7, c3, d10), (a6, b7, c3, d11), (a6, b7, c3, d12), (a6, b7, c4, d1), (a6, b7, c4, d2), (a6, b7, c4, d3), (a6, b7, c4, d4), (a6, b7, c4, d5), (a6, b7, c4, d6), (a6, b7, c4, d7), (a6, b7, c4, d8), (a6, b7, c4, d9), (a6, b7, c4, d10), (a6, b7, c4, . d11), (a6, b7, c4, d12), (a6, b7, c5, d1), (a6, b7, c5, d2), (a6, b7, c5, d3), (a6, b7, c5, d4), (a6, b7, c5, d5), (a6, b7, c5, d6), (a6, b7, c5, d7), (a6, b7, c5, d8), (a6, b7, c5, d9), (a6, b7, c5, d10), (a6, b7, c5, d11) (a6, b7, c5, d12), (a6, b7, c6, d1), (a6, b7, c6, d2), (a6, b7, c6, d3), (a6, b7, c6, d4), (a6, b7, c6, d5), (a6, b7, c6, d6), (a6, b7, c6, d7), (a6, b7, c6, d8), (a6, b7, c6, d9), (a6, b7, c6, d10), (a6, b7, c6, d11), (a6, b7, c6, d12), (a6, b7, c7, d1), (a6, b7, c7, d2), (a6, b7, c7, d3), (a6, b7, c7, d4), (a6, b7, c7, d5), (a6, b7, c7, d6), (a6, b7, c7, d7), (a6, b7, c7, d8), (a6, b7, c7, d9), (a6, b7, c7, d10), (a6, b7, c7, d11), (a6, b7, c7, d12), (a6, b7, c8, d1), (a6, b7, c8, d2), (a6, b7, c8, d3), (a6, b7, c8, d4), (a6, b7, c8, d5), (a6, b7, c8, d6), (a6, b7, c8, d7), (a6, b7, c8, d8), (a6, b7, c8, d9), (a6, b7, c8, d10), (a6, b7, c8, d11), (a6, b7, c8, d12), (a6, b7, c9, d1), (a6, b7, c9, d2), (a6, b7, c9, d3), (a6, b7, c9, d4), (a6, b7, c9, d5), (a6, b7, c9, d6), (a6, b7, c9, d7), (a6, b7, c9, d8), (a6, b7, c9, d9), (a6, b7, c9, d10), (a6, b7, c9, d11) (a6, b7, c9, d12), (a6, b7, c10, d1), (a6, b7, c10, d2), (a6, b7, c10, d3), (a6, b7, c10, d4), (a6, b7, c10, d5), (a6, b7, c10, d6), (a6, b7, c10, d7), (a6, b7, c10, d8), (a6, b7, c10, d9), (a6, b7, c10, d10), (a6, b7, c10, d11), (a6, b7, c10, d12), (a6, b7, c11, d1), (a6, b7, c11, d2), (a6, b7, c11, d3), (a6, b7, c11, d4), (a6, b7, c11, d5), (a6, b7, c11, d6), (a6, b7, c11, d7), (a6, b7, c11, d8), (a6, b7, c11, d9), (a6, b7, c11, d10), (a6, b7, c11, d11), (a6, b7, c11, d12), (a6, b7, c12, d1), (a6, b7, c12, d2), (a6, b7, c12, d3), (a6, b7, c12, d4), (a6, b7, c12, d5), (a6, b7, c12, d6), (a6, b7, c12, d7), (a6, b7, c12, d8), (a6, b7, c12, d9), (a6, b7, c12, d10), (a6, b7, c12, d11), (a6, b7, c12, d12), (a6, b7, c13, d1), (a6, b7, c13, d2), (a6, b7, c13, d3), (a6, b7, c13, d4), (a6, b7, c13, d5), (a6, b7, c13, d6), (a6, b7, c13, d7), (a6, b7, c13, d8), (a6, b7, c13, d9), (a6, b7, c13, d10), (a6, b7, c13, d11), (a6, b7, c13, d12), (a6, b8, c1, d1), (a6, b8, c1, d2), (a6, b8, c1, d3), (a6, b8, c1, d4), (a6, b8, c1, d5), (a6, b8, c1, d6), (a6, b8, c1, d7), (a6, b8, c1, d8), (a6, b8, c1, d9), (a6, b8, c1, d10), (a6, b8, c1, d11), (a6, b8, c1, d12), (a6, b8, c2, d1), (a6, b8, c2, d2), (a6, b8, c2, d3), (a6, b8, c2, d4), (a6, b8, c2, d5), (a6, b8, c2, d6), (a6, b8, c2, d7), (a6, b8, c2, d8), (a6, b8, c2, d9), (a6, b8, c2, d10), (a6, b8, c2, d11), (a6, b8, c2, d12), (a6, b8, c3, d1), (a6, b8, c3, d2), (a6, b8, c3, d3), (a6, b8, c3, d4), (a6, b8, c3, d5), (a6, b8, c3, d6), (a6, b8, c3, d7), (a6, b8, c3, d8), (a6, b8, c3, d9), (a6, b8, c3, d10), (a6, b8, c3, d11), (a6, b8, c3, d12), (a6, b8, c4, d1), (a6, b8, c4, d2), (a6, b8, c4, d3), (a6, b8, c4, d4), (a6, b8, c4, d5), (a6, b8, c4, d6), (a6, b8, c4, d7), (a6, b8, c4, d8), (a6, b8, c4, d9), (a6, b8, c4, d10), (a6, b8, c4, d11), (a6, b8, c4, d12), (a6, b8, c5, d1), (a6, b8, c5, d2), (a6, b8, c5, d3), (a6, b8, c5, d4), (a6, b8, c5, d5), (a6, b8, c5, d6), (a6, b8, c5, d7), (a6, b8, c5, d8), (a6, b8, c5, d9), (a6, b8, c5, d10), (a6, b8, c5, d11), (a6, b8, c5, d12), (a6, b8, c6, d1), (a6, b8, c6, d2), (a6, b8, c6, d3), (a6, b8, c6, d4), (a6, b8, c6, d5), (a6, b8, c6, d6), (a6, b8, c6, d7), (a6, b8, c6, d8), (a6, b8, c6, d9), (a6, b8, c6, d10), (a6, b8, c6, d11), (a6, b8, c6, d12), (a6, b8, c7, d1), (a6, b8, c7, d2), (a6, b8, c7, d3), (a6, b8, c7, d4), (a6, b8, c7, d5), (a6, b8, c7, d6), (a6, b8, c7, d7), (a6, b8, c7, d8), (a6, b8, c7, d9), (a6, b8, c7, d10), (a6, b8, c7, d11), (a6, b8, c7, d12), (a6, b8, c8, d1), (a6, b8, c8, d2), (a6, b8, c8, d3), (a6, b8, c8, d4), (a6, b8, c8, d5), (a6, b8, c8, d6), (a6, b8, c8, d7), (a6, b8, c8, d8), (a6, b8, c8, d9), (a6, b8, c8, d10), (a6, b8, c8, d11), (a6, b8, c8, d12), (a6, b8, c9, d1), (a6, b8, c9, d2), (a6, b8, c9, d3), (a6, b8, c9, d4), (a6, b8, c9, d5), (a6, b8, c9, d6), (a6, b8, c9, d7), (a6, b8, c9, d8), (a6, b8, c9, d9), (a6, b8, c9, d10), (a6, b8, c9, d11), (a6, b8, c9, d12), (a6, b8, c10, d1), (a6, b8, c10, d2), (a6, b8, c10, d3), (a6, b8, c10, d4), (a6, b8, c10, d5), (a6, b8, c10, d6), (a6, b8, c10, d7), (a6, b8, c10, d8), (a6, b8, c10, d9), (a6, b8, c10, d10), (a6, b8, c10, d11), (a6, b8, c10, d12), (a6, b8, c11, d1), (a6, b8, c11, d2), (a6, b8, c11, d3), (a6, b8, c11, d4), (a6, b8, c11, d5), (a6, b8, c11, d6), (a6, b8, c11, d7), (a6, b8, c11, d8), (a6, b8, c11, d9), (a6, b8, c11, d10), (a6, b8, c11, d11), (a6, b8, c11, d12), (a6, b8, c12, d1), (a6, b8, c12, d2), (a6, b8, c12, d3), (a6, b8, c12, d4), (a6, b8, c12, d5), (a6, b8, c12, d6), (a6, b8, c12, d7), (a6, b8, c12, d8), (a6, b8, c12, d9), (a6, b8, c12, d10), (a6, b8, c12, d11), (a6, b8, c12, d12), (a6, b8, c13, d1), (a6, b8, c13, d2), (a6, b8, c13, d3), (a6, b8, c13, d4), (a6, b8, c13, d5), (a6, b8, c13, d6), (a6, b8, c13, d7), (a6, b8, c13, d8), (a6, b8, c13, d9), (a6, b8, c13, d10), (a6, b8, c13, d11), (a6, b8, c13, d12), (a6, b9, c1, d1), (a6, b9, c1 d2), (a6, b9, c1 d3), (a6, b9, c1, d4), (a6, b9, c1 d5), (a6, b9, c1, d6), (a6, b9, c1 d7), (a6, b9, c1, d8), (a6, b9, c1, d9), (a6, b9, c1, d10), (a6, b9, c1, d11), (a6, b9, c1 d12), (a6, b9, c2, d1), (a6, b9, c2, d2), (a6, b9, c2, d3), (a6, b9, c2, d4), (a6, b9, c2, d5), (a6, b9, c2, d6), (a6, b9, c2, d7), (a6, b9, c2, d8), (a6, b9, c2, d9), (a6, b9, c2, d10), (a6, b9, c2, d11), (a6, b9, c2, d12), (a6, b9, c3, d1), (a6, b9, c3, d2), (a6, b9, c3, d3), (a6, b9, c3, d4), (a6, b9, c3, d5), (a6, b9, c3, d6), (a6, b9, c3, d7), (a6, b9, c3, d8), (a6, b9, c3, d9), (a6, b9, c3, d10), (a6, b9, c3, d11), (a6, b9, c3, d12), (a6, b9, c4, d1), (a6, b9, c4, d2), (a6, b9, c4, d3), (a6, b9, c4, d4), (a6, b9, c4, d5), (a6, b9, c4, d6), (a6, b9, c4, d7), (a6, b9, c4, d8), (a6, b9, c4, d9), (a6, b9, c4, d10), (a6, b9, c4, d11), (a6, b9, c4, d12), (a6, b9, c5, d1), (a6, b9, c5, d2), (a6, b9, c5, d3), (a6, b9, c5, d4), (a6, b9, c5, d5), (a6, b9, c5, d6), (a6, b9, c5, d7), (a6, b9, c5, d8), (a6, b9, c5, d9), (a6, b9, c5, d10), (a6, b9, c5, d11), (a6, b9, c5, d12), (a6, b9, c6, d1), (a6, b9, c6, d2), (a6, b9, c6, d3), (a6, b9, c6, d4), (a6, b9, c6, d5), (a6, b9, c6, d6), (a6, b9, c6, d7), (a6, b9, c6, d8), (a6, b9, c6, d9), (a6, b9, c6, d10), (a6, b9, c6, d11), (a6, b9, c6, d12), (a6, b9, c7, d1), (a6, b9, c7, d2), (a6, b9, c7, d3), (a6, b9, c7, d4), (a6, b9, c7, d5), (a6, b9, c7, d6), (a6, b9, c7, d7), (a6, b9, c7, d8), (a6, b9, c7, d9), (a6, b9, c7, d10), (a6, b9, c7, d11), (a6, b9, c7, d12), (a6, b9, c8, d1), (a6, b9, c8, d2), (a6, b9, c8, d3), (a6, b9, c8, d4), (a6, b9, c8, d5), (a6, b9, c8, d6), (a6, b9, c8, d7), (a6, b9, c8, d8), (a6, b9, c8, d9), (a6, b9, c8, d10), (a6, b9, c8, d11), (a6, b9, c8, d12), (a6, b9, c9, d1), (a6, b9, c9, d2), (a6, b9, c9, d3), (a6, b9, c9, d4), (a6, b9, c9, d5), (a6, b9, c9, d6), (a6, b9, c9, d7), (a6, b9, c9, d8), (a6, b9, c9, d9), (a6, b9, c9, d10), (a6, b9, c9, d11), (a6, b9, c9, d12), (a6, b9, c10, d1), (a6, b9, c10, d2), (a6, b9, c10, d3), (a6, b9, c10, d4), (a6, b9, c10, d5), (a6, b9, c10, d6), (a6, b9, c10, d7), (a6, b9, c10, d8),

(a6, b9, c10, d9), (a6, b9, c10, d10), (a6, b9, c10, d11), (a6, b9, c10, d12), (a6, b9, c11, d1), (a6, b9, c11, d2), (a6, b9, c11, d3), (a6, b9, c11, d4), (a6, b9, c11, d5), (a6, b9, c11, d6), (a6, b9, c11, d7), (a6, b9, c11, d8), (a6, b9, c11, d9), (a6, b9, c11, d10), (a6, b9, c11, d11), (a6, b9, c11, d12), (a6, b9, c12, d1), (a6, b9, c12, d2), (a6, b9, c12, d3), (a6, b9, c12, d4), (a6, b9, c12, d5), (a6, b9, c12, d6), (a6, b9, c12, d7), (a6, b9, c12, d8), (a6, b9, c12, d9), (a6, b9, c12, d10), (a6, b9, c12, d11), (a6, b9, c12, d12), (a6, b9, c13, d1), (a6, b9, c13, d2), (a6, b9, c13, d3), (a6, b9, c13, d4), (a6, b9, c13, d5), (a6, b9, c13, d6), (a6, b9, c13, d7), (a6, b9, c13, d8), (a6, b9, c13, d9), (a6, b9, c13, d10), (a6, b9, c13, d11), (a6, b9, c13, d12), (a6, b10, c1, d1), (a6, b10, c1, d2), (a6, b10, c1, d3), (a6, b10, c1 d4), (a6, b10, c1, d5), (a6, b10, c1, d6), (a6, b10, c1, d7), (a6, b10, c1, d8), (a6, b10, c1, d9), (a6, b10, c1, d10), (a6, b10, c1, d11), (a6, b10, c1, d12), (a6, b10, c2, d1), (a6, b10, c2, d2), (a6, b10, c2, d3), (a6, b10, c2, d4), (a6, b10, c2, d5), (a6, b10, c2, d6), (a6, b10, c2, d7), (a6, b10, c2, d8), (a6, b10, c2, d9), (a6, b10, c2, d10), (a6, b10, c2, d11), (a6, b10, c2, d12), (a6, b10, c3, d1), (a6, b10, c3, d2), (a6, b10, c3, d3), (a6, b10, c3, d4), (a6, b10, c3, d5), (a6, b10, c3, d6), (a6, b10, c3, d7), (a6, b10, c3, d8), (a6, b10, c3, d9), (a6, b10, c3, d10), (a6, b10, c3, d11), (a6, b10, c3, d12), (a6, b10, c4, d1), (a6, b10, c4, d2), (a6, b10, c4, d3), (a6, b10, c4, d4), (a6, b10, c4, d5), (a6, b10, c4, d6), (a6, b10, c4, d7), (a6, b10, c4, d8), (a6, b10, c4, d9), (a6, b10, c4, d10), (a6, b10, c4, d11), (a6, b10, c4, d12), (a6, b10, c5, d1), (a6, b10, c5, d2), (a6, b10, c5, d3), (a6, b10, c5, d4), (a6, b10, c5, d5), (a6, b10, c5, d6), (a6, b10, c5, d7), (a6, b10, c5, d8), (a6, b10, c5, d9), (a6, b10, c5, d10), (a6, b10, c5, d11), (a6, b10, c5, d12), (a6, b10, c6, d1), (a6, b10, c6, d2), (a6, b10, c6, d3), (a6, b10, c6, d4), (a6, b10, c6, d5), (a6, b10, c6, d6), (a6, b10, c6, d7), (a6, b10, c6, d8), (a6, b10, c6, d9), (a6, b10, c6, d10), (a6, b10, c6, d11), (a6, b10, c6, d12), (a6, b10, c7, d1), (a6, b10, c7, d2), (a6, b10, c7, d3), (a6, b10, c7, d4), (a6, b10, c7, d5), (a6, b10, c7, d6), (a6, b10, c7, d7), (a6, b10, c7, d8), (a6, b10, c7, d9), (a6, b10, c7, d10), (a6, b10, c7, d11), (a6, b10, c7, d12), (a6, b10, c8, d1), (a6, b10, c8, d2), (a6, b10, c8, d3), (a6, b10, c8, d4), (a6, b10, c8, d5), (a6, b10, c8, d6), (a6, b10, c8, d7), (a6, b10, c8, d8), (a6, b10, c8, d9), (a6, b10, c8, d10), (a6, b10, c8, d11), (a6, b10, c8, d12), (a6, b10, c9, d1), (a6, b10, c9, d2), (a6, b10, c9, d3), (a6, b10, c9, d4), (a6, b10, c9, d5), (a6, b10, c9, d6), (a6, b10, c9, d7), (a6, b10, c9, d8), (a6, b10, c9, d9), (a6, b10, c9, d10), (a6, b10, c9, d11), (a6, b10, c9, d12), (a6, b10, c10, d1), (a6, b10, c10, d2), (a6, b10, c10, d3), (a6, b10, c10, d4), (a6, b10, c10, d5), (a6, b10, c10, d6), (a6, b10, c10, d7), (a6, b10, c10, d8), (a6, b10, c10, d9), (a6, b10, c10, d10), (a6, b10, c10, d11), (a6, b10, c10, d12), (a6, b10, c11, d1), (a6, b10, c11, d2), (a6, b10, c11, d3), (a6, b10, c11, d4), (a6, b10, c11, d5), (a6, b10, c11, d6), (a6, b10, c11, d7), (a6, b10, c11, d8), (a6, b10, c11, d9), (a6, b10, c11, d10), (a6, b10, c11, d11), (a6, b10, c11, d12), (a6, b10, c12, d1), (a6, b10, c12, d2), (a6, b10, c12, d3), (a6, b10, c12, d4), (a6, b10, c12, d5), (a6, b10, c12, d6), (a6, b10, c12, d7), (a6, b10, c12, d8), (a6, b10, c12, d9), (a6, b10, c12, d10), (a6, b10, c12, d11), (a6, b10, c12, d12), (a6, b10, c13, d1), (a6, b10, c13, d2), (a6, b10, c13, d3), (a6, b10, c13, d4), (a6, b10, c13, d5), (a6, b10, c13, d6), (a6, b10, c13, d7), (a6, b10, c13, d8), (a6, b10, c13, d9), (a6, b10, c13, d10), (a6, b10, c13, d11), (a6, b10, c13, d12), (a6, b11, c1, d1), (a6, b11, c1, d2), (a6, b11, c1, d3), (a6, b11, c1, d4), (a6, b11, c1, d5), (a6, b11, c1, d6), (a6, b11, c1, d7), (a6, b11 c1, d8), (a6, b11, c1, d9), (a6, b11, c1, d10), (a6, b11, c1, d11), (a6, b11, c1, d12), (a6, b11, c2, d1), (a6, b11, c2, d2), (a6, b11, c2, d3), (a6, b11, c2, d4), (a6, b11, c2, d5), (a6, b11, c2, d6), (a6, b11, c2, d7), (a6, b11, c2, d8), (a6, b11, c2, d9), (a6, b11, c2, d10), (a6, b11, c2, d11), (a6, b11, c2, d12), (a6, b11, c3, d1), (a6, b11, c3, d2), (a6, b11, c3, d3), (a6, b11, c3, d4), (a6, b11, c3, d5), (a6, b11, c3, d6), (a6, b11, c3, d7), (a6, b11, c3, d8), (a6, b11, c3, d9), (a6, b11, c3, d10), (a6, b11, c3, d11), (a6, b11, c3, d12), (a6, b11, c4, d1), (a6, b11, c4, d2), (a6, b11, c4, d3), (a6, b11, c4, d4), (a6, b11, c4, d5), (a6, b11, c4, d6), (a6, b11, c4, d7), (a6, b11, c4, d8), (a6, b11, c4, d9), (a6, b11, c4, d10), (a6, b11, c4, d11), (a6, b11 c4, d12), (a6, b11, c5, d1), (a6, b11, c5, d2), (a6, b11, c5, d3), (a6, b11, c5, d4), (a6, b11, c5, d5), (a6, b11, c5, d6), (a6, b11, c5, d7), (a6, b11, c5, d8), (a6, b11, c5, d9), (a6, b11, c5, d10), (a6, b11, c5, d11), (a6, b11, c5, d12), (a6, b11, c6, d1), (a6, b11, c6, d2), (a6, b11, c6, d3), (a6, b11, c6, d4), (a6, b11, c6, d5), (a6, b11, c6, d6), (a6, b11, c6, d7), (a6, b11, c6, d8), (a6, b11, c6, d9), (a6, b11, c6, d10), (a6, b11, c6, d11), (a6, b11, c6, d12), (a6, b11, c7, d1), (a6, b11, c7, d2), (a6, b11, c7, d3), (a6, b11, c7, d4), (a6, b11, c7, d5), (a6, b11, c7, d6), (a6, b11, c7, d7), (a6, b11, c7, d8), (a6, b11, c7, d9), (a6, b11, c7, d10), (a6, b11, c7, d11), (a6, b11, c7, d12), (a6, b11, c8, d1), (a6, b11, c8, d2), (a6, b11, c8, d3), (a6, b11, c8, d4), (a6, b11, c8, d5), (a6, b11, c8, d6), (a6, b11, c8, d7), (a6, b11, c8, d8), (a6, b11, c8, d9), (a6, b11, c8, d10), (a6, b11, c8, d11), (a6, b11, c8, d12), (a6, b11, c9, d1), (a6, b11, c9, d2), (a6, b11, c9, d3), (a6, b11, c9, d4), (a6, b11, c9, d5), (a6, b11, c9, d6), (a6, b11, c9, d7), (a6, b11, c9, d8), (a6, b11, c9, d9), (a6, b11, c9, d10), (a6, b11, c9, d11), (a6, b11, c9, d12), (a6, b11, c10, d1), (a6, b11, c10, d2), (a6, b11, c10, d3), (a6, b11, c10, d4), (a6, b11, c10, d5), (a6, b11, c10, d6), (a6, b11, c10, d7), (a6, b11, c10, d8), (a6, b11, c10, d9), (a6, b11, c10, d10), (a6, b11, c10, d11), (a6, b11, c10, d12), (a6, b11, c11, d1), (a6, b11, c11, d2), (a6, b11, c11, d3), (a6, b11, c11, d4), (a6, b11, c11, d5), (a6, b11, c11, d6), (a6, b11, c11, d7), (a6, b11, c11, d8), (a6, b11, c11, d9), (a6, b11, c11, d10), (a6, b11, c11, d11), (a6, b11, c11, d12), (a6, b11, c12, d1), (a6, b11, c12, d2), (a6, b11, c12, d3), (a6, b11, c12, d4), (a6, b11, c12, d5), (a6, b11, c12, d6), (a6, b11, c12, d7), (a6, b11, c12, d8), (a6, b11, c12, d9), (a6, b11, c12, d10), (a6, b11, c12, d11), (a6, b11, c12, d12), (a6, b11, c13, d1), (a6, b11, c13, d2), (a6, b11, c13, d3), (a6, b11, c13, d4), (a6, b11, c13, d5), (a6, b11, c13, d6), (a6, b11, c13, d7), (a6, b11, c13, d8), (a6, b11, c13, d9), (a6, b11, c13, d10), (a6, b11, c13, d11), (a6, b11, c13, d12), (a6, b12, c1, d1), (a6, b12, c1, d2), (a6, b12, c1, d3), (a6, b12, c1, d4), (a6, b12, c1, d5), (a6, b12, c1, d6), (a6, b12, c1, d7), (a6, b12, c1, d8), (a6, b12, c1, d9), (a6, b12, c1, d10), (a6, b12, c1, d11), (a6, b12, c1, d12), (a6, b12, c2, d1), (a6, b12, c2, d2), (a6, b12, c2, d3), (a6, b12, c2, d4), (a6, b12, c2, d5), (a6, b12, c2, d6), (a6, b12, c2, d7), (a6, b12, c2, d8), (a6, b12, c2, d9), (a6, b12, c2, d10), (a6,

b12, c2, d11), (a6, b12, c2, d12), (a6, b12, c3, d1), (a6, b12, c3, d2), (a6, b12, c3, d3), (a6, b12, c3, d4), (a6, b12, c3, d5), (a6, b12, c3, d6), (a6, b12, c3, d7), (a6, b12, c3, d8), (a6, b12, c3, d9), (a6, b12, c3, d10), (a6, b12, c3, d11), (a6, b12, c3, d12), (a6, b12, c4, d1), (a6, b12, c4, d2), (a6, b12, c4, d3), (a6, b12, c4, d4), (a6, b12, c4, d5), (a6, b12, c4, d6), (a6, b12, c4, d7), (a6, b12, c4, d8), (a6, b12, c4, d9), (a6, b12, c4, d10), (a6, b12, c4, d11), (a6, b12, c4, d12), (a6, b12, c5, d1), (a6, b12, c5, d2), (a6, b12, c5, d3), (a6, b12, c5, d4), (a6, b12, c5, d5), (a6, b12, c5, d6), (a6, b12, c5, d7), (a6, b12, c5, d8), (a6, b12, c5, d9), (a6, b12, c5, d10), (a6, b12, c5, d11), (a6, b12, c5, d12), (a6, b12, c6, d1), (a6, b12, c6, d2), (a6, b12, c6, d3), (a6, b12, c6, d4), (a6, b12, c6, d5), (a6, b12, c6, d6), (a6, b12, c6, d7), (a6, b12, c6, d8), (a6, b12, c6, d9), (a6, b12, c6, d10), (a6, b12, c6, d11), (a6, b12, c6, d12), (a6, b12, c7, d1), (a6, b12, c7, d2), (a6, b12, c7, d3), (a6, b12, c7, d4), (a6, b12, c7, d5), (a6, b12, c7, d6), (a6, b12, c7, d7), (a6, b12, c7, d8), (a6, b12, c7, d9), (a6, b12, c7, d10), (a6, b12, c7, d11), (a6, b12, c7, d12), (a6, b12, c8, d1), (a6, b12, c8, d2), (a6, b12, c8, d3), (a6, b12, c8, d4), (a6, b12, c8, d5), (a6, b12, c8, d6), (a6, b12, c8, d7), (a6, b12, c8, d8), (a6, b12, c8, d9), (a6, b12, c8, d10), (a6, b12, c8, d11), (a6, b12, c8, d12), (a6, b12, c9, d1), (a6, b12, c9, d2), (a6, b 12, c9, d3), (a6, b 12, c9, d4), (a6, b 12, c9, d5), (a6, b12, c9, d6), (a6, b 12, c9, d7), (a6, b12, c9, d8), (a6, b12, c9, d9), (a6, b12, c9, d10), (a6, b12, c9, d11), (a6, b12, c9, d12), (a6, b12, c10, d1), (a6, b12, c10, d2), (a6, b12, c10, d3), (a6, b12, c10, d4), (a6, b12, c10, d5), (a6, b12, c10, d6), (a6, b12, c10, d7), (a6, b12, c10, d8), (a6, b12, c10, d9), (a6, b12, c10, d10), (a6, b12, c10, d11), (a6, b12, c10, d12), (a6, b12, c11, d1), (a6, b12, c11, d2), (a6, b12, c11, d3), (a6, b12, c11, d4), (a6, b12, c11, d5), (a6, b12, c11, d6), (a6, b12, c11, d7), (a6, b12, c11, d8), (a6, b12, c11, d9), (a6, b12, c11, d10), (a6, b12, c11, d11), (a6, b12, c11, d12), (a6, b12, c12, d1), (a6, b12, c12, d2), (a6, b12, c12, d3), (a6, b12, c12, d4), (a6, b12, c12, d5), (a6, b12, c12, d6), (a6, b12, c12, d7), (a6, b12, c12, d8), (a6, b12, c12, d9), (a6, b12, c12, d10), (a6, b12, c12, d11), (a6, b12, c12, d12), (a6, b12, c13, d1), (a6, b12, c13, d2), (a6, b12, c13, d3), (a6, b12, c13, d4), (a6, b12, c13, d5), (a6, b12, c13, d6), (a6, b12, c13, d7), (a6, b12, c13, d8), (a6, b12, c13, d9), (a6, b12, c13, d10), (a6, b12, c13, d11), (a6, b12, c13, d12), (a6, b13, c1, d1), (a6, b13, c1, d2), (a6, b13, c1, d3), (a6, b13, c1, d4), (a6, b13, c1, d5), (a6, b13, c1, d6), (a6, b13, c1, d7), (a6, b13, c1, d8), (a6, b13, c1, d9), (a6, b13, c1, d10), (a6, b13, c1, d11), (a6, b13, c1, d12), (a6, b13, c2, d1), (a6, b13, c2, d2), (a6, b13, c2, d3), (a6, b13, c2, d4), (a6, b13, c2, d5), (a6, b13, c2, d6), (a6, b13, c2, d7), (a6, b13, c2, d8), (a6, b13, c2, d9), (a6, b13, c2, d10), (a6, b13, c2, d11), (a6, b13, c2, d12), (a6, b13, c3, d1), (a6, b13, c3, d2), (a6, b13, c3, d3), (a6, b13, c3, d4), (a6, b13, c3, d5), (a6, b13, c3, d6), (a6, b13, c3, d7), (a6, b13, c3, d8), (a6, b13, c3, d9), (a6, b13, c3, d10), (a6, b13, c3, d11), (a6, b13, c3, d12), (a6, b13, c4, d1), (a6, b13, c4, d2), (a6, b13, c4, d3), (a6, b13, c4, d4), (a6, b13, c4, d5), (a6, b13, c4, d6), (a6, b13, c4, d7), (a6, b13, c4, d8), (a6, b13, c4, d9), (a6, b13, c4, d10), (a6, b13, c4, d11), (a6, b13, c4, d12), (a6, b13, c5, d1), (a6, b13, c5, d2), (a6, b13, c5, d3), (a6, b13, c5, d4), (a6, b13, c5, d5), (a6, b13, c5, d6), (a6, b13, c5, d7), (a6, b13, c5, d8), (a6, b13, c5, d9), (a6, b13, c5, d10), (a6, b13, c5, d11), (a6, b13, c5, d12), (a6, b13, c6, d1), (a6, b13, c6, d2), (a6, b13, c6, d3), (a6, b13, c6, d4), (a6, b13, c6, d5), (a6, b13, c6, d6), (a6, b13, c6, d7), (a6, b13, c6, d8), (a6, b13, c6, d9), (a6, b13, c6, d10), (a6, b13, c6, d11), (a6, b13, c6, d12), (a6, b13, c7, d1), (a6, b13, c7, d2), (a6, b13, c7, d3), (a6, b13, c7, d4), (a6, b13, c7, d5), (a6, b13, c7, d6), (a6, b13, c7, d7), (a6, b13, c7, d8), (a6, b13, c7, d9), (a6, b13, c7, d10), (a6, b13, c7, d11), (a6, b13, c7, d12), (a6, b13, c8, d1), (a6, b13, c8, d2), (a6, b13, c8, d3), (a6, b13, c8, d4), (a6, b13, c8, d5), (a6, b13, c8, d6), (a6, b13, c8, d7), (a6, b13, c8, d8), (a6, b13, c8, d9), (a6, b13, c8, d10), (a6, b13, c8, d11), (a6, b13, c8, d12), (a6, b13, c9, d1), (a6, b13, c9, d2), (a6, b13, c9, d3), (a6, b13, c9, d4), (a6, b13, c9, d5), (a6, b13, c9, d6), (a6, b13, c9, d7), (a6, b13, c9, d8), (a6, b13, c9, d9), (a6, b13, c9, d10), (a6, b13, c9, d11), (a6, b13, c9, d12), (a6, b13, c10, d1), (a6, b13, c10, d2), (a6, b13, c10, d3), (a6, b13, c10, d4), (a6, b13, c10, d5), (a6, b13, c10, d6), (a6, b13, c10, d7), (a6, b13, c10, d8), (a6, b13, c10, d9), (a6, b13, c10, d10), (a6, b13, c10, d11), (a6, b13, c10, d12), (a6, b13, c11, d1), (a6, b13, c11, d2), (a6, b13, c11, d3), (a6, b13, c11, d4), (a6, b13, c11, d5), (a6, b13, c11, d6), (a6, b13, c11, d7), (a6, b13, c11, d8), (a6, b13, c11, d9), (a6, b13, c11, d10), (a6, b13, c11, d11), (a6, b13, c11, d12), (a6, b13, c12, d1), (a6, b13, c12, d2), (a6, b13, c12, d3), (a6, b13, c12, d4), (a6, b13, c12, d5), (a6, b13, c12, d6), (a6, b13, c12, d7), (a6, b13, c12, d8), (a6, b13, c12, d9), (a6, b13, c12, d10), (a6, b13, c12, d11), (a6, b13, c12, d12), (a6, b13, c13, d1), (a6, b13, c13, d2), (a6, b13, c13, d3), (a6, b13, c13, d4), (a6, b13, c13, d5), (a6, b13, c13, d6), (a6, b13, c13, d7), (a6, b13, c13, d8), (a6, b13, c13, d9), (a6, b13, c13, d10), (a6, b13, c13, d11), (a6, b13, c13, d12), (a6, b14, c1, d1), (a6, b14, c1, d2), (a6, b14, c1, d3), (a6, b14, c1, d4), (a6, b14, c1, d5), (a6, b14, c1, d6), (a6, b14, c1, d7), (a6, b14, c1, d8), (a6, b14, c1, d9), (a6, b14, c1, d10), (a6, b14, c1, d11), (a6, b14, c1, d12), (a6, b14, c2, d1), (a6, b14, c2, d2), (a6, b14, c2, d3), (a6, b14, c2, d4), (a6, b14, c2, d5), (a6, b14, c2, d6), (a6, b14, c2, d7), (a6, b14, c2, d8), (a6, b14, c2, d9), (a6, b14, c2, d10), (a6, b14, c2, d11), (a6, b14, c2, d12), (a6, b14, c3, d1), (a6, b14, c3, d2), (a6, b14, c3, d3), (a6, b14, c3, d4), (a6, b14, c3, d5), (a6, b14, c3, d6), (a6, b14, c3, d7), (a6, b14, c3, d8), (a6, b14, c3, d9), (a6, b14, c3, d10), (a6, b14, c3, d11), (a6, b14, c3, d12), (a6, b14, c4, d1), (a6, b14, c4, d2), (a6, b14, c4, d3), (a6, b14, c4, d4), (a6, b14, c4, d5), (a6, b14, c4, d6), (a6, b14, c4, d7), (a6, b14, c4, d8), (a6, b14, c4, d9), (a6, b14, c4, d10), (a6, b14, c4, d11), (a6, b14, c4, d12), (a6, b14, c5, d1), (a6, b14, c5, d2), (a6, b14, c5, d3), (a6, b14, c5, d4), (a6, b14, c5, d5), (a6, b14, c5, d6), (a6, b14, c5, d7), (a6, b14, c5, d8), (a6, b14, c5, d9), (a6, b14, c5, d10), (a6, b14, c5, d11), (a6, b14, c5, d12), (a6, b14, c6, d1), (a6, b14, c6, d2), (a6, b14, c6, d3), (a6, b14, c6, d4), (a6, b14, c6, d5), (a6, b14, c6, d6), (a6, b14, c6, d7), (a6, b14, c6, d8), (a6, b14, c6, d9), (a6, b14, c6, d10), (a6, b14, c6, d11), (a6, b14, c6, d12), (a6, b14, c7, d1), (a6, b14, c7, d2), (a6, b14, c7, d3), (a6, b14, c7, d4), (a6, b14, c7, d5), (a6, b14, c7, d6), (a6, b14, c7, d7), (a6, b14, c7, d8), (a6, b14, c7, d9), (a6, b14, c7, d10), (a6, b14, c7, d11), (a6, b14, c7, d12), (a6, b14, c8,

d1), (a6, b14, c8, d2), (a6, b14, c8, d3), (a6, b14, c8, d4), (a6, b14, c8, d5), (a6, b14, c8, d6), (a6, b14, c8, d7), (a6, b14, c8, d8), (a6, b14, c8, d9), (a6, b14, c8, d10), (a6, b14, c8, d11), (a6, b14, c8, d12), (a6, b14, c9, d1), (a6, b14, c9, d2), (a6, b14, c9, d3), (a6, b14, c9, d4), (a6, b14, c9, d5), (a6, b14, c9, d6), (a6, b14, c9, d7), (a6, b14, c9, d8), (a6, b14, c9, d9), (a6, b14, c9, d10), (a6, b14, c9, d11), (a6, b14, c9, d12), (a6, b14, c10, d1), (a6, b14, c10, d2), (a6, b14, c10, d3), (a6, b14, c10, d4), (a6, b14, c10, d5), (a6, b14, c10, d6), (a6, b14, c10, d7), (a6, b14, c10, d8), (a6, b14, c10, d9), (a6, b14, c10, d10), (a6, b14, c10, d11), (a6, b14, c10, d12), (a6, b14, c11, d1), (a6, b14, c11, d2), (a6, b14, c11, d3), (a6, b14, c11, d4), (a6, b14, c11, d5), (a6, b14, c11, d6), (a6, b14, c11, d7), (a6, b14, c11, d8), (a6, b14, c11, d9), (a6, b14, c11, d10), (a6, b14, c11, d11), (a6, b14, c11, d12), (a6, b14, c12, d1), (a6, b14, c12, d2), (a6, b14, c12, d3), (a6, b14, c12, d4), (a6, b14, c12, d5), (a6, b14, c12, d6), (a6, b14, c12, d7), (a6, b14, c12, d8), (a6, b14, c12, d9), (a6, b14, c12, d10), (a6, b14, c12, d11), (a6, b14, c12, d12), (a6, b14, c13, d1), (a6, b14, c13, d2), (a6, b14, c13, d3), (a6, b14, c13, d4), (a6, b14, c13, d5), (a6, b14, c13, d6), (a6, b14, c13, d7), (a6, b14, c13, d8), (a6, b14, c13, d9), (a6, b14, c13, d10), (a6, b14, c13, d11), (a6, b14, c13, d12), (a6, b15, c1, d1), (a6, b15, c1, d2), (a6, b15, c1, d3), (a6, b15, c1, d4), (a6, b15, c1, d5), (a6, b15, c1, d6), (a6, b15, c1, d7), (a6, b15, c1, d8), (a6, b15, c1, d9), (a6, b15, c1, d10), (a6, b15, c1, d11), (a6, b15, c1, d12), (a6, b15, c2, d1), (a6, b15, c2, d2), (a6, b15, c2, d3), (a6, b15, c2, d4), (a6, b15, c2, d5), (a6, b15, c2, d6), (a6, b15, c2, d7), (a6, b15, c2, d8), (a6, b15, c2, d9), (a6, b15, c2, d10), (a6, b15, c2, d11), (a6, b15, c2, d12), (a6, b15, c3, d1), (a6, b15, c3, d2), (a6, b15, c3, d3), (a6, b15, c3, d4), (a6, b15, c3, d5), (a6, b15, c3, d6), (a6, b15, c3, d7), (a6, b15, c3, d8), (a6, b15, c3, d9), (a6, b15, c3, d10), (a6, b15, c3, d11), (a6, b15, c3, d12), (a6, b15, c4, d1), (a6, b15, c4, d2), (a6, b15, c4, d3), (a6, b15, c4, d4), (a6, b15, c4, d5), (a6, b15, c4, d6), (a6, b15, c4, d7), (a6, b15, c4, d8), (a6, b15, c4, d9), (a6, b15, c4, d10), (a6, b15, c4, d11), (a6, b15, c4, d12), (a6, b15, c5, d1), (a6, b15, c5, d2), (a6, b15, c5, d3), (a6, b15, c5, d4), (a6, b15, c5, d5), (a6, b15, c5, d6), (a6, b15, c5, d7), (a6, b15, c5, d8), (a6, b15, c5, d9), (a6, b15, c5, d10), (a6, b15, c5, d11), (a6, b15, c5, d12), (a6, b15, c6, d1), (a6, b15, c6, d2), (a6, b15, c6, d3), (a6, b15, c6, d4), (a6, b15, c6, d5), (a6, b15, c6, d6), (a6, b15, c6, d7), (a6, b15, c6, d8), (a6, b5, c6, d9), (a6, b15, c6, d10), (a6, b15, c6, d11), (a6, b15, c6, d12), (a6, b15, c7, d1), (a6, b15, c7, d2), (a6, b15, c7, d3), (a6, b15, c7, d4), (a6, b15, c7, d5), (a6, b15, c7, d6), (a6, b15, c7, d7), (a6, b15, c7, d8), (a6, b15, c7,-d9), (a6, b15, c7, d10), (a6, b15, c7, d11), (a6, b15, c7, d12), (a6, b15, c8, d1), (a6, b15, c8, d2), (a6, b15, c8, d3), (a6, b15, c8, d4), (a6, b15, c8, d5), (a6, b15, c8, d6), (a6, b15, c8, d7), (a6, b15, c8, d8), (a6, b15, c8, d9), (a6, b15, c8, d10), (a6, b15, c8, d11), (a6, b15, c8, d12), (a6, b15, c9, d1), (a6, b15, c9, d2), (a6, b15, c9, d3), (a6, b15, c9, d4), (a6, b15, c9, d5), (a6, b15, c9, d6), (a6, b15, c9, d7), (a6, b15, c9, d8), (a6, b15, c9, d9), (a6, b15, c9, d10), (a6, b15, c9, d11), (a6, b15, c9, d12), (a6, b15, c10, d1), (a6, b15, c10, d2), (a6, b15, c10, d3), (a6, b15, c10, d4), (a6, b15, c10, d5), (a6, b15, c10, d6), (a6, b15, c10, d7), (a6, b15, c10, d8), (a6, b15, c10, d9), (a6, b15, c10, d10), (a6, b15, c10, d11), (a6, b15, c10, d12), (a6, b15, c11, d1), (a6, b15, c11, d2), (a6, b15, c11, d3), (a6, b15, c11, d4), (a6, b15, c11, d5), (a6, b15, c11, d6), (a6, b15, c11, d7), (a6, b15, c11, d8), (a6, b15, c11, d9), (a6, b15, c11, d10), (a6, b15, c11, d11), (a6, b15, c11, d12), (a6, b15, c12, d1), (a6, b15, c12, d2), (a6, b15, c12, d3), (a6, b15, c12, d4), (a6, b15, c12, d5), (a6, b15, c12, d6), (a6, b15, c12, d7), (a6, b15, c12, d8), (a6, b15, c12, d9), (a6, b15, c12, d10), (a6, b15, c12, d11), (a6, b15, c12, d12), (a6, b15, c13, d1), (a6, b15, c13, d2), (a6, b15, c13, d3), (a6, b15, c13, d4), (a6, b15, c13, d5), (a6, b15, c13, d6), (a6, b15, c13, d7), (a6, b15, c13, d8), (a6, b15, c13, d9), (a6, b15, c13, d10), (a6, b15, c13, d11), (a6, b15, c13, d12) are synthesized.

Test Example 1 Preparation of MMP

Various human MMPs (MMP-2, MMP-9 and MMP-13) were purchased from Calbiochem (EMD/MERCK Biosciences).
Since the purchased MMP-13 was Proenzyme, the activated one was used, which was incubated at 37°C for 2 hours after the successive addition of an assay buffer (containing 50 mM Tris-HCl (pH7.6), 0.3 M NaCl, 10 mM $CaCl_2$, 0.005% Brij35) and APMA (final concentration 1mM) to the enzyme solution.

Test Example 2 Method for measuring enzyme inhibiting activity of various MMPs

The enzyme activity of MMPs was measured according to the method described in C. Graham Knight, Frances Willenbrock and Gillian Murphy: A novel coumarin-labelled peptide for sensitive continuous assays of the matrix metalloproteinases: FEBS LETT., 296, (1992), 263-266. As a substrate, MOCAc-Pro-Leu-Gly-Leu-$A_2$Pr(DNP)-Ala-Arg-NHz (Peptide Institute, Inc. Osaka, Japan) was used, and the following four assays are performed per compound (inhibitor) for measuring the inhibiting activity.

(A) Substrate (synthetic substrate), enzyme (MMPs), inhibitor
(B) Substrate (synthetic substrate), inhibitor
(C) Substrate (synthetic substrate), enzyme (MMPs)
(D) Substrate (synthetic substrate)

In each assay, the fluorescent intensity was measured, and inhibition (%) was calculated by the following equation.

$$\text{Inhibition (\%)} = [I-(A-B)/(C-D)] \times 100$$

$IC_{50}$ indicates a concentration at which inhibition (%) is 50%.
Inhibiting activity values for MMP-2, MMP-9 and MMP-13 measured by the aforementioned method are shown in Table 1.
[Table 1]

| No | IC50 (uM) | | |
|---|---|---|---|
| | MMP-2 | MMP-9 | MMP-13 |
| I-1 | >10 | >10 | 0.121 |
| I-5 | >10 | >10 | 0.110 |
| I-11 | >10 | >10 | 0.0549 |
| I-13 | >10 | >10 | 0.0443 |
| I-15 | 1-10 | >10 | 0.126 |
| I-19 | >10 | >10 | 0.142 |
| I-20 | 1-10 | >10 | 0.0945 |
| I-21 | >10 | >10 | 0.176 |
| I-24 | >10 | >10 | 0.138 |
| I-25 | >10 | >10 | 0.145 |
| I-27 | >10 | >10 | 0.0423 |
| I-28 | >10 | >10 | 0.0771 |
| I-32 | 1-10 | >10 | 0.0685 |
| I-45 | 1-10 | >10 | 0.186 |
| I-47 | >10 | >10 | 0.131 |
| I-49 | 1-10 | >10 | 0.0965 |
| I-53 | 1-10 | >10 | 0.107 |
| I-66 | >10 | >10 | 0.0396 |

Preparation Examples

Preparation Example 1

A granule containing the following ingredients is produced.

| | | |
|---|---|---|
| Ingredient | Active ingredient | 10 mg |
| | Lactose | 700 mg |
| | Corn starch | 274 mg |
| | HPC-L | 16 mg |
| | | 1000 mg |

The active ingredient and lactose are passed through a 60 mesh sieve. Corn starch is passed through a 120 mesh sieve. These are mixed by a V-type mixing machine. To the mixed powder is added a HPC-L (low viscosity hydroxypropylcellulose) aqueous solution, the materials are kneaded, granulated (extrusion granulation, pore diameter 0.5-1 mm), and dried. The resulting dried granule is passed through a vibration sieve (12/60 mesh) to give a granule.

Preparation Examples 2

A filling powder into capsules, having the following ingredients, is produced.

| | | |
|---|---|---|
| Ingredient | Active ingredient | 10 mg |
| | Lactose | 79 mg |
| | Corn starch | 10 mg |
| | Magnesium stearate | 1 mg |
| | | 100 mg |

The active ingredient and lactose are passed through a 60 mesh sieve. Corn starch is passed through a 120 mesh sieve. These are mixed with magnesium stearate are by a V-type mixing machine. The 10% powder, 100 mg, is filled into a No.5 hard-gelatin capsule.

Preparation Examples 3

A tablet having the following ingredients is produced.

| | | |
|---|---|---|
| Ingredient | Active ingredient | 10 mg |
| | Lactose | 90 mg |
| | Microcrystalline cellulose | 30 mg |
| | CMC-Na | 15 mg |
| | Magnesium stearate | 5 mg |
| | | 150 mg |

The active ingredient, lactose, microcrystalline cellulose, and CMC-Na (carboxymethylcellulose sodium salt) are passed through a 60 mesh sieve, and mixed. Magnesium stearate is mixed with the above powder to give the mixed powder for preparing a tablet. The obtained mixed powder is compressed to give a 150-mg tablet.

Preparation Example 4

An aerosol solution containing the following ingredients is produced.

| | Weight |
|---|---|
| Active ingredient | 0.25 |
| Ethanol | 25.75 |
| Propellant 22 (chlorodifluoromethane) | 74.00 |
| Total | 100.00 |

The active ingredient and ethanol are mixed and added to a portionof the propellant 22. After being cooled to -30°C, the mixture is transferred to a filling device. Then, a required amount is supplied to a stainless steel container, and is diluted with a rest of propellant. A valve unit is attached to the container.

Preparation Example 5

An intravenous preparation for vein is produced as follows:

| | |
|---|---|
| Active ingredient | 100 mg |
| Physiological saline | 1000 ml |

A solution of the aforementioned ingredients is intravenously administered to a patient usually at a rate of 1 ml per minutes.

Preparation Example 6

A transdermal preparation containing the following ingredients is produced.

| | |
|---|---|
| Active ingredient | 10 mg |
| Isopropyl myristate | 990 mg |
| | 1000 mg |

After the compound represented by the formula (I) is dispersed into isopropyl myristate, the dispersion is mixed with an acryl-based adhesive (e.g. Nicazol), and then a transdermal preparation was obtained by applying to an application support.

Preparation Example 7

An ointment containing the following ingredients is produced.

| | |
|---|---|
| Active ingredient | 10 mg |
| Liquid paraffin | 75 mg |
| White vaseline | 925 mg |
| | 1000 mg |

The compound represented by the formula (I) and liquid paraffin are dispersed, and kneaded with white vaseline to give an ointment.

[Industrial applicability]

The sulfonamide derivatives having the MMP-13 selective inhibiting activity of the present invention are useful as a medicine for treating or preventing a disease associated with MMP-13, particularly osteoarthritis.

## Claims

1. A compound represented by the general formula (I):

[Chemical formula 1]

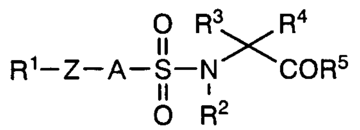

(I)

wherein $R^1$ is optionally substituted aryl, optionally substituted heteroaryl, an optionally substituted non-aromatic carbocyclic group, or an optionally substituted non-aromatic heterocyclic group;
Z is -O-, -S-, -SO-, $-SO_2-$, $-N(R^{10})-$, $-N(R^{10})-C(=O)-$, $-C(=O)-N(R^{10})-$, $-N(R^{10})-SO_2-$, $-SO_2-N(R^{10})-$, -C(=O)-, -O-C(=O)-, -C(=O)-O-, $-N(R^{10})-C(=O)-N(R^{10})-$, $-N(R^{10})-C(=S)-N(R^{10})-$, $-O-N=C(R^8)-$, $-C(R^8)=N-O-$, $-O-C(=O)-N(R^{10})-$, $-N(R^{10})-C(=O)-O-$, -C(=O)C(=O)-, an optionally substituted non-aromatic carbocyclic group, an optionally substituted non-aromatic heterocyclic group, C1-C5 alkylene which is optionally substituted and may be intervened with a substituent selected from Substituent group a, C1-C5 alkenylene which is optionally substituted, and may be intervened with a substituent selected from Substituent group a, or C1-C5 alkynylene which is optionally substituted, and may be intervened with a substituent selected from Substituent group a, wherein the Substituent group a consists of -O-, -S-, - SO-, $-SO_2-$, $-N(R^{10})-$, $-N(R^{10})-C(=O)-$, $-C(=O)-N(R^{10})-$, $-N(R^{10})-SO_2-$, $-SO_2-N(R^{10})-$, -C(=O)-, -O-C(=O)-, -C(=O)-O-, $-N(R^{10})-C(=O)-N(R^{10})-$, $-N(R^{10})-C(=S)-N(R^{10})-$, $-O-N=C(R^8)-$, $-C(R^8)=N-O-$, $-O-C(=O)-N(R^{10})-$, $-N(R^{10})-C(=O)-O-$, and - C(=O)-C(=O)-,
$R^8$ and $R^{10}$ are each independently a hydrogen atom or lower alkyl;

A is a group represented by the formula;

[Chemical formula 2]

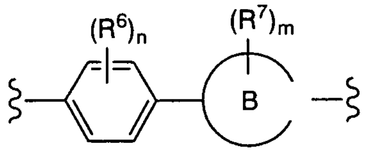

or

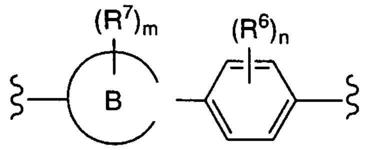

wherein $R^6$ and $R^7$ are each independently halogen, lower alkyl, cycloalkyl, lower alkenyl, lower alkynyl, lower alkyloxy, lower alkenyloxy, lower alkylthio, halo lower alkyl, halo lower alkyloxy, halo lower alkylthio, hydroxy, hydroxy lower alkyl, mercapto, carboxy, lower alkyloxycarbonyl, lower alkylsulfonyl, acyl, acyloxy, nitro, cyano, optionally substituted amino, or optionally substituted aminocarbonyl;
B ring is a nitrogen-containing heterocycle optionally further containing a nitrogen atom, an oxygen atom, and/or a sulfur atom in the ring;
m and n are each independently an integer of 0 to 3;
$R^2$ is a hydrogen atom, optionally substituted lower alkyl, optionally substituted aralkyl, optionally substituted heteroarylalkyl, optionally substituted aryl, or optionally substituted heteroaryl;
$R^3$ is a hydrogen atom, optionally substituted lower alkyl, optionally substituted aralkyl, optionally substituted heteroarylalkyl, optionally substituted aryl, or optionally substituted heteroaryl;
$R^4$ is a hydrogen atom, or
$R^3$ and $R^4$ may be taken together with an adjacent carbon atom to form a 5-to 6-membered non-aromatic carbocyclic ring or a 5- to 6-membered non-aromatic heterocyclic ring;
$R^5$ is hydroxy, lower alkyloxy, or -NHOH;
provided that when B ring is piperazine and Z is $-CH_2-O-$, $R^1$ is not pyridyl, an optically active isomer, a pharmaceutically acceptable salt, or a solvate thereof.

2. The compound according to claim 1, which is represented by the general formula (II):

[Chemical formula 3]

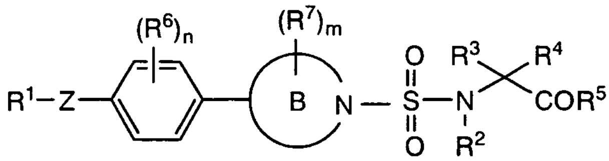

(II)

wherein $R^1$, $R^2$, $R^3$, $R^4$, $R^5$, $R^6$, $R^7$, Z, m, n and B ring are as defined in claim 1, an optically active isomer, a pharmaceutically acceptable salt, or a solvate thereof.

3. The compound according to claim 1, which is represented by the general formula (III):

[Chemical formula 4]

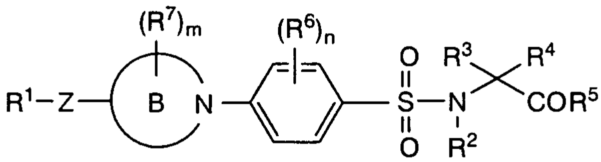

(III)

wherein $R^1$, $R^2$, $R^3$, $R^4$, $R^5$, $R^6$, $R^7$, Z, m, n and B ring are as defined in claim 1, an optically active isomer, a pharmaceutically acceptable salt, or a solvate thereof.

**4.** The compound according to claim 1, wherein $R^1$ is optionally substituted naphthyl or optionally substituted carbazolyl, an optically active isomer, a pharmaceutically acceptable salt, or a solvate thereof.

**5.** The compound according to claim 1, wherein $R^1$ is a group represented by the formula:

[Chemical formula 5]

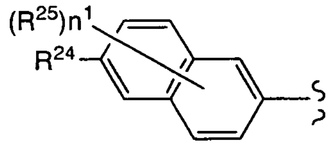

wherein $R^{24}$ is a hydrogen atom; lower alkyl optionally substituted with carboxy or lower alkyloxyimino; lower alkenyl optionally substituted with carboxy; hydroxy; lower alkyloxy optionally susbstituted with cycloalkyl, hydroxy, lower alkyloxy, carboxy, or aminocarbonyl optionally substituted with 1 or 2 lower alkyl(s); carboxy; acyl; amino optionally substitute with 1 or 2 acyl(s) or lower alkylsulfonyl(s); aminocarbonyl optionally substituted with 1 or 2 lower alkyl (s); or a non-aromatic heterocycle;
$R^{25}$ is halogen, cyano, or acyl;
$n^1$ is an integer of 0 to 2, and $R^{25}$ may be substituted on all replaceable constituent carbon atoms, an optically active isomer, a pharmaceutically acceptable salt, or a solvate thereof.

**6.** The compound according to claim 1, wherein Z is -C($R^8$)($R^9$)-, -O-, -S-, - SO-, -$SO_2$-, -N($R^{10}$)-, -C($R^8$)($R^9$)-C($R^8$)($R^9$)-, -C($R^8$)=C($R^9$)-, -C≡C-, -O-C($R^8$)($R^9$)-, -C($R^8$)($R^9$)-O-, -S-C($R^8$)($R^9$)-, -C($R^8$)($R^9$)-S-, -SO-C($R^8$)($R^9$)-, - C($R^8$)($R^9$)-SO-, -$SO_2$-C($R^8$)($R^9$)-, -C($R^8$)($R^9$)-$SO_2$-, -N($R^{10}$)-C($R^8$) ($R^9$)-, - C($R^8$)($R^9$)-N($R^{10}$)-, -N($R^{10}$)-C(=O)-, -C(=O)-N($R^{10}$)-, -N($R^{10}$)-$SO_2$-, -$SO_2$-N($R^{10}$)-, -C($R^8$)($R^9$)-C(=O)-, -C(=O)-C($R^8$)($R^9$)-, -C(=O)-C(=O)-, -C($R^8$)($R^9$)-C($R^8$)($R^9$)-C($R^8$)($R^9$)-, -C($R^8$)($R^9$)-C($R^8$)=C($R^9$)-, -C($R^8$)=C($R^9$)-C($R^8$)($R^9$)-, -C($R^8$)($R^9$)-C≡C-, -C≡C-C($R^8$)($R^9$)-, -O-C($R^8$)($R^9$)-C($R^8$)($R^9$)-, -C($R^8$)($R^9$)-O-C($R^8$)($R^9$)-, -C(R8)(R9)-C($R^8$)($R^9$)-O-, -S-C($R^8$)($R^9$)-C($R^8$)($R^9$)-, -C($R^8$)($R^9$)-S-C($R^8$)($R^9$)-, -C($R^8$)($R^9$)-C($R^8$)($R^9$)-S-, -SO-C($R^8$)($R^9$)-C($R^8$)($R^9$)-, -C($R^8$)($R^9$)-SO-C($R^8$)($R^9$)-, -C(R8)(R9)-C($R^8$)($R^9$)-SO-, -$SO_2$-C($R^8$)($R^9$)-C($R^8$)($R^9$)-, -C($R^8$)($R^9$)-$SO_2$-C($R^8$)($R^9$)-, - C($R^8$)($R^9$)-C($R^8$)($R^9$)-$SO_2$-, -N($R^{10}$)-C($R^8$)($R^9$)-C($R^8$)($R^9$)-, -C($R^8$)($R^9$)-N($R^{10}$)-C($R^8$)($R^9$)-, -C($R^8$)($R^9$)-C($R^8$)($R^9$)-N($R^{10}$)-, -C(=O)-C($R^8$)($R^9$)-C($R^8$)($R^9$)-, - C($R^8$)($R^9$)-C(=O)-C($R^8$)($R^9$)-, -C($R^8$)($R^9$)-C($R^8$)($R^9$)-C(=O)-, -C($R^8$)($R^9$)-N($R^{10}$)-C(=O)-, -N($R^{10}$)-C(=O)-C($R^8$)($R^9$)-, -C($R^8$)($R^9$)-C(=O)-N($R^{10}$)-, -C(=O)-N($R^{10}$)-C($R^8$)($R^9$)-, -C($R^8$)($R^9$)-N($R^{10}$)-$SO_2$-, -N($R^{10}$)-$SO_2$-C($R^8$)($R^9$)-, -C($R^8$)($R^9$)-$SO_2$-N($R^{10}$)-, -$SO_2$-N($R^{10}$)-C($R^8$)($R^9$)-, -N($R^{10}$)-C(=O)-N($R^{10}$)-, -N($R^{10}$)-C(=S)-N($R^{10}$)-, -O-N=C($R^8$)-, -C($R^8$)=N-O-, -O-C(=O)-C($R^8$)($R^9$)-, -C($R^8$)($R^9$)-C(=O)-O-, -O-C(=O)-N($R^{10}$)-, or -N($R^{10}$)-C(=O)-O-, wherein $R^8$, $R^9$ and $R^{10}$ are each independently a hydrogen atom or lower alkyl, an optically active isomer, a pharmaceutically acceptable salt, or a solvate thereof.

**7.** The compound according to claim 1, wherein a group represented by the formula:

[Chemical formula 6]

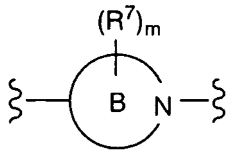

is a group represented by the formula:

[Chemical formula 7]

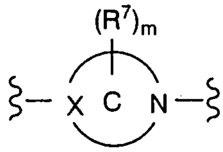

wherein X is a carbon atom or a nitrogen atom, C ring is a 5- to 8-membered nitrogten-containing heterocycle, and $R^7$ and m are as defined in claim 1, an optically active isomer, a pharmaceutically acceptable salt, or a solvate thereof.

**8.** The compound according to claim 1, wherein $R^2$ is a hydrogen atom, an optically active isomer, a pharmaceutically acceptable salt, or a solvate thereof.

**9.** The compound according to claim 1, wherein $R^3$ is a hydrogen atom, methyl, ethyl, n-propyl, isopropyl, n-butyl, s-butyl, isobutyl, t-butyl, hydroxymethyl, 1-hydroxyethyl, 2-hydroxyethyl, carboxymethyl, carboxyethyl, methyloxymethyl, methylthiomethyl, 2-methylthioethyl, phenyl, 4-hydroxyphenyl, 4-fluorophenyl, 4-methyloxyphenyl, benzyl, 4-hydroxybenzyl, 4-fluorobenzyl, 4-chlorobenzyl, 4-methoxybenzyl, indol-3-ylmethyl, 1-carboxymethylindol-3-ylmethyl, thiazol-4-ylmethyl, carboxymethyloxybenzyl, or cyclopropylmethyl;
$R^4$ is a hydrogen atom; or
$R^3$ and $R^4$ may be taken together with an adjacent carbon to form a ring represented by the formula:

[Chemical formula 8]

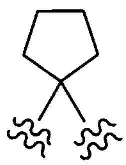 , 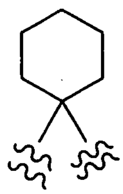 or 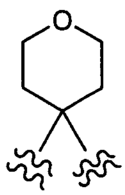

an optically active isomer, a pharmaceutically acceptable salt, or a solvate thereof.

**10.** The compound according to claim 1, wherein $R^5$ is hydroxy, an optically active isomer, a pharmaceutically acceptable salt, or a solvate thereof.

**11.** A pharmaceutical composition containing the compound as defined in any one of claims 1 to 10 as an active ingredient.

**12.** A pharmaceutical composition for inhibiting MMP-13 which contains the compound as defined in any one of claims 1 to 10 as an active ingredient.

**13.** A method of treating a disease resulting from, or associated with MMP-13 of a mammal, comprising administering an amount for exhibiting the therapeutic effect of the compound as defined in any one of claims 1 to 10 to a mammal including a human.

**14.** Use of the compound as defined in any one of claims 1 to 10, for preparing a medicament for treating a disease resulting from or associated with MMP-13.

**INTERNATIONAL SEARCH REPORT**

International application No.
PCT/JP2007/053899

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D209/86, A61K31/451, A61K31/495, A61K31/496, A61P1/02, A61P19/02, A61P19/10, A61P27/02, A61P29/00, A61P31/12, A61P31/18, A61P35/00, A61P43/00, C07D211/96, C07D243/08, C07D295/22

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAOLD(STN), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | JP 2001-523222 A (F. Hoffmann-La Roche AG.), 20 November, 2001 (20.11.01), Full text<br>& WO 98/32748 A1 & DE 19802350 A<br>& CA 2278694 A & AU 9866140 A<br>& EP 958287 A1 & BR 9807508 A<br>& NZ 336625 A & HU 200000941 A<br>& ZA 9800376 A & FR 2758559 A<br>& GB 2321641 A & ES 2136037 A<br>& NO 9903587 A & MX 9906822 A | 1,2,4-9,11, 12,14<br>10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>24 April, 2007 (24.04.07) | Date of mailing of the international search report<br>15 May, 2007 (15.05.07) |
| Name and mailing address of the ISA/<br>Japanese Patent Office<br><br>Facsimile No. | Authorized officer<br><br>Telephone No. |

Form PCT/ISA/210 (second sheet) (April 2005)

**INTERNATIONAL SEARCH REPORT**

International application No.
PCT/JP2007/053899

C (Continuation). DOCUMENTS CONSIDERED TO BE RELEVANT

| Category* | Citation of document, with indication, where appropriate, of the relevant passages | Relevant to claim No. |
|---|---|---|
| X | WO 97/19068 A1 (WARNER-LAMBERT CO.), 29 May, 1997 (29.05.97), Full text; particularly, example 12; page 8, lines 1 to 11<br>& JP 2000-514779 A & CA 2233560 A<br>& AU 9674641 A & EP 874836 A1<br>& BR 9611487 A & HU 9902083 A<br>& NZ 321293 A & IL 123901 A<br>& ZA 9609584 A & US 5977141 A<br>& NO 9802223 A & US 6297247 B1 | 1,3-12,14 |
| Y | JP 2001-163786 A (Sankyo Co., Ltd.), 19 June, 2001 (19.06.01), Claims; particularly, definitions concerning R1 (Family: none) | 10 |
| Y | WO 03/080042 A1 (Shionogi & Co., Ltd.), 02 October, 2003 (02.10.03), Claims; particularly, definitions concerning R1<br>& US 2005/0227994 A1 & EP 1491190 A1 | 10 |

Form PCT/ISA/210 (continuation of second sheet) (April 2005)

**INTERNATIONAL SEARCH REPORT**

International application No.
PCT/JP2007/053899

Continuation of A. CLASSIFICATION OF SUBJECT MATTER
(International Patent Classification (IPC))

*C07D209/86*(2006.01)i, *A61K31/451*(2006.01)i, *A61K31/495*(2006.01)i, *A61K31/496*(2006.01)i, *A61P1/02*(2006.01)i, *A61P19/02*(2006.01)i, *A61P19/10*(2006.01)i, *A61P27/02*(2006.01)i, *A61P29/00*(2006.01)i, *A61P31/12*(2006.01)i, *A61P31/18*(2006.01)i, *A61P35/00*(2006.01)i, *A61P43/00*(2006.01)i, *C07D211/96*(2006.01)i, *C07D243/08*(2006.01)i, *C07D295/22*(2006.01)i

(According to International Patent Classification (IPC) or to both national classification and IPC)

Form PCT/ISA/210 (extra sheet) (April 2005)

**INTERNATIONAL SEARCH REPORT**

International application No.
PCT/JP2007/053899

**Box No. II Observations where certain claims were found unsearchable (Continuation of item 2 of first sheet)**

This international search report has not been established in respect of certain claims under Article 17(2)(a) for the following reasons:

1. [X] Claims Nos.: 13
because they relate to subject matter not required to be searched by this Authority, namely:
The invention as set forth in claim 13 pertains to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9727174 A
- WO 9904780 A
- WO 0015213 A
- WO 0183431 A
- WO 0228844 A
- WO 0183461 A
- WO 03080042 A
- JP 2000319250 A
- WO 05061459 A
- WO 9832748 A
- WO 0046189 A


### Non-patent literature cited in the description


- **YOSHINORI TAMURA et al.** *J. Med. Chem.,* 1998, vol. 41 (4), 640-649
- *Tetrahedron Lett.,* 1998, vol. 39, 617-620
- Experimental Chemistry Course. Chemical Society of Japan, vol. 22
- **GARY M. KSANDER.** *J. Med. Chem.,* 1995, vol. 38, 1689-1700
- *New Experimental Chemistry Course,* 1978, vol. 14, 1787
- **TATSUO HAMADA.** *Synthesis,* 1986, 852-854
- **THEODORA W GREEN.** Protective Groups in Organic Synthesis. John Wiley & Sons
- *Tetrahedron Lett.,* 1996, vol. 37, 6965-6968
- *Angew Chem, Int Ed,* 1998, vol. 37, 325-329
- **MEACOCK et al.** *J. Exp. Path.,* 1990, vol. 71, 279-293
- **FLETCHER et al.** *J. Pharmacol. Exp. Ther.,* vol. 284 (2), 714-721
- *J. Org. Chem.,* 2003, vol. 68, 115
- **C. GRAHAM KNIGHT ; FRANCES WILLENBROCK ; GILLIAN MURPHY.** A novel coumarin-labelled peptide for sensitive continuous assays of the matrix metalloproteinases. *FEBS LETT.,* 1992, vol. 296, 263-266